(19) Europäisches Patentamt
European Patent Office
Office européen des brevets

(11) EP 3 429 345 B1

(12) **EUROPEAN PATENT SPECIFICATION**

(45) Date of publication and mention of the grant of the patent:
**11.09.2019 Bulletin 2019/37**

(21) Application number: **17786853.6**

(22) Date of filing: **04.10.2017**

(51) Int Cl.:
***A01N 1/02*** *(2006.01)*

(86) International application number:
**PCT/EP2017/075257**

(87) International publication number:
**WO 2018/065491 (12.04.2018 Gazette 2018/15)**

(54) **USES OF RECOMBINANT YEAST-DERIVED SERUM ALBUMIN**

VERWENDUNGEN VON REKOMBINANTEM, VON HEFE ABGELEITETEM SERUMALBUMIN

UTILISATION DE SERUMALBUMINE HUMAINE RECOMBINEE DERIVEE PAR LA LEVURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.10.2016 EP 16192276**

(43) Date of publication of application:
**23.01.2019 Bulletin 2019/04**

(60) Divisional application:
**19182203.0**

(73) Proprietor: **Albumedix Ltd.**
**Nottingham NG7 1FD (GB)**

(72) Inventor: **JØRGENSEN, Eva Balslev**
**3500 Vaerloese (DK)**

(74) Representative: **Potter Clarkson**
**The Belgrave Centre**
**Talbot Street**
**Nottingham NG1 5GG (GB)**

(56) References cited:
**US-A1- 2013 157 356 US-A1- 2015 175 955**

- **Anonymous: "Albumin in Cell Culture Media - An examination of quality and function - Cell Culture Dish - Cell Culture Dish", , 25 May 2016 (2016-05-25), XP055338218, Retrieved from the Internet: URL:http://cellculturedish.com/2016/05/albumin-cell-culture-media-examination-quality-function/ [retrieved on 2017-01-24]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 429 345 B1

Printed by Jouve, 75001 PARIS (FR)

## Description

### Reference to a Sequence Listing

This application contains a Sequence Listing in computer readable form.

### Field of Invention

The present invention relates to methods and uses for protecting cells from the downstream effects of physiological shock, and to compositions comprising recombinant yeast-derived serum albumin useful therefor. In particular, the present invention relates to the preservation of stem cells in a viable form, in particular for extending the viability of cryopreserved stem cells following freezing and thawing, and during post-thawing storage.

### Background of Invention

Stem cells have been used in a clinical setting for many years. Haematopoietic stem cells have been used for the treatment of both haematological and non-haematological disease; while more recently mesenchymal stem cells derived from bone marrow have been the subject of both laboratory and early clinical studies. These cells show both multipotency and expansion potential. Human embryonic stem cells are pluripotent cells, capable of forming stable cell lines which retain the capacity to differentiate into cells from all three germ layers. This makes them of special significance in both regenerative medicine and toxicology. Induced pluripotent stem (iPS) cells may also provide a similar breadth of utility without some of the confounding ethical issues surrounding embryonic stem cells.

US 2013/157356 was published on 20 June 2013, and is entitled "Methods & Compositions for Improving Protein Production". Its disclosure relates to compositions, and uses thereof, which are beneficial for eukaryotic cells in culture, and methods for their use in promoting cell growth, viability and recombinant protein expression. The methods disclosed therein are taught to be useful, for example, for improving cell viability and in accelerating the rate of cell growth of cells grown in culture. In one aspect, the supplements described therein are indicated to be useful for improving or enhancing the yield of the recombinant proteins from the cell cultures.

An essential pre-requisite to the commercial and clinical application of stem cells are suitable cryopreservation protocols for long-term storage.

This routine procedure generally involves slow cooling in the presence of a cryoprotectant to avoid the damaging effects of intracellular ice formation. Dimethyl sulphoxide (DMSO) is a common cryoprotectant.

Whilst the current cryopreservation protocols are clinically effective, questions still remain as to whether or not they are optimal. DMSO is known to be toxic to tissues and cells, with toxicity being time-, temperature- and concentration-dependent. Toxicity varies from cell type to cell type, and the accepted practice has been to introduce the cryoprotectant at low temperatures (+4 °C) for as short a period as is considered practical.

After thawing of the cells, washing procedures are generally used, for example based on that originally developed by Rubenstein et al., 1995, Proc. Natl. Acad. Sci. USA., 92:10119-10122, in order to transfer the thawed cells to a DMSO-free medium.

However, after thawing of cryopreserved stems cells, and transfer to a fresh (ideally DMSO-free) medium, there is a very limited period of time (typically 24 hours or less) in which the stem cells remain viable and suitable for subsequent use.

Accordingly, it is an object of the present invention to address challenges in cryopreservation methods to improve the ability for prolonged storage of stem cells post-freezing, and to improve the stability of clinical grade stem cells during and post-freezing without compromising viability, identity and multipotentiality.

### Summary of the Invention

The applicant has surprisingly found that recombinant yeast-derived serum albumin preparations are particularly effective (compared, for example, to plasma-derived serum albumin) in protecting cells from the downstream effects of physiological shock. That is, it has been found that recombinant yeast-derived serum albumin preparations, such as the AlbIX®, Recombumin® Alpha and Recombumin® Prime products available from Albumedix Ltd., are particularly effective, compared for example to other forms of albumin preparation such as plasma-derived serum albumin preparations, in protecting cells (such as stem cells) from moving from an early to late apoptotic state following a physiological shock, such as the freeze/thaw steps used in cryopreservation.

It has consequently been demonstrated by the applicant that recombinant yeast-derived serum albumin preparations, such as the AlbIX®, Recombumin® Alpha and Recombumin® Prime products available from Albumedix Ltd., are particularly effective components in cryopreservation media and/or storage media in order to substantially extend the period of viability of stem cells that have been subjected to freeze/thaw steps used in cryopreservation, and then stored prior to use. The extension of the period of time in which previously-cryopreserved stem cells can be kept in storage after thawing, and maintained in a viable state, provides an important improvement and added flexibility.

Specifically, by increasing post-freeze stability, recombinant yeast-derived serum albumin preparations can solve challenges of storage and transport and add flexibility to the clinical practitioners and patients in the use of stem cell therapies.

Accordingly, a first aspect of the present invention provides a method for the preservation of stem cells, the method comprising the steps of combining the stem cells with a cryopreservation medium to produce a mixture, and freezing the mixture to produce a frozen stem cell product,

optionally, wherein the method further comprises the steps of thawing the frozen stem cell product, transferring the thawed cells to a storage medium, and storing stem cells in the storage medium,
wherein the cryopreservation medium and/or the storage medium comprises a recombinant yeast-derived serum albumin preparation.

The first aspect of the present invention also provides a method for the preservation of stem cells, the method comprising storing stem cells in a storage medium,

wherein the stem cells have been frozen in a cryopreservation medium, thawed, and then transferred to the storage medium prior to storage; and
wherein the cryopreservation medium and/or the storage medium comprises a recombinant yeast-derived serum albumin preparation.

Preferably, the method of the first aspect of the present invention comprises the steps of freezing stem cells in the cryopreservation medium to produce a frozen stem cell product, thawing the frozen stem cell product, transferring the thawed cells to the storage medium, and storing stem cells in the storage medium, wherein the cryopreservation medium and/or the storage medium comprises a recombinant yeast-derived serum albumin preparation.

It may be preferred that the recombinant yeast-derived serum albumin preparation is present in the cryopreservation medium and/or the storage medium, in accordance with the first aspect of the present invention, when mixed with the stem cells, in an amount suitable to provide a concentration of the recombinant yeast-derived serum albumin protein that is greater than about 0.01 % (w/v) and less than 10% (w/v), less than about 9% (w/v), less than about 8% (w/v), less than about 7% (w/v) or less than about 6% (w/v), such as at a concentration of from about 0.1 % (w/v) to about 5% (w/v), preferably at about 1% (w/v), about 2% (w/v), about 3 (w/v) or about 4% (w/v). The term "about" in this context, can include meaning of ± 50%, 40%, 30%, 20%, 10%, 5%, 4%, 3%, 2% or 1% of the stated value; for example, 10% (w/v) ± 10% of the stated value is 9 to 11 % (w/v).

The recombinant yeast-derived serum albumin preparation may preferably be present in the cryopreservation medium and is also present in the storage medium.

Optionally, in a preferred embodiment of the first aspect of the present invention, the stem cells are stored in the storage medium at a temperature of 2-8°C, such as at about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

For example, the stem cells may be stored in the storage medium for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days); and optionally, the stem cells are stored at a temperature of 2-8°C for a period of time greater than 24 hours, such as up to about 48 hours, for example up to about 72 hours, or more, and in which the viability of the stem cells at the end of the storage period is greater than 5%, 10%, 15%, 20%, 25%, 30%, 35%, 40%, 45%, 50%, 55%, 60%, 70%, 75%, 80%, 85%, 86%, 87%, 88%, 89%, 90%, 91%, 92%, 93%, 94%, 95% or more, such as about 60%, 70%, 75%, 80%, 85%, 86%, 87%, 88%, 89%, 90%, 91%, 92%, 93%, 94%, 95% or more. The term "about" as used in the context of time periods, can include meaning of ± 50%, 40%, 30%, 20%, 10%, 5%, 4%, 3%, 2% or 1% of the stated value. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

Preferably, in accordance with the first aspect of the present invention, the recombinant yeast-derived serum albumin protein present in the cryopreservation medium and/or the storage medium exhibits one or more of the following properties:

(a) less than 0.5% (w/w) binds to Concanavalin A, preferably less than 0.4%, 0.3%, 0.2% or 0.15%; and/or
(b) a glycation level of less than 0.6 moles hexose / mole of protein, and preferably less than 0.10, 0.075 or 0.05 moles hexose / mole of protein.

The recombinant yeast-derived serum albumin protein present in the cryopreservation medium and/or the storage medium used in accordance with the first aspect of the present invention:

(a) may be at least about 95%, 96%, 97%, 98%, more preferably at least about 99.5% monomeric and dimeric, preferably essentially 100% monomeric and dimeric (as used in this context, the term "about", can include meaning of ± 1%, 0.5%, 0.4%, 0.3%, 0.3%, 0.1% or less);
(b) may be at least about 93%, 94%, 95%, 96% or 97% monomeric (as used in this context, the term "about", can include meaning of ± 1%, 0.5%, 0.4%, 0.3%, 0.3%, 0.1% or less); and/or
(c) may have an albumin polymer content of not greater, and preferably less, than about 1.0% (w/w) 0.1 % (w/w) or 0.01% (w/w). As used in this context, the term "about", can include meaning of ± 50%, 40%, 30%, 20%, 10%, 5% 1%, 0.5%, 0.4%, 0.3%, 0.3%, 0.1% or less of the stated value; e.g. 1.0 % (w/v) ± 50% is the range of 0.5 to 1.5 % (w/v). As used in this context, the term "polymer" as applied to recombinant yeast-derived serum albumin protein is distinct from monomeric and dimeric forms.

Optionally, the recombinant yeast-derived serum albumin preparation used in the formation of the cryopreservation medium and/or the storage medium in accordance with the first aspect of the present invention, may comprise, consist essentially of, or consist of, yeast-derived serum albumin protein, cations (such as sodium, potassium, calcium, magnesium, ammonium, preferably sodium) and balancing anions (such as chloride, phosphate, sulfate, citrate or acetate, preferably chloride or phosphate), water, and optionally octanoate and polysorbate 80.

Optionally, the recombinant yeast-derived serum albumin preparation used in the formation of the cryopreservation medium and/or the storage medium in accordance with the first aspect of the present invention, may comprise octanoate at less than 35mM, 32.5mM, 30 mM, 28 mM, 26 mM, 24 mM, 22 mM, 20 mM, 18 mM, 16 mM, 15 mM, 14 mM, 12 mM, 10 mM, 8 mM, 6 mM, 5 mM, 4 mM, 3 mM, 2 mM, 1 mM, 0.5 mM, 0.4 mM, 0.3 mM, 0.2 mM, 0.1 mM, 0.01 mM, 0.001 mM, is substantially free of octanoate, or is free of octanoate.

Further optionally, the cryopreservation medium and/or the storage medium used in accordance with the first aspect of the present invention, and which comprises the recombinant yeast-derived serum albumin preparation and one or more other components, optionally including stem cells, comprises octanoate at less than 35mM, 32.5mM, 30 mM, 28 mM, 26 mM, 24 mM, 22 mM, 20 mM, 18 mM, 16 mM, 15 mM, 14 mM, 12 mM, 10 mM, 8 mM, 6 mM, 5 mM, 4 mM, 3 mM, 2 mM, 1 mM, 0.5 mM, 0.4 mM, 0.3 mM, 0.2 mM, 0.1 mM, 0.01 mM, 0.001 mM, is substantially free of octanoate, or is free of octanoate.

Optionally, the recombinant yeast-derived serum albumin preparation used in the formation of the cryopreservation medium and/or the storage medium in accordance with the first aspect of the present invention, may have an overall fatty acid content less than or equal to 35mM, 32.5mM, 30 mM, 28 mM, 26 mM, 24 mM, 22 mM, 20 mM, 15 mM, 10 mM, 5 mM, 4 mM, 3 mM, 2 mM, 1 mM, is substantially free of fatty acids, or is free of fatty acids.

Further optionally, the cryopreservation medium and/or the storage medium used in accordance with the first aspect of the present invention, and which comprises the recombinant yeast-derived serum albumin preparation and one or more other components, optionally including stem cells, has an overall fatty acid content less than or equal to 35mM, 32.5mM, 30 mM, 28 mM, 26 mM, 24 mM, 22 mM, 20 mM, 15 mM, 10 mM, 5 mM, 4 mM, 3 mM, 2 mM, 1 mM, is substantially free of fatty acids, or is free of fatty acids.

Optionally, the recombinant yeast-derived serum albumin preparation used in the formation of the cryopreservation medium and/or the storage medium in accordance with the first aspect of the present invention, may comprise detergent, such as polysorbate (preferably polysorbate 80) at a concentration less than 200 $mg.L^{-1}$, 150 $mg.L^{-1}$, 100 $mg.L^{-1}$, 90 $mg.L^{-1}$, 80 $mg.L^{-1}$, 70 $mg.L^{-1}$, 60 $mg.L^{-1}$, 50 $mg.L^{-1}$, 40 $mg.L^{-1}$, 30 $mg.L^{-1}$, 20 $mg.L^{-1}$, 15 $mg.L^{-1}$, 10 $mg.L^{-1}$, 5 $mg.L^{-1}$, 4 $mg.L^{-1}$, 3 $mg.L^{-1}$, 2 $mg.L^{-1}$, 1 $mg.L^{-1}$, 0.5 $mg.L^{-1}$, 0.1 $mg.L^{-1}$, 0.01 $mg.L^{-1}$, 0.001 $mg.L^{-1}$, is substantially free of the detergent, or is free of the detergent.

Further optionally, the cryopreservation medium and/or the storage medium used in accordance with the first aspect of the present invention, and which comprises the recombinant yeast-derived serum albumin preparation and one or more other components, optionally including stem cells, comprises detergent, such as polysorbate (preferably polysorbate 80) at a concentration less than 200 $mg.L^{-1}$, 150 $mg.L^{-1}$, 100 $mg.L^{-1}$, 90 $mg.L^{-1}$, 80 $mg.L^{-1}$, 70 $mg.L^{-1}$, 60 $mg.L^{-1}$, 50 $mg.L^{-1}$, 40 $mg.L^{-1}$, 30 $mg.L^{-1}$, 20 $mg.L^{-1}$, 15 $mg.L^{-1}$, 10 $mg.L^{-1}$, 5 $mg.L^{-1}$, 4 $mg.L^{-1}$, 3 $mg.L^{-1}$, 2 $mg.L^{-1}$, 1 $mg.L^{-1}$, 0.5 $mg.L^{-1}$, 0.1 $mg.L^{-1}$, 0.01 $mg.L^{-1}$, 0.001 $mg.L^{-1}$, is substantially free of detergent, such as polysorbate (preferably polysorbate 80), or is free of the detergent (preferably is free of polysorbate 80).

Optionally, the recombinant yeast-derived serum albumin preparation used in the formation of the cryopreservation medium and/or the storage medium in accordance with the first aspect of the present invention, may comprise total free amino acid level and/or N-acetyl tryptophan levels less than 35mM, 32.5mM, 30 mM, 28 mM, 26 mM, 24 mM, 22 mM, 20 mM, 15 mM, 10 mM, 5 mM, 4 mM, 3 mM, 2 mM, 1 mM, 0.5 mM, 0.1 mM, 0.01 mM, 0.005 mM, 0.001 mM, is substantially free of free amino acids and/or N-acetyl tryptophan in particular, or is free of free amino acids and/or of N-acetyl tryptophan in particular.

Further optionally, the cryopreservation medium and/or the storage medium used in accordance with the first aspect of the present invention, and which comprises the recombinant yeast-derived serum albumin preparation and one or more other components, optionally including stem cells, comprises total free amino acid level and/or N-acetyl tryptophan levels less than 35mM, 32.5mM, 30 mM, 28 mM, 26 mM, 24 mM, 22 mM, 20 mM, 15 mM, 10 mM, 5 mM, 4 mM, 3 mM, 2 mM, 1 mM, 0.5 mM, 0.1 mM, 0.01 mM, 0.005 mM, 0.001 mM, is substantially free of free amino acids and/or N-acetyl tryptophan in particular, or is free of free amino acids and/or of N-acetyl tryptophan in particular.

In one preferred embodiment, the recombinant yeast-derived serum albumin preparation used in the formation of the cryopreservation medium and/or the storage medium in accordance with the first aspect of the present invention, may be substantially free of, or completely free of, all of octanoate, free amino acids and/or N-acetyl tryptophan in particular, and detergent (such as polysorbate 80).

In a further preferred embodiment, the cryopreservation medium and/or the storage medium used in accordance with the first aspect of the present invention, and which comprises the recombinant yeast-derived serum albumin preparation and one or more other components, optionally including stem cells, is substantially free of, or completely free of, all of octanoate, free amino acids and/or N-acetyl tryptophan in particular, and detergent (such as polysorbate 80).

In a further preferred embodiment, the recombinant yeast-derived serum albumin preparation used in the formation of the cryopreservation medium and/or the storage medium in accordance with the first aspect of the present invention, may be selected from: Recombumin® Prime, or a preparation that is similar thereto; Recombumin® Alpha, or a preparation that is similar thereto; or AlbIX®, or a preparation that is similar thereto.

Typically, the recombinant yeast-derived serum albumin preparation used in the formation of the cryopreservation medium and/or the storage medium in accordance with the first aspect of the present invention, is free of one or more, such all, components selected from: haem, prekallikrein activator, pyrogens, hepatitis C and/or human viruses. Further, typically, the recombinant yeast-derived serum albumin preparation used in the formation of the cryopreservation medium and/or the storage medium in accordance with the first aspect of the present invention, has an aluminium concentration of less than 200 $\mu g.L^{-1}$, such as less than 180 $\mu g.L^{-1}$, 160 $\mu g.L^{-1}$, 140 $\mu g.L^{-1}$, 120 $\mu g.L^{-1}$, 100 $\mu g.L^{-1}$, 90 $\mu g.L^{-1}$, 80 $\mu g.L^{-1}$, 70 $\mu g.L^{-1}$, 60 $\mu g.L^{-1}$, 50 $\mu g.L^{-1}$, or 40 $\mu g.L^{-1}$, more typically within the range of about 10 $\mu g.L^{-1}$ to about 30 $\mu g.L^{-1}$. As used in this context, the term "about", can include meaning of $\pm$ 10$\mu g.L^{-1}$, 5$\mu g.L^{-1}$, 4$\mu g.L^{-1}$, 3$\mu g.L^{-1}$, 2$\mu g.L^{-1}$, 1$\mu g.L^{-1}$, 0.5$\mu g.L^{-1}$, 0.1$\mu g.L^{-1}$ or less of the stated value.

Further typically, the cryopreservation medium and/or the storage medium used in accordance with the first aspect of the present invention, and which comprises the recombinant yeast-derived serum albumin preparation and one or more other components, optionally including stem cells, is free of one or more, such all, components selected from: haem, prekallikrein activator, pyrogens, hepatitis C and/or human viruses). The cryopreservation medium and/or the storage medium may additionally or alternatively have an aluminium concentration of less than 200 $\mu g.L^{-1}$, such as less than 180 $\mu g.L^{-1}$, 160 $\mu g.L^{-1}$, 140 $\mu g.L^{-1}$, 120 $\mu g.L^{-1}$, 100 $\mu g.L^{-1}$, 90 $\mu g.L^{-1}$, 80 $\mu g.L^{-1}$, 70 $\mu g.L^{-1}$, 60 $\mu g.L^{-1}$, 50 $\mu g.L^{-1}$, or 40 $\mu g.L^{-1}$, more typically within the range of about 10 $\mu g.L^{-1}$ to about 30 $\mu g.L^{-1}$. As used in this context, the term "about", can include meaning of $\pm$ 10$\mu g.L^{-1}$, 5$\mu g.L^{-1}$, 4$\mu g.L^{-1}$, 3$\mu g.L^{-1}$, 2$\mu g.L^{-1}$, 1$\mu g.L^{-1}$, 0.5$\mu g.L^{-1}$, 0.1$\mu g.L^{-1}$ or less of the stated value.

Further typically, the cryopreservation medium and/or the storage medium used in accordance with the first aspect (or any other aspect of the present invention) of the present invention, and which comprises the recombinant yeast-derived serum albumin preparation and one or more other components, optionally including stem cells, is free of, or essentially free of, energy substrates selected from a group comprising Trehalose, Hydroxyethyl Starch, or a combination thereof and/or is free of, or essentially free of, an anti-ageing agent, which may be a combination of L-Glutamine, Poly-L-Lysine and Ectoine. In this context, "essentially free" of an energy substrate includes the meaning less than about 0.25% v/v, such as less than 0.1 % v/v, less than 0.01 % v/v, less than 0.001 % v/v, less than 0.001 % v/v, or 0 % v/v. "Essentially free" of an anti-ageing agent includes the meaning less than about 0.0005 % v/v, such as less than $5x10^{-5}$ % v/v, less than $5x10^{-6}$% v/v, less than $5x10^{-7}$ % v/v, or 0 % v/v.

Typically, the recombinant yeast-derived serum albumin preparation used in the formation of the cryopreservation medium and/or the storage medium in accordance with the first aspect of the present invention, possesses an intact or substantially intact N-terminal sequence.

Typically, the recombinant yeast-derived serum albumin preparation used in the formation of the cryopreservation medium and/or the storage medium in accordance with the first aspect of the present invention, comprises albumin protein that has a free thiol group content that is greater than about 62%, such as at least about 69%, at least about 70%, at least about 75%, at least about 80%, at least about 85%, at least about 90%, at least about 95%, about 96%, about 97%. As used in this context, the term "about", can include meaning of $\pm$ 50%, 40%, 30%, 20%, 10%, 5%, 4%, 3%, 2%, 1 % or less of the stated value; e.g. 80% $\pm$ 10% refers to the range of 72 to 88%.

Typically, the recombinant yeast-derived serum albumin preparation used in the formation of the cryopreservation medium and/or the storage medium in accordance with the first aspect of the present invention, comprise albumin protein that, when tested by size exclusion chromatography (SEC), displays an SEC profile excluding peaks with a peak retention time under 14 minutes and over 19 minutes, and more preferably excludes peaks with a peak retention time under 14 or 15 minutes and over 18 minutes.

Typically, the recombinant yeast-derived serum albumin preparation used in the formation of the cryopreservation medium and/or the storage medium in accordance with the first aspect of the present invention, comprise albumin protein that, when tested by reversed phase high performance liquid chromatography (RP-HPLC), displays a single major peak, corresponding to albumin in the native monomeric form.

Typically, the recombinant yeast-derived serum albumin preparation used in the formation of the cryopreservation medium and/or the storage medium in accordance with the first aspect of the present invention, comprises albumin protein that, when tested by mass spectrometry, is a product that displays fewer than about 13, about 12, about 11, about 10, about 9, about 8, about 7, about 6, such as about 1 to about 11, 1 to about 8, or 1 to about 5, 1 to about 4, 1 to about 3, 1 to about 2, about 1 or less than 1 hexose modified lysine and/or arginine residues per protein. As used in this context, the term "about", can include meaning of $\pm$ 5, 4, 3, 2 or 1 hexose modified lysine and/or arginine residues per protein.

Typically, the recombinant yeast-derived serum albumin preparation used in the formation of the cryopreservation medium and/or the storage medium in accordance with the first aspect of the present invention, comprises albumin protein that is not glycated with plant-specific sugars, such as $\alpha$-1,3-fucose and/or $\beta$-1,2-xylose.

For the avoidance of doubt, the recombinant yeast-derived serum albumin preparation used in the formation of the cryopreservation medium and/or the storage medium in accordance with the first aspect (and all other aspects) of the present invention, and the media, such as cryopreservation media and/or the storage media, produced thereby, will be essentially free of, or not contain, plant protein hydrolysate.

The term "plant protein hydrolysate" refers to a substance containing amino acids or/and peptides, in which a substance containing amino acids or/and peptides are prepared by hydrolysis of plant proteins. The plant protein hydrolysates may be prepared by hydrolysis of plant proteins using a particular enzyme, etc., but are not limited thereto. Examples thereof may be tobacco, rice, or bean protein hydrolysates. The plant protein hydrolysates may be the product of direct hydrolysis of plant proteins using an enzyme, etc., or commercially available plant protein hydrolysates. A further example is hydrolyzed bean proteins, and ULTRAPEP SOY or ULTRAPEP YE manufactured by Sheffield.

The term "essentially free of" in this context means that the recombinant yeast-derived serum albumin preparation used in the formation of the cryopreservation medium and/or the storage medium in accordance with the first aspect (and all other aspects) of the present invention, and the media, such as cryopreservation media and/or the storage media, produced thereby, contain plant protein hydrolysate or components thereof at a level that is less than 1 part by weight to 50 parts by weight, preferably less than 1 part by weight to 100 parts by weight, more preferably less than 1 part by weight to 1000 parts by weight, based on 100 parts by weight of the total composition, and most typically zero plant protein hydrolysate.

The plant protein hydrolysates include essential amino acids or/and non-essential amino acids which may be used as a basic energy source of cells, thus providing nutrients for cells, increasing their activity upon freezing and thawing. Without being bound by theory, the applicant believes that the absence of such components in cryopreservation media and storage media for use in the present invention contributes to the ability of the present invention to maintain the viability of stem cells after thawing, as it retains the cells in a form of stasis during storage and reduces the cells' ability to progress into late stage apoptosis. It is further considered likely that the high purity of the albumin protein in the recombinant yeast-derived serum albumin products used in the present invention (compared to the lower purity found in albumin preparations from other sources) is a contributory factor to shielding the stem cells from signalling 'noise' created by factors present in less pure preparations, and that this can further contribute to minimising changes in cells during storage.

Optionally, the cryopreservation medium for use in accordance with the first aspect of the present invention comprises a recombinant serum albumin preparation and a separate cryopreservant. Further optionally, the cryopreservation medium comprises, consists essentially of, or consists of an aqueous solution of the recombinant yeast-derived serum albumin preparation, a cryopreservant, and an ionic buffer. Preferably the ionic buffer comprises, consists essentially of, or consists of, an aqueous solution of electrolytes, for example wherein the electrolytes are selected from the group consisting of sodium ions, potassium ions, magnesium ions, chloride ions, acetate ions, phosphate ions, and/or gluconate ions, and more preferably, wherein the ionic buffer possesses electrolyte concentrations, osmolality and/or pH that mimics that of human physiological plasma. For example, the ionic buffer may be a sterile, nonpyrogenic isotonic solution that contains, per 100 mL, about 526 mg of Sodium Chloride, USP (NaCl); about 502 mg of Sodium Gluconate ($C_6H_{11}NaO_7$); about 368 mg of Sodium Acetate Trihydrate, USP ($C_2H_3NaO_2 \cdot 3H_2O$); about 37 mg of Potassium Chloride, USP (KCl); and about 30 mg of Magnesium Chloride, USP ($MgCl_2 \cdot 6H_2O$), such as an isotonic buffer that is substantially equivalent to Plasmalyte®. Most preferably the ionic buffer is substantially isotonic to the stem cells and/or the ionic buffer is Plasmalyte®.

For the avoidance of doubt, it is to be noted that the cryopreservation medium for use in accordance with the first aspect of the present invention is not a stem cell culture growth media. It preferably does not support the growth of stem cells. For example, cell growth observed under standard growth conditions would be typically less than 50%, 40%, 30%, 20%, 10%, 5%, 4%, 3%, 2%, 1% or 0% of that observed for the same cells, under the same conditions, when grown in a standard stem cell culture growth medium such as DMEM.

More preferably the cryopreservation medium for use in accordance with the first aspect of the present invention includes substantially no, or no, levels of any one or more (such as all) of the components of a typical stem cell culture medium such as vitamins, hormones growth factors, iron sources, free amino acids and/or glucose.

As used herein, "vitamins" may include one or more of choline chloride, folic acid, myoinositol, niacinamide, d-pantothenic acid (hemicalcium), pyridoxal, pyridoxine, riboflavin and/or thiamine.

As used herein, "hormones" may include one or more of triiodothyronine, parathormone, tyrotrophin releasing hormone, somatomedin, estrogens, prolactin, growth hormone, testosterone, and/or hydrocortisone.

As used herein, "iron sources" may include transferrin.

As used herein, "growth factors" may include one or more of adrenomedullin (AM); angiopoietin (Ang); autocrine motility factor; bone morphogenetic proteins (BMPs); one or more members of the ciliary neurotrophic factor family (including, but not limited to ciliary neurotrophic factor (CNTF), leukemia inhibitory factor (LIF) and/or interleukin-6 (IL-6)); one or more colony-stimulating factors (including, but not limited to macrophage colony-stimulating factor (m-CSF), granulocyte colony-stimulating factor (G-CSF) and/or granulocyte macrophage colony-stimulating factor (GM-CSF)); epidermal growth factor (EGF); one or more ephrins (including, but not limited to Ephrin A1, Ephrin A2, Ephrin A3, Ephrin A4, Ephrin A5, Ephrin B1, Ephrin B2, Ephrin B3); erythropoietin (EPO); fibroblast growth factor (FGF); foetal bovine somatotrophin (FBS); one or more GDNF family of ligands (including, but not limited to, glial cell line-derived neurotrophic factor (GDNF), neurturin, persephin and/or artemin); growth differentiation factor-9 (GDF9); hepatocyte growth factor (HGF); hepatoma-derived growth factor (HDGF); insulin; one or more insulin-like growth factors (including, but not limited to, insulin-like growth factor-1 (IGF-1), and/or insulin-like growth factor-2 (IGF-2)); one or more Interleukins (including, but not limited to, IL-1, IL-2, IL-3, IL-4, IL-5, IL-6 and/or IL-7); keratinocyte growth factor (KGF); migration-stimulating factor (MSF); macrophage-stimulating protein (MSP); myostatin (GDF-8); one or more neuregulins (including, but not limited to neuregulin 1 (NRG1), neuregulin 2 (NRG2), neuregulin 3 (NRG3), and/or neuregulin 4 (NRG4)); one or more neurotrophins (including, but not limited to, brain-derived neurotrophic factor (BDNF), nerve growth factor (NGF); neurotrophin-3 (NT-3) and/or Neurotrophin-4 (NT-4)); placental growth factor (PGF); platelet-derived growth factor (PDGF); renalase (RNLS); T-cell growth factor (TCGF); thrombopoietin (TPO); one or more transforming growth factors (including, but not limited to transforming growth factor alpha (TGF-$\alpha$) and/or transforming growth factor beta (TGF-$\beta$)); tumor necrosis factor-alpha (TNF-$\alpha$); vascular endothelial growth factor (VEGF); and/or Wnt Signaling Pathway.

As used herein, "free amino acids" may include one or more of L-Arginine, L-Cystine, Glycine. L-Histidine, L-Isoleucine, L-Leucine, L-Lysine, L-Methionine, L-Phenylalanine, L-Serine, L-Threonine, L-Tryptophan, L-Tyrosine, and/or L-Valine.

The cryopreservant used in the cryopreservation medium is distinct from the recombinant yeast-derived serum albumin and may, for example, be selected from the group consisting of dimethyl sulphoxide (DMSO), glycerol, Polyethylene glycol (PEG), ethylene glycol (EG), polyvinylpyrrolidone (PVP), and Trehalose. DMSO may be particularly preferred. The cryoprotectant may be present in the cryopreservation medium, when mixed with the stem cells, at a concentration suitable to provide a cryopreservative effect. In the case of DMSO, this may be about 10% (w/v) $\pm$ 5%, 4%, 3%, 2%, or 1%. The skilled person can readily determine a suitable amount of cryopreservant using routine testing.

In accordance with a method according to the first aspect of the present invention, the storage medium may comprise the recombinant yeast-derived serum albumin preparation, and optionally, the storage medium may comprise, consist essentially of, or consist of an aqueous solution of the recombinant yeast-derived serum albumin preparation and an ionic buffer, preferably wherein the ionic buffer comprises, consists essentially of, or consists of, an aqueous solution of electrolytes, for example wherein the electrolytes are selected from the group consisting of sodium ions, potassium ions, magnesium ions, chloride ions, acetate ions, phosphate ions, and/or gluconate ions, and more preferably, wherein the ionic buffer possesses electrolyte concentrations, osmolality and/or pH that mimics that of human physiological plasma. For example, the ionic buffer may be a sterile, nonpyrogenic isotonic solution that contains, per 100 mL, about 526 mg of Sodium Chloride, USP (NaCl); about 502 mg of Sodium Gluconate ($C_6H_{11}NaO_7$); about 368 mg of Sodium Acetate Trihydrate, USP ($C_2H_3NaO_2 \bullet 3H_2O$); about 37 mg of Potassium Chloride, USP (KCl); and about 30 mg of Magnesium Chloride, USP ($MgCl_2 \bullet 6H_2O$), such as an isotonic buffer that is substantially equivalent to Plasmalyte®. Most preferably the ionic buffer is substantially isotonic to the stem cells and/or the ionic buffer is Plasmalyte®.

For the avoidance of doubt, it is to be noted that the storage medium for use in accordance with the first aspect of the present invention is not a stem cell culture growth medium. It preferably does not support the growth of stem cells. For example, cell growth observed under standard growth conditions would be typically less than 50%, 40%, 30%, 20%, 10%. 5%, 4%, 3%, 2%, 1% or 0% of that observed for the same cells, under the same conditions, when grown in a standard stem cell culture growth medium such as DMEM.

More preferably the storage medium for use in accordance with the first aspect of the present invention includes substantially no, or no, levels of any one or more (such as all) of the components of a typical stem cell culture medium such as vitamins, hormones, growth factors, iron sources, free amino acids and/or glucose. As used herein, the terms "vitamins", "hormones", "iron sources", "growth factors", "free amino acids" may be as defined further above.

It is particularly preferred that the cryopreservation medium and/or the storage medium used in accordance with the first aspect of the present invention individually or both do not comprise one or more components of a serum-derived albumin preparation, for example one or more components (such as all) selected from the list consisting of: haem, prekallikrein activator, pyrogens, hepatitis C human viruses and/or N-acetyl tryptophan, and is preferably substantially free of, or completely free of, octanoate and/or detergent (such as polysorbate 80).

The method of the first aspect of the present invention may comprise storing stem cells in the storage medium, optionally at a temperature of 2-8°C (e.g. at about 2°C, 3°C, 4°C, 5°C, 6°C, 7°C or 8 °C, wherein the term "about" can include the meaning of ± 0.9, 0.8, 0.7, 0.6, 0.5, 0.4, 0.3, 0.2, or 0.1 °C), and further optionally for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days), and optionally wherein, directly or indirectly after the step of storing stem cells in the storage medium, the method further comprises one or more steps, selected from the steps of:

- culturing the stem cells;
- expanding a culture of the stem cells;
- differentiation of the stem cells;
- immobilization of the stem cells, or cultured and/or differentiated cells derived therefrom, for example into tissue or a medical implant;
- formulating stem cells, or cultured and/or differentiated cells or other products derived therefrom, in a pharmaceutically acceptable composition or veterinarially acceptable composition; and/or
- administering the stem cells, or cultured and/or differentiated cells or other products derived therefrom, to a patient.

Optionally, a method in accordance with the first aspect of the present invention comprises storing stem cells in the storage medium, and after the storage, the stem cells are differentiated, for example into a cell type selected from osteocytes, cardiocytes, pancreatic beta cells, neurons, fibroblasts, cardiomyocytes, osteoblasts and/or chondrocytes.

A second aspect of the present invention provides a cryopreservation medium for the cryopreservation of stem cells, wherein the cryopreservation medium comprises a recombinant yeast-derived serum albumin preparation and a cryopreservant. The cryopreservant may, for example, be selected from the group consisting of dimethyl sulphoxide (DMSO), glycerol, Polyethylene glycol (PEG), ethylene glycol (EG), polyvinylpyrrolidone (PVP), and Trehalose. Preferably, the cryopreservation medium which comprises, consists essentially of, or consists of an aqueous solution of the recombinant serum albumin preparation, the cryopreservant, and an ionic buffer. Preferably the ionic buffer comprises, consists essentially of, or consists of, an aqueous solution of electrolytes, for example wherein the electrolytes are selected from the group consisting of sodium ions, potassium ions, magnesium ions, chloride ions, acetate ions, phosphate ions, and/or gluconate ions, and more preferably, wherein the ionic buffer possesses electrolyte concentrations, osmolality and/or pH that mimics that of human physiological plasma. For example, the ionic buffer may be a sterile, nonpyrogenic isotonic solution that contains, per 100 mL, about 526 mg of Sodium Chloride, USP (NaCl); about 502 mg of Sodium Gluconate ($C_6H_{11}NaO_7$); about 368 mg of Sodium Acetate Trihydrate, USP ($C_2H_3NaO_2•3H_2O$); about 37 mg of Potassium Chloride, USP (KCl); and about 30 mg of Magnesium Chloride, USP ($MgCl_2•6H_2O$), such as an isotonic buffer that is substantially equivalent to Plasmalyte®. Most preferably the ionic buffer is substantially isotonic to the stem cells and/or the ionic buffer is Plasmalyte®.

For the avoidance of doubt, it is to be noted that the cryopreservation medium of the second aspect of the present invention is not a stem cell culture growth media. It preferably does not support the growth of stem cells. For example, cell growth observed in the cryopreservation medium under standard growth conditions would be typically less than 50%, 40%, 30%, 20%, 10%. 5%, 4%, 3%, 2%, 1% or 0% of that observed for the same cells, under the same conditions, when grown in a standard stem cell culture growth medium such as DMEM.

More preferably the cryopreservation medium of the second aspect of the present invention includes substantially no, or no, levels of any one or more (such as all) of the components of a typical stem cell culture medium such as vitamins, hormones, growth factors, iron sources, free amino acids and/or glucose. As used herein, the terms "vitamins", "hormones", "iron sources", "growth factors", "free amino acids" may be as defined further above

The cryopreservation medium of the second aspect of the present invention may further comprise stem cells, and optionally the stem cells may be selected from the group consisting of pluripotent stem cells (such as embryonic stem cells, embryonic germ cells, induced pluripotent stem cells), multipotent stem cells (such as adult stem cells, for example, mesenchymal stem cells which may optionally be derived from fat, bone marrow, umbilical cord blood, or umbilical cord; hematopoietic stem cells, which may optionally be derived from bone marrow or peripheral blood; neural stem cells; or germ stem cells) or unipotent stem cells (such as committed stem cells for hepatocytes).

Optionally, the cryopreservation medium of the second aspect of the present invention, which may comprise stem cells, is in a frozen form, for example, in a form that is below 0°C, and more preferably in a form that is between about -80°C and about -196°C. As used in this context, the term "about" may include ± 20, 15, 10, 9, 8, 7, 6, 5, 4, 3, 2, 1 °C. In accordance with this embodiment, any stem cells present in the frozen composition are preferably in a state of suspended animation. The cryopreservation medium comprising the stem cells, in a frozen form, may be maintained in a frozen form for 1, 2, 3, 4, 5, 5, 6, or 7 days, 1, 2, 3, 4, 5, 6, 7, 8, 9, 10 weeks, 1, 2, 3, 4, 5, 6, 7, 8, 9, 10, 11, 12 months, 1, 2, 3, 4, 5, 6, 7, 8, 9, 10 years or longer.

Alternatively, the cryopreservation medium of the second aspect of the present invention is not in a frozen form, but may comprise a population of stem cells which has been frozen and then thawed. Stem cell populations that have been frozen and thawed can be distinguished from stem cell populations that have not been through the freeze/thaw process. The freezing causes stress to the cell and will typically initiate programmed cell death (apoptosis). The data in the present examples show how the stages of apoptosis can be followed by specific markers, such as Annexin V binding, and PI and/or 7AAD inclusion, as discussed herein. Stem cell populations that have been frozen and thawed can, for example, be distinguished from stem cell populations that have not been through the freeze/thaw process by measuring the level of early stage and late stage apoptosis within the cell population. All cell populations will have a percentage of cells in apoptotic stage, but after freezing and thawing the level is increased, particularly when stored in a storage solution as described herein at 2-8°C for a period of greater than 24 hours. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

A cryopreservation medium of the second aspect of the present invention will preferably possess one or more (such as all) of the characteristics described above for cryopreservation media used in respect of the first aspect of the present invention.

Preferably, a recombinant yeast-derived serum albumin preparation is present in the cryopreservation medium of the second aspect of the present invention, when mixed with the stem cells, in an amount suitable to provide a concentration of the recombinant yeast-derived serum albumin protein that is greater than about 0.01 % (w/v) and less than 10% (w/v), less than about 9% (w/v), less than about 8% (w/v), less than about 7% (w/v) or less than about 6% (w/v), such as at a concentration of from about 0.1 % (w/v) to about 5% (w/v), preferably at about 1% (w/v), about 2% (w/v), about 3 (w/v) or about 4% (w/v). The term "about" in this context, can include meaning of ± 50%, 40%, 30%, 20%, 10%, 5%, 4%, 3%, 2% or 1% of the stated value; for example, 0.1% (w/v) ± 10% of the stated value is 0.9 to 0.11 % (w/v).

Preferably, the cryopreservation medium of the second aspect of the present invention, comprises recombinant yeast-derived serum albumin protein that exhibits one or more of the following properties:

(a) less than 0.5% (w/w) binds to Concanavalin A, preferably less than 0.4%, 0.3%, 0.2% or 0.15%; and/or
(b) a glycation level of less than 0.6 moles hexose / mole of protein, and preferably less than 0.10, 0.075 or 0.05 moles hexose / mole of protein.

Preferably, the cryopreservation medium of the second aspect of the present invention, comprises recombinant yeast-derived serum albumin protein that:

(a) is at least about 95%, 96%, 97%, 98%, more preferably at least about 99.5% monomeric and dimeric, preferably essentially 100% monomeric and dimeric;
(b) is at least about 93%, 94%, 95%, 96% or 97% monomeric; and/or
(c) has an albumin polymer content of not greater, and preferably less, than about 1.0% (w/w), 0.1 % (w/w) or 0.01% (w/w). As used in this context, the term "polymer" as applied to recombinant yeast-derived serum albumin protein is distinct from monomeric and dimeric forms.

Preferably, in accordance with the second aspect of the present invention:

(a) the recombinant yeast-derived serum albumin preparation present in the cryopreservation medium comprises, consists essentially of, or consists of, yeast-derived serum albumin protein, cations (such as sodium, potassium, calcium, magnesium, ammonium, preferably sodium) and balancing anions (such as chloride, phosphate, sulfate, citrate or acetate, preferably chloride or phosphate), water, and optionally octanoate and polysorbate 80;
(b) the cryopreservation medium comprising the recombinant yeast-derived serum albumin preparation and one or more other components, optionally including stem cells, comprises octanoate at less than 35mM, 32.5mM, 30 mM, 28 mM, 26 mM, 24 mM, 22 mM, 20 mM, 18 mM, 16 mM, 15 mM, 14 mM, 12 mM, 10 mM, 8 mM, 6 mM, 5 mM, 4 mM, 3 mM, 2 mM, 1 mM, 0.5 mM, 0.4 mM, 0.3 mM, 0.2 mM, 0.1 mM, 0.01 mM, 0.001 mM, is substantially free of octanoate, or is free of octanoate;

(c) the cryopreservation medium comprising the recombinant yeast-derived serum albumin preparation and one or more other components optionally including stem cells, has an overall fatty acid content less than or equal to 35mM, 32.5mM, 30 mM, 28 mM, 26 mM, 24 mM, 22 mM, 20 mM, 15 mM, 10 mM, 5 mM, 4 mM, 3 mM, 2 mM, 1 mM, is substantially free of fatty acids, or is free of fatty acids;

(d) the cryopreservation medium comprising the recombinant yeast-derived serum albumin preparation and one or more other components, optionally including stem cells, comprises detergent, such as polysorbate (preferably polysorbate 80) at a concentration less than 200 $mg.L^{-1}$, 150 $mg.L^{-1}$, 100 $mg.L^{-1}$, 90 $mg.L^{-1}$, 80 $mg.L^{-1}$, 70 $mg.L^{-1}$, 60 $mg.L^{-1}$, 50 $mg.L^{-1}$, 40 $mg.L^{-1}$, 30 $mg.L^{-1}$, 20 $mg.L^{-1}$, 15 $mg.L^{-1}$, 10 $mg.L^{-1}$, 5 $mg.L^{-1}$, 4 $mg.L^{-1}$, 3 $mg.L^{-1}$, 2 $mg.L^{-1}$, 1 $mg.L^{-1}$, 0.5 $mg.L^{-1}$, 0.1 $mg.L^{-1}$, 0.01 $mg.L^{-1}$, 0.001 $mg.L^{-1}$, is substantially free of detergent, such as polysorbate (preferably polysorbate 80), or is free of the detergent (preferably is free of polysorbate 80);

(e) the cryopreservation medium comprising the recombinant yeast-derived serum albumin preparation and one or more other components, optionally including stem cells, comprises total free amino acid levels and/or N-acetyl tryptophan levels less than 35mM, 32.5mM, 30 mM, 28 mM, 26 mM, 24 mM, 22 mM, 20 mM, 15 mM, 10 mM, 5 mM, 4 mM, 3 mM, 2 mM, 1 mM, 0.5 mM, 0.1 mM, 0.01 mM, 0.005 mM, 0.001 mM, is substantially free of free amino acids and/or N-acetyl tryptophan in particular, or is free of free amino acids and/or of N-acetyl tryptophan in particular;

(f) the cryopreservation medium comprising the recombinant yeast-derived serum albumin preparation and one or more other components, optionally including stem cells, is substantially free of, or completely free of, all of octanoate, free amino acids and/or N-acetyl tryptophan in particular, and detergent (such as polysorbate 80);

(g) the recombinant yeast-derived serum albumin protein present in the cryopreservation medium is a preparation selected from: Recombumin® Prime, or a preparation that is similar thereto; Recombumin® Alpha, or a preparation that is similar thereto; or AlbIX®, or a preparation that is similar thereto;

(h) the cryopreservation medium comprising the recombinant yeast-derived serum albumin preparation and one or more other components, optionally including stem cells, is free of one or more, such all, components selected from: haem, prekallikrein activator, pyrogens, hepatitis C and/or human viruses) and/or is has an aluminium concentration of less than 200 $\mu g.L^{-1}$, such as less than 180 $\mu g.L^{-1}$, 160 $\mu g.L^{-1}$, 140 $\mu g.L^{-1}$, 120 $\mu g.L^{-1}$, 100 $\mu g.L^{-1}$, 90 $\mu g.L^{-1}$, 80 $\mu g.L^{-1}$, 70 $\mu g.L^{-1}$, 60 $\mu g.L^{-1}$, 50 $\mu g.L^{-1}$, or 40 $\mu g.L^{-1}$, more typically within the range of about 10 $\mu g.L^{-1}$ to about 30 $\mu g.L^{-1}$;

(i) the recombinant yeast-derived serum albumin protein present in the cryopreservation medium possesses an intact or substantially intact N-terminal sequence;

(j) the recombinant yeast-derived serum albumin protein present in the cryopreservation medium comprise albumin protein that has a free thiol group content that is greater than 62%, such as at least 69%, at least 70%, at least 75%, at least 80%, at least 85%, at least 90%, at least 95%, about 96%, about 97%;

(k) the recombinant yeast-derived serum albumin preparation present in the cryopreservation medium comprises albumin protein that, when tested by size exclusion chromatography (SEC), displays an SEC profile excluding peaks with a peak retention time under 14 minutes and over 19 minutes, and more preferably excludes peaks with a peak retention time under 14 or 15 minutes and over 18 minutes;

(l) the recombinant yeast-derived serum albumin preparation present in the cryopreservation medium comprises albumin protein that, when tested by reversed phase high performance liquid chromatography (RP-HPLC), displays a single major peak, corresponding to albumin in the native monomeric form;

(m) the recombinant yeast-derived serum albumin preparation present in the cryopreservation medium comprises albumin protein that, when tested by mass spectrometry, is a product that displays fewer than 13, 12, 11, 10, 9, 8, 7, 6, such as about 1 to 11, 1 to 8, 1 to 5, 1 to 4, 1 to 3, 1 to 2, 1 or less than 1, hexose modified lysine and/or arginine residues per protein; and/or

(n) the recombinant yeast-derived serum albumin preparation present in the cryopreservation medium comprise albumin protein that is not glycated with plant-specific sugars, such as $\alpha$-1,3-fucose and/or $\beta$-1,2-xylose.

A third aspect of the present invention provides a storage medium for the storage of stem cells that have been frozen in a cryopreservation medium, thawed, and then transferred to the storage medium, wherein the storage medium comprises a recombinant yeast-derived serum albumin preparation. Preferably, the storage medium comprises, consists essentially of, or consists of an aqueous solution of the recombinant yeast-derived serum albumin preparation and an ionic buffer. The ionic buffer comprises, consists essentially of, or consists of, an aqueous solution of electrolytes, for example wherein the electrolytes are selected from the group consisting of sodium ions, potassium ions, magnesium ions, chloride ions, acetate ions, phosphate ions, and/or gluconate ions, and preferably, wherein the ionic buffer possesses electrolyte concentrations, osmolality and/or pH that mimics that of human physiological plasma. For example, the ionic buffer may be a sterile, nonpyrogenic isotonic solution that contains, per 100 mL, about 526 mg of Sodium Chloride, USP (NaCl); about 502 mg of Sodium Gluconate ($C_6H_{11}NaO_7$); about 368 mg of Sodium Acetate Trihydrate, USP ($C_2H_3NaO_2$•$3H_2O$); about 37 mg of Potassium Chloride, USP (KCl); and about 30 mg of Magnesium Chloride, USP ($MgCl_2$•$6H_2O$), such as an isotonic buffer that is substantially equivalent to Plasmalyte®. Most preferably the ionic buffer is substantially isotonic to the stem cells and/or the ionic buffer is Plasmalyte®.

For the avoidance of doubt, it is to be noted that the storage medium of the third aspect of the present invention is not a stem cell culture growth media. It preferably does not support the growth of stem cells. For example, cell growth observed in the storage medium under standard growth conditions would be typically less than 50%, 40%, 30%, 20%, 10%. 5%, 4%, 3%, 2%, 1% or 0% of that observed for the same cells, under the same conditions, when grown in a standard stem cell culture growth medium such as DMEM.

More preferably the storage medium of the third aspect of the present invention includes substantially no, or no, levels of any one or more (such as all) of the components of a typical stem cell culture medium such as vitamins, hormones, growth factors, iron sources, free amino acids and/or glucose. As used herein, the terms "vitamins", "hormones", "iron sources", "growth factors", "free amino acids" may be as defined further above.

A storage medium of the third aspect of the present invention will preferably possess one or more (such as all) of the characteristics described above for storage media used in respect of the first aspect of the present invention.

Preferably, the recombinant yeast-derived serum albumin preparation is present in the storage medium of the third aspect of the present invention, when mixed with the stem cells, in an amount suitable to provide a concentration of the recombinant yeast-derived serum albumin protein that is greater than about 0.01 % (w/v) and less than 10% (w/v), less than about 9% (w/v), less than about 8% (w/v), less than about 7% (w/v) or less than about 6% (w/v), such as at a concentration of from about 0.1 % (w/v) to about 5% (w/v), preferably at about 1% (w/v), about 2% (w/v), about 3 (w/v) or about 4% (w/v).

Typically, the recombinant yeast-derived serum albumin protein present in the storage medium of the third aspect of the present invention exhibits one or more of the following properties:

(a) less than 0.5% (w/w) binds to Concanavalin A, preferably less than 0.4%, 0.3%, 0.2% or 0.15%; and/or

(b) a glycation level of less than 0.6 moles hexose / mole of protein, and preferably less than 0.10, 0.075 or 0.05 moles hexose / mole of protein.

Typically, the recombinant yeast-derived serum albumin protein present in the storage medium of the third aspect of the present invention:

(a) is at least about 95%, 96%, 97%, 98%, more preferably at least about 99.5% monomeric and dimeric, preferably essentially 100% monomeric and dimeric;

(b) is at least about 93%, 94%, 95%, 96% or 97% monomeric; and/or

(c) has an albumin polymer content of not greater, and preferably less, than about 1.0% (w/w), 0.1 % (w/w) or 0.01% (w/w). As used in this context, the term "polymer" as applied to recombinant yeast-derived serum albumin protein is distinct from monomeric and dimeric forms.

Preferably, in the storage medium of the third aspect of the present invention:

(a) the recombinant yeast-derived serum albumin preparation present in the storage medium comprises, consists essentially of, or consists of, yeast-derived serum albumin protein, cations (such as sodium, potassium, calcium, magnesium, ammonium, preferably sodium) and balancing anions (such as chloride, phosphate, sulfate, citrate or acetate, preferably chloride or phosphate), water, and optionally octanoate and polysorbate 80;

(b) the storage medium comprising the recombinant yeast-derived serum albumin preparation and one or more other components, optionally including stem cells, comprises octanoate at less than 35mM, 32.5mM, 30 mM, 28 mM, 26 mM, 24 mM, 22 mM, 20 mM, 18 mM, 16 mM, 15 mM, 14 mM, 12 mM, 10 mM, 8 mM, 6 mM, 5 mM, 4 mM, 3 mM, 2 mM, 1 mM, 0.5 mM, 0.4 mM, 0.3 mM, 0.2 mM, 0.1 mM, 0.01 mM, 0.001 mM, is substantially free of octanoate, or is free of octanoate;

(c) the storage medium comprising the recombinant yeast-derived serum albumin preparation and one or more other components optionally including stem cells, has an overall fatty acid content less than or equal to 35mM, 32.5mM, 30 mM, 28 mM, 26 mM, 24 mM, 22 mM, 20 mM, 15 mM, 10 mM, 5 mM, 4 mM, 3 mM, 2 mM, 1 mM, is substantially free of fatty acids, or is free of fatty acids;

(d) the storage medium comprising the recombinant yeast-derived serum albumin preparation and one or more other components, optionally including stem cells, comprises detergent, such as polysorbate (preferably polysorbate 80) at a concentration less than 200 $mg.L^{-1}$, 150 $mg.L^{-1}$, 100 $mg.L^{-1}$, 90 $mg.L^{-1}$, 80 $mg.L^{-1}$, 70 $mg.L^{-1}$, 60 $mg.L^{-1}$, 50 $mg.L^{-1}$, 40 $mg.L^{-1}$, 30 $mg.L^{-1}$, 20 $mg.L^{-1}$, 15 $mg.L^{-1}$, 10 $mg.L^{-1}$, 5 $mg.L^{-1}$, 4 $mg.L^{-1}$, 3 $mg.L^{-1}$, 2 $mg.L^{-1}$, 1 $mg.L^{-1}$, 0.5 $mg.L^{-1}$, 0.1 $mg.L^{-1}$, 0.01 $mg.L^{-1}$, 0.001 $mg.L^{-1}$, is substantially free of detergent, such as polysorbate (preferably polysorbate 80), or is free of the detergent (preferably is free of polysorbate 80);

(e) the storage medium comprising the recombinant yeast-derived serum albumin preparation and one or more other components, optionally including stem cells, comprises total free amino acid levels and/or N-acetyl tryptophan levels less than 30 mM, 28 mM, 26 mM, 24 mM, 22 mM, 20 mM, 15 mM, 10 mM, 5 mM, 4 mM, 3 mM, 2 mM, 1 mM, 0.5 mM, 0.1 mM, 0.01 mM, 0.005 mM, 0.001 mM, is substantially free of free amino acids and/or N-acetyl tryptophan in particular, or is free of free amino acids and/or of N-acetyl tryptophan in particular;

(f) the storage medium comprising the recombinant yeast-derived serum albumin preparation and one or more other components, optionally including stem cells, is substantially free of, or completely free of, all of octanoate, free amino acids and/or N-acetyl tryptophan in particular, and detergent (such as polysorbate 80);

(g) the recombinant yeast-derived serum albumin protein present in the storage medium is a preparation selected from: Recombumin® Prime, or a preparation that is similar thereto; Recombumin® Alpha, or a preparation that is similar thereto; or AlbIX®, or a preparation that is similar thereto;

(h) the storage medium comprising the recombinant yeast-derived serum albumin preparation and one or more other components, optionally including stem cells, is free of one or more, such all, components selected from: haem, prekallikrein activator, pyrogens, hepatitis C and/or human viruses) and/or has an aluminium concentration of less than 200 $\mu g.L^{-1}$, such as less than 180 $\mu g.L^{-1}$, 160 $\mu g.L^{-1}$, 140 $\mu g.L^{-1}$, 120 $\mu g.L^{-1}$, 100 $\mu g.L^{-1}$, 90 $\mu g.L^{-1}$, 80 $\mu g.L^{-1}$, 70 $\mu g.L^{-1}$, 60 $\mu g.L^{-1}$, 50 $\mu g.L^{-1}$, or 40 $\mu g.L^{-1}$, more typically within the range of about 10 $\mu g.L^{-1}$ to about 30 $\mu g.L^{-1}$;

(i) the recombinant yeast-derived serum albumin protein present in the storage medium possesses an intact or substantially intact N-terminal sequence;

(j) the recombinant yeast-derived serum albumin protein present in the storage medium comprises albumin protein that has a free thiol group content that is greater than 62%, such as at least 69%, at least 70%, at least 75%, at least 80%, at least 85%, at least 90%, at least 95%, about 96%, about 97%;

(k) the recombinant yeast-derived serum albumin preparation present in the storage medium comprises albumin protein that, when tested by size exclusion chromatography (SEC), displays an SEC profile excluding peaks with a peak retention time under 14 minutes and over 19 minutes, and more preferably excludes peaks with a peak retention time under 14 or 15 minutes and over 18 minutes;

(l) the recombinant yeast-derived serum albumin preparation present in the storage medium comprises albumin protein that, when tested by reversed phase high performance liquid chromatography (RP-HPLC), displays a single major peak, corresponding to albumin in the native monomeric form;

(m) the recombinant yeast-derived serum albumin preparation present in the storage medium comprises albumin protein that, when tested by mass spectrometry, is a product that displays fewer than 13, 12, 11, 10, 9, 8, 7, 6, such as about 1 to 11, 1 to 8, 1 to 5, 1 to 4, 1 to 3, 1 to 2, 1 or less than 1 hexose modified lysine and/or arginine residues per protein; and/or

(n) the recombinant yeast-derived serum albumin preparation present in the storage medium comprise albumin protein that is not glycated with plant-specific sugars, such as $\alpha$-1,3-fucose and/or $\beta$-1,2-xylose.

In a preferred embodiment, the storage medium of the third aspect of the present invention further comprises stem cells. Optionally, the stem cells may be are selected from the group consisting of pluripotent stem cells (such as embryonic stem cells, embryonic germ cells, induced pluripotent stem cells), multipotent stem cells (such as adult stem cells, for example, mesenchymal stem cells which may optionally be derived from fat, bone marrow, umbilical cord blood, or umbilical cord; hematopoietic stem cells, which may optionally be derived from bone marrow or peripheral blood; neural stem cells; or germ stem cells) or unipotent stem cells (such as committed stem cells for hepatocytes).

Optionally, the storage medium of the third aspect of the present invention further comprises stem cells that have been frozen in a cryopreservation medium (preferably, a cryopreservation medium as defined by the first and/or second aspect of the present invention), thawed, and then transferred to the storage medium. As noted above, stem cell populations that have been frozen and thawed can be distinguished from stem cell populations that have not been through the freeze/thaw process. The freezing causes stress to the cell and will typically initiate programmed cell death (apoptosis). The data in the present examples show how the stages of apoptosis can be followed by specific markers, such as Annexin V binding, and PI and/or 7AAD inclusion, as discussed herein. Stem cell populations that have been frozen and thawed can, for example, be distinguished from stem cell populations that have not been through the freeze/thaw process by measuring the level of early stage and late stage apoptosis within the cell population. All cell populations will have a percentage of cells in apoptotic stage, but after freezing and thawing the level is increased, particularly when stored in a storage solution as described herein at 2-8°C for a period of greater than 24 hours. Optionally, the storage temperature is about 2°C, 3°C, 4°C, 5°C, 6°C, 7°C or 8 °C. The term "about" as used in this context, can include the meaning of $\pm$ 0.9, 0.8, 0.7, 0.6, 0.5, 0.4, 0.3, 0.2, or 0.1 °C.

Optionally, the storage medium of the third aspect of the present invention comprises stem cells that have been frozen in a cryopreservation medium of the first and/or second aspect of the present invention, thawed, and then transferred to the storage medium.

Typically, the storage medium of the third aspect of the present invention, which further comprises stem cells stored in the storage medium, is stored in a refrigerator and/or as at a temperature of 2-8°C. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

The storage medium of the third aspect of the present invention, which further comprises stem cells, may be optionally characterised in that the stem cells have been frozen in a cryopreservation medium, thawed, and then transferred to the storage medium, and the stem cells are stored in the storage medium (typically in a refrigerator and/or at a temperature of 2-8°C) for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days). Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

In accordance with an embodiment of the third aspect of the present invention, the storage medium further comprises stem cells (optionally stem cells that have been frozen in a cryopreservation medium, thawed, and then transferred to the storage medium, or subjected to another physiological shock prior to being transferred to the storage medium), that have been stored in the storage medium at a temperature of 2-8°C for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days), and in which the viability of the stem cells at the end of the storage period is greater than 5%, 10%, 15%, 20%, 25%, 30%, 35%, 40%, 45%, 50%, 55%, 60%, 70%, 75%, 80%, 85%, 86%, 87%, 88%, 89%, 90%, 91%, 92%, 93%, 94%, 95% or more, such as about 60%, 70%, 75%, 80%, 85%, 86%, 87%, 88%, 89%, 90%, 91%, 92%, 93%, 94%, 95% or more. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

For example, a viability of about 60%, 70%, 75%, 80%, 85%, 86%, 87%, 88%, 89%, 90%, 91%, 92%, 93%, 94%, 95% or more is preferred after about 48 hours in storage.

A viability of about 60%, 70%, 75%, 80%, 85%, 86%, 87%, 88%, 89%, 90%, 91%, 92%, 93%, 94%, 95% or more is preferred after about 72 hours in storage.

Viability may, for example, be determined using markers such as Annexin V binding and PI inclusion, as discussed below in respect of Fig 6 and example 1.

As surprisingly demonstrated in the present examples, post-thaw viability was extended further when using an albumin concentration of around 2% (w/v), rather than 5% (w/v) in the tests. Even so, the benefit of using recombinant yeast-derived serum albumin, rather than plasma-derived serum albumin, was even more pronounced when using 5% (w/v) albumin.

Accordingly, in one embodiment of the third aspect of the present invention, the recombinant yeast-derived serum albumin protein is present in the cryopreservation and/or storage medium at about 2% (w/v) ± 1.5, 1.4, 1.3, 1.2, 1.1, 1.0, 0.9, 0.8, 0.7, 0.6, 0.5, 0.4, 0.3, 0.2 or 0.1 % (w/v), when mixed with the stem cells, and the storage medium further comprises the stem cells (optionally stem cells that have been frozen in a cryopreservation medium, thawed, and then transferred to the storage medium, or subjected to another physiological shock prior to being transferred to the storage medium), that have been stored in the storage medium for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days), and in which the viability of the stem cells at the end of the storage period is greater than 5%, 10%, 15%, 20%, 25%, 30%, 35%, 40%, 45%, 50%, 55%, 60%, 70%, 75%, 80%, 85%, 86%, 87%, 88%, 89%, 90%, 91%, 92%, 93%, 94%, 95% or more, such as about 60%, 70%, 75%, 80%, 85%, 86%, 87%, 88%, 89%, 90%, 91%, 92%, 93%, 94%, 95% or more.

In another embodiment of the third aspect of the present invention, the recombinant yeast-derived serum albumin protein is present in the cryopreservation and/or storage medium at 5% (w/v) ± 1.5, 1.4, 1.3, 1.2, 1.1, 1.0, 0.9, 0.8, 0.7, 0.6, 0.5, 0.4, 0.3, 0.2 or 0.1 % (w/v), when mixed with the stem cells, and the storage medium comprises further comprises the stem cells (optionally stem cells that have been frozen in a cryopreservation medium, thawed, and then transferred to the storage medium, or subjected to another physiological shock prior to being transferred to the storage medium), that have been stored in the storage medium for a period of time greater than 24 hours, such as up to about 48 hours, for example up to about 72 hours, or more, and in which the viability of the stem cells at the end of the storage period is greater than 5%, 10%, 15%, 20%, 25%, 30%, 35%, 40%, 45%, 50%, 55%, 56%, 57%, 58%, 59%, 60%, 61%, 62%, 63%, 64%, 65% or more, such as about 55%, 56%, 57%, 58%, 59%, 60%, 61%, 62%, 63%, 64%, 65% or more.

Uses of cryopreservation media and storage media are also provided by the present invention.

Accordingly, in a fourth aspect, the present invention provides for the use of a cryopreservation medium according to second aspect of the present invention for the preservation of stem cells.

The use of the fourth aspect of the present invention may be for the preservation of stem cells in a viable state following storage of the stem cells at 2-8°C for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days). Optionally, the viability of the stem cells at the end of the storage period is greater than 5%, 10%, 15%, 20%, 25%, 30%, 35%, 40%, 45%, 50%, 55%, 60%, 70%, 75%, 80%, 85%, 86%, 87%, 88%, 89%, 90%, 91%, 92%, 93%, 94%, 95% or more, such as about 60%, 70%, 75%, 80%, 85%, 86%, 87%, 88%, 89%, 90%, 91%, 92%, 93%, 94%, 95% or more. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

Optionally, in accordance with the use of the fourth aspect of the present invention the recombinant yeast-derived serum albumin protein is present in the cryopreservation medium at about 2% (w/v) ± 1.5, 1.4, 1.3, 1.2, 1.1, 1.0, 0.9, 0.8, 0.7, 0.6, 0.5, 0.4, 0.3, 0.2 or 0.1 % (w/v), when mixed with the stem cells. In an alternative option, the recombinant yeast-derived serum albumin protein may be present in the cryopreservation medium at 5% (w/v) ± 1.5, 1.4, 1.3, 1.2, 1.1, 1.0, 0.9, 0.8, 0.7, 0.6, 0.5, 0.4, 0.3, 0.2 or 0.1 % (w/v), when mixed with the stem cells.

In one embodiment, the use of the fourth aspect of the present invention is for the preservation of stem cells by combining the stem cells with the cryopreservation medium to produce a mixture, and freezing the mixture to produce a frozen stem cell product (or subjecting the stem cells to another physiological shock), prior to storage at 2-8°C for a period of time greater than 24 hours, such as up to about 48 hours, for example up to about 72 hours, or more. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

- This may optionally comprise the steps of thawing the frozen stem cell product, transferring the thawed cells to a storage medium, and storing stem cells in the storage medium at 2-8°C for a period of time greater than 24 hours, such as up to about 48 hours, for example up to about 72 hours, or more.
- In a further alternative, this may optionally be for the preservation of stem cells by combining the stem cells with the cryopreservation medium to produce a mixture, subjecting the stem cells to a physiological shock, transferring the cells to a storage medium, and storing stem cells in the storage medium at 2-8°C for a period of time greater than 24 hours, such as up to about 48 hours, for example up to about 72 hours, or more.

Preferably with either alternative, the storage medium is a storage medium according the third aspect of the present invention.

A fifth aspect of the present invention provides for the use of a storage medium according to the third aspect of the present invention for the preservation of stem cells, by storing stem cells in the storage medium. Optionally, the stem cells have been frozen in a cryopreservation medium, thawed, and then transferred to the storage medium prior to storage. Alternatively, the stem cells may have been mixed with a cryopreservation medium, subjected a physiological shock, and then transferred to the storage medium prior to storage. With either alternative, it may be preferred that the cryopreservation medium is a cryopreservation medium according to the second aspect of the present invention.

A sixth aspect of the present invention provides for the use of recombinant yeast-derived serum albumin for improving the post-thawing viability of cryopreserved stem cells. The improvement is typically compared to the level of post-thawing viability of cryopreserved stem cells observed when using plasma-derived serum albumin in place of the recombinant yeast-derived serum albumin, in the same concentration. The improvement may, for example, be observable in the post-thawed stem cells, when stored in a storage medium at 2-8°C for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days). Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

In accordance with the sixth aspect of the present invention, the recombinant yeast-derived serum albumin may be used by formulating it into a cryopreservation medium and mixing the cryopreservation medium with stem cells prior to freezing, and optionally wherein the cryopreservation medium is a medium as defined by the second aspect of the present invention.

In accordance with the sixth aspect of the present invention, the recombinant yeast-derived serum albumin may additionally or alternatively be used by formulating it into a storage medium and mixing the storage medium with stem cells after thawing, and optionally wherein the storage medium is a medium as defined by the third aspect of the present invention.

A seventh aspect of the present invention provides for the use of recombinant yeast-derived serum albumin for improving the viability of cells that are subjected to physiological shock. The cells may be, for example, animal cells, mammalian cells, human cells, and/or preferably stem cells or lymphocytes. The improvement is typically compared to the level of post-shock viability of the cells (e.g. stem cells) observed when using plasma-derived serum albumin in place of the recombinant yeast-derived serum albumin, in the same concentration. The improvement may be observable in the post-shock cells (e.g. stem cells), when stored in a storage medium at 2-8°C for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days). Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

In accordance with the seventh aspect of the present invention, the recombinant yeast-derived serum albumin may be used by formulating it into a cryopreservation medium and mixing the cryopreservation medium with the cells (e.g. stem cells) prior to the physiological shock, and optionally the cryopreservation medium may be a medium as defined by the second aspect of the present invention.

In accordance with the seventh aspect of the present invention, the recombinant yeast-derived serum albumin may additionally, or alternatively, be used by formulating it into a storage medium and mixing the storage medium with cells (e.g. stem cells) after receiving the physiological shock, optionally wherein the storage medium is a medium as defined by the third aspect of the present invention.

The present examples also surprisingly show the benefits of recombinant yeast-derived serum albumin in maintaining stressed cells in an early apoptotic phase rather than a late apoptotic phase. Cells in an early apoptotic phase are not committed to apoptosis, and can revert, under favourable conditions, into a rescued form that can be used as viable cells. For example, as discussed in Vives et al, 2003, Met. Eng., 5: 124-132, although cells can respond to several insults by activating the apoptosis program, the viability and growth of protected cells may be recovered when re-exposed to non-inducing apoptosis conditions, whereas cells not prevented from entering late-stage apoptosis will irremediably die. Tintó et al, J. Biotechnol., 95: 205-214 exemplifies the recovery of hybridoma cell growth, using caspase inhibitors to protect against apoptosis-inducing culture conditions, and shows a return to normal growth after reversion to favourable culture conditions.

Accordingly, an eighth aspect of the present invention provides a method for preventing, delaying, or reducing, the switch of cells from early stage apoptosis to late stage apoptosis, the method comprising mixing the cells with a medium comprising recombinant yeast-derived serum albumin preparation.

The eighth aspect of the present invention also provides for the use of recombinant yeast-derived serum albumin preparation, for example in the form of a medium comprising the recombinant yeast-derived serum albumin preparation, for preventing, delaying, or reducing, the switch of cells from early stage apoptosis to late stage apoptosis.

The cells to be treated in accordance with the eighth aspect of the present invention may any suitable cell type that can undergo apoptosis, and may for example, be selected from animal cells, or human cells. Preferably, the cells are stem cells. Examples of such stem cells include pluripotent stem cells (such as embryonic stem cells, embryonic germ cells, induced pluripotent stem cells), multipotent stem cells (such as adult stem cells, for example, mesenchymal stem cells which may optionally be derived from fat, bone marrow, umbilical cord blood, or umbilical cord; hematopoietic stem cells, which may optionally be derived from bone marrow or peripheral blood; neural stem cells; or germ stem cells) or unipotent stem cells (such as committed stem cells for hepatocytes). Optionally, the cells to be treated be treated in accordance with the eighth aspect of the present invention may be *ex vivo* cells.

In accordance with the eighth aspect of the present invention, the cells may be mixed with the medium comprising recombinant yeast-derived serum albumin preparation prior to and/or after receiving a physiological shock. It may be preferred for the recombinant yeast-derived serum albumin preparation to be presented to the cells both before and after the physiological shock. By "physiological shock" we include physical and chemical changes in the environment of the cells which can induce apoptosis in the population of cells to be treated. Preferably, a physiological shock, within the meaning of this aspect of the invention, will induce apoptosis in at least 10%, 20%, 30%, 40%, 50%, 60%, 70%, 80%, 90%, 95% or more of the cells in the population, in the absence the recombinant yeast-derived serum albumin preparation, when stored in a storage medium at 2-8°C for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days), even if in the presence of plasma-derived serum albumin. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

Examples of physiological shock include, without limitation, heat shock (e.g. greater than 37, 38, 39, 40, 41, 42, 43, 44, 45, 56, 47, 48, 49 or 50°C), cold shock (e.g. lower than 2, 1, 0, -1, -2, -3, -4, -5, -6, -7, -8, -9, -10, -20, -80, -100°C, or lower), osmotic shock (e.g. exposure to conditions that are substantially non-isotonic with the cells), nutrient deprivation, exposure to toxic compounds, exposure to and/or deprivation of metabolites, exposure to and/or deprivation of enzymes, chemical shock, pH shock, exposure to organic solutions and other shocks such as exposure to shearing forces, surface exposure and/or loss of surface exposure. Optionally, the physiological shock may be shock resulting from one or more of the steps of freezing and/or thawing during cryopreservation.

In accordance with the eighth aspect of the present invention, without limitation, early stage apoptosis may be characterised by cells which display Annexin (such as Anneixn V) binding but no propidium iodide (PI) and/or 7-aminoactinomycin D (7AAD) inclusion, for example as determined using flow cytometry. Early stage apoptosis may be further characterised by mitochondrial permeability that is higher than the level observed in the same batch of cells that has not received a physiological shock, in combination with Annexin binding but no PI and/or 7AAD inclusion. Other characterising features of early-stage apoptosis are well known in the art (examples of which are discussed elsewhere within this application), and may also be used as additional or alternative markers.

In accordance with the eighth aspect of the present invention, without limitation, late stage apoptosis may be characterised by cells which display Annexin (such as Annexin V) binding and also displays propidium iodide (PI) inclusion and/or 7-aminoactinomycin D (7AAD) inclusion, for example as determined using flow cytometry. Other characterising features of late-stage apoptosis are well known in the art (examples of which are discussed elsewhere within this application), and may also be used as additional or alternative markers.

In accordance with the eighth aspect of the present invention, the recombinant yeast-derived serum albumin preparation may be mixed with the cells, in an amount suitable to provide a concentration of the recombinant yeast-derived serum albumin protein that is greater than about 0.01 % (w/v) and less than 10% (w/v), less than about 9% (w/v), less than about 8% (w/v), less than about 7% (w/v) or less than about 6% (w/v), such as at a concentration of from about 0.1 % (w/v) to about 5% (w/v), preferably at about 1% (w/v), about 2% (w/v), about 3 (w/v) or about 4% (w/v). Concentrations of about 2% (w/v) ± 1.5, 1.4, 1.3, 1.2, 1.1, 1.0, 0.9, 0.8, 0.7, 0.6, 0.5, 0.4, 0.3, 0.2 or 0.1 % (w/v), when mixed with the stem cells; or 5% (w/v) ± 1.5, 1.4, 1.3, 1.2, 1.1, 1.0, 0.9, 0.8, 0.7, 0.6, 0.5, 0.4, 0.3, 0.2 or 0.1 % (w/v), when mixed with the cells, may be preferred.

Optionally, in accordance with the eighth aspect of the present invention, the cells may be stored in the medium comprising the recombinant yeast-derived serum albumin preparation at a temperature of 2-8°C. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C. The storage may be for a time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days). The prevention, delay, or reduction, in the switching of cells from early stage apoptosis to late stage apoptosis may be observable during the storage period.

Accordingly, it may be preferred that the cells are stored in the medium comprising the recombinant yeast-derived serum albumin preparation for a period of time greater than 24 hours (for example, at least, or about, 25, 26, 27, 28, 29, 30, 31, 32, 33, 34, 35, 36, 37, 38, 39, 40, 41, 42, 43, 44, 45, 46, 47), such as up to about 48 hours, for example up to about 72 hours (for example, at least, or about, 49, 50, 51, 52, 53, 54, 55, 56, 57, 58, 59, 60, 61, 62, 63, 64, 65, 66, 67, 68, 69, 70, 71 or 72 hours), or more (such as up to 3, 4, 5, 6 or 7 days). Optionally, cells are stored at a temperature of 2-8°C for a period of time greater than 24 hours, such as up to about 48 hours, for example up to about 72 hours, or more, and the percentage of cells which are at the early stage of apoptosis at the end of the storage period is greater than 5%, 10%, 15%, 20%, 25%, 30%, 35%, 40%, 45%, 50%, 55%, 60%, 70%, 75%, 80%, 85%, 86%, 87%, 88%, 89%, 90%, 91%, 92%, 93%, 94%, 95% or more, such as about 60%, 70%, 75%, 80%, 85%, 86%, 87%, 88%, 89%, 90%, 91%, 92%, 93%, 94%, 95% or more. Optionally, the storage temperature is about 2°C, 3°C, 4°C, 5°C, 6°C, 7°C or 8 °C. The term "about" as used in this context, can include the meaning of ± 0.9, 0.8, 0.7, 0.6, 0.5, 0.4, 0.3, 0.2, or 0.1 °C.

Typically, the recombinant yeast-derived serum albumin protein that is used in the medium according to the eighth aspect of the present invention exhibits one or more of the following properties:

(a) less than 0.5% (w/w) binds to Concanavalin A, preferably less than 0.4%, 0.3%, 0.2% or 0.15%; and/or
(b) a glycation level of less than 0.6 moles hexose / mole of protein, and preferably less than 0.10, 0.075 or 0.05 moles hexose / mole of protein.

Typically, the recombinant yeast-derived serum albumin protein that is used in the medium according to the eighth aspect of the present invention:

(a) is at least about 95%, 96%, 97%, 98%, more preferably at least about 99.5% monomeric and dimeric, preferably essentially 100% monomeric and dimeric;
(b) is at least about 93%, 94%, 95%, 96% or 97% monomeric; and/or
(c) has an albumin polymer content of not greater, and preferably less, than about 1.0% (w/w), 0.1 % (w/w) or 0.01% (w/w). As used in this context, the term "polymer" as applied to recombinant yeast-derived serum albumin protein is distinct from monomeric and dimeric forms.

Typically, according to the eighth aspect of the present invention:

(a) the recombinant yeast-derived serum albumin preparation comprises, consists essentially of, or consists of, yeast-derived serum albumin protein, cations (such as sodium, potassium, calcium, magnesium, ammonium, preferably sodium) and balancing anions (such as chloride, phosphate, sulfate, citrate or acetate, preferably chloride or phosphate), water, and optionally octanoate and polysorbate 80;

(b) the recombinant yeast-derived serum albumin preparation and/or medium comprising the recombinant yeast-derived serum albumin preparation and one or more other components, optionally including the cells, comprises octanoate at less than 35mM, 32.5mM, 30 mM, 28 mM, 26 mM, 24 mM, 22 mM, 20 mM, 18 mM, 16 mM, 15 mM, 14 mM, 12 mM, 10 mM, 8 mM, 6 mM, 5 mM, 4 mM, 3 mM, 2 mM, 1 mM, 0.5 mM, 0.4 mM, 0.3 mM, 0.2 mM, 0.1 mM, 0.01 mM, 0.001 mM, is substantially free of octanoate, or is free of octanoate;

(c) the recombinant yeast-derived serum albumin preparation and/or medium comprising the recombinant yeast-derived serum albumin preparation and one or more other components, optionally including the cells, has an overall fatty acid content less than or equal to 35mM, 32.5mM, 30 mM, 28 mM, 26 mM, 24 mM, 22 mM, 20 mM, 15 mM, 10 mM, 5 mM, 4 mM, 3 mM, 2 mM, 1 mM, is substantially free of fatty acids, or is free of fatty acids;

(d) the recombinant yeast-derived serum albumin preparation and/or medium comprising the recombinant yeast-derived serum albumin preparation and one or more other components, optionally including the cells, comprises detergent, such as polysorbate (preferably polysorbate 80) at a concentration less than 200 $mg.L^{-1}$, 150 $mg.L^{-1}$, 100 $mg.L^{-1}$, 90 $mg.L^{-1}$, 80 $mg.L^{-1}$, 70 $mg.L^{-1}$, 60 $mg.L^{-1}$, 50 $mg.L^{-1}$, 40 $mg.L^{-1}$, 30 $mg.L^{-1}$, 20 $mg.L^{-1}$, 15 $mg.L^{-1}$, 10 $mg.L^{-1}$, 5 $mg.L^{-1}$, 4 $mg.L^{-1}$, 3 $mg.L^{-1}$, 2 $mg.L^{-1}$, 1 $mg.L^{-1}$, 0.5 $mg.L^{-1}$, 0.1 $mg.L^{-1}$, 0.01 $mg.L^{-1}$, 0.001 $mg.L^{-1}$, is substantially free of the detergent, or is free of the detergent;

(e) the recombinant yeast-derived serum albumin preparation and/or medium comprising the recombinant yeast-derived serum albumin preparation and one or more other components, optionally including the cells, comprises total free amino acid level and/or N-acetyl tryptophan levels less than 30 mM, 28 mM, 26 mM, 24 mM, 22 mM, 20 mM, 15 mM, 10 mM, 5 mM, 4 mM, 3 mM, 2 mM, 1 mM, 0.5 mM, 0.1 mM, 0.01 mM, 0.005 mM, 0.001 mM, is substantially free of free amino acids and/or N-acetyl tryptophan in particular, or is free of free amino acids and/or of N-acetyl tryptophan in particular;

(f) the recombinant yeast-derived serum albumin preparation and/or medium comprising the recombinant yeast-derived serum albumin preparation and one or more other components, optionally including the cells, is substantially free of, or completely free of, all of octanoate, free amino acids and/or N-acetyl tryptophan in particular, and detergent (such as polysorbate 80);

(g) the recombinant yeast-derived serum albumin protein preparation is a preparation selected from: Recombumin® Prime, or a preparation that is similar thereto; Recombumin® Alpha, or a preparation that is similar thereto; or AlbIX®, or a preparation that is similar thereto;

(h) the recombinant yeast-derived serum albumin protein preparation and/or medium comprising the recombinant yeast-derived serum albumin preparation and one or more other components, optionally including the cells, is free of one or more, such all, components selected from: haem, prekallikrein activator, pyrogens, hepatitis C and/or human viruses and/or has an aluminium concentration of less than 200 $\mu g.L^{-1}$, such as less than 180 $\mu g.L^{-1}$, 160 $\mu g.L^{-1}$, 140 $\mu g.L^{-1}$, 120 $\mu g.L^{-1}$, 100 $\mu g.L^{-1}$, 90 $\mu g.L^{-1}$, 80 $\mu g.L^{-1}$, 70 $\mu g.L^{-1}$, 60 $\mu g.L^{-1}$, 50 $\mu g.L^{-1}$, or 40 $\mu g.L^{-1}$, more typically within the range of about 10 $\mu g.L^{-1}$ to about 30 $\mu g.L^{-1}$;

(i) the recombinant yeast-derived serum albumin protein present in the medium possesses an intact or substantially intact N-terminal sequence;

(j) the recombinant yeast-derived serum albumin preparation present in the medium comprises albumin protein that has a free thiol group content that is greater than 62%, such as at least 69%, at least 70%, at least 75%, at least 80%, at least 85%, at least 90%, at least 95%, about 96%, about 97%;

(k) the recombinant yeast-derived serum albumin preparation present in the medium comprises albumin protein that, when tested by size exclusion chromatography (SEC), displays an SEC profile excluding peaks with a peak retention time under 14 minutes and over 19 minutes, and more preferably excludes peaks with a peak retention time under 14 or 15 minutes and over 18 minutes;

(l) the recombinant yeast-derived serum albumin preparation present in the medium comprises albumin protein that, when tested by reversed phase high performance liquid chromatography (RP-HPLC), displays a single major peak, corresponding to albumin in the native monomeric form;

(m) the recombinant yeast-derived serum albumin preparation present in the medium comprise albumin protein that, when tested by mass spectrometry, is a product that displays fewer than 13, 12, 11, 10, 9, 8, 7, 6, such as about 1 to 11, 1 to 8, 1 to 5, 1 to 4, 1 to 3, 1 to 2, 1 or less than 1 hexose modified lysine and/or arginine residues per protein; and/or

(n) the recombinant yeast-derived serum albumin preparation present in the medium comprise albumin protein that is not glycated with plant-specific sugars, such as $\alpha$-1,3-fucose and/or $\beta$-1,2-xylose.

**Description of the Figures:**

**Fig 1** shows mass spectrometry analysis of commercial albumin products as reported in Example 5: (a) Recombumin® Prime and Recombumin® Alpha; (b) Commercial albumin 1 (plant-derived); (c) Commercial albumin 2 (plant-derived); (d) Commercial albumin 3 (derived from the yeast, *Pichia*).

**Fig 2** shows comparative gel electrophoresis data recorded from commercial albumin products as described in Example 5: (a) Recombumin® Prime; (b) Recombumin® Alpha; (c) Commercial albumin 1; (d) Commercial albumin 3; (e) Commercial albumin 2.

**Fig 3** shows a comparison of the pigmentation of the recombinant albumins as described in Example 5.

**Fig 4** shows a scheme of the experimental phase followed in the study described in Example 1.

**Fig 5** shows the configuration used in stability tests at 2-8°C, in the study described in Example 1.

**Fig 6** shows the results of the apoptotic assay in post-thawing stability at 2-8°C, based on Annexin V binding (horizontal axis) and PI inclusion (vertical axis).

**Fig 7** shows % surface markers for identity of hMSC at pre-freezing and post-thawing times, comparing Albutein® (RI) or AlbIX® (TI) cryopreservation solutions, as discussed in Example 2.

**Fig 8** shows **(A)** Flow cytometric analysis. % Viability along the stability time points for Albutein® (RI) or AlbIX® (TI) additives in cell cultures expanded with hSERB (dashed line indicates 70% viability specification limit). For 72h the % of reduction considering time 0 as reference is showed. Results of ANOVA Tukey Multiple comparison test are shown. **(B)** Flow cytometric analysis. % Viability along the stability time points for Albutein® (RI) or AlbIX® (TI) additives in cell cultures expanded with PL (dashed line indicates 70% viability specification limit). For 72h the % of reduction considering time 0 as reference is shown. Results of ANOVA Tukey Multiple comparison test are shown. **(C)** and **(D)** Flow cytometry analysis. %Viability reduction with respect to time 0, for Albutein® (RI) and AlbIX® (TI) cryopreservation conditions, and for hSERB **(C)**, and PL **(D)**, expansion strategies. ANOVA non parametric Sidak's multiple comparison test was used for statistical analysis.

**Fig 9** shows shows a fatty acid profile of an albumin formulation of a recombinant yeast-derived albumin preparation according to the third embodiment of the invention.

**Fig 10** shows a metal ion profile, by ICP-OES, of an albumin formulation of a recombinant yeast-derived albumin preparation according to the third embodiment of the invention.

**Fig 11** shows % surface markers for identity of hMSC at different time points of the stability assessment comparing Albutein® (RI) or AlbIX® (TI) cryopreservation solutions for cell cultures expanded with hSERB culture media.

**Fig 12** shows early and late stage apoptotic states of hMSC cells at different time points of the stability assessment comparing Albutein® (control) or AlbIX® cryopreservation solutions.

**Fig 13** shows the protective effect of yeast-derived recombinant human serum albumin, immediately post-thawing, when used either in the cryopreservation medium, the storage medium, or both. (**A**) shows the percentage of viability for all the items (RI, TI1, TI2, TI3; n=3 per item) obtained with Flow Cytometry. The dashed line corresponds to the routine acceptance criteria for viability after thawing. Comparison was performed with the one-way ANOVA, Dunnetts multiple test. (**B**) shows the percentage of viability for all the items (RI, TI1, TI2, TI3; n=3 per item) obtained with NucleoCounter. The dashed line corresponds to the routine acceptance criteria for viability after thawing. Comparison was performed with the one-way ANOVA, Dunnetts multiple test.

**Fig 14** shows the protective effect of yeast-derived recombinant human serum albumin, from immediately post-thawing to 72 hours post-thawing, when used either in the cryopreservation medium, the storage medium, or both. (**A**) shows the percentage of viability obtained by flow cytometry. (**B**) shows the percentage of viability obtained by the nucleocounter. Both representations show the viability along the stability study associated with the standard deviation of all the items (n=3 per item). In both of Figs 14A and 14B, ● is the Reference Item, ■ is Test Item 1, ▲ is Test Item 1, and ▼ is Test Item 3.

**Fig 15** shows a comparison of RI and TI2 samples, assessed for post-thaw viability (%) when stored at 2-8°C. (**A**) shows the results as determined by flow cytometry and (**B**) shows the results as determined by nucleocounter.

**Fig 16** shows a comparison of RI and TI2 samples, assessed for reduction in post-thaw viability (%) over time, when stored at 2-8°C, and using normalized data, in which the time viability at point 0h for each sample was considered as the time point with 100% of viability and viability reduction from this time point was evaluated with respect to this value. (**A**) shows the results as determined by flow cytometry and (**B**) shows the results as determined by nucleocounter.

**Fig 17** shows a comparison of TI1 and TI3 samples, assessed for post-thaw viability (%) when stored at 2-8°C. (**A**) shows the results as determined by flow cytometry and (**B**) shows the results as determined by nucleocounter.

**Fig 18** shows a comparison of RI and TI3 samples, assessed for post-thaw viability (%) when stored at 2-8°C. (**A**) shows the results as determined by flow cytometry and (**B**) shows the results as determined by nucleocounter.

**Fig 19** shows a representation of the percentage of early apoptotic cells and late apoptotic cells. (**A**) shows the comparison of the Reference Item (RI) and the Test Item 2 (TI2), (**B**) shows the comparison of the Test Item 1 (TI1) and the Test Item 3 (TI3), and (**C**) shows the comparison of the Reference Item (RI) and the Test Item 3 (TI3), at each time point of the stability study.

## Detailed Description of the Invention

### *Definitions*

Freezing: The term "freezing" as used in the present application in the context of freezing cell preparations, includes exposing the cells to a temperature that is low enough to generate ice crystals or render the cell preparation as an entirely frozen solid form, for example, in a form that is below 0°C. The term "freezing" preferably refers to cryopreservation.

Cryopreservation: Cryopreservation is a method for keeping biological material (cells, tissues and microorganisms) in good condition, by freezing at very low temperatures, generally between -80°C and -196°C. In this way, the vital functions are reduced and they can be maintained in a state of suspended animation for a long time.

As used herein, the term "cryopreservation" refers to stably maintaining cells for a long period of time via freezing. Generally, when cells are cultured, a mutation occurs in a ratio of one to ten thousands, and when cells go through a long-term subculture, cell populations change and become different from the original populations. In severe cases, cells may lose their own particular functions by subculture. Further, cells may be infected with mycoplasma, etc. during subculture. Because of such problems, cell cryopreservation is performed to freeze and preserve cells before losing their intrinsic characteristics, and to use them again when needed. In particular, if stem cells are used for therapy, it is necessary to be able to immediately use healthy stem cells when needed. Thus, effective cryopreservation is considered especially important for stem cells.

Freezing stem cells that are treated with a cryopreservation media of the present invention may be performed by any conventional method of freezing stem cells known in the art, and examples thereof may include a vitrification method and a slow freezing method, but are not limited thereto. The vitrification method is performed, for example, by constantly decreasing a temperature at a rate (e.g. of 1°C. per 1 minute) using an apparatus such as a controlled-rate freezer (CRF). Preferably, when the temperature reaches -80° C., cells are immediately stored in a nitrogen tank. The slow freezing method may be performed by placing a vial containing a cryopreservation media containing cells in a freezer container box containing isopropyl alcohol, and by constantly decreasing the temperature over a specific period of time (e.g. for 12 hours to 24 hours) in an ultra-low freezer at -70° C. or lower, without being limited thereto. Further, the frozen cells can be stored in a liquid nitrogen tank, and used again when needed.

Suitable exemplary methods are also described in the examples.

Cryopreservation Solution: Freezing of cellular products is carried out using one or more cryoprotectants in a cryopreservation solution. The use of said solution is generally based on the preservation of cellular viability during the freezing process by cellular dehydration in order to prevent the formation of crystals of frozen water in the intracellular compartments of cells.

Cryopreservant: As used herein, the term "cryopreservant" refers to a substance that is used to minimize cell damage caused by freezing and thawing processes which are inevitably accompanied by ice crystal formation and ionic and osmotic imbalance when cells, tissues, or organs are preserved at an ultra-low temperature of -80 °C to -200°C.

For the purposes of the present invention, the "cryopreservant" is not recombinant yeast-derived serum albumin' or any other form of albumin protein. Otherwise, the cryoprotectant is not limited to a certain substance, as long as it is able to reduce cell damage during cryopreservation.

Without limitation, a cryopreservant present in a cryopreservation solution may include: dimethyl sulphoxide (DMSO), glycerol, Polyethylene glycol (PEG), ethylene glycol (EG), polyvinylpyrrolidone (PVP), and Trehalose. DMSO may be particularly preferred. The cryoprotectant may be present in the cryopreservation medium, when mixed with the stem cells, at a concentration suitable to provide a cryopreservative effect. In the case of DMSO, this may be about 10% (w/v) ± 5%, 4%, 3%, 2%, or 1%. The skilled person can readily determine a suitable amount of cryopreservant using routine testing.

Stem cells: As used herein, the term "stem cell" refers to an undifferentiated cell having self-renewal and differentiation capacity. Stem cells include subpopulations of totipotent stem cells, pluripotent stem cells, multipotent stem cells, and unipotent stem cells according to their differentiation capacity. Totipotent stem cells refer to cells that can differentiate into any cell in an organism including embryonic tissue and placental cells. Pluripotent stem cells refer to cells that have potency to differentiate into all tissues or cells that constitute a living organism. Multipotent stem cells refer to cells that do not have potency to differentiate into all kinds but into plural kinds of tissues or cells. Unipotent stem cells refer to cells that have potency to differentiate into a particular tissue or cell.

Pluripotent stem cells may include embryonic stem cells (ES cells), embryonic germ cells (EG cells), induced pluripotent stem cells (iPS cells), etc.

Multipotent stem cells may include adult stem cells such as mesenchymal stem cells (derived from fat, bone marrow, umbilical cord blood, or umbilical cord, etc.), hematopoietic stem cells (derived from bone marrow or peripheral blood), neural stem cells, germ stem cells, etc.

Unipotent stem cells may include committed stem cells for hepatocytes, which are normally quiescent with low self-renewal capacity, but vigorously differentiate into hepatocytes under certain conditions. In an embodiment of the present invention, bone marrow mesenchymal stem cells and umbilical cord stem cells were used to examine that the composition of the present invention may be used for cryopreservation of stem cells with safety and stability.

In accordance with the present invention, the term "stem cells" typically does not include primary cells. As used herein, the term "primary cell" refers to a cell that is isolated from a tissue of an individual without any genetic manipulation, etc., which represents functions of an organ/tissue of a living organism. Primary cells are isolated from skin or vascular endothelium, bone marrow, fat, cartilage, etc., and used for studying functions of corresponding tissues and cells, or as therapeutic agents for restoring lost tissues.

The origins of the stem cells and primary cells are not particularly limited, as long as cells may be stably cryopreserved by the composition of the present invention. Examples thereof may include cells derived from human, monkey, pig, horse, cow, sheep, dog, cat, mouse, or rabbit. The stem cells or primary cells are preferably human stem cells or primary cells, but are not limited thereto.

Optionally, the methods and uses of the present invention (in particular those methods and uses in which the stem cells used are human embryonic stem cells (hESCs)) exclude the uses of human embryos for any purpose, including for industrial or commercial purposes. As such, it may be preferred that any hESC used in accordance with the present invention is not obtained directly or indirectly through the destruction of a human embryo. To put it another way, it may be preferred that any hESC used in accordance with the present invention is obtained via means that do not require the prior destruction of human embryos or their use as base material, whatever the stage at which that takes place. For example, it may be preferred that any hESC for use in the present invention is derived from a parthenote and/or other cell or collection of cells which does not have the inherent capacity of developing into a human being.

Apoptosis: Apoptosis is a process of programmed cell death that occurs in multicellular organisms. Biochemical events lead to characteristic cell changes (morphology) and death. These changes can include one or more characteristics selected from blebbing, cell shrinkage, nuclear fragmentation, chromatin condensation, chromosomal DNA fragmentation, and global mRNA decay. Apoptosis is distinct to necrosis, the latter of which is a form of traumatic cell death that results from acute cellular injury. In contrast, apoptosis is a highly regulated and controlled process. Unlike necrosis, apoptosis produces cell fragments called apoptotic bodies that phagocytic cells are able to engulf and quickly remove before the contents of the cell can spill out onto surrounding cells and cause damage.

Methods are well known in the art for distinguishing apoptotic and necrotic cells. This may include, for example, analysis of morphology by time-lapse microscopy, flow fluorocytometry, and transmission electron microscopy. There are also various biochemical techniques for analysis of cell surface markers (phosphatidylserine exposure versus cell permeability by flow fluorocytometry), cellular markers such as DNA fragmentation (flow fluorocytometry), caspase activation, Bid cleavage, and cytochrome c release (Western blotting). Primary and secondary necrotic cells can be distinguished by analysis of supernatant for caspases, HMGB1, and release of cytokeratin 18. However, no distinct surface or biochemical markers of necrotic cell death have been identified yet, and only negative markers are available. These include absence of apoptotic markers (caspase activation, cytochrome c release, and oligonucleosomal DNA fragmentation) and differential kinetics of cell death markers (phosphatidylserine exposure and cell membrane permeabilization). A selection of techniques that can be used to distinguish apoptosis from necroptotic cells are well known.

Annexins are a family of calcium-dependent phospholipid-binding proteins, which bind to phosphatidylserine (PS) to identify apoptotic cells. In healthy cells, PS is predominantly located along the cytosolic side of the plasma membrane. Upon initiation of apoptosis, PS loses its asymmetric distribution in the phospholipid bilayer and translocates to the extracellular membrane, which is detectable with fluorescently labelled Annexin V.

- In early stages of apoptosis, the plasma membrane excludes viability dyes such as propidium iodide (PI) and 7AAD, therefore cells which display only Annexin V staining (PI and/or 7AAD negative) are in early stages of apoptosis. Cells in an early apoptotic phase are not committed to apoptosis, and can revert, under favourable conditions, into a rescued form that can be used as viable cells. As such, cells in the early stage of apoptosis are considered, for the purposes of the present application, to be "viable".

- During late-stage apoptosis, loss of cell membrane integrity allows Annexin V binding to cytosolic PS, as well as cell uptake of PI and/or 7AAD. Cells in the late stage of apoptosis are not considered, for the purposes of the present application, to be "viable".

Annexin V staining, paired with 7AAD or PI is widely used to identify apoptotic stages, for example by flow cytometry.

An additional measure of early-stage apoptosis can include, for example, determination of mitochondrial permeability, such as using the Mitoscreen kit as described in Milian et al, 2015, J. Biotech., 209: 58-67 (see in particular, section 2.2 and Fig 5A of Milian *et al*). Further, caspase 3 activity assay may be assayed, for example using the methodology of Tintó et al, 2002, J. Biotech., 95: 205-214.

Tintó *et al*, *supra,* also describes additional useful assays that may be applied to characterise late-stage apoptosis, including DNA ladder analysis (see Figures 3B, E, H of Tintó *et al*), and confocal imaging, for example as described in Figure 1C of Tintó *et al*.

Recombinant Yeast-Derived Serum Albumin: The present invention relates to 'recombinant yeast-derived serum albumin' protein and preparations thereof.

That is to say, the serum albumin protein is derived from a yeast culture medium obtained by culturing a yeast transformed with an albumin-encoding nucleotide sequence in a fermentation medium, whereby said yeast expresses the albumin protein and secretes it into the medium. It is preferably manufactured without the use of animal- or human-derived materials. The recombinant yeast-derived serum albumin protein is then recovered therefrom in a form that is substantially purified, in order to produce the recombinant yeast-derived serum albumin preparation.

The yeast is preferably of the genus *Saccharomyces* (eg *Saccharomyces cerevisiae),* the genus *Kluyveromyces* (eg *Kluyveromyces lactis)* or the genus *Pichia* (eg *Pichia pastoris).* Methods for the production of recombinant yeast-derived serum albumin protein are well known in the art, for example in *Kluyveromyces* (Fleer 1991, Bio/technology 9, 968-975), *Pichia* (Kobayashi 1998 Therapeutic Apheresis 2, 257-262) and *Saccharomyces* (Sleep 1990, Bio/technology 8, 42-46)). Most preferably the genus is *Saccharomyces,* and most preferably *Saccharomyces cerevisiae.*

Suitable methods for the production of exemplary recombinant yeast-derived serum albumin preparations, and such preparations *per se,* are described in WO 2000/044772 and/or WO 2013/006675.

EP1329460 (A1), EP1329461 (A1), EP1329462 (A1) and EP1710250 (A1) also describe methods suitable for the production of recombinant yeast-derived serum albumin preparations, and such preparations *per se,* which may be potentially suitable for use in the present invention.

As discussed below, the production of recombinant serum albumin in yeast, and recovery therefrom, provides a recombinant yeast-derived serum albumin preparation that is physically distinct from serum albumin compositions obtained from other sources (e.g. from plasma, or from other recombinant sources such as plants). Those distinctions are numerous, and include physical differences in the structure of the serum albumin protein itself, and in the identity and/or level of the accompanying components with which the albumin protein is co-purified in the preparation. At least in part, that is because, in comparison to serum albumin obtained from other sources, the albumin protein in the recombinant yeast-derived serum albumin the original albumin molecule is relatively devoid of changes and alterations imposed on the protein by the source.

Albumin has been described and characterized from a large number of mammals and birds (*e.g.* albumins listed in WO2010/092135 (particularly Table 1) and PCT/EP11/055577 published as WO 2011/124718 (particularly page 9 and SEQ ID No: 2, 4-19 and 31)).

A recombinant yeast-derived serum albumin preparation for use in the present invention may comprise one or more (several) albumins. Preferably the composition comprises an albumin selected from human albumin (*e.g.* AAA98797 or P02768-1, SEQ ID NO: 2 (mature), SEQ ID NO: 3 (immature)), non-human primate albumin, (such as chimpanzee albumin (*e.g.* predicted sequence XP_517233.2 SEQ ID NO: 4), gorilla albumin or macaque albumin (*e.g.*

NP_001182578, SEQ ID NO: 5), rodent albumin (such as hamster albumin (*e.g.* A6YF56, SEQ ID NO: 6), guinea pig albumin (*e.g.* Q6WDN9-1, SEQ ID NO: 7), mouse albumin (*e.g.* AAH49971 or P07724-1 Version 3, SEQ ID NO: 8, or the mature sequence SEQ ID NO: 19) and rat albumin (*e.g.* AAH85359 or P02770-1 Version 2, SEQ ID NO: 9))), bovine albumin (*e.g.* cow albumin P02769-1, SEQ ID NO: 10), equine albumin such as horse albumin (*e.g.* P35747-1, SEQ ID NO: 11) or donkey albumin (*e.g.* Q5XLE4-1, SEQ ID NO: 12), rabbit albumin (*e.g.* P49065-1 Version 2, SEQ ID NO: 13), goat albumin (*e.g.* ACF10391, SEQ ID NO: 14), sheep albumin (*e.g.* P14639-1, SEQ ID NO: 15), dog albumin (*e.g.* P49822-1, SEQ ID NO: 16), chicken albumin (*e.g.* P19121-1 Version 2, SEQ ID NO: 17) and pig albumin (*e.g.* P08835-1 Version 2, SEQ ID NO: 18). Mature forms of albumin are particularly preferred and the skilled person is able to identify mature forms using publicly available information such as protein databanks and/or by using signal peptide recognition software such as SignalP (*e.g.*, SignalP (Nielsen et al., 1997, Protein Engineering 10: 1-6)). SignalP Version 4.0 is preferred (Petersen *et al* (2011) Nature methods (8): 785-786).

The recombinant yeast-derived serum albumin is preferably a protein having the same and/or very similar tertiary structure as human serum albumin (HSA) or HSA domains and has similar properties of HSA or the relevant domains.

Human albumin as disclosed in SEQ ID NO: 2 or any naturally occurring allele thereof, is the preferred recombinant yeast-derived serum albumin protein for use according to the invention. SEQ ID No: 2 may be encoded by the nucleotide sequence of SEQ ID No: 1.

Particularly preferred forms of recombinant yeast-derived human serum albumin preparations for use in accordance with the present invention include the known commercial presentations of recombinant yeast-derived Recombumin® Alpha (formerly Albucult®), Recombumin® Prime (formerly Recombumin®) and/or AlbIX® (all from Albumedix Ltd.).

Alternatively, the recombinant yeast-derived serum albumin may be a protein having a sequence that has a very similar tertiary structure to HSA or HSA domains and has similar properties of HSA or the relevant domains.

Similar tertiary structures are for example the structures of the albumins from the species mentioned under parent albumin. Some of the major properties of albumin are i) its ability to regulate of plasma volume, ii) a long plasma half-life of around 19 days ± 5 days, iii) ligand-binding, *e.g.* binding of endogenous molecules such as acidic, lipophilic compounds including bilirubin fatty acids, hemin and thyroxine (see also Table 1 of Kragh-Hansen et al, 2002, Biol. Pharm. Bull. 25, 695), iv) binding of small organic compounds with acidic or electronegative features *e.g.* drugs such as warfarin, diazepam, ibuprofen and paclitaxel (see also Table 1 of Kragh-Hansen et al, 2002, Biol. Pharm. Bull. 25, 695). Not all of these properties need to be fulfilled to in order to characterize a protein or fragment as an albumin. If a fragment, for example, does not comprise a domain responsible for binding of certain ligands or organic compounds the variant of such a fragment will not be expected to have these properties either. The term albumin includes variants, and/or derivatives such as fusions and/or conjugations of an albumin or of an albumin variant.

The term "parent" or "parent albumin" means an albumin to which an alteration is made to produce the albumin variants which may be used in the present invention. The parent may be a naturally occurring (wild-type) polypeptide or an allele thereof or a variant thereof such as a variant described in PCT/EP2010/066572 published as WO 2011/051489, or a variant or derivative described in PCT/EP2011/055577 published as WO 2011/124718.

The term "variant" means a polypeptide derived from a parent albumin comprising an alteration, *i.e.*, a substitution, insertion, and/or deletion, at one or more (several) positions. A substitution means a replacement of an amino acid occupying a position with a different amino acid; a deletion means removal of an amino acid occupying a position; and an insertion means adding 1-3 amino acids adjacent to an amino acid occupying a position. The altered polypeptide (variant) can be obtained through human intervention by modification of the polynucleotide sequence encoding the parental albumin.

The recombinant yeast-derived serum albumin protein, particularly the human albumin, may be a variant, or a derivative such as fusion of conjugation of an albumin or of an albumin variant. It is preferred that the albumin has at least 70% identity to HSA (SEQ ID No: 2), more preferably at least 72, 73, 75, 80, 85, 90, 95, 96, 97, 98, 99, 99.5 % identity to HSA. The albumin variant may have one or more point (several) mutations, *e.g.* K573P, K573Y, K573W, K500A compared to a parent albumin such as those provided in the sequence listing, particularly SEQ ID No: 2 (mutations are described in relation to SEQ ID No: 2 and the skilled person can identify equivalent mutations in other albumins by aligning an albumin sequence against SEQ ID No: 2 using the EMBOSS software described herein). For an albumin having about 70 to 80 % identity to SEQ ID No: 2 (such as mouse albumin *e.g.* SEQ ID NO: 19), it is more preferred that the cation is present from at least 250 mM.

The variant albumin is preferably at least 70%, preferably at least 75%, more preferably at least 80%, more preferably at least 85%, even more preferably at least 90%, most preferably at least 95%, at least 96%, at least 97%, at least 98%, at least 99%, or 100% identical to SEQ ID NO: 2 and maintains at least one of the major properties of the parent albumin or a similar tertiary structure as HSA.

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

The variant may possess altered binding affinity to FcRn and/or an altered rate of transcytosis across endothelia, epithelia and/or mesothelia mono cell-layer when compared to the parent albumin. The variant polypeptide sequence is preferably one which is not found in nature. A variant includes a fragment, *e.g.* comprising or consisting of at least 100, 150, 200, 250, 300, 350, 450, 500, 550 contiguous amino acids of an albumin.

The term "wild-type" (WT) albumin means an albumin having the same amino acid sequence as the albumins naturally found in an animal or in a human being. SEQ ID NO: 2 is an example of a wild-type albumin from *Homo sapiens.*

In accordance with one embodiment, the recombinant yeast-derived serum albumin preparation (most preferably derived by recombinant DNA expression in *Saccharomyces cerevisiae*) comprises recombinant yeast-derived serum albumin which exhibits one or more of the following properties:

(1) less than 0.5% (w/w) binds to Concanavalin A, preferably less than 0.4%, 0.3%, 0.2% or 0.15%; and/or
(2) a glycation level of less than 0.6 moles hexose / mole of protein, and preferably less than 0.10, 0.075 or 0.05 moles hexose / mole of protein.

A suitable Concanavalin A assay is described, for example, in WO 2000/044772.

The recombinant yeast-derived serum albumin preparation (most preferably derived by recombinant DNA expression in *Saccharomyces cerevisiae)* may be at least 95%, 96%, 97%, 98%, more preferably at least 99.5% monomeric and dimeric, preferably essentially 100% monomeric and dimeric. Up to 0.5%, preferably 0.2% trimer is tolerable but larger forms of albumin are generally absent.

In certain preferred embodiments, the recombinant yeast-derived serum albumin preparation may be:

i. the commercially-available Recombumin® Prime (formerly Recombumin®) product from Albumedix Ltd., or a preparation that is similar thereto, as described below in a first particularly preferred embodiment;
ii. the commercially-available Recombumin® Alpha (formerly Albucult®) product from Albumedix Ltd., or a preparation that is similar thereto, as described below in a second particularly preferred embodiment; or
iii. most preferably, the commercially-available AlbIX® product from Albumedix Ltd., or a preparation that is similar thereto, as described below in a second particularly preferred embodiment.

Accordingly, in a first particularly preferred embodiment, the recombinant yeast-derived serum albumin preparation (most preferably derived by recombinant DNA expression in *Saccharomyces cerevisiae)* is the commercially-available Recombumin® Prime (formerly Recombumin®) product from Albumedix Ltd., or a preparation that is similar thereto that may be characterised by one or more of the following characteristics.

i. It may have a nickel ion level of less than 100ng, based on one gram of albumin;
ii. it may have a glycation level of less than 0.6, preferably less than 0.10, 0.075 or 0.05 moles hexose/mole protein as measured in the Amadori product assay;
iii. it may have an intact, i.e. homogeneous, C-terminus;
iv. it may have a content of conA-binding albumin of less than 0.5% (w/w), preferably less than 0.4%, 0.3%, 0.2% or 0.15%;
v. it may have a free thiol content of at least 0.85 mole SH/mole protein;
vi. it may contain substantially no C18 or C20 fatty acids; and/or
vii. at least 99%, preferably at least 99.9%, by weight of the protein in the recombinant yeast-derived serum albumin preparation may be the recombinant yeast-derived serum albumin protein.

Additionally, or alternatively, in accordance with the first particularly preferred embodiment, the recombinant yeast-derived serum albumin preparation (most preferably derived by recombinant DNA expression in *Saccharomyces cerevisiae)* is the commercially-available Recombumin® Prime (formerly Recombumin®) product from Albumedix Ltd., or a preparation that is similar thereto, and may comprise, consist essentially of, or consist of the following components:

i. Yeast-derived recombinant human albumin as the "Active" ingredient. For example, the yeast derived yeast-derived recombinant human albumin may be present in the preparation at a concentration of about 10 to 400 g/L, such as about 20, 30, 40 50, 60, 70, 80, 90, 100, 110, 120, 130, 140, 150, 160, 170, 180, 190 or 200 g/L.
ii. Sodium (for example, added in the form of NaCl) in order to adjust the tonicity. The sodium may be present, for example, at a concentration of about 145 mM, ± 100 mM, 80 mM, 60 mM, 40 mM, 20 mM, 10 mM, or 5 mM.

iii. Octanoate as a stabilizing agent. The octanoate may be present, for example, at a concentration of about 1.6 mM per 10 g/L concentration of yeast-derived recombinant human albumin, e.g. about 32 mM for a 200 g/L preparation. In this context, the term "about" is intended to mean ± 1.0, 0.8, 0.6, 0.4, or 0.2 mM of the stated value.

iv. Polysorbate 80 as a stabilizing agent, for example at a concentration of about 15 mg/L for a recombinant human albumin concentration of 200 g/L, or adjusted proportionally for other concentrations of recombinant human albumin. In this context, the term "about" is intended to mean around 0.1, 0.2, 0.3, 0.4, 0.5, 0.75, 1, 1.5, 2, 2.5, 3, 3.5, 4, 5, 6, 7, 8, 9, or 10 mg/L polysorbate 80 is added per 10 g/L of concentration of recombinant human albumin.

v. Water as a diluent/vehicle.

Additionally, or alternatively, in accordance with the first preferred embodiment, the recombinant yeast-derived serum albumin preparation (most preferably derived by recombinant DNA expression in *Saccharomyces cerevisiae)* is the commercially-available Recombumin® Prime (formerly Recombumin®) product from Albumedix Ltd., or a preparation that is similar thereto that, and may be characterised by one or more (such as all) of the following characteristics:

(i) A mass analysis of the recombinant human albumin showing a theoretical mass ± 20 Da (66418 to 66458);
(ii) Endotoxin content not greater (preferably less) than 0.50 EU/ml, for example as determined by the LAL assay;
(iii) Sterility that meets requirements of the USP<71> test;
(iv) pH of 6.7-7.3, for example as determined by a standard quality control method;
(v) a protein purity of at least (preferably greater than) 99.0% (w/w), for example as determined by native PAGE;
(vi) an albumin polymer content of not greater (preferably less) than 1.0% (w/w), for example as determined by GP HPLC;
(vii) Protein 19.0-21.0% Kjeldahl;
(viii) a sodium content of 130-160 mM, for example as determined by atomic absorption spectroscopy;
(ix) an appearance, in a glass vial prior to addition to the cryopreservation and/or storage medium, that is free from defects, containing a slightly viscous, clear straw to amber coloured solution practically free from visible contamination, for example as determined by visual inspection;
(x) host cell protein content of not greater (preferably less) than 0.15 μg/g albumin protein, for example as determined by ELISA;
(xi) Con A-binding species of recombinant yeast-derived serum albumin of not greater (preferably less) than 0.30% (w/w) protein, for example as determined by Con A Chromatography;
(xii) Nickel content of not greater (preferably less) than 0.5 μg/g protein, for example as determined by atomic absorption spectroscopy;
(xiii) Potassium content of not greater (preferably less) than 0.01 mmol/g protein, for example as determined by flame atomic absorption spectroscopy;
(xiv) Delta Blue Matrix (DBA; as described in WO 96/37515) leachates: Dye Fragment (DAAS) of not greater (preferably less) than 0.1 μg/g protein, Dye base of not greater (preferably less) than 0.1 μg/g protein, and Dye + spacer of not greater (preferably less) than 0.4 μg/g protein, for example each as determined by RP-HPLC;
(xv) aminophenylboronate (PBA) leachate of not greater (preferably less) than 0.18 μg/g protein RP-HPLC; (xvi) Octanoate content in the range of from about 28.8 mM to about 35.2 mM, for example as determined by gas chromatography; and/or
(xvi) Polysorbate 80 content in the range of from about 10 mg/L to about 20 mg/L, for example as determined by SEC-HPLC.

In accordance with a second particularly preferred embodiment, the recombinant yeast-derived serum albumin preparation (most preferably derived by recombinant DNA expression in *Saccharomyces cerevisiae)* is the commercially-available Recombumin® Alpha (formerly Albucult®) product from Albumedix Ltd., or a preparation that is similar thereto, and may comprise, consist essentially of, or consist of the following components:

i. Yeast-derived recombinant human albumin as the "Active" ingredient. For example, the yeast derived yeast-derived recombinant human albumin may be present in the preparation at a concentration of about 10 to 400 g/L, such as about 20, 30, 40 50, 60, 70, 80, 90, 100, 110, 120, 130, 140, 150, 160, 170, 180, 190 or 200 g/L.

ii. Sodium (for example, added in the form of NaCl) in order to adjust the tonicity. The sodium may be present, for example, at a concentration of about 145 mM, ± 100 mM, 80 mM, 60 mM, 40 mM, 20 mM, 10 mM, or 5 mM.

iii. Octanoate as a stabilizing agent. The octanoate may be present, for example, at a concentration of about 0.8 mM per 10 g/L concentration of yeast-derived recombinant human albumin, e.g. about 8 mM for a 100 g/L preparation and about 16 mM for a 200 g/L preparation. In this context, the term "about" is intended to mean ± 0.6, 0.5, 0.4, 0.3, 0.2 or 0.1 mM of the stated value.

iv. Polysorbate 80 as a stabilizing agent, for example at a concentration of about 50 mg/L for a recombinant human albumin concentration of 100 g/L, or adjusted proportionally for other concentrations of recombinant human albumin. In this context, the term "about" is intended to mean around 0.1, 0.2, 0.3, 0.4, 0.5, 0.75, 1, 1.5, 2, 2.5, 3, 3.5, 4, 5, 6, 7, 8, 9, or 10 mg/L polysorbate 80 is added per 10 g/L of concentration of recombinant human albumin.
v. Water as a diluent/vehicle.

Additionally, or alternatively, accordance with the second preferred embodiment, the recombinant yeast-derived serum albumin preparation (most preferably derived by recombinant DNA expression in *Saccharomyces cerevisiae)* is the commercially-available Recombumin® Alpha (formerly Albucult®) product from Albumedix Ltd., or a preparation that is similar thereto, and may be characterised by one or more (such as all) of the following characteristics:

(i) A mass analysis of the recombinant human albumin showing a theoretical mass ± 20 Da (66418 to 66458);
(ii) Endotoxin content not greater (preferably less) than 0.50 EU/ml, for example as determined by the LAL assay;
(iii) Sterility that meets requirements of the USP<71> test;
(iv) pH of 6.4-7.4, for example as determined by a standard quality control method;
(v) a protein purity of at least (preferably greater than) 99.0% (w/w), for example as determined by native PAGE;
(vi) an albumin polymer content of not greater (preferably less) than 1.0% (w/w), for example as determined by GP HPLC;
(vii) a sodium content of 120-160 mM, for example as determined by atomic absorption spectroscopy;
(viii) an appearance, in a glass vial prior to addition to the cryopreservation and/or storage medium, that is free from defects, containing a slightly viscous, clear straw to amber coloured solution practically free from visible contamination, for example as determined by visual inspection;
(ix) host cell protein content of not greater (preferably less) than less than about 200 ng or 150 ng host cell protein per gram of albumin as measured by ELISA e.g. YA53M less than or equal to about 15 ng/g albumin and/or YA53H less than or equal to about 150 ng/g albumin; and/or
(x) Con A-binding species of recombinant yeast-derived serum albumin of not greater (preferably less) than 0.30% (w/w) protein, for example as determined by Con A Chromatography.

In accordance with a third embodiment, the recombinant yeast-derived serum albumin preparation (most preferably derived by recombinant DNA expression in *Saccharomyces cerevisiae*) is the commercially-available AlbIX® product from Albumedix Ltd., or a preparation that is similar thereto, which may comprise, consist essentially of, or consist of recombinant yeast-derived serum albumin protein, a solvent, at least 175 mM cations, having a pH from about 5.0 to about 9.0 and wherein the preparation comprises equal to or less than 30 mM octanoate.

It is preferred that the preparation of the third embodiment contains anions to balance the cations.

The solvent in the preparation of the third embodiment may be an inorganic solvent such as water or an inorganic buffer such as a phosphate buffer such as sodium phosphate, potassium phosphate, or an organic buffer such as sodium acetate or sodium citrate. The buffer may stabilize pH. Sodium phosphate (*e.g.* $NaH_2PO_4$) is a preferred pH buffer, such as pH 5.0, 5.5., 6.0, 6.5, 7.0, 7.5, 8.0.

The preparation of the third embodiment comprises low levels of octanoate. For example, it is preferred that the preparation comprises less than 30 mM octanoate, more preferably less than about 28, 26, 24, 22, 20, 18, 16, 15, 14, 12, 10, 8 mM octanoate, even more preferably less than about 6, 5, 4, 3 mM octanoate, most preferably less than about 2, 1, 0.5, 0.4, 0.3, 0.2, 0.1, 0.01, or 0.001 mM octanoate. It is preferred that the preparation is substantially free of octanoate. Most preferably the preparation is free of octanoate (0 mM octanoate).

Preferred parameters for fatty acids in the preparation of the third embodiment are provided below. The fatty acid content is preferably an average of multiple samples, for example 2, 3, 4 or 5 samples:

| Fatty Acid | Preferred range (mM) |
|---|---|
| C6:0 | ≤ 0.1 mM, more preferably ≤0.01 mM, most preferably 0 mM |
| C8:0 | ≤ 2.5 mM, more preferably ≤0.23 mM, most preferably 0 mM |
| C9:0 | ≤ 0.1 mM, more preferably ≤0.01 mM, most preferably 0 mM |
| C10:0 | ≤ 0.1 mM, more preferably ≤0.01 mM, most preferably 0 mM |
| C11:0 | ≤ 0.1 mM, more preferably ≤0.01 mM, most preferably 0 mM |
| C12:0 | ≤ 0.5 mM, more preferably ≤ 0.05 mM, most preferably 0 mM |
| C13:0 | ≤ 0.1 mM, more preferably ≤0.01 mM, most preferably 0 mM |

(continued)

| Fatty Acid | Preferred range (mM) |
|---|---|
| C14:0 | ≤ 10 mM, more preferably 1 ≤ mM, most preferably 0 mM |
| C14:1 | ≤ 0.1 mM, more preferably ≤0.01 mM, most preferably 0 mM |
| C15:0 | ≤ 0.4 mM, more preferably ≤ 0.04 mM, most preferably 0 mM |
| C15:1 | ≤ 0.1, more preferably ≤0.01 mM, most preferably 0 mM |
| C16:0 | ≤ 34 mM, more preferably ≤ 3.38 mM, most preferably 0 mM |
| C16:1n7 | ≤ 0.9 mM, more preferably ≤ 0.09 mM, most preferably 0 mM |
| C16:2n4 | ≤ 0.1 mM, more preferably ≤0.01 mM, most preferably 0 mM |
| C16:3n4 | ≤ 0.5 mM, more preferably ≤ 0.05 mM, most preferably 0 mM |
| C17:0 | ≤ 0.5 mM, more preferably ≤ 0.05 mM, most preferably 0 mM |
| C17:1 | ≤ 0.1, more preferably ≤0.01 mM, most preferably 0 mM |
| C18:0 | ≤ 20 mM, more preferably ≤ 2.05 mM, most preferably 0 mM |
| C18:1n7 | ≤ 0.2 mM, more preferably ≤ 0.02 mM, most preferably 0 mM |
| C18:1n9c | ≤ 8 mM, more preferably ≤ 0.8 mM, most preferably 0 mM |
| C18:1n9t | ≤ 1.7 mM, more preferably ≤ 0.17 mM, most preferably 0 mM |
| C18:2n6c | ≤ 4.2 mM, more preferably ≤ 042 mM, most preferably 0 mM |
| C18:2n6t | ≤ 0.1 mM, more preferably ≤0.01 mM, most preferably 0 mM |
| C18:3n3 | ≤ 0.1 mM, more preferably ≤0.01 mM, most preferably 0 mM |
| C18:4n3 | ≤ 0.1 mM, more preferably ≤0.01 mM, most preferably 0 mM |
| C19:0 | ≤ 0.1 mM, more preferably ≤0.01 mM, most preferably 0 mM |
| C20:0 | ≤ 6 mM, more preferably ≤ 0.6 mM, most preferably 0 mM |
| C20:1n9 | ≤ 0.1 mM, more preferably ≤0.01 mM, most preferably 0 mM |
| C20:2n6 | ≤ 0.1 mM, more preferably ≤0.01 mM, most preferably 0 mM |
| C20:3n3 | ≤ 0.1 mM, more preferably ≤0.01 mM, most preferably 0 mM |
| C20:3n6 | ≤ 0.1 mM, more preferably ≤0.01 mM, most preferably 0 mM |
| C20:4n6 | ≤ 0.1 mM, more preferably ≤0.01 mM, most preferably 0 mM |
| C20:5n3 | ≤ 0.1 mM, more preferably ≤0.01 mM, most preferably 0 mM |
| C22:0 | ≤ 5.7 mM, more preferably ≤ 0.57 mM, most preferably 0 mM |
| C22:1n11 | ≤ 0.1 mM, more preferably ≤0.01 mM, most preferably 0 mM |
| C22:1n9 | ≤ 0.1 mM, more preferably ≤0.01 mM, most preferably 0 mM |
| C22:2n6 | ≤ 0.1 mM, more preferably ≤0.01 mM, most preferably 0 mM |

It is also preferred that the overall fatty acid content of the preparation of the third embodiment is less than or equal to 35mM, 32.5mM, 30 mM, 28 mM, 26 mM, 24 mM, 22 mM, 20 mM, more preferably less than or equal to 15, 10, 5, 4, 3, 2 or 1 mM. It is more preferred that the composition is substantially free of fatty acids, more preferably free of fatty acids.

A fatty acid profile and a metal ion profile of a yeast-derived recombinant albumin preparation of the third embodiment comprising 100g.$L^{-1}$ albumin, ≤ 1 mM octanoate, 250 mM $Na^+$ and having a pH of about 6.5 are provided in Figures 9 and 10, respectively. These are particularly preferred profiles of a yeast-derived recombinant albumin preparation of the third embodiment. The albumin preparation may comply with one or both of the profiles of Fig 9 and Fig 10.

It is preferred that the cations are present in the yeast-derived recombinant albumin preparation of the third embodiment from at least about 175 mM, for example from at least about 200, 225, 250, 275, 300, 325, 350, 375, 400, 425, 450, 475, 500, 525, 550, 575, 600, 650, 700, 750, 800, 850, 900, 950, 1000 mM. Preferred maximum cation concentrations include 1000, 950, 900, 850, 800, 750, 700, 650, 600, 575, 550, 525, 500, 475, 450, 425, 400, 375, 350, 325, 300, 275 and 250 mM. Preferred cation concentrations include 200 to 500 mM. More preferred is a cation concentration of about 200 to 350 mM. Most preferred is a cation concentration of about 250 mM.

The pH of a yeast-derived recombinant albumin preparation of the third embodiment may be between about 5.0 and about 9.0, for example from about 5.0, 5.25, 5.5, 5.75, 6.0, 6.25, 6.5, 6.75, 7.0, 7.25, 7.5, 7.75, 8.0, 8.25, or 8.5 to about 5.5, 5.75, 6.0, 6.25, 6.5, 6.75, 7.0, 7.25, 7.5, 7.75, 8.0, 8.25, 8.5, 8.75 or 9.0. It is preferred that pH is from about 5.0 to 8.0, such as from about 6.0 to about 8.0, more preferably from about 6.0 to about 7.0 or 6.0 to 6.5. Most preferred the pH is about 6.5.

The cations of a yeast-derived recombinant albumin preparation of the third embodiment may be provided by any cation and may be provided by one or more (several) classes or species as described below. For example, the cations may be either mono or bivalent, monoatomic or polyatomic and may be provided by one or more (several) of an alkali metal (such as sodium, potassium), an alkaline earth metal (such as calcium, magnesium) or ammonium. It is preferred that the cations are provided by sodium and/or potassium and/or magnesium, most preferably sodium or magnesium.

Cations may be provided to a yeast-derived recombinant albumin preparation of the third embodiment by a salt of an inorganic acid (e.g. a group 1 or 2 metal or ammonium salt such as sodium chloride), a salt of a divalent acid (e.g. a group 1 or group 2 metal or ammonium sulphate or phosphate such as sodium sulphate) or a salt of an organic acid (e.g. a group 1 or group 2 metal or ammonium salt of acetate or citrate such as sodium acetate).

Cations and anions used to stabilize the albumin in a yeast-derived recombinant albumin preparation of the third embodiment may be provided by (i) salts and/or (ii) pH buffers such as described herein. Therefore, there may be more than one (several) species of cation or anion, such as 2, or 3 species. There may be more than one (several) source of a single cation, for example Na which may be provided by both a pH buffer (such as sodium phosphate) and a salt (such as NaCl).

Anions useful to a yeast-derived recombinant albumin preparation of the third embodiment include inorganic anions such as phosphate, and halides such as chloride, and organic anions such as acetate and citrate. Anions may be either mono or bivalent, monoatomic or polyatomic. Preferred anions include sulphate, acetate phosphate and chloride, particularly chloride, sulphate and acetate.

Therefore, a yeast-derived recombinant albumin preparation of the third embodiment may comprise one or more (several) of an alkali metal phosphate or chloride (such as sodium phosphate, potassium phosphate, sodium chloride or potassium chloride), an alkaline earth metal phosphate (such as calcium phosphate, magnesium phosphate, calcium chloride, magnesium chloride) or ammonium phosphate or ammonium chloride.

The yeast-derived recombinant albumin preparation of the third embodiment may have an overall ionic strength of at least 175 $mmol.L^{-1}$. For example, from about 175 to 1000 $mmol.L^{-1}$ such as from about 175, 200, 225, 250, 275, 300, 325, 350, 375, 400, 425, 450, 475, 500, 525, 550, 575, 600, 650, 700, 750, 800, 850, 900, 950, 1000 $mmol.L^{-1}$ to about 1000, 950, 900, 850, 800, 750, 700, 650, 600, 575, 550, 525, 500, 475, 450, 425, 400, 375, 350, 325, 300, 275, 250 $mmol.L^{-1}$. More preferred is an overall ionic strength of about 200 to 350 $mmol.L^{-1}$. Most preferred is an ionic strength of about 250 $mmol.L^{-1}$.

It is preferred that a yeast-derived recombinant albumin preparation of the third embodiment comprises less than 20 $mg.L^{-1}$ detergent (*e.g.* polysorbate 80), preferably less than 15, 10, 5, 4, 3, 2, 1, 0.5, 0.1, 0.01, 0.001 $mg.L^{-1}$ detergent (*e.g.* polysorbate 80). Even more preferably, the preparation is substantially free of detergent (*e.g.* polysorbate 80). Most preferably the preparation is free of detergent (*e.g.* polysorbate 80). Detergent (*e.g.* polysorbate 80) levels can be assayed by techniques known to the skilled person for example, but not limited to, the assay disclosed in WO 2004/099234.

The yeast-derived recombinant albumin preparation of the third embodiment may be an albumin composition which has low levels of amino acids (*e.g.* N-acetyl tryptophan), is substantially free of amino acids (*e.g.* N-acetyl tryptophan) or is free of amino acids (*e.g.* N-acetyl tryptophan). It may be preferred that the yeast-derived recombinant albumin preparation of the third embodiment comprises less than 5 mM amino acids (*e.g.* N-acetyl tryptophan), preferably less than 4, 3, 2, 1, 0.5, 0.1, 0.01, 0.005, 0.001 mM amino acids (*e.g.* N-acetyl tryptophan). Even more preferably, the yeast-derived recombinant albumin preparation of the third embodiment is substantially free of amino acids (*e.g.* N-acetyl tryptophan). Most preferably the preparation is free of amino acids (*e.g.* N-acetyl tryptophan).

It is even more preferred that the yeast-derived recombinant albumin preparation of the third embodiment is substantially free of, or completely free of, octanoate, amino acids (*e.g.* N-acetyl tryptophan) and detergent (*e.g.* polysorbate 80).

It is preferred that the stability of the yeast-derived recombinant albumin preparation of the third embodiment is higher than that of equivalent albumin in water or in 150 mM Na. One method to compare stability, particularly related to the formation of insoluble aggregates of albumin, is:

i) place an aliquot (*e.g.* 1mL) of the albumin preparation in a cuvette (*e.g.* a polystyrene cuvette, such as Sarstedt 10x4x45mm);

ii) place the cuvette in a temperature controlled spectrophotometer that has been pre-equilibrated and controlled at a desired temperature, *e.g.* 65°C;

iii) Monitor / measure the absorbance of the composition at 350nm, referenced against an empty cuvette over a desired time period (*e.g.* 2 hours) by taking a reading at defined intervals (*e.g.* every 18 seconds)

iv) Process the data by taking the first several (*e.g.* seven) data points, average the data point readings and subtract this data point from all data points in order to provide base absorbance values of around 0.

v) Determine and/or record the time taken for the processed absorbance values to increase by 0.1 AU (Absorbance Units) above this baseline.

It is preferred that stability analysis is performed in duplicate.
It is preferred that the stability of the yeast-derived recombinant albumin preparation of the third embodiment is sufficiently high so that the time taken for the measured absorbance to increase by 0.1 AU above the baseline (according to the above described test carried out at 65°C), compared to a control solution of albumin at the same concentration in a solvent such as 150 mM Na or water and measured under the same conditions is at least 10% better. It is more preferred that the stability is at least 20, 30, 40, 50, 60, 70, 80, 90 or 100% better.

An alternative or additional stability test, particularly for the formation of soluble aggregates of albumin, is to monitor the formation of soluble albumin polymer by GP-HPLC over time at a set temperature. One suitable stability study with measurement by GP HPLC includes:

i) Placing 10mL sterilely (*e.g.* by filtration through a sterile 0.22μm filter) of each sample to be investigated into sterile vials (*e.g.* baked 10mL glass vials) which are then stoppered (*e.g.* with a sterile butyl rubber seal and optionally over-sealed).

ii) A T0 sample of ~200μL is then taken and the vial is incubated at a specified temperature (e.g. placed in a water bath that is set at a specified temperature (*e.g.* at 40°C)).

iii) Samples (~200μL) are then taken from each of the vials after certain time points (*e.g.* 14 days).

iv) injecting an aliquot (*e.g.* 25μL) of the albumin sample taken out of the vial (at <50mg/mL) onto a GP-HPLC column (*e.g.* 7.8mm id x 300mm length TSK G3000SWXL column, (Tosoh Bioscience), with a 6.0mm id x 40mm length TSK SW guard column (Tosoh Bioscience));

v) chromatographing the aliquot in a suitable buffer (*e.g.* 25 mM sodium phosphate, 100 mM sodium sulphate, 0.05% (w/v) sodium azide, pH 7.0) at a suitable speed (*e.g.* 1mL/min)

vi) monitoring the chromatograph procedure *e.g.* by UV detection at 280nm;

vii) quantifying one or more (several), or all, of monomer, dimer, trimer and polymer content of the aliquot as % (w/w) by identifying their respective peak area relative to the total peak area.

It is preferred that the test is carried out in triplicate.

Therefore, the yeast-derived recombinant albumin preparation of the third embodiment preferably possesses stability as defined in one or both of the above-mentioned tests.

A preferred yeast-derived recombinant albumin preparation of the third embodiment comprises 50 to 250 $g.L^{-1}$ yeast-derived recombinant albumin protein, 200 to 300 mM $Na^+$ (for example, 225 to 275 mM $Na^+$), 20 to 30 mM phosphate, comprises less than 2 mM octanoate and has a pH between about 6.0 and 7.0 (for example, about pH 6.5). A particularly preferred preparation comprises 50 to 150 $g.L^{-}1$ yeast-derived recombinant albumin protein, 225 to 275 mM $Na^+$, 20 to 30 mM phosphate, comprises less than 1 mM octanoate and has a pH of about 6.5.

In accordance with the third particularly preferred embodiment, the recombinant yeast-derived serum albumin preparation (most preferably derived by recombinant DNA expression in *Saccharomyces cerevisiae*) is the commercially-available AlbIX® product from Albumedix Ltd., or a preparation that is similar thereto, and may comprise, consist essentially of, or consist of the following components:

i. Yeast-derived recombinant human albumin as the "Active" ingredient. For example, the yeast derived yeast-derived recombinant human albumin may be present in the preparation at a concentration of about 10 to 400 g/L, such as about 20, 30, 40 50, 60, 70, 80, 90, 100, 110, 120, 130, 140, 150, 160, 170, 180, 190 or 200 g/L.

ii. Sodium (for example, added in the form of NaCl) in order to adjust the tonicity. The sodium may be present, for example, at a concentration of at least about 150 mM, more typically about 200 to about 300 mM.

iii. Water or buffer as a diluent/vehicle.

iv. Is substantially free of, or completely free of, octanoate, amino acids (*e.g.* N-acetyl tryptophan) and detergent (*e.g.* polysorbate 80).

Additionally, or alternatively, accordance with the third preferred embodiment, the recombinant yeast-derived serum albumin preparation (most preferably derived by recombinant DNA expression in *Saccharomyces cerevisiae*) is the commercially-available AlbIX® product from Albumedix Ltd., or a preparation that is similar thereto, and may be characterised by one or more (such as all) of the following characteristics:

(i) A recombinant yeast-derived serum albumin protein purity of at least (preferably greater than) 99.0% (w/w), for example as determined by native PAGE;
(ii) Endotoxin content not greater (preferably less) than 0.50 EU/ml, and more preferably not greater (preferably less) than 0.01 EU/mg of recombinant yeast-derived serum albumin protein, for example as determined by the USP <85> test;
(iii) pH of 6.0-7.0, for example as determined by the USP <791> test;
(iv) a sodium content of 200-300 mM, for example as determined by atomic absorption spectroscopy;
(v) an appearance that is a clear, pale straw to amber coloured solution, for example as determined by visual inspection;
(vi) Con A-binding species of recombinant yeast-derived serum albumin of not greater (preferably less) than 0.30% (w/w) protein, for example as determined by Con A Chromatography.

For the avoidance of doubt, the term 'recombinant yeast-derived serum albumin preparation' as used herein specifically excludes plasma-derived albumin products. It also specifically excludes recombinant albumin products derived from non-yeast sources, such as by recombinant expression in plants.

Historically, the classic way of sourcing of human albumin for the manufacture of serum albumin preparations, has been to purify it from human blood, thereby creating a plasma-derived serum albumin preparation. Human albumin obtained from plasma is governed in the US by the Code of Federal Regulations (CFR) Title 21, Food And Drugs, Chapter IFood And Drug Administration Department Of Health And Human Services, Subchapter F Biologics, Part 640 Additional Standards For Human Blood And Blood Products, Subpart H Albumin (Human), Sections 640.80 to 86. This standard has very few requirement, but specifies, for example, that (i) the formulation must contain either 0.08 ± 0.016 millimole sodium caprylate (i.e. sodium octanoate), or 0.08 ± 0.016 millimole sodium acetyltryptophanate and 0.08 ± 0.016 millimole sodium caprylate per gram of protein, present as a stabilizer(s); (ii) the formulation must be heat treated by heating continuously for not less than 10, or more than 11 hours, at an attained temperature of 60 ± 0.5 deg. C; (iii) the protein composition shall be at least 96% albumin of the total protein in the final product; (iv) the pH shall be 6.9 ± 0.5 when measured in a solution of the final product diluted to a concentration of 1 percent protein with 0.15 molar sodium chloride; (v) the sodium concentration of the final product shall be 130 to 160 milliequivalents per liter; (vi) the potassium concentration of the final product shall not exceed 2 milliequivalents per liter; (vii) it shall demonstrate heat stability, as indicated when a final container sample remains unchanged, as determined by visual inspection, after heating at 57 deg. C for 50 hours, when compared to its control consisting of a sample, from the same lot, which has not undergone this heating.

The US Pharmacopeia (USP) refers to the above noted CFR entry as the basis of its requirements, and refers to the product as a sterile, non-pyrogenic preparation of serum albumin obtained by fractionating material (source blood, plasma, serum or placentas) from heathy human donors, the source material being tested for the absence of hepatits B surface antigen. It is stated to contain sodium acetyltryptophanate with or without sodium caprylate as a stabilizing agent, and a sodium content of not less than 130 mEq per L and not more than 160 mEq per L. It is stated to possess a heme content such that the absorbance of a solution, diluted to contain 1 percent of protein, in a 1 cm holding cell, measured at a wavelength of 403 nm, is not more than 0.25.

The European Pharmacopoeia (EP) monograph 0255 describes human albumin solutions as an aqueous solution of protein obtained from plasma that complies with the requirements of the monograph on Plasma for fractionation, human (0853). It is specified to contain a suitable stabiliser against the effects of heat, such as sodium caprylate (sodium octanoate) or N-acetyltryptophan or a combination of these two. It specifies that the preparation is produced by a method that involves the preparation being passed through a bacteria-retentive filter and distributed aseptically into sterile containers which are then closed so as to prevent contamination; wherein thereafter the solution in its final container is heated to 60 ± 1.0 °C and maintained at this temperature for not less than 10 h. The containers are then incubated at 30-32 °C for not less than 14 days or at 20-25 °C for not less than 4 weeks and examined visually for evidence of microbial contamination.

In all cases it is permitted by the CFR, USP and EP for plasma-derived serum albumin preparations to show the presence of small quantities of other plasma proteins and other contaminants. For example, plasma-derived serum albumin preparations typically contain:

- Up to 4%, or 5% of source-derived protein that is distinct from monomeric human albumin.
- Haem content that is not greater than 0.15 (as determined by the method below),

- Prekallikrein activator content to a maximum of 35 IU/ml,
- Aluminium to a maximum 200 μg of Al per litre,
- Potassium to a maximum 0.05 mmol of K per gram of protein, and
- Sodium: maximum 160 mmol of Na per litre.

Haem content in an albumin preparation can be tested using the following method. Dilute the preparation to be examined using a 9 g/l solution of sodium chloride R to obtain a solution containing 10 g/l of protein. The absorbance of the solution measured at 403 nm using water R as the compensation liquid.

Aluminium content in an albumin preparation can be tested using the following method. Atomic absorption spectrometry: Use a furnace as atomic generator. Use plastic containers for preparation of the solutions. Wash equipment in nitric acid (200 g/l $HNO_3$) before use. Test solution: Use the preparation to be examined. Validation solution: Use human albumin for aluminium validation BRP. Reference solutions: Prepare a suitable range of reference solutions by adding suitable volumes of aluminium standard solution (10 ppm Al) R to known volumes of water R. Dilute the solutions as necessary using nitric acid (10 g/l HNO3) containing 1.7 g/l of magnesium nitrate R and 0.05 per cent V/V of octoxinol 10 R. Measure the absorbance at 309.3 nm. The test is valid if the aluminium content determined for human albumin for aluminium validation BRP is within 20 per cent of the value stated in the leaflet accompanying the reference preparation.

Potassium content in an albumin preparation can be tested using atomic emission spectrometry, Wavelength: 766.5 nm.

Sodium content in an albumin preparation can be tested using atomic emission spectrometry, Wavelength: 589 nm.

The recombinant yeast-derived serum albumin protein, and the preparation thereof, is "serum-free". That is, the recombinant yeast-derived serum albumin contains no serum (*e.g.*, human serum, fetal bovine serum (FBS), horse serum, goat serum, or any other animal-derived serum known to one skilled in the art). The recombinant yeast-derived serum albumin preparation will, therefore, generally be totally free of serum-derived contaminants, since none are present in the starting material.

The recombinant yeast-derived serum albumin protein, and the preparation thereof, is free of components specifically derived from source material used in the preparation of serum albumin from naturally-occurring biological sources of serum albumin, such as blood, plasma, serum or placentas. Components specifically derived from source material include haem, prekallikrein activator and/or other non-albumin proteins, peptides or amino acids derived from naturally-occurring biological sources of serum albumin, such as blood, plasma, serum or placentas. Preparations of serum albumin from naturally-occurring biological sources of serum albumin, such as blood, plasma, serum or placentas may also contain pyrogens and/or endotoxin. They may also contain infectious agents, such as viruses (including hepatitis C), that can cause disease. Although typically, the risk that such products will transmit an infectious agent has been reduced by screening plasma donors for prior exposure to certain viruses, by testing for the presence of certain current virus infections, and by inactivating and/or removing certain viruses, despite these measures, such products can still potentially transmit disease. There is also the possibility that unknown infectious agents may be present in such products.

In contrast, for example, the recombinant yeast-derived serum albumin protein, and the preparation thereof, may be free of haem, whereas haem will be detected in a plasma-derived serum albumin preparation, for example when tested by the absorbance of a solution, diluted to contain 1 percent of protein, in a 1 cm holding cell, measured at a wavelength of 403 nm.

The recombinant yeast-derived serum albumin protein, and the preparation thereof, may be free of prekallikrein activator, whereas prekallikrein activator will be detected in a plasma-derived serum albumin preparation.

Pyrogens may be detected in a plasma-derived serum albumin preparation, whereas pyrogens are typically at much lower levels in the recombinant yeast-derived serum albumin protein, and the preparation thereof, for use in the present invention.

The recombinant yeast-derived serum albumin protein, and the preparation thereof, as well as media (e.g. cryopreservation media and/or storage media) prepared therefrom and used in accordance with the present invention, may be free of human viruses (including hepatitis C), whereas human viruses (including hepatitis C) may be detected in a plasma-derived serum albumin preparation.

The recombinant yeast-derived serum albumin protein, and the preparation thereof, as well as media (e.g. cryopreservation media and/or storage media) prepared therefrom and used in accordance with the present invention, may comprises low levels of octanoate. For example, it may be preferred that the recombinant yeast-derived serum albumin protein comprises less than 3.0, 2.5, 2.0, 1.5, 1, 0.9, 0.8, 0.7, 0.6, 0.5, 0.4, 0.3 0.2, 0.1, 0.09, 0.08, 0.07, 0.06, 0.05, 0.04, 0.03, 0.02, 0.01, 0.009, 0.008, 0.007, 0.006, 0.005, 0.04, 0.003, 0.002, or 0.001 mM octanoate, is substantially free of octanoate, or is free of octanoate (0 mM octanaote). In contrast, plasma-derived serum albumin preparations typically contain 0.08 ± 0.016 millimole sodium caprylate per gram of protein (for a 20% w/v plasma-derived serum albumin preparation, this corresponds to a sodium caprylate concentration of 16 mM ± 3.2 mM; for a 4% w/v plasma-derived serum albumin preparation, this corresponds to a sodium caprylate concentration of 3.2 mM ± 0.64 mM).

The recombinant yeast-derived serum albumin protein, and the preparation thereof, as well as media (e.g. cryopreservation media and/or storage media) prepared therefrom and used in accordance with the present invention, may comprises low levels of N-acetyl tryptophan. For example, it may be preferred that the recombinant yeast-derived serum albumin protein comprises less than 3.0, 2.5, 2.0, 1.5, 1, 0.9, 0.8, 0.7, 0.6, 0.5, 0.4, 0.3 0.2, 0.1, 0.09, 0.08, 0.07, 0.06, 0.05, 0.04, 0.03, 0.02, 0.01, 0.009, 0.008, 0.007, 0.006, 0.005, 0.04, 0.003, 0.002, or 0.001 mM N-acetyl tryptophan, is substantially free of N-acetyl tryptophan, or is free of N-acetyl tryptophan (0 mM N-acetyl tryptophan). In contrast, plasma-derived serum albumin preparations typically contain 0.08 $\pm$ 0.016 millimole N-acetyl tryptophan per gram of protein (for a 20% w/v plasma-derived serum albumin preparation, this corresponds to a N-acetyl tryptophan concentration of 16 mM $\pm$ 3.2 mM; for a 4% w/v plasma-derived serum albumin preparation, this corresponds to a N-acetyl tryptophan concentration of 3.2 mM $\pm$ 0.64 mM).

It is even more preferred that a yeast-derived recombinant albumin preparation for use in accordance with the present invention, as well as media (e.g. cryopreservation media and/or storage media) prepared therefrom and used in accordance with the present invention, is substantially free of, or completely free of, octanoate and N-acetyl tryptophan.

Further, typically, the recombinant yeast-derived serum albumin preparation used in the formation of the cryopreservation medium and/or the storage medium in accordance with the present invention, as well as media (e.g. cryopreservation media and/or storage media) prepared therefrom and used in accordance with the present invention, has an aluminium concentration of less than 200 $\mu g.L^{-1}$, such as less than 180 $\mu g.L^{-1}$, 160 $\mu g.L^{-1}$, 140 $\mu g.L^{-1}$, 120 $\mu g.L^{-1}$, 100 $\mu g.L^{-1}$, 90 $\mu g.L^{-1}$, 80 $\mu g.L^{-1}$, 70 $\mu g.L^{-1}$, 60 $\mu g.L^{-1}$, 50 $\mu g.L^{-1}$, or 40 $\mu g.L^{-1}$, more typically within the range of about 10 $\mu g.L^{-1}$ to about 30 $\mu g.L^{-1}$.

Therefore, recombinant yeast-derived serum albumin preparations for use in the present invention are physically distinct from serum albumin compositions obtained from naturally-occurring biological sources of serum albumin, such as blood, plasma, serum or placentas.

Recombinant yeast-derived serum albumin preparations for use in the present invention are also physically distinct from serum albumin compositions obtained from other sources (e.g. by recombinant expression in plants). At least in part, that is because, in comparison to serum albumin obtained from other sources, the albumin protein in the recombinant yeast-derived serum albumin is relatively devoid of changes and alterations imposed on the protein by the source.

For example, it is known that plasma-derived serum albumin preparations, and recombinant plant-derived serum albumin preparations typically lose one, or more commonly two, N-terminal amino acids from the albumin protein. Without being bound by theory, it is believed that this is due to the use of heating in the production processes used in the production of plasma-derived serum albumin preparations, and recombinant plant-derived serum albumin preparations. In contrast, recombinant yeast-derived serum albumin protein typically possesses an intact N-terminal amino acid sequence. Therefore, it may be preferred that the recombinant yeast-derived serum albumin protein, and preparations thereof, for use in the present invention possess an intact or substantially intact N-terminal sequence. In that regard, a protein can be defined as having an intact N-terminal sequence if no N-terminal loss is observable when tested using intact mass spectrometry with a level of quantitation of 1.5%.

Moreover, the data in Example 5 demonstrates clear physical differences in recombinant yeast-derived serum albumin preparation and recombinant plant-derived serum albumin preparation.

More particularly, as shown in Example 5, mass spectrometry profiling of yeast-derived serum albumin preparations demonstrates a single species at about 66.4 kDa that is representative of the recombinant yeast-derived serum albumin preparation comprising predominantly a native intact human serum albumin molecule, with a reduced Cys34 residue. As shown in Figure 1 of this application, all recombinant yeast-derived serum albumin preparations tested by intact mass spectrometry demonstrated a single main peak at about 66.4 kDa that is representative of native intact human serum albumin molecule, and very low levels of any other peaks. In contrast, the two recombinant plant-derived serum albumin preparations tested demonstrated high numbers of peaks distinct from the main peak at about 66.4 kDa that is representative of native intact human serum albumin molecule.

It may therefore be preferred that that the yeast-derived recombinant serum albumin preparation used in accordance with the present invention is a preparation that, when tested by mass spectrometry, is a product that (compared to recombinant plant-derived serum albumin protein, such as the samples shown in Fig 1) displays substantially fewer peaks (such as lower than 50%, 40%, 30%, 20%, 10%, 5% or less) distinct from the main peak at about 66.4 kDa that is representative of native intact human serum albumin molecule.

Further, as shown in Example 5, different recombinant serum albumin products demonstrate different levels of pigmentation. The images presented in Figure 3, demonstrate that Recombumin® Prime and Recombumin® Alpha are the least pigmented of the products evaluated; with both products having a clear/straw colour. The alternative products showed increasing levels of pigmentation with the albumin from Supplier 1, a rice-derived product, showing the greatest pigmentation and the final product having an orange/amber colour.

It may therefore be preferred that that the yeast-derived recombinant serum albumin preparation used in accordance with the present invention is a preparation that has too little pigmentation to present as an orange/amber colour, and is most preferably a clear, straw, or straw to amber colour.

Further, as shown in Example 5, yeast-derived recombinant albumin preparations comprised albumin protein having a notably higher free thiol group content than plant-derived recombinant albumin preparations. It may therefore be preferred that the yeast-derived recombinant serum albumin preparation comprise albumin protein that has a free thiol group content (specifically, at Cys34) that is greater than 62%, such as at least 69%, at least 70%, at least 75%, at least 80%, at least 85%, at least 90%, at least 95%, about 96%, about 97%. In practice, the free thiol group content may be about 85% or about 97%. The term "about" in this context is intended to optionally include ± 3, 2, or 1%.

In addition, Frahm et al, 2014, PLOS One, 9(1): e109893 reported a study in which the properties of yeast-derived recombinant serum albumin were compared to plasma-derived serum albumin and recombinant plant-derived serum albumin products. Specifically:

- the yeast-derived recombinant serum albumin products tested were Recombumin® Alpha (formerly Albucult®) and Recombumin® Prime (formerly Recombumin®) commercially available from Albumedix Ltd., which are both expressed in *Saccharomyces cerevisae*, and Albagen, which is expressed in *Pichia pastoris* and was sourced from Sigma-Aldrich - these were referred to by Frahm *et al* as "ScrHSA", "Recombumin®", and "PrPHSA", respectively;
- the plant-derived recombinant serum albumin products tested were four different lots of serum albumin expressed in *Orzya sativa* (rice) from Cellastim (referred to by Frahm *et al* as "OsrHSA-sig-C", "OsrHSA-sig-G", "Osr-sig-H" and "OsrHSA-sig-J"); and three further different rice-derived recombinant serum albumin products from eEnzyme LLC ("OrsHSA-phy"), ScienCell Research Laboratories ("OsrHSA-sci") and amsbio LLC (OsrHSA-ams"); and
- the plasma-derived serum albumin product was essentially FA-free albumin from Sigma-Aldrich ("pHSA").

Frahm *et al* found numerous differences between yeast-derived recombinant serum albumin products, on the one hand, and plasma-derived serum albumin and recombinant plant-derived serum albumin products on the other hand.

For example, when assessed by size exclusion chromatography (SEC), Frahm *et al* showed that yeast-derived recombinant serum albumin products produced a characteristic single focused peak. The results were as follows:

**Table S1: reproduced from Frahm *et al*, 2014, *PLOS One*, 9(1): e109893**

| Sample/Peak# | Peak retention time (min)/Percent Area of Chromatogram | | | | | | | |
|---|---|---|---|---|---|---|---|---|
| | 1 | 2 | 3 | 4 | 5 | 6 | 7 | 8 |
| pHSA | 13.1/0.1 | 13.8/0.6 | 14.3/2.5 | 15.4/12.2 | 17.4/84.6 | | | |
| Recombumin® Prime | | | 14.3/0.2 | 15.4/4 | 17.4/95.9 | | | |
| Recombumin® Alpha | | | | 15.3/0.6 | 17.4/99.4 | | | |
| PprHSA | | | 14.4/1.6 | 15.4/9.36 | 17.4/84.5 | 18.2/4.3 | | |
| OsrHSA-sig-C | 13.0/0.6 | | 14.4/5.1 | 15.3/14.9 | 17.3/66.6 | 18.4/8.5 | 19.8/1.7 | 20.4/2.5 |
| OsrHSA-sig-G | 13.0/1.0 | | 14.4/3.1 | 15.3/12.1 | 17.3/69.9 | 18.4/9.5 | 19.7/1.9 | 20.4/2.7 |
| OsrHSA-sig-H | 12.9/1.2 | | 14.4/0.9 | 15.4/6.2 | 17.3/80.1 | 18.4/7.7 | 19.8/1.4 | 20.5/1.9 |
| OsrHSA-sig-J | 12.9/1.2 | | 14.4/0.9 | 15.4/6.2 | 17.3/80.6 | 18.4/7.7 | 19.8/1.4 | 20.5/1.9 |
| OsrHSA-sci | 12.9/0.5 | | 14.3/3.7 | 15.4/12.7 | 17.4/83.1 | | | |
| OsrHSA-phy | 12.9/0.7 | 13.8/0.9 | 14.3/3.3 | 15.4/13.1 | 17.4/82.0 | | | |
| OsrHSA-ams | 12.9/0.3 | 13.7/0.2 | 14.4/1.6 | 15.4/9.1 | 17.4/88.8 | | | |

Therefore, whereas all samples showed a main peak eluting at approximately 17.3-17.4 minutes, only the yeast-derived recombinant serum albumin products displayed an SEC profile excluding peaks corresponding to high molecular weight contaminants with a peak retention time under 14 minutes and peaks corresponding to low molecular weight contaminants with a peak retention time over 19 minutes.

Furthermore, the yeast-derived recombinant serum albumin products derived from *Saccharomyces cerevisae* and commercially available from Albumedix Ltd. (i.e. Recombumin® Alpha and Recombumin® Prime, which are referred to by Frahm *et al* as "ScrHSA" and "Recombumin®", respectively) are the only products tested that displayed an SEC profile excluding peaks with a peak retention time under 14 minutes and over 18 minutes (in fact, the ScrHSA product displayed an SEC profile excluding peaks with a peak retention time under 15 minutes and over 18 minutes). They are also the only products tested that displayed a main peak that represented a relative quantity that is greater than 90%.

It may, therefore, be preferred that the yeast-derived recombinant serum albumin preparation used in accordance with the present invention is a preparation that, when tested by SEC, is a product that displays an SEC profile excluding peaks with a peak retention time under 14 minutes and over 19 minutes, and more preferably excludes peaks with a peak retention time under 14 or 15 minutes and over 18 minutes. It may also be preferred that the yeast-derived recombinant serum albumin preparation used in accordance with the present invention is a preparation that, when tested by SEC, is a product that displays a main peak that represents a relative quantity that is greater than 90%, such as greater than 91%, 92%, 93%, 94%, 95%, 96%, 97%, 98%, 99%, 99.1%, 99.2%, 99.3% or at least about 99.4%.

In the foregoing definition, it may be preferred that the SEC technique used for such measurement is the methodology of Frahm et al, 2014, PLOS One, 9(1): e109893. Specifically, the size-exclusion chromatography system may be a Waters Alliance 2695 Separations Module fitted with a Waters 2996 Photodiode Array Detector (Waters Corporation, Milford, MA, USA). Instrument operation and data acquisition and manipulation may be carried out with Waters Empower 2 Chromatography Manager (Waters Corporation). A YMC-Pack Diol-200 column (Product# DL20S05-5008WT, YMC America, Inc., Allentown, PA, USA) with internal dimensions of 500×8.0 mm may be used at a flow rate of 0.8 ml/min. The mobile phase can contain 0.1 M sodium phosphate, 0.15 M sodium chloride (pH 7.0) and peaks may be detected at a wavelength of 214 nm.

Frahm *et al* further reported that RP-HPLC analysis showed plasma-derived HSA (i.e. "pHSA") to be heterogeneous displaying two major peaks, 1 and 2, each of which has been previously shown to consist of several components with native HSA eluting as part of the more hydrophobic peak 2. RP-HPLC analysis of the yeast-derived preparations Recombumin® Alpha and Recombumin® Prime (referred to by Frahm *et al* as "ScrHSA" and "Recombumin®", respectively) and PprHSA showed the major peak to be peak 2, suggesting that the majority of HSA in the yeast-derived preparations was present in native forms. RP-HPLC of Sigma-Aldrich sourced OsrHSA showed the major peak of all lots to be peak 1, suggesting that the majority of Sigma-Aldrich sourced plant-derived OsrHSA was present as modified forms. All other forms of OsrHSA (OsrHSA-sci, -phy, and -ams) also showed peaks corresponding to peak 1.

It may, therefore, be preferred that the yeast-derived recombinant serum albumin preparation used in accordance with the present invention is a preparation that, when tested by RP-HPLC, is a product that displays a single major peak, corresponding to peak 2 of pHSA, and indicative that the majority of the serum albumin in the yeast-derived recombinant serum albumin preparation is present in native form.

In the foregoing definition, it may be preferred that the RP-HPLC technique used for such measurement is the methodology of Frahm et al, 2014, PLOS One, 9(1): e109893. Specifically, the HPLC system may use a Waters Alliance 2695 chromatograph equipped with a column heater and an autosampler with a sample cooling device, coupled to a Waters 2996 U/V-Vis photodiode array detector. Data acquisition and integration can, for example, be performed with Empower Pro Software from Waters. Separation conditions can be as described in Girard et al., Biomed. Chromatogr., 12: 183-184. Briefly, the column may be an Aquapore RP-300, C8, 7 μm, 220×2.1 mm i.d. (Brownlee) and may be maintained at 50°C. Mobile Phase A (MP A) can consist of 0.05% trifluoroacetic acid (TFA) in 10% acetonitrile/90% water; Mobile Phase B (MP B) can be 0.05% TFA in 90% acetonitrile/10% water; Mobile Phase C (MP C) can be 0.05% TFA in acetonitrile. The column may be equilibrated with a mixture of MP A and MP B (70:30) until a stable baseline is obtained. Elution may be carried out using a multi-step gradient consisting of MP A/MP B (70:30) for 1 min (at 0.7 ml/min), linear gradient to MP A/MP B (65:35) over 5 min (at 0.7 ml/min), linear gradient to MP A/MP B (61:39) over 19 min (at 0.7 mL/min), linear gradient to MP A/MP B (50:50) over 10 min (at 0.7 mL/min), linear gradient to MP C over 5 min (at 1.0 mL/min), MP C for 12 min, linear gradient to MP A/MP B (70:30) over 3 min. The effluent may be monitored at 220 nm.

Frahm *et al* also used Mass Spectrometry to assess differential glycation of lysine/arginine residues. The results showed lower numbers of hexose modified lysine or arginine residues in all yeast-derived recombinant serum albumin preparations compared to the plasma sample (pHSA) and all plant-derived (OsrHSA) recombinant serum albumin preparations. The results were as follows:

**Table 1: reproduced from Frahm *et al*, 2014, *PLOS One*, 9(1): e109893**

| Sample | Sequence Coverage %[*] | Number of Unique Hex(K) and Hex(R) Identified |
|---|---|---|
| pHSA | 95 | 15 |
| Recombumin® Prime | 96 | 8 |
| Recombumin® Alpha | 97 | 5 |
| PprHSA | 96 | 11 |
| OsrHSA-sig-C | 95 | 18 |

(continued)

| Sample | Sequence Coverage %* | Number of Unique Hex(K) and Hex(R) Identified |
|---|---|---|
| OsrHSA-sig-G | 95 | 23 |
| OsrHSA-sig-H | 96 | 13 |
| OsrHSA-sig-J | 95 | 13 |
| OsrHSA-sci | 95 | 23 |
| OsrHSA-phy | 95 | 21 |
| OsrHSA-ams | 95 | 17 |
| * Excluding leader sequence. | | |

The applicant further notes that, although Frahm *et al* finds 8 hexose modified in Recombumin® Prime and 5 in Recombumin® Alpha, these are defined across a complete population of molecules in a preparation, and they will not all be modified in one albumin protein molecule. An individual Recombumin® Prime albumin molecule will more likely only have 1 or 2, perhaps even 3, but not all 8 hexose modified K and R residues.

It may, therefore, be preferred that the yeast-derived recombinant serum albumin preparation used in accordance with the present invention is a preparation that, when tested by Mass Spectrometry, is a product that displays fewer than 13, more preferably fewer than 12, 11, 10, 9, 8, 7, or 6, such as about 1 to 11, 1 to 8, 1 to 5, 1 to 4, 1 to 3, 1 to 2, 1 or less than 1, hexose modified lysine or arginine residues per protein. In the foregoing definition, it may be preferred that the Mass Spectrometry technique used for such measurement is the methodology of Frahm et al, 2014, PLOS One, 9(1): e109893.

In particular, Frahm *et al* found (Table S2 thereof) that the following resides were rarely glycated in yeast-derived recombinant serum albumin, compared to plasma-derived serum albumin and plant-derived recombinant serum albumin: K51; K64; K73; K137; K159; K174; K181; K225; K233; K240; K262; K466; K525; K545; and K574.

Conversely, Frahm *et al* found (Table S2 thereof), that the following resides were commonly more glycated in yeast-derived recombinant serum albumin, compared to plasma-derived serum albumin and plant-derived recombinant serum albumin: R485; K500.

It may, therefore, be preferred that the yeast-derived recombinant serum albumin preparation used in accordance with the present invention is a preparation that displays levels of glycation at any one, or more (such as all), of K51; K64; K73; K137; K159; K174; K181; K225; K233; K240; K262; K466; K525; K545; K574, R485; and/or K500 that is substantially identical the levels observed for Recombumin® Alpha or Recombumin® Prime (referred to by Frahm *et al* as "ScrHSA" and "Recombumin®", respectively) or PprHSA, as reported in Frahm *et al.* The measured level of glycation may be based on an average of multiple, such as 2, 3, 4, 5, 10 or more samples.

Frahm *et al* concluded that there are clear differences in glycation between recombinant serum albumin expressed in yeast expression systems compared to plasma-derived serum albumin and plant-derived recombinant serum albumin. It is thought that glycation occurs via a slow non-enzymatic Maillard reaction in which residues with free amine groups are modified with sugars. Up to 10% of plasma-derived HSA is believed to be glycated in healthy individuals and up to 30% in individuals with hyperglycaemia. Hexose modification of lysine and arginine residues in plasma-derived albumin is believed to occur over the long (26-31 day) circulation lifetime of the protein whereas *in vitro* glycation of lysine and arginine is considered to require elevated temperature and sugar concentrations as well as a time scale on the order of days or weeks. In contrast, yeast-expressed recombinant serum albumin is typically secreted during expression and, subsequently, the sugars in the growth media (which may be around 2% glucose) could provide an environment suitable for the glycation of the secreted protein. However, it has been suggested that the glycation mechanisms in plants may involve light/dark cycles or light stress to the plants and glycation of plant proteins is not unexpected as higher order plants possess homologues of animal enzymes that repair early glycation adducts. Frahm *et al* noted that the OsrHSA used in their experiments were expressed in the endosperm of *O. sativa* which contains up to 19 mg of glucose per g total weight and it was suggested that the presence of this monosaccharide may allow for the glycation of the protein over the growth period of the plant (approximately 30 days for grain ripening) and variations in growth conditions of the plant may account for the lot-to-lot variability observed. Additionally, it was noted that plant-derived recombinant serum albumin protein may be glycated with plant-specific sugars, such as α-1,3-fucose and/or β-1,2-xylose. This can be a further physical distinction between plant-derived recombinant serum albumin protein and serum albumin protein derived from other sources, such as plasma or by recombinant expression in yeast.

Detergent: Detergents can be classified into four groups, depending on the electrical charge - anionic detergents, cationic detergents, non-ionic detergents and zwitterionic detergents.

Typical anionic detergents include alkylbenzenesulfonates. The alkylbenzene portion of these anions is lipophilic and the sulfonate is hydrophilic. Examples of types of anionic detergents include branched sodium dodecylbenzenesulfonate, linear sodium dodecylbenzenesulfonate, and soap.

Recombinant yeast-derived serum albumin preparations, and media (such as cryopreservation media, storage media, and other media) which comprise the recombinant yeast-derived serum albumin preparations, for use in accordance with any of the aspects of the present invention, may comprises less than 0.01, preferably less than 0.001, more preferably less than 0.0001 % (w/v) anionic detergent.

Cationic detergents are similar to the anionic detergents, with a hydrophobic component, but, instead of the anionic sulfonate group, the cationic surfactants have quaternary ammonium (i.e. positively charged group) as the polar moiety.

Recombinant yeast-derived serum albumin preparations, and media (such as cryopreservation media, storage media, and other media) which comprise the recombinant yeast-derived serum albumin preparations, for use in accordance with any of the aspects of the present invention, may comprises less than 0.01, preferably less than 0.001 %, more preferably less than 0.0001 (w/v) cationic detergent.

Zwitterionic detergents possess a net zero charge arising from the presence of equal numbers of +1 and -1 charged chemical groups. One example of a zwitterionic detergent is CHAPS (3-[(3-Cholamidopropyl)dimethylammonio]-1-propanesulfonate). In one embodiment, recombinant yeast-derived serum albumin preparations, and media (such as cryopreservation media, storage media, and other media) which comprise the recombinant yeast-derived serum albumin preparations, for use in accordance with any of the aspects of the present invention, may comprises less than 0.001 % (w/v) zwitterionic detergent and may be essentially free of zwitterionic detergents.

Non-ionic detergents are characterized by their uncharged, hydrophilic headgroups. Typical non-ionic detergents are based on polyoxyethylene or a glycoside. Common examples of the former include polysorbate 80 (e.g. Tween®), 4-(1,1,3,3-Tetramethylbutyl)phenyl-polyethylene glycol, t-Octylphenoxypolyethoxyethanol (e.g. Triton® X-100), and the Brij® series. These materials are also known as ethoxylates or PEGylates. Glycosides have a sugar as their uncharged hydrophilic head-group. Examples include octyl-thioglucoside and maltosides. Hydroxyethylglucamide (HEGA) and methylglucamide (MEGA) series detergents are similar, possessing a sugar alcohol as the head-group.

In one preferred embodiment, recombinant yeast-derived serum albumin preparations, and media (such as cryopreservation media, storage media, and other media) which comprise the recombinant yeast-derived serum albumin preparations, for use in accordance with any of the aspects of the present invention, may comprises less than 0.01, preferably less than 0.001, more preferably less than 0.0001 % (w/v) nonionic detergent.

In a further embodiment, recombinant yeast-derived serum albumin preparations, and media (such as cryopreservation media, storage media, and other media) which comprise the recombinant yeast-derived serum albumin preparations, for use in accordance with any of the aspects of the present invention, may comprises less than 0.01, preferably less than 0.001%, more preferably less than 0.0001 % (w/v) polysorbate 80 and may be essentially free of polysorbate 80. For example, the composition may comprise less than or equal to $3.325*10^{-4}$ % (w/v) polysorbate 80 or 20, such as less than or equal to $2.85*10^{-4}$ % (w/v) polysorbate 80 or 20, such as less than or equal to $2.375*10^{-4}$ % (w/v) polysorbate 80 or 20, such as less than or equal to $1.425*10^{-4}$ % (w/v) polysorbate 80 or 20, such as less than or equal to $9*10^{-5}$ % (w/v) polysorbate 80 or 20, such as less than or equal to $6.625*10^{-5}$ % (w/v) polysorbate 80 or 20, such as less than or equal to $5.7*10^{-5}$ % (w/v) polysorbate 80 or 20, such as less than or equal to $5*10^{-5}$ % (w/v) polysorbate 80 or 20, such as less than or equal to $4.75*10^{-5}$ % (w/v) polysorbate 80 or 20, such as less than or equal to $4.5*10^{-5}$ % (w/v) polysorbate 80 or 20, such as less than or equal to $4.75*10^{-5}$ % (w/v) polysorbate 80 or 20, such as less than or equal to $2.85*10^{-5}$ % (w/v) polysorbate 80 or 20, such as less than or equal to $2.5*10^{-5}$ % (w/v) polysorbate 80 or 20, such as less than or equal to $1.9*10^{-5}$ % (w/v) polysorbate 80 or 20, such as less than or equal to $1.8*10^{-5}$ % (w/v) polysorbate 80 or 20, such as less than or equal to $1*10^{-5}$ % (w/v) polysorbate 80 or 20, such as less than or equal to $9.5*10^{-6}$ % (w/v) polysorbate 80 or 20, such as less than or equal to $9*10^{-6}$ % (w/v) polysorbate 80 or 20, such as less than or equal to $5*10^{-6}$ % (w/v) polysorbate 80 or 20, most preferably substantially free of polysorbate 80 or 20. In another embodiment, the composition of the present invention comprises less 0.01, preferably less than 0.001, more preferably less than 0.0001 % (w/v) polysorbate 20 and may be free of polysorbate 20. In another embodiment, the composition may comprises less than 0.01, preferably less than 0.001, more preferably less than 0.0001% (w/v) poloxamer and may be free of poloxamer.

In one embodiment, recombinant yeast-derived serum albumin preparations, and media (such as cryopreservation media, storage media, and other media) which comprise the recombinant yeast-derived serum albumin preparations, for use in accordance with any of the aspects of the present invention, may comprises from 0.001, such as from 0.002, such as from 0.003, such as from 0.004, such as from 0.005, such as from 0.006, such as from 0.007, such as from 0.008, such as from 0.009, such as from 0.01, such as from 0.02, such as from 0.03, such as from 0.04, such as from 0.05, such as from 0.06, such as from 0.07, such as from 0.08, such as from 0.09, such as from 0.1, such as from 0.2, such as from 0.3, such as from 0.4, such as from 0.5, such as from 0.6, such as from 0.7, such as from 0.8, such as from 0.9 % (w/v) non-ionic detergent to 0.002, such as to 0.003, such as to 0.004, such as to 0.005, such as to 0.006, such as to 0.007, such as to 0.008, such as to 0.009, such as to 0.01, such as to 0.02, such as to 0.03, such as to 0.04, such as to 0.05, such as to 0.06, such as to 0.07, such as to 0.08, such as to 0.09, such as to 0.1, such as to 0.2, such as to 0.3, such as to 0.4, such as to 0.5, such as to 0.6, such as to 0.7, such as to 0.8, such as to 0.9, such as to 1 % (w/v) of non-ionic detergents.

In one embodiment, the non-ionic detergent is selected from polysorbate 80, polysorbate 20 and poloxamer.

In one embodiment, the recombinant yeast-derived serum albumin preparations, and media (such as cryopreservation media, storage media, and other media) which comprise the recombinant yeast-derived serum albumin preparations, for use in accordance with any of the aspects of the present invention, may comprise up to 0.01, preferably up to 0.001, more preferably up to 0.0001 % (w/v) of non-ionic detergents such as but not limited to polysorbate 80, polysorbate 20 and poloxamer.

In one embodiment, the recombinant yeast-derived serum albumin preparations, and media (such as cryopreservation media, storage media, and other media) which comprise the recombinant yeast-derived serum albumin preparations, for use in accordance with any of the aspects of the present invention, may be essentially detergent free.

Fatty acids: Typically the recombinant yeast-derived serum albumin preparations, and media (such as cryopreservation media, storage media, and other media) which comprise the recombinant yeast-derived serum albumin preparations, for use in accordance with any of the aspects of the present invention, may comprise less than or equal to 25 mM fatty acids. The fatty acid can be any fatty acid such as saturated or unsaturated fatty acids as well as salts thereof. Preferably the fatty acid is one or more saturated fatty acids such as a fatty acid selected from the group consisting of propanoic acid, butanoic acid, pentanoic acid, hexanoic acid, heptanoic acid, octanoic acid, nonanoic acid, decanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, eicosanoic acid, heneicosanoic acid, docosanoic acid, tricosanoic acid, tetracosanoic acid, pentacosanoic acid, hexacosanoic acid, heptacosanoic acid, octacosanoic acid, nonacosanoic acid, triacontanoic acid, henatriacontanoic acid, dotriacontanoic acid, tritriacontanoic acid, tetratriacontanoic acid, pentatriacontanoic acid and hexatriacontanoic acid.

In one embodiment the recombinant yeast-derived serum albumin preparations, and media (such as cryopreservation media, storage media, and other media) which comprise the recombinant yeast-derived serum albumin preparations, for use in accordance with any of the aspects of the present invention, may comprise less than or equal to 25 mM fatty acids, such as less than or equal to 20 mM fatty acids, such as less than or equal to 15 mM fatty acids, such as less than or equal to 10 mM fatty acids, such as less than or equal to 5 mM fatty acids, such as less than or equal to 2 mM fatty acids, such as less than or equal to 1 mM fatty acids.

In one embodiment the recombinant yeast-derived serum albumin preparations, and media (such as cryopreservation media, storage media, and other media) which comprise the recombinant yeast-derived serum albumin preparations, for use in accordance with any of the aspects of the present invention, may comprise less than 25 mM fatty acids, such as less than 20 mM fatty acids, such as less than 15 mM fatty acids, such as less than 15 mM fatty acids, such as less than 14 mM fatty acids, such as less than 13 mM fatty acids, such as less than 12 mM fatty acids, such as less than 11 mM fatty acids, such as less than 10 mM fatty acids, such as less than 9 mM fatty acids, such as less than 8 mM fatty acids, such as less than 7 mM fatty acids, such as less than 6 mM fatty acids, such as less than 5 mM fatty acids, such as less than 4 mM fatty acids, such as less than 3 mM fatty acids, such as less than 2 mM fatty acids, such as less than 1 mM fatty acids, such as less than 0.5 mM fatty acids, such as less than 0.1 mM fatty acids, such as less than 0.05 mM fatty acids, such as less than 0.01 mM fatty acids, such as wherein the composition is essentially free of fatty acids.

In a preferred embodiment, the fatty acid is octanoate (octanoic acid). In one embodiment the recombinant yeast-derived serum albumin preparations, and media (such as cryopreservation media, storage media, and other media) which comprise the recombinant yeast-derived serum albumin preparations, for use in accordance with any of the aspects of the present invention, may comprise less than or equal to 25 mM octanoate, such as less than or equal to 20 mM octanoate, such as less than or equal to 15 mM octanoate, such as less than or equal to 10 mM octanoate, such as less than or equal to 5 mM octanoate, such as less than or equal to 2 mM octanoate, such as less than or equal to 1 mM octanoate.

In one embodiment the recombinant yeast-derived serum albumin preparations, and media (such as cryopreservation media, storage media, and other media) which comprise the recombinant yeast-derived serum albumin preparations, for use in accordance with any of the aspects of the present invention, may comprise less than 25 mM octanoate, such as less than 20 mM octanoate, such as less than 15 mM octanoate, such as less than 15 mM octanoate, such as less than 14 mM octanoate, such as less than 13 mM octanoate, such as less than 12 mM octanoate, such as less than 11 mM octanoate, such as less than 10 mM octanoate, such as less than 9 mM octanoate, such as less than 8 mM octanoate, such as less than 7 mM octanoate, such as less than 6 mM octanoate, such as less than 5 mM octanoate, such as less than 4 mM octanoate, such as less than 3 mM octanoate, such as less than 2 mM octanoate, such as less than 1 mM octanoate, such as less than 0.5 mM octanoate, such as less than 0.1 mM octanoate, such as less than 0.05 mM octanoate, such as less than 0.01 mM octanoate, such as wherein the composition is essentially free of octanoate.

For example, the recombinant yeast-derived serum albumin preparations, and media (such as cryopreservation media, storage media, and other media) which comprise the recombinant yeast-derived serum albumin preparations, for use in accordance with any of the aspects of the present invention, may comprise less than or equal to 25 mM octanoate or total fatty acids is less than or equal to 20 mM octanoate or total fatty acids, such as less than or equal to 15 mM octanoate or total fatty acids, such as less than or equal to 10 mM octanoate or total fatty acids, such as less than or equal to 5 mM octanoate or total fatty acids, such as less than or equal to 2.28 mM octanoate or total fatty acids, such as less than or equal to 2.16 mM octanoate or total fatty acids, such as less than or equal to 2 mM octanoate or total fatty acids, such as less than or equal to 1.52 mM octanoate or total fatty acids, such as less than or equal to 1.44 mM octanoate or total fatty acids, such as less than or equal to 1.2 mM octanoate or total fatty acids, such as less than or equal to 1 mM octanoate or total fatty acids, such as less than or equal to 800 uM octanoate or total fatty acids, such as less than or equal to 720 uM octanoate or total fatty acids, such as less than or equal to 456 uM octanoate or total fatty acids, such as less than or equal to 400 mM octanoate or total fatty acids, such as less than or equal to 304 mM octanoate or total fatty acids, such as less than or equal to 288 mM octanoate or total fatty acids, such as less than or equal to 240 uM octanoate or total fatty acids, such as less than or equal to 160 uM octanoate or total fatty acids, such as less than or equal to 152 uM octanoate or total fatty acids, such as less than or equal to 144 uM octanoate or total fatty acids, such as less than or equal to 80 uM octanoate or total fatty acids.

The recombinant yeast-derived serum albumin preparations, and media (such as cryopreservation media, storage media, and other media) which comprise the recombinant yeast-derived serum albumin preparations, for use in accordance with any of the aspects of the present invention, may comprise a molar ratio of octanoate to albumin that is less than or equal to 20:1, 19:1, 18:1, 17:1, 16:1, 15:1, 14:1, 13:1, 12:1, 11:1, 10:1, 9:1, 8:1, 7:1, 6:1, 5:1, 4:1, 3: 1, 2:1 or 1:1. A molar ratio of less than or equal to 16:1, 11:1 or 5:1 is preferred.

In one embodiment the recombinant yeast-derived serum albumin preparations, and media (such as cryopreservation media, storage media, and other media) which comprise the recombinant yeast-derived serum albumin preparations, for use in accordance with any of the aspects of the present invention, may comprise less than 25 mM hydrophobic molecules e.g. phospholipids, such as less than 20 mM hydrophobic molecules e.g. phospholipids, such as less than 15 mM hydrophobic molecules e.g. phospholipids, such as less than 15 mM hydrophobic molecules e.g. phospholipids, such as less than 14 mM hydrophobic molecules e.g. phospholipids, such as less than 13 mM hydrophobic molecules e.g. phospholipids, such as less than 12 mM hydrophobic molecules e.g. phospholipids, such as less than 11 mM hydrophobic molecules e.g. phospholipids, such as less than 10 mM hydrophobic molecules e.g. phospholipids, such as less than 9 mM hydrophobic molecules e.g. phospholipids, such as less than 8 mM hydrophobic molecules e.g. phospholipids, such as less than 7 mM hydrophobic molecules e.g. phospholipids, such as less than 6 mM hydrophobic molecules e.g. phospholipids, such as less than 5 mM hydrophobic molecules e.g. phospholipids, such as less than 4 mM hydrophobic molecules e.g. phospholipids, such as less than 3 mM hydrophobic molecules e.g. phospholipids, such as less than 2 mM hydrophobic molecules e.g. phospholipids, such as less than 1 mM hydrophobic molecules e.g. phospholipids, such as less than 0.5 mM hydrophobic molecules e.g. phospholipids, such as less than 0.1 mM hydrophobic molecules e.g. phospholipids, such as less than 0.05 mM hydrophobic molecules e.g. phospholipids, such as less than 0.01 mM hydrophobic molecules e.g. phospholipids, such as wherein the composition is essentially free of hydrophobic molecules e.g. phospholipids.

In one embodiment, the term hydrophobic molecules include fatty acids such as octanoate, but excludes detergents such as non-ionic detergents, such as polysorbate 80.

The recombinant yeast-derived serum albumin preparations, and media (such as cryopreservation media, storage media, and other media) which comprise the recombinant yeast-derived serum albumin preparations, for use in accordance with any of the aspects of the present invention, may include or exclude various amphiphilic compounds. For example, one type of amphiphilic compound may be included but exclude another.

Alternatively, the recombinant yeast-derived serum albumin preparations, and media (such as cryopreservation media, storage media, and other media) which comprise the recombinant yeast-derived serum albumin preparations, for use in accordance with any of the aspects of the present invention, may exclude essentially all amphiphilic compounds such as detergents, fatty acids and/or phospholipids.

In one embodiment the recombinant yeast-derived serum albumin preparations, and media (such as cryopreservation media, storage media, and other media) which comprise the recombinant yeast-derived serum albumin preparations, for use in accordance with any of the aspects of the present invention, may comprise less than or equal to 25 mM amphiphilic compounds.

In another embodiment the recombinant yeast-derived serum albumin preparations, and media (such as cryopreservation media, storage media, and other media) which comprise the recombinant yeast-derived serum albumin preparations, for use in accordance with any of the aspects of the present invention, may be essentially free from amphiphilic compounds.

Free amino acids: The recombinant yeast-derived serum albumin preparations, and media (such as cryopreservation media, storage media, and other media) which comprise the recombinant yeast-derived serum albumin preparations, for use in accordance with any of the aspects of the present invention, typically comprises less than 5 mM free amino acids such as less than 4 mM free amino acids, such as less than 3 mM free amino acids, such as less than 2 mM free amino acids, such as less than 1 mM free amino acids, such as less than 0.5, such as less than 0.1, such as less than 0.01, such as less than 0.005, such as less than 0.001 mM free amino acids or essentially no free amino acids.

Free amino acids may include one or more free amino acids, including natural amino acids selected from the group consisting of tryptophan, phenylalanine, tyrosine, glycine, alanine, valine, leucine, isoleucine, methionine, proline, serine, threonine, cysteine, asparagine, glutamine, aspartate, glutamate, lysine, arginine and histidine, or modified and non-natural amino acids. Any one of the amino acids of the composition of the present invention may be either an L-amino acid or a D-amino acid.

In one embodiment the recombinant yeast-derived serum albumin preparations, and media (such as cryopreservation media, storage media, and other media) which comprise the recombinant yeast-derived serum albumin preparations, for use in accordance with any of the aspects of the present invention, is essentially free from free amino acids.

The recombinant yeast-derived serum albumin preparations, and media (such as cryopreservation media, storage media, and other media) which comprise the recombinant yeast-derived serum albumin preparations, for use in accordance with any of the aspects of the present invention, typically comprises less than 5, such as less than 4, such as less than 3, such as less than 2, such as less than 1, such as less than 0.5, such as less than 0.1, such as less than 0.01, such as less than 0.005, such as less than 0.001 mM tryptophan or N-acetyl tryptophan, or essentially no tryptophan or N-acetyl tryptophan. Preferably it does not comprise free tryptophan or N-acetyl-tryptophan.

Salt: The recombinant yeast-derived serum albumin preparations, and media (such as cryopreservation media, storage media, and other media) which comprise the recombinant yeast-derived serum albumin preparations, for use in accordance with any of the aspects of the present invention, may include any suitable salt such as but not limited to bromide, chloride, fluoride, hydride, iodide, nitride, oxide, phosphide, sulfide, peroxide, borate, bromate, hypobromite, carbonate, hydrogen carbonate, bicarbonate, chlorate, perchlorate, chlorite, hypochlorite, chromate, iodate, nitrate, nitrite, phosphate, hydrogen phosphate, dihydrogen phosphate, phosphite, sulfate, thiosulfate, hydrogen sulfate, bisulfate, sulfite, hydrogen sulfite, bisulfite, acetate, formate, oxalate, hydrogen oxalate, bioxalate, hydrogen sulfide, bisulfide, telluride, amide, thiocyanate, muriate (HCl), succinate and maleate salts or any combination thereof. Alternatively salts of the composition of the present invention may also include derivatives from nontoxic inorganic acids such as hydrochloric, nitric, phosphoric, sulphuric, hydrobromic, hydriodic, hydrofluoric, phosphorous and the like, as well as the salts derived from nontoxic organic acids, such as aliphatic mono and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, tartrate, methanesulfonate, and the like.

Salts of the invention may include salts of metals, such as monovalent (e.g. Group 1) metals and divalent (e.g. Group 2 and transition element) metals, and salts of ammonium. Salts include NaCl, and KCl.

Metal ions: In a further embodiment, recombinant yeast-derived serum albumin preparations, and media (such as cryopreservation media, storage media, and other media) which comprise the recombinant yeast-derived serum albumin preparations, for use in accordance with any of the aspects of the present invention, may be essentially metal ion free, such as essentially free from $Zn^{2+}$, $Ca^{2+}$, $Mg^{2+}$, $Mn^{2+}$, $Fe^{2+}$, $Cu^{2+}$, $Co^{2+}$ and/or $Ni^{2+}$ ions.

Acid/Base considerations: The recombinant yeast-derived serum albumin preparations, and media (such as cryopreservation media, storage media, and other media) which comprise the recombinant yeast-derived serum albumin preparations, for use in accordance with any of the aspects of the present invention, may have a pH of between 4 and 9; such as between 4 and 8; such as between 4 and 7; such as between 5 and 8; such as between 6 and 8; preferably a pH of from 6.4 to 7.4; from 6.0 to 7.0; from 6.7 to 7.3 or between 6.5 and 7.5 such as wherein said composition has a pH of about 7.

Buffer: The recombinant yeast-derived serum albumin preparations, and media (such as cryopreservation media, storage media, and other media) which comprise the recombinant yeast-derived serum albumin preparations, for use in accordance with any of the aspects of the present invention, may comprise a buffer such as a citrate buffer, a phosphate buffer or a histidine buffer. Phosphate buffer or histidine buffer are preferred. The buffer concentration may be from about 10 to about 150 mM, such as from about 30 to about 150 mM, such as from about 10, 20, 30, 40, 50, 60, 70, 80, 90, 100, 110, 120, 130, or 140 to about 20, 30, 40, 50, 60, 70, 80, 90, 100, 110, 120, 130, 140 or about 150 mM.

The present invention is further described by the following examples that should not be construed as limiting the scope of the invention.

## Examples

### Example 1

Stem cells are cryopreserved prior to conditioning into their final formulation. Currently, a standard cryopreservation solution used consists of a combination of DMSO, at 10%, and a solution of human serum albumin (HSA, Albutein® from Grifols., S.A) at 2%. A subject of interest in the cell therapy field, particularly in the allogeneic context, is the search of new cryoprotective agents that improve the recovery of cells after freezing-thawing cycles and extends stability of the cell suspension.

The purpose of this study was to evaluate the potential use of two different recombinant albumins (AlbIX® and Recombumin® Alpha) developed by Novozymes A/S, and now available as commercial products from Albumedix Ltd., in combination with human Mesenchymal Stromal Cells (hMSC).

Albutein®, the albumin solution derived from human blood commercialized by Grifols, S.A. was used as the reference item.

TEST ITEMS:

| | |
|---|---|
| **Name** | **TEST 1: AlbIX®** |
| **Composition** | A) For stability assessment: cells were conditioned with Plasmalyte® + 2% (w/v) and 5% (w/v) AlbIX®.<br>B) For post-thawing stability assessment: cells were cryopreserved using two cryopreservation solutions with 2% (w/v) and 5% (w/v) AlbIX® concentration for 21 days. |

| | |
|---|---|
| **Name** | **TEST 2: Recombumin® Alpha** |
| **Composition** | A) For stability assessment: cells were conditioned with Plasmalyte® + 2% (w/v) and 5% (w/v) Recombumin® Alpha.<br>B) For post-thawing stability assessment: cells were cryopreserved using two cryopreservation solutions with 2% (w/v) and 5% (w/v) Recombumin® Alpha concentration for 21 days. |

REFEREMCE ITEM

| | |
|---|---|
| **Name** | **REFERENCE: Albutein®** |
| **Composition** | A) For stability assessment: cells were conditioned with Plasmalyte® + 2% (w/v) and 5% (w/v) Albutein®.<br>B) For post-thawing stability assessment: cells were cryopreserved using two cryopreservation solutions with 2% (w/v) and 5% (w/v) Albutein® concentration for 21 days. |

EXPERIMENTAL DESIGN:

The experimental phase of the study was performed as it is showed in Figure 4.

For cell expansion and conditioning, hMSC were thawed, seeded and expanded for 27 days first in a one-tiered CellStack and thereafter in a five-tiered CellStack. Cells were harvested and distributed in three 50mL Falcon tubes (filled with 52mL of cell suspension) and three 15mL Falcon tubes (filled with 13mL of cell suspension). After centrifuging the tubes, supernatants were removed by decantation, and cells were resuspended in Plasmalyte + each studied albumin to achieve a cell concentration of 15M/mL (as it is shown in Figure 4).

**FREEZING PROTOCOL:**

**Equipment**

- Laminar flow booth of class IIA
- System of tubes for non-programmed freezing
- Biological Ultrafreezer -80°C
- Cylinder of liquid nitrogen (if applicable)
- Automatic pipettor

- Centrifuge
- Pipettes of 1, 2, 5, 10, 25 and 50 mL
- Tubes of 15 and 50 mL
- Cryopreservation tubes of 1, 1.5 or 2 mL
- Chronometer
- Ice packs (replaceable with ice)

**Reagents**

- Plasmalyte®
- Albutein® human plasma albumin from Grifols., S.A 20%
- AlbIX® 10% and Recombumin® Alpha 20% developed by Novozymes Biopharma DK A/S and/or Delta Biotechnology Ltd., and now available as commercial products from Albumedix Ltd.,
- DMSO
- Isopropanol

**Procedure**

**1. Preparation of the cryopreservation solution:**

A. Prepare the required volume of cryopreservation solution, as shown in Fig 4. If the required volume is not known exactly, prepare an excess volume greater than is considered necessary (generally 5 or 10 mL). The following shows the volumes for preparing 10 mL of cryopreservation solution, as well as the final concentration of the reagents used: Volumes and concentration of the reagents used in the preparation of the cryopreservation solution:

| Reagent | Initial concentration (*Ci*) (stock reagent) | Final concentration (*Cf*) in the cryopreservation solution |
|---|---|---|
| **Plasmalyte** | 100% | NA |
| **Albumin** | 10% or 20% (w/v)) | 2% or 5% w/v |
| **DMSO** | 100% | 20% v/v |
| | | **Final Volume** (*Vf*): |

B. To find the initial volume required for each reagent it is recommended to perform these calculations using the following equation (Eq) 1:

$$C_i \times V_i = C_f \times V_f \rightarrow \text{Eq 1.}$$

C. The cryopreservation solution is stored in the range 2-8°C until the moment of use; as a maximum, it may be stored in the range 2-8°C for one day.

**2. Obtaining and preparing the cellular suspension:**

D. The mesenchymal cells to be frozen come from a cellular suspension of known volume and concentration and are collected in a correctly labelled sterile tube, by centrifugation of cultured cells, removal of supernatants, and resuspension in Plasmalyte + each studied albumin to achieve a cell concentration of 15M/mL (as it is shown in Figure 4).
E. The number of cells to freeze per cryotube, the number of cryotubes, the cellular concentration for freezing (Cc), and the volume per cryotube, are decided as a function of the amount of cells available, or the use for which they are intended.

F. Calculate the final volume for freezing (Vc) from equation 2:

$$Vc = \frac{Volume}{cryotube} \times number\ of\ crytotubes \rightarrow \text{Eq 2.}$$

G. The freezing process comprises 1:1 dilution of the cellular suspension (i.e. cells resuspended in Plasmalyte + each studied albumin) with the cryopreservation solution (i.e. Plasmalyte + each studied albumin + 20% w/v DMSO). For this reason, both the volume of cellular suspension to be prepared for freezing, and the volume of cryopreservation solution to be added, will be ½ of the volume calculated in Eq 2.

**3. Addition of the cryopreservation solution:**

H. Taking into account that the total volume of cryopreservation solution to add (Vca) is ½ the volume calculated in Eq 2, and that said volume corresponds exactly to the volume of cellular suspension to be frozen, calculate the percentage of cryopreservation solution to add in each step, to generate the freezing ramp shown in the following table.

Ramp for addition of cryopreservation solution:

| Step | Percentage of cryopreservation solution to add | Time (minutes) | Temperature (°C) |
|---|---|---|---|
| 1 | 10% of Vca | 2 | 2-8 |
| 2 | 10% of Vca | 2 | 2-8 |
| 3 | 10% of Vca | 2 | 2-8 |
| 4 | 20% of Vca | 2 | 2-8 |
| 5 | 25% of Vca | 2 | 2-8 |
| 6 | 25% of Vca | Up to max. 30 min | 2-8 |

I. Slowly add the cryopreservation solution (cooled beforehand) to the cellular suspension following the addition ramp specified in the 'ramp' scheme shown in the foregoing table. Gradual addition of the cryopreservation solution avoids possible cellular osmotic shock.

**4. Aliquoting of the cellular suspension:**

J. Aliquoting of the cellular suspension is a procedure that must be performed as quickly as possible, since DMSO is a component that is toxic for the cells. The aliquoting process begins once the volume of cryopreservation solution specified in step 6 of the 'ramp' scheme (shown in the foregoing table) has been added.

K. Distribute the final volume of the cellular sample in previously labelled cryopreservation tubes (according to the volume per cryotube stipulated in section E).

**5. Freezing**

L. Put the cryopreservation tubes with the cellular suspension in the system of tubes for non-programmed freezing (the "system"), which contains isopropanol at the correct level. Ideally, it is preferable for the system container to equilibrate at a temperature in the range 2-8°C before each use, although it may also be used if it has been equilibrated at room temperature.

M. Put the system container in the -80°C freezer in order to freeze it and keep it there at least overnight and for a maximum of one month.

N. Once the tubes have been removed from the system container, they are put in the corresponding box of the -80°C freezer or of the liquid nitrogen cylinder.

**THAWING PROTOCOL:**

**Equipment**

- Laminar flow booth of class IIA
- Thermostatic bath with distilled water or alternative heat source
- Automatic pipettor
- Incubator
- Centrifuge
- Chronometer

**Perishable material and reagents**

- Pipettes of 1, 2, 5, 10, 25 and 50 mL
- Falcon tubes of 15 and 50 mL
- Plasmalyte + each studied albumin
- Ice packs if applicable (if unavailable, ice may be used)

**Procedure**

**i. Preparation of the thawing solution**

A. Prepare the required volume of thawing solution, consisting of Plasmalyte + each studied albumin.
B. The thawing solution must be stored in the range 2-8°C until the moment of use.

**e.ii. Thawing**

C. Select the cryotube(s) to be thawed and withdrawn from the -80°C freezer or from the liquid nitrogen cylinder.
D. Transfer the frozen cryotubes to the thermostatic bath (or equivalent heat source), preheated to 37°C. It is important for thawing to be effected quickly. The cryotube must be withdrawn from the heat source at the precise moment that it is transformed to the liquid state (it may, however, be withdrawn while a very minor proportion is still in the solid, granular state).
E. Once the cellular suspension has thawed, transfer the contents of the cryotube to a 15-mL or 50-mL tube, as appropriate. If thawing more than one cryotube obtained for the same parent cellular suspension, it is recommended to combine all the thawed fractions, instead of carrying out the thawing process for each cryotube.
F. Dilute the cellular suspension 1:1 with the thawing solution, adding the same volume of thawing solution as the volume of cellular suspension recovered. The thawing solution is added slowly, following the strategy shown in the following table.

Strategy for adding the thawing solution:

| Step | Percentage (v/v) of thawing solution | Time (minutes) | Temperature (°C) |
|---|---|---|---|
| 1 | 25% of the volume to be added | 2 | 2-8 |
| 2 | 25% of the volume to be added | 2 | 2-8 |
| 3 | 50% of the volume to be added | 2 | 2-8 |

G. Dilute the cellular suspension with the thawing solution until the dilution is 1:10 of the initial volume of thawed suspension. An example of calculation is shown below:
*If the initial volume of thawed cellular suspension is 1 mL:*

*1 mL x10= 10 mL (final volume for 1:10 dilution)*

*As it will previously have been diluted with 1 mL of thawing solution (according to the instructions in section F), the volume to be added in this step will be:*

*10 mL-1 mL (initial volume of suspension) – 1 mL (volume of solution added for 1:1 dilution) = 8 mL*

*In this step, for an initial volume of 1 mL, it will be necessary to add* 8 *mL of thawing solution.*

H. By pipetting, determine the total volume of thawed solution and then homogenize the suspension thoroughly (to prevent appearance of bubbles, which may make it difficult to measure the volume).

**STABILITY TESTING:**

A total of 6 criovials were frozen following the freezing protocol discussed above, as it is shown in Figure 4 and stored for 21 days at -196°C in a liquid nitrogen tank. Then they were thawed following the thawing protocol discussed above, using the corresponding albumin solution in each case.

During the post-thawing cell stability at 2-8°C, each albumin was tested at two concentrations: 2% (w/v) and 5% (w/v).

To assess cell stability at 2-8°C, cell suspensions conditioned with 2% (w/v) of each albumin (AlbIX®, Recombumin® Alpha or Albutein®) were assessed. Three syringes were set up using in each case one of the cells suspensions to be proven as is shown in Figure 4. To achieve the desired cell concentration of 6.5M/mL, capped syringes were loaded through the top with 0.87 mL of the corresponding stock cell solution (15M/mL) and 1.13 mL of the analogous conditioning solution (Plasmalyte + 2% of corresponding albumin). Then the plunges were assembled and syringe's volumes were set at 2.8 mL (2mL liquid and 0.8 mL air) as it is shown in Figure 5. Finally, syringes were stored in a fridge during the test to maintain the target temperature.

**ANALYSIS OF THE RESULTS:**

NucleoView NC-3000™ software (Chemometec) was used for the interpretation and analysis of the NucleoCounter's (Chemometec) results of cell counting, cell viability and cell aggregation as well as to determine apoptosis in the cell population.

Microsoft® Excel 2007 (Microsoft Corporation) and GraphPad Prism v5 for Windows (GraphPad Software Inc.) were used for analysis of the results and plot generation.

**RESULTS AND DISCUSSION:**

The human albumin protein is widely used in the advanced therapy field. It guarantees the stability of cell-based products by protecting cells from the damages associated to the freezing-thawing cycles. Therefore, cell stability has to be proven to confirm the usefulness of different forms of albumin and their optimal working concentration. In this case, cell stability was assessed by means of cell counting, cell viability, cell aggregation and cell non-apoptotic, apoptotic and dead populations using the NucleoCounter® NC-3000™ and its manufacturer's protocols and instructions.

RESULTS OF STABILITY AT 2-8°C:

*(a) Stability without pre-treatment with freezing and thawing:*

hMSC cells that had been expanded, centrifuged, supernatants removed, and cells resuspended in Plasmalyte + each studied albumin to achieve a cell concentration of 15M/mL were tested, without freezing and thawing, for stability at 2-8°C. No significant differences among the different tested albumin solutions were observed in terms of cell concentration and cell viability (data not shown) when tested after 0 hours, 23.7 hours, 44 hours, 51.5 hours and 93.5 hours of storage. Cell concentrations remained constant and cell viability was greater than 97% until 52 hours.

The results obtained by the apoptotis assay over 44 hours (data not shown) identified two different cell populations in all cases: viable cells and dead cells. No apoptotic population was observed; therefore it is considered that cells became necrotic without previously suffering apoptosis. There were no significant differences among albumins tested; the same pattern was observed in all three cases according with the results obtained for cell concentration and cell viability.

*(b) Stability following pre-treatment with freezing and thawing:*

hMSC cells that had been expanded, centrifuged, supernatants removed, and cells resuspended in Plasmalyte + each studied albumin to achieve a cell concentration of 15M/mL were tested, following freezing and thawing, for stability at 2-8°C.

Samples were removed after 20 cycles of homogenization by inversion at the following timepoints: 0 hours, 23.5 hours, 45.5 hours and 72 hours.

As shown in Figure 6, the cells tested in the three albumins showed a progressive decrease of their viabilities over time, with the recombinant yeast-derived albumin preparations providing the greatest protection for the cells.

The data of Fig 6 can be summarised as follows:

| **Albumin** | **Conc'n** | **Parameter** | **0 hours** | **23.5 hours** | **45.5 hours** |
|---|---|---|---|---|---|
| AlbIX® | 2% | Apoptotic cells | 4.9% | 6.8% | 5.6% |
| | 2% | Viable cells | 92.6% | 90.3% | **92.9%** |
| Recombumin® Alpha | 2% | Apoptotic cells | 9.0% | 16.7% | 37.6% |
| | 2% | Viable cells | 87.3% | 81.2% | **59.1%** |
| Albutein® | 2% | Apoptotic cells | 14.6% | 12.2% | 56.0% |
| | 2% | Viable cells | 83.6% | 85.6% | **40.7%** |

| **Albumin** | **Conc'n** | **Parameter** | **0 hours** | **23.5 hours** | **45.5 hours** |
|---|---|---|---|---|---|
| AlbIX® | 5% | Apoptotic cells | 8.6% | 22.2% | 37.4% |
| | 5% | Viable cells | 88.5% | 76.3% | **63.2%** |
| Recombumin® Alpha | 5% | Apoptotic cells | 21.9% | 47.3% | 61.1% |
| | 5% | Viable cells | 76.6% | 51.1% | **34.5%** |
| Albutein® | 5% | Apoptotic cells | 17.4% | 33.4% | 79.9% |
| | 5% | Viable cells | 79.0% | 63.4% | **4.5%** |

The data show that:

- At 2% albumin, after 45.5 hours of storage at 2-8 °C, the level of viable cells was best for AlbIX® (92.9%), then for Recombumin® Alpha (59.1%), and worst for the plasma-derived Albutein® (40.7%).

- Although the viabilities were lower overall when using 5% albumin, the difference between the level of viable cells after 45.5 hours of storage at 2-8 °C was even more pronounced, with the best result for AlbIX® (63.2%), then for Recombumin® Alpha (34.5%), and again worst for the plasma-derived Albutein® (only 4.5%)

It is therefore concluded that the recombinant yeast-derived albumin preparations providing the greatest protection for the cells during storage, compared to plasma-derived albumin products, when used to protect cells during and/or after a physiological shock, such as freezing and thawing. It is noted that the differences are not necessarily apparent after 23.5 hours of storage at 2-8 °C. This is considered to be due to the fact that, after that short period of time, the cells have had inadequate time to react fully to the physiological shock of freezing and thawing.

It is also concluded that a 2% albumin concentration is preferable to a 5% albumin concentration to maximise cell viability during storage, after a physiological shock, such as freezing and thawing.

**Example 2**

Further testing was conducted to confirm the beneficial effect of AlbIX® as an additive for the cryopreservation of hMSC, and the comparison with Albutein® (a commercial plasma-derived albumin solution manufactured by Grifols S.A.).

A comprehensive analysis was done in different cell cultures from different donors expanded using two strategies: the standard, 10% hSERB (human Sera B), and the exploratory, 5%PL (Platelet Lysate) and evaluating the efficiency of the thawing-freezing process and the stability of the cell suspension, in terms of viability, identity and multipotentiality.

Stem cells were cryopreserved prior to conditioning into their final formulation. A standard cryopreservation solution used consisted of a combination of DMSO, at 10%, and a solution of human serum albumin (HSA, Albutein® from Grifols., S.A) at 2%.

REAGENTS AND SOLUTIONS.:

Critical reagents and solutions used in the experimental phases of the study are listed below:

- AlbIX® (Albumedix Ltd, batch:AK190201)
- Albutein® (Grifols S.A., batch:MPAB5HA001)
- Plasmalyte® + 2% w/v Albutein®
- Plasmalyte® + 2% w/v AlbIX®

REFERENCE ITEM (RI):

| | |
|---|---|
| **Name** | **HSA-MSC (Albutein®)** |
| **Composition** | *Ex vivo* expanded BM-hMSC cryopreserved in a Plasmalyte® at 2% Albutein® w/v and 10% v/v DMSO solution. |
| **Presentation** | 2 Cryovials for each cell batch at 10%hSERB expansion; 1 Cryovial for each cell batch at 5%PL expansion. |
| **Acceptance criteria** | Cryovial cell concentration: $7.5x10^6$ viable hMSC/mL ± 20% ($6.0x10^6$ - $9.0x10^6$ viable hMSC/mL).<br><br>10%hSERB (human Sera B):<br>XCC14040: $7.50x10^6$ viable hMSC/mL<br>XCO15048: $7.10x10^6$ viable hMSC/mL<br>XCO13008: $7.50x10^6$ viable hMSC/mL<br><br>5%PL (Platelet Lysate):<br>XCC14040: $8.01x10^6$ viable hMSC/mL<br>XCO15048: $7.50x10^6$ viable hMSC/mL<br>XCO15054: $8.65x10^6$ viable hMSC/mL |

TEST ITEM (TI)

| | |
|---|---|
| **Name** | **AlbIX®-MSC** |
| **Composition** | *Ex vivo* expanded BM-hMSC cryopreserved in a Plasmalyte® at 2% AlbIX w/v and 10% v/v DMSO solution. |
| **Presentation** | 2 Cryovials for each cell batch at 10%hSERB expansion; 1 Cryovial for each cell batch at 5%PL expansion. |

(continued)

| Name | AlbIX®-MSC |
|---|---|
| Acceptance criteria | Cryovial cell concentration: $7.5x10^6$ viable hMSC/mL ± 20% ($6.0x10^6$ - $9.0x10^6$ viable hMSC/mL).<br><br>10%hSERB (human Sera B):<br>XCC14040: $7.50x10^6$ viable hMSC/mL<br>XCO15048: $7.10x10^6$ viable hMSC/mL<br>XCO13008: $7.50x10^6$ viable hMSC/mL<br><br>5%PL (Platelet Lysate):<br>XCC14040: $8.30x10^6$ viable hMSC/mL<br>XCO15048: $7.50x10^6$ viable hMSC/mL<br>XCO15054: $7.76x10^6$ viable hMSC/mL |

EXPERIMENTAL DESIGN:

**Cell Expansion**

Four different primary hMSC cell culture test systems were thawed, seeded, and expanded, to produce sufficient number of cells to perform the experiments for the evaluation of AlbIX® and its comparison to Albutein®.

Six different expansions were performed following different strategies. For the first one, the primary cell cultures named XCC14040, XCO15048 and XCO13008 were cultured using the standard media formulation: DMEM+10% human Sera B (hSERB); whereas for the second one, the primary cell cultures named XCC14040, XCO15048, XCO15054 were cultured with DMEM+5% Platelet Lysate (PL).

The kinetic parameters obtained during the expansion of the different primary cell cultures, with the different conditions: 10%hSERB, and 5% PL, were evaluated and are shown in the following table:

| Batch id | Passage | CPD | Doubling time (days)* |
|---|---|---|---|
| **10% hSerB** | | | |
| XCC14040 | 02 | 9.8 | 2.3 |
| XCO15048 | 03 | 14.9 | 3.1 |
| XCO13008 | 05 | 21.6 | 5.3 |
| **5% PL** | | | |
| XCC14040 | 03 | 6.1 | 3.6 |
| XCO15048 | 01 | 9.9 | 9.1 |
| XCO15054 | 01 | 9.5 | 2.8 |
| *Doubling time for the last sub-culturing step before cryopreservation. | | | |

The foregoing table shows the kinetic features of the cell lines used during the study (CPD: Cumulative Populations Doublings). The CPDs were calculated taking into account that the cells had already been expanded prior to current study. Therefore, a standard factor of 3 for each anterior sub-culturing step or passage was added to the calculated CPD.

The initial passage considered for these hMSC was -02, which is the starting point in the isolation of hMSC from bone marrow (BM) samples. For all the expansions the CPDs were lower than the Hayflick limit of 50 CPDs.

In the comparison of the primary cell cultures that are common for both culture strategies, XCC14040 and XCO15048, differences were detected in the doubling time, showing that the growth rate was slower for the 5%PL and then considering this as a sub-optimal culture condition for the cells. The information obtained in this less favourable culture condition contributed to a better comparison between the cryoprotective agents.

Periodical visual inspections with an inverted microscope were performed during the cell expansion in order to rule out the presence of bacterial contamination or changes in cell morphology. In the case of the PL condition of expansion, a complementary analysis of *Mycoplasma* detection was performed, showing a negative result and discarding this as a cause of the slower growth (data not shown).

**Cell Freezing & Thawing**

Cells were trypsinized once confluence was reached (about 70%), and cell counting, viability and phenotype was determined by flow cytometric techniques prior to freezing. Taking into consideration the number of viable cells determined in the cytometric analyses, cells from the different expansions were split into two different parts, were centrifuged, media supernatants were discarded, and cellular pellets of each part were reconstituted at a concentration of $15x10^6$ viable hMSC/mL $\pm$ 20%. The solutions for pellet reconstitution were: Plasmalyte® at 2% w/v Albutein®, for the RI condition, and Plasmalyte® at 2% w/v AlbIX®, for TI condition.

These cellular suspensions were frozen following the procedure and steps detailed in the Freezing Protocol given in Example 1, adding, during the cooling rate, specific cryopreservation solutions. The final concentration achieved in each cryovial was $7.5x10^6$ viable hMSC/mL $\pm$ 20%, and the composition of the final cryopreserved cell suspension was 10%v/v DMSO and 2% w/v Albutein® in Plasmalyte, for RI, and 10% v/v DMSO and 2% w/v AlbIX® in Plasmalyte, for TI.

After a minimum period of 7 days, cells were thawed following the Thawing Protocol given in Example 1 but using different specific thawing solutions: Albutein® 2% w/v in Plasmalyte® for RI, and AlbIX®2%w/v in Plasmalyte for TI.

The study of the albumin solutions was divided in two parts: the cryopreservation process and the stability.

**Cryopreservation Protection**

For the evaluation of the cryoprotective effect that the albumin solutions could provide to hMSC, cytometric analysis were repeated after thawing and were compared to the pre-freezing results. In the case of the cells cultured with PL, which is the complementary assessment, only cell counting and viability were performed.

The post-cryopreservation flow cytometric analyses performed constituted the time 0 of the stability assessment.

**Stability Assessment: Cell Viability and Cell Identity**

hMSC suspension were packaged in 10 or 20 mL syringes, for cells expanded with PL or hSERB respectively, maintaining a 0.4 ratio of air volume vs liquid. Samples were stored at 2-8°C and diverse evaluations were performed at different time-points: 0h, 24h 48h and 72h depending on the culture strategy used.

<u>PL expanded cultures.</u>

Stability assessment for cells expanded with PL consisted in viability determinations along the mentioned time-points using two different methodologies:

- Flow cytometry: The viability determinations performed considered both necrotic and apoptotic cells using 7AAD and Annexin V markers respectively.

- NC3000™ NucleoCounter (Chemometec) technology based on fluorescent cell analysis through multiple image acquisition: Two different protocols were followed, the first one or the standard allows the determination of cell concentration, aggregation, and viability; the second protocol was used for a more complete study of the apoptotic process given that early and late apoptotic cells were measured using Annexin V binding and PI inclusion.

<u>hSERB expanded cultures:</u>

Stability assessment for cells expanded with hSERB consisted on the evaluation not only of the viability but also the identity. For the viability determinations, in the same way as for the culture expanded with PL, two different methods were performed:

- Flow cytometry at 0h, 24h, 48h and 72h.

- NuceloCounter determinations with the standard procedure that only involves the cell counting, aggregation and viability at 0h, 24h and 72h.

Identity determinations were also evaluated by flow cytometry, at time points: 0h, 24h and 72h. The surface marker expression considered was adapted from the ISCT (International Society for Cellular Therapy) and acceptance criteria was set also according to experience: at least 95.0 for CD105, CD73 and CD90 antigens; not greater than 5% for CD45 and CD31; and not greater than 20% for HLA-DR.

**Stability Assessment: Multipotency**

Cell potency was only evaluated for the samples obtained from cell cultures expanded with 10% hSerB supplemented-media, and at the time points: 0h, 24h and 72h. The ability of the cells to differentiate into the three different lineages (adipogenic, osteogenic and chondrogenic) was assessed.

Two 48 well-format plates, one for the basal condition and another for the differentiated were seeded for each staining protocol performed. The basal condition was stained 0-2 days after seeding, whereas the differentiated one was stained after culturing cells in the specific differentiation media, under standard culture conditions (37°C, 5% $CO_2$ and 95% of Relative Humidity (RH)), and media was replaced every 3-4 days.

For both osteogenic and adipogenic differentiation assays, the time that cells were exposed to the differentiation stimuli and the cell densities used, were 1-4 weeks and $3x10^3$-$1x10^5$ cells/$cm^2$.

In the case of chondrogenic differentiation, the seeding density employed was $8.0x10^4 \pm 12.5\%$ cells/micromass at 72 h and for some cell cultures due to the low number of viable cells it was not possible.

ANALYSIS OF THE RESULTS:

Microsoft Excel 2007 (Microsoft Corporation) and GraphPad Prism v6.01 for Windows (GraphPad Software Inc.) were used for analysis of the results and plot generation.

NucleoView NC-3000™ software (Chemometec) was used for the interpretation and analysis of the Nucelo-Counter's (Chemometec) results of cell counting, cell viability and cell aggregation as well as to determine apoptosis in the cell population.

BD Cell Quest v5.2.1 was used for cytometric analyses of cell counting and cell identity evaluation.

RESULTS AND DISCUSSION:

**Cryopreservation Protection Of AlbIX® Vs Albutein®**

The cryoprotective effect of the albumin solutions studied, AlbIX® (TI) and Albutein® (RI), on hMSC was evaluated in terms of viability and identity by flow cytometry. Viability assessment was performed in both culture expansion strategies, hSERB and PL, whereas identity only was evaluated for hSERB expansion.

The % of viability at pre-freeezing and immediately post-thawing in each condition, Albutein® or AlbIX®, for each primary cell culture and expansion strategy used was assessed.

The variability provided by the primary cell cultures studied and expansion strategies used was reduced after data normalization given that only the viability reduction was considered. The results after this normalization (data not shown) showed that there were no significant statistical differences between both cryopreservation solutions (Unpaired t-Test statistical analysis used). Taken together all the previous results confirmed that, *just after thawing*, the cryoprotective effect of both additives evaluated Albutein® and AlbIX® is comparable.

**Phenotype Pre Vs Post Freezing**

The identity of hMSC was compared before freezing and after thawing checking the % of expression of the different surface markers considered (see Figure 7).

For all the conditions explored, pre-freezing and post-thawing with Albutein® or AlbIX® cryopreservation protocols, the currently accepted criteria for the identity of hMSC was met: at least 95.0 for CD105, CD73 and CD90 antigens; not greater than 5% for CD45 and CD31; and not greater than 20% for HLA-DR. Moreover, statistical analysis performed (ANOVA Tukey's multiple comparison test used) did not find any statistical significant differences between the cryoprotective agents evaluated.

Therefore, the cryopreservation process did not alter the identity of surface markers and either AlbIX® or Albutein® solutions maintained the original phenotype of hMSC.

**Stability Effect Of Albix Vs Albutein**

The stabilization effect of the albumin solutions studied, AlbIX® (TI) and Albutein® (RI), on hMSC after cryopreservation was evaluated in terms of viability, identity and multipotency.

(a) Viability:

The viability was evaluated by flow cytometry. For both, hSERB and PL culture expansion strategies, viability was evaluated at the different time points 0, 24, 48 and 72h, with a double staining protocol that considered both necrotic (7AAD) and apoptotic cells (Annexin V).

In Figure 8A (for hSERB expanded cells), and Figure 8B (for PL expanded cells), the % of viability along the different time points and cryopreservation conditions, AlbIX® and Albutien® are shown.

At time t= 0h, directly after thawing, all the cells were compliant with release criteria (Viability =70%) independently of the cell culture strategy followed (hSERB or PL) and the cryopreservation condition (AlbIX® or Albutein®).

For hSerB cell culture strategy and AlbIX condition (Figure 8A), all the cells showed viabilities higher than 70% along the different time-points except for XCO13008 that became out of specification (OOS) from t=24h. This fact might be consequence to an incident that occurring during thawing in which the cells were exposed longer to DMSO, and this may have resulted in lower viability already at t=0h. For Albutein® condition, from t=24h to the end of the stability assessment two primary cell cultures out of three were OOS, and at 72h all the primary cell cultures were non-compliant. Statistical analyses (ANOVA, Tukey multiple comparison test) showed that the decrease in viability was significant for Albutein® between time-points 0h and 72h ($p<0.05$), whereas for AlbIX® condition the reduction in viability was not statistically significant. Values of viability reduction and the results of statistical analysis demonstrate an extended stability for the AlbIX® condition in the cells expanded with hSERB.

For the sub-optimal cell culture strategy with PL (Figure 8B), AlbIX® condition also showed better results than Albutein® but in this case results were more obvious. From 24h to 72h all the cells cryopreserved with Albutein® were OOS, whereas for AlbIX® treatment viability became lower than 70% for only one primary cell culture and not until the last time point. Statistical analyses, (ANOVA, Tukey multiple comparison test) showed similar results than those obtained for hSERB expanded cells: non-significant differences for AlbIX® condition along stability time-points, whereas Albutein® showed differences at 24, 48 and 72h ($p<0.05$). These results are in accordance with the cells expanded with hSERB and strengthened the conclusion that AlbIX® treatment provides a better performance than Albutein® treatment.

The data combining both cell culture strategies for the comparison of the cryopreservation studies (not shown) demonstrated that the mean viability values from 24 to 72 h were OOS for the Albutein® solution, whereas the results were compliant with the specification along all of the stability time points studies for the AlbIX® solution.

In order to better compare the %viability for AlbIX® and Albutein® treatment conditions, the variability observed in the different primary cell lines and expansion strategies was normalized. The normalization consisted on considering the T=0 of the stability as the 100% of viability and calculating the percentage of reduction along time with respect to this value. Graphical representations of these percentages of viability reduction are shown in Figure 8C and 8D.

The viability reduction after data normalization was always higher for Albutein® than for AlbIX™ condition in all the cell lines and culture strategies tested. Reductions in viability were progressively increasing along time achieving values of 60-80% at 72h for PL cells or 45-85% for hSERB cells. Statistical analysis (ANOVA non parametric Sidak's multiple comparison test used) were performed in the comparison between cryopreservation conditions and resulted in the detection of significant differences at 72h ($p<0.05$) for the hSERB culture strategy, and a t=24 ($p< 0.05$), 48 and 72h ($p<0.001$) for the PL culture expansion strategy.

Altogether, these results corroborated the previous conclusion and confirmed that AlbIX™ improves post-thaw product stability regarding viability compared to Albutein®.

**Phenotype**

The identity of hMSC was compared along the different stability time points (0, 24 and 72h) for the cells lines expanded with hSERB checking the % of expression of the different surface markers considered (see Figure 11).

For the time-points 0 and 24h, and for Albutein® or AlbIX® cryopreservation protocols, the currently accepted criteria for identifying hMSC were met: at least 95.0 for CD105, CD73 and CD90 antigens; not greater than 5% for CD45 and CD31; and not greater than 20% for HLA-DR. In the case of 72h time-point, the primary cell cultures XCO13008, for both cryopreservation conditions studied Albutein® and AlbIX®, was OOS for the positive surface markers: CD105, CD73 and CD90. The expression of the mentioned antigens was below the accepted limit of at least 95.0. This result was consequence of the low viability of this primary cell culture: 10%. Indeed, when cells are dead, they release a number of intracellular proteins or components in the environment which might be bound by the monoclonal antibody conjugates, potentially leading to erroneous conclusions, particularly when cell frequencies are low as was this case.

Statistical analysis performed (ANOVA Tukey's multiple comparison test was used) did not reveal any significant differences between the cryoprotective agents evaluated for the stability assessment. The stability of the cell suspension after cryopreservation was equivalent for AlbIX® and Albutein® solutions *in terms of identity.*

**Multipotency Protection of AlbIX® Vs Albutein®**

The multipotency of hMSC, in other words their ability to differentiate into different mesodermal lineages (adipogenic, osteogenic and chondrogenic) was studied after thawing and along the different stability time points (0, 24 and 72h) for the primary cell cultures expanded with hSERB and cryopreserved with Albutein® or AlbIX® conditions.

The data obtained (not shown) indicated that the multipotentiality of hMSC was stable after cryopreservation and during 72h post-thaw for both AlbIX® and Albutein® conditions. Lack of differentiation potential was detected in some conditions and this was associated to low number of viable cells or cell culture dependent but not related to the cryopreservation solution used.

CONCLUSIONS:

**Cryoprotective Effect**

No significant differences between the cryopreservation solutions AlbIX® (TI) and Albutein® (RI) were detected in terms of viability and identity when the pre-freezing and immediate post-thawing cell suspensions were compared.

**Stability After Cryopreservation Effect**

After thawing and along the stability time-points assessed, AlbIX® ensured the best cell viability results:

- Cell culture expanded with hSERB showed OOS (viability =70%) at 48H for Albutein®, whereas the first OOS were detected at 72H for AlbIX®. Significant statistical differences were only detected for Albutein® in the comparison of t=0 with respect to t=72h.

- Primary cell culture expanded with the sub-optimal condition PL strengthened the better performance of AlbIX® in respect to Albutein®. Viability OOS were already detected at 24h for Albutein®, whereas the first OOS were detected at 72h for AlbIX®. Significant statistical differences were detected at 24h for Albutein® in the comparison to the time 0, and were not detected for AlbIX®.

- After data normalization, the percentage in viability reduction confirmed previous data: lower reductions were detected in AlbIX® condition, and statistically significant differences were observed when it was compared with Albutein® at time 72h, for the hSERB expanded cells, and for all the time-points studied 24, 48 and 72h, for PL expanded cells.

Regarding identity and multipotency of hMSC, the solutions AlbIX® and Albutein® showed comparable results along the different time-points of study maintaining the result obtained at time 0.

**Example 3**

The Albutein®-treated (control) and AlbIX®-treated cells of Example 2 were assessed for early and late stage apoptosis following post-thawing storage for time points T=0, 24, 48 and 72 hours after thawing, when stored in the relevant storage media at 2-8°C. Early and late stage apoptosis were assessed using an NC3000™ NucleoCounter. The results are shown in Figure 12.

Figure 12A shows that AlbIX® is effective in reducing the number of cells entering late stage apoptosis following storage for periods longer than 24 hours, showing clear differences at 48 and 72 hours.

Figure 12B shows that AlbIX® is effective in increasing the number of cells retained in early stage apoptosis at all time points assessed.

The apoptotic study may explain AlbIX®'s mode of action by preventing cells to progress into late apoptosis compared to control.

**Example 4**

Previous examples demonstrated the beneficial effect of the tested yeast-derived recombinant serum albumin, AlbIX®, when present in both the cryopreservation media and the storage media, on stem cells during a cryopreservation freeze/thawing cycle.

In this example, the role of the yeast-derived recombinant serum albumin was further studied to determine whether it exerts its beneficial effects as a stabilizer after thawing, or as a cryoprotector during freezing, or both.

To do so, four different conditions were set up. Three test items employed a representative yeast-derived recombinant serum albumin (AlbIX®), that was either used as cryoprotector only, as stabilizer only, or as both cryoprotector and stabilizer. The fourth condition, provided as a reference item (RI), was the use of Albutein® an albumin solution derived from human blood and commercialised by Grifols, in which the Albutein® was included as both a cryoprotector and a stabilizer.

This can be further summarised as follows:

| **Sample** | **Cryopreservation Solution** | **Post-Thaw Storage Solution** |
|---|---|---|
| Reference Item (**RI**) | Plasmalyte®, 2% **Albutein®** and 10% DMSO solution | Plasmalyte® and 2% **Albutein®** |
| Test Item 1 (**TI1**) | Plasmalyte®, 2% **AlbIX®** and 10% DMSO solution | Plasmalyte® and 2% **Albutein®** |
| Test Item 2 (**TI2**) | Plasmalyte®, 2% **Albutein®** and 10% DMSO solution | Plasmalyte® and 2% **AlbIX®** |
| Test Item 3 (**TI3**) | Plasmalyte®, 2% **AlbIX®** and 10% DMSO solution | Plasmalyte® and 2% **AlbIX®** |

Viability was evaluated prefreezing, immediately post-thawing, and during a stability study, as recommended by GMP regulations when performing manufacturing changes. An apoptosis study was also performed to further investigate a potential mechanism of action of the yeast-derived recombinant serum albumin.

**MSC cultures:**

Clinical grade MSC derived from bone marrow (BM) were produced following a Good Manufacturing Practice (GMP)-compliant bioprocess with appropriate donor informed consent, as described elsewhere (Codinach et al., 2016, Cytotherapy, 18(9):1197-1208). MSC were further expanded *in vitro* by using "expansion medium", which is composed of Dulbecco's Modified Eagle's Medium (DMEM; Gibco, Grand Island, New York; USA) containing 2 mM glutamine and supplemented with 10% (v/v) human Serum B (hSerB; Banc de Sang i Teixits, Barcelona, Spain). All cultures were maintained at 37 °C and 5% $CO_2$ in humidified incubators and whole media replacement was performed every 3-4 days.

**Freezing/Post thawing formulation:**

Following large scale expansion, cells were cryopreserved in a solution composed of Dulbecco's Phosphate-Buffered Saline (DPBS, Gibco, Grand Island, New York; USA) supplemented with 10% (v/v) dimethyl sulfoxide (DMSO; OriGen Biomedical, Austin, Texas, USA) and 2% (w/v) of Albutein® or AlbIX®. Depending on the conditions, cells were either thawed with solution of 2% Albutein® or AlbIX® (w/v) in Plasmalyte® (Baxter). Cells were then conditioned in 10 mL syringe to reproduce the clinical dose and stored at 2-8°C for the stability study.

**Stability assessment:**

Cell concentration and viability were evaluated with two techniques: flow cytometry and with an automated cell counter the NucleoCounter™ 3000™ (ChemoMetec, Allerod, Denmark). Cell concentration was evaluated by flow cytometry using Perfect-Count Microsphere™ (Cytognos). Nucleocounter viability assay and cell counting were performed according to the manufacturer's instructions. The percentage of cell viability was determined with flow cytometry by a double staining using 7-aminoactinomycin D (7AAD; BD Biosciences, San Diego, California, USA) and ANNEXIN-V. While 7AAD binds specifically to DNA when the cell membrane is damaged by necrotic processes, ANNEXIN-V binds to the phosphatidylserine which is normally expressed in the intra leaflet of the plasma membrane and is translocated externally during the early stage of the apoptosis.

**Apoptosis assay:**

The percentage of early and late apoptotic cells was evaluated using an automated cell counter the NucleoCounter NC-3000TM (ChemoMetec, Allerod, Denmark) as described in the manufacturer's instructions. Briefly, cells were stained with Hoescht 33342 to select the total cell population, ANNEXIN-V binding was used for specific staining of early apoptotic cells and finally cells were incubated with PI to quantify necrotic cells. NucleoView NC3000 software (ChemoMetec A/S, Allerod, Denmark) was used to analyse and interpret results from the NucleoCounter™.

**References:**

Application note No. 3017 Rev. 1.4 entitled "Annexin V Assay using the NucleoCounter® NC-3000™ System", published by ChemoMetec A/S, of Gydevang 43, DK-3450 Allerod, Denmark, and available online, for example, at https://chemometec.com/wp-content/uploads/2015/04/994-3017-Annexin-V-Assay.pdf.

**Results:**

*Viability analysis:*

Pre-freezing and post freezing viability was compared for all the items with two devices: flow cytometer and Nucleocounter.

Results from both techniques are shown in the **Fig 13**. Similar trends were observed with both devices. Immediately after thawing, all the test items (TI1, TI2 & TI3) met the acceptance criteria (viability> 70 %) while 2 out of three of the reference items (RI) were already out of specification. This demonstrates that all the test items with AlbIX® showed better viability immediately post thawing, compared to the reference item (RI) which used Albutein® in both the cryopreservation and storage media.

It is notable that the percentage viability of TI3, as determined by nucleocounter technique, is not significantly different to the level of viability in the pre-freezing sample, indicating a particularly high degree of cell protection when yeast-derived recombinant serum albumin, such as AlbIX®, is used in both the cryopreservation and storage media.

These data indicate that yeast-derived recombinant serum albumin, such as AlbIX®, improves post-thawing viability. The beneficial effect was observed when the yeast-derived recombinant serum albumin was included in the cryopreservation medium alone, the storage medium alone, or in both for the maximal effect.

It is interesting to note the measurable difference in viability immediately after thawing (t=0hrs) observed in the data in Fig 13. In contrast, in Example 2, we reported that viability immediately after thawing was comparable between the use of Albutein® and AlbIX® in the cryopreservation and storage media. Without being bound by theory, it is believed that this difference may be attributable to differences in the expansion strategies used to provide the cells for testing in Examples 2 and 4. The use of optimal media and conditions for cell expansion, as in Example 2, appears to lead to equivalence in detected viability immediately after thawing (although differences became apparent after longer storage periods, such as 24 hours or greater), whereas cells expanded in sub-optimal and/or stressed conditions prior to cryopreservation appear to demonstrate more notable differences in viability immediately after thawing, depending on the use of AlbIX® or Albuetin®.

Viability was also assessed over a longer period of post-thaw storage at 2-8 °C, from immediately after thawing to 72 hours. The results are provided in **Fig 14**.

The results in Fig 13 and 14 indicate that yeast-derived recombinant serum albumin provided the greatest beneficial protective effect when included in both the cryopreservation medium and the storage medium, although a beneficial protective effect was also observed when the yeast-derived recombinant serum albumin was included in either the cryopreservation medium alone, or the storage medium alone, compared to the reference item.

A direct comparison of RI and TI2 shows the benefit of providing yeast-derived recombinant serum albumin solely in the storage medium. Viability was evaluated at the following time point after thawing: 0h, 24h, 48h and 72h with a double staining protocol that considered both necrotic (7AAD) and apoptotic cells (ANNEXIN V) for the flow cytometry. A triple staining was used for the nucleocounter which considered also the necrotic cells (PI) and apoptotic cells (ANNEXIN V). While flow cytometry gated the cells according to their size and morphology, the nucleocounter selected the cells with a third staining Hoechst. Results are shown in **Fig 15**.

The results show that, after thawing, only one cell line out of three from the RI sample met the routine acceptance criteria (viability >70%). In contrast, all the cell lines from TI2 showed a viability higher than 70%. TI2 thawed and conditioned with AlbIX® showed higher percentage of viability at all tested time points.

Data for RI and TI2 were normalized, to evaluate the variation of viability over time without being affected by the initial % of viability observed immediately post-thawing, at t=0h. For the purposes of this test, the time point 0h was considered as the time point with 100% of viability for each sample, and viability reduction from this time point was evaluated with respect to this value. Data were plotted for each cell line as shown in **Fig 16**.

Similar trends in viability reductions were observed with both flow cytometry and nucleocounter. For both devices, a significant difference in the normalised viability reduction was observed from t=48h, for which viability reduction was lower for TI2 thawed with AlbIX® than RI thawed with Albutein™. While viability reduction for the reference item was already significant at t= 24h, viability reduction became significant from t=48h (data not shown).

The data show that yeast-derived recombinant serum albumin, such as AlbIX, significantly increased the stability of the product compared to plasma derived albumin, such as Albutein®, by diminishing viability reduction occurring at 2-8°C.

Additional testing was conducted to determine whether the beneficial effects of yeast-derived recombinant serum albumin, such as AlbIX®, in the storage medium are modified by additionally including the yeast-derived recombinant serum albumin in the cryopreservation medium. Results of an apoptosis assay comparing TI1 and TI3 are provided in **Fig 17**.

As noted above, TI1 is characterised by using AlbIX® in the cryopreservation medium but Albutein™ in the storage medium; whereas TI3 is characterised by using AlbIX® in both of the cryopreservation medium and the storage medium.

The results show that TI3 had higher viability along all of the time points. After data normalization (not shown), the data indicated that viability reduction was lower for TI3 than for TI1 at each time point. These results demonstrate the beneficial effects of yeast-derived recombinant serum albumin, such as AlbIX®, in the storage medium. The beneficial effect is not limited solely to its inclusion in the cryopreservation medium, and nor is it dependent on the specific identity of the cryopreservation medium (although the best results were obtained with AlbIX® in both the cryopreservation and the storage media).

A comparison of RI and TI3 shows directly the benefit of including recombinant serum albumin, such as AlbIX®, in both the cryopreservation medium and the storage medium (TI3), compared to the use of Albutein™ in both media (RI). Results are shown in **Fig 18**, which indicates that viability was significantly higher in the stability study for the TI3, at every time point. These differences tended to increase over longer periods of post-thaw storage.

Following normalization of the data comparing RI and TI3 (data not shown), it was observed that viability reduction at each time point was greater for RI than for TI3, the latter of which appeared to be the best condition with the lowest viability reduction over time compared not only to the RI but the other test items assessed.

It is concluded that yeast-derived recombinant serum albumin, such as AlbIX®, improves the stability of the product. Beneficial effects can be obtained when the yeast-derived recombinant serum albumin is included in the cryopreservation medium alone, the storage medium alone, or in both.

*Apoptotic analysis:*

Results of the comparison of RI and TI2 samples by the apoptosis assay are provided in **Fig 19A.** From t= 24h, a clear significant difference was observed between the two items (ANOVA Sidak's multiple comparison test). After data normalization, from t= 0h to 72h, a significant decrease of early apoptotic cells was observed for both items, indicating a general process of switching from early to late apoptosis during the storage period. However, there was a clear difference between RI and TI2 at t=24 and t=48h. The data show a reduction in the switch to late apoptosis for TI2, compared to the RI sample, supporting a conclusion that yeast-derived recombinant serum albumin, such as AlbIX®, can slow down the process of the cells entering late apoptosis.

From these results, we conclude that yeast-derived recombinant serum albumin, such as AlbIX®, supports the maintenance of cryopreserved stem cells in post-thawing storage in the early apoptotic state, reduces switching to late stage apoptosis, and increases the stability of the cell products when it is used as stabilizer in the storage medium.

Results of the comparison of TI1 and TI3 samples by the apoptosis assay are provided in **Fig 19B**. The data further support the conclusion that yeast-derived recombinant serum albumin, such as AlbIX® as a stabilizer in the storage medium, can slow down the switching of cells toward a late apoptotic state, irrespective of the identity of albumin in the cryopreservation medium.

Results of the comparison of RI and TI3 samples by the apoptosis assay are provided in **Fig 19C**. The results show that the percentage of early apoptotic cells was significantly different between the RI and TI3 at every time point during the stability test. Similarly, the difference in percentage of late apoptotic cells between these two items was also significant. These data further confirm that that yeast-derived recombinant serum albumin, such as AlbIX®, has the ability to slow down the entry of the cells into late apoptosis during post-thaw storage.

## Example 5

### Comparison of Recombumin® Prime and Recombumin® Alpha to Plant-Derived Recombinant Albumin Products

As noted above, in the description of this application, Frahm et al, 2014, PLOS One, 9(1): e109893 reported a study in which the properties of recombinant yeast-derived serum albumin (in particular, Recombumin® Prime and Recombumin® Alpha) were compared to another recombinant plant-derived serum albumin product, to plasma-derived serum albumin and recombinant plant-derived serum albumin products.

The recombinant yeast-derived serum albumin products displayed numerous physical differences from serum albumin compositions obtained from other sources (e.g. from plasma, or from other recombinant sources such as plants). Further, the Recombumin® Prime and Recombumin® Alpha products showed some clear differences to the other recombinant yeast-derived serum albumin obtained from *Pichia.*

The present example demonstrates further differences.

A summary of the products is presented in the following table. All of the recombinant albumins described are intended for biopharmaceutical use.

| Albumin | Source | Labelled Purity |
|---|---|---|
| Recombumin® Prime | Yeast- *Saccharomyces* | ≥99% |
| Recombumin® Alpha | Yeast- *Saccharomyces* | ≥99% |
| Commercial Albumin 1 | Rice-*Oryza* | >99% |
| Commercial Albumin 2 | Rice-*Oryza* | >95% |
| Commercial Albumin 3 | Yeast-*Pichia* | >98% |

The column headed "labelled purity" refers to the level of purity indicated by the manufacturer on the product sheet.

**Purity and homogeneity:**

Understanding the purity of a protein excipient such as a recombinant albumin is complex. In addition to understanding the presence of other components in the final formulation, such as host cell protein levels and excipients, attention must also be given to the homogeneity of the protein itself. The presence of modified forms of the protein, in significant quantities, such as glycosylated or partially degraded protein can have a significant effect on the functionality of the protein, as well as decreasing the acceptability of the material to regulatory authorities.

All of the commercial recombinant albumins tested in this example have a manufacturer-indicated protein purity specification of 95-99%, based on electrophoresis. While this is a standard technique for the determination of this parameter, it will not fully resolve the heterogeneity of the protein. We have performed a detailed analysis of the commercial recombinant albumins by mass spectrometry and the data is presented in Figure 1. These data are presented along with comparative gel electrophoresis data in Figure 2.

Despite the relatively similarly labelled purity claims by gel electrophoresis, the mass spectrometry analysis reveals a more interesting picture. Recombumin® Prime and Recombumin® Alpha (Fig 1(a)) both demonstrated a predominantly single species at 66.4 kDa that is representative of the native human serum albumin molecule. In comparison, all of the other commercial recombinant albumin samples evaluated contained multiple peaks that were indicative of partially degraded or glycosylated forms of the protein in the final formulation. Perhaps the most striking mass spectrum was recorded for the rice-derived product from Supplier 1 (Fig 1(b)). This product displayed -40 peaks in the mass range evaluated. On the basis of this analysis it is clear that only a small proportion of the protein present is native human serum albumin. This is despite the labelled purity claim of >96% (w/w).

**Visual Appearance:**

Visual appearance is often one of the first analytical tests performed to examine product colour and clarity. This parameter is important since it is often considered a direct indication of product quality and purity. In instances where the material is likely to be present in relatively large quantities, a significant increase in product pigmentation due to the excipient may be undesirable.

The images presented in Figure 3, demonstrate that Recombumin® Prime and Recombumin® Alpha are the least pigmented of the products evaluated; with both products having a clear/straw colour. The alternative products showed increasing levels of pigmentation with the albumin from Supplier 1, a rice-derived product, showing the greatest pigmentation and the final product having an orange/amber colour.

**Functional characterization:**

The albumin molecule contains a natural free thiol group that is a natural antioxidant for formulation stabilization. Whilst this group is intrinsic to the native albumin molecule, it can become oxidized and deactivated due to poor storage or processing.

The free thiol content of the commercial recombinant albumins described in this Example is presented in the following table.

| Albumin | Free Thiol Content (%) |
|---|---|
| Recombumin® Alpha | 97 |
| Recombumin® Prime | 85 |
| Commercial Albumin 3 | 69 |
| Commercial Albumin 2 | 62 |
| Commercial Albumin 1 | 2 |

It is obvious that free thiol levels vary between products, with yeast-derived recombinant albumin products showing the highest levels of free thiol content. Recombumin® Prime and Recombumin® Alpha showed the highest levels of all the albumins tested. Commercial albumin 1, derived from rice, was almost completely blocked.

**Example 6**

**Comparison of Stem Cell Culture in the Presence of Yeast-Derived Recombinant Albumin, Plant-Derived Recombinant Albumin Product, or Plasma-Derived Albumin**

The aim of this study was to evaluate the suitability of 6 different human serum albumin (HSA) samples for use as a cell culture supplement in human embryonic stem cell (hESC) culture applications.
The samples tested were:

- Samples 1, 3, 4 and 5: different lots of yeast-derived recombinant HSA (AlbIX®).
- Sample 2: plasma-derived HSA available from Sigma (A 9080).
- Sample 6: plant-derived recombinant HSA, Cellastim™, available from Sigma (A9731).

Two different hESC lines (SA121 and SA181), previously cultured in a xenofree cell culture system for 8 passages, were then transferred into xenofree medium containing each of the different test samples and grown for a further 7 passages before they were analysed for expression of pluripotency markers by immunocytochemistry and QPCR. DEF-CS™ (a commercially available chemically defined human iPS cell culture medium) and xenofree medium containing AlbIX® were used as controls in the study.
Cells were counted at each passage and the doubling time calculated using the formula Td = t x [log2 / log (density of cells at harvest / density at seeding)], where t = time between passages (hours). The mean doubling time was then calculated for each of the test media and data were analysed by one-way ANOVA with Tukey's pairwise comparison.

**Results**

Yeast-derived recombinant HSA (Samples 1, 3, 4 and 5) were found to be suitable for use as a medium supplement for hESCs as they supported the undifferentiated growth of both SA121 and SA181 cell lines for 7 passages. QPCR and immunocytochemical analyses demonstrated uniform expression of pluripotency markers in these cultures with low levels of expression of differentiation markers that were examined. The results obtained with samples 1, 3, 4 and 5 compare favourably with both controls.
Plasma-derived HSA (Sample 2) was found to be unsuitable as it failed to support the undifferentiated growth of SA181 cell line, however, SA121 cells did grow well in medium containing this HSA.
The plant-derived recombinant HSA, Cellastim™ (Sample 6) was also found to be unsuitable for hESC culture applications as it could not support the growth of either SA121 or SA181 cell lines, with cultures failing before the first passage in each case.

## Claims

1. A method for the preservation of stem cells, the method comprising the steps of:

(i) combining the stem cells with a cryopreservation medium to produce a mixture, and freezing the mixture to produce a frozen stem cell product,

(ii) combining the stem cells with a cryopreservation medium to produce a mixture, and freezing the mixture to produce a frozen stem cell product, wherein the method further comprises the steps of thawing the frozen stem cell product, transferring the thawed cells to a storage medium, and storing stem cells in the storage medium, or
(iii) storing the stem cells in a storage medium, wherein the stem cells have been frozen in a cryopreservation medium, thawed, and then transferred to the storage medium prior to storage
wherein the cryopreservation medium and/or the storage medium comprises a recombinant yeast-derived serum albumin preparation.

2. The method of Claim 1, wherein the method comprises the steps of freezing stem cells in the cryopreservation medium to produce a frozen stem cell product, thawing the frozen stem cell product, transferring the thawed cells to the storage medium, and storing stem cells in the storage medium,
wherein the cryopreservation medium and/or the storage medium comprises a recombinant yeast-derived serum albumin preparation.

3. The method of any preceding claim wherein the recombinant yeast-derived serum albumin preparation is present in the cryopreservation medium and/or the storage medium, when mixed with the stem cells, in an amount suitable to provide a concentration of the recombinant yeast-derived serum albumin protein that is a concentration selected from: (i) greater than about 0.01 % (w/v) and less than 10% (w/v), (ii) from about 0.1 % (w/v) to about 5% (w/v), or (iii) about 2% (w/v).

4. The method of any preceding claim, wherein:

(a) the stem cells are stored in the storage medium at a temperature of 2-8°C; and/or
(b) wherein the stem cells are stored in the storage medium for a period of time selected from: greater than 24 hours; greater than 24 hours and up to about 48 hours; greater than 24 hours and up to about 72 hours; or more than about 72 hours; or
(c) wherein the stem cells are stored in the storage medium at a temperature of 2-8°C for a period of time selected from: greater than 24 hours; greater than 24 hours and up to about 48 hours; greater than 24 hours and up to about 72 hours; or more than about 72 hours, and in which the viability of the stem cells at the end of the storage period is greater than 5%, 10%, 20%, 30%, 40%, 50%, 60%, 70%, 75%, 80%, 85%, 86%, 87%, 88%, 89%, 90%, 91%, 92%, 93%, 94%, 95% or more.

5. The method of any preceding claim wherein the recombinant yeast-derived serum albumin protein present in the cryopreservation medium and/or the storage medium exhibits one or more of the following properties:

(a) less than 0.5% (w/w), 0.4% (w/w), 0.3% (w/w), 0.2% (w/w) or 0.15% (w/w) binds to Concanavalin A; and/or
(b) a glycation level of less than 0.6, 0.10, 0.075 or 0.05 moles hexose / mole of protein.

6. The method of any preceding claim wherein the recombinant yeast-derived serum albumin protein present in the cryopreservation medium and/or the storage medium:

(a) is at least about 95%, 96%, 97%, 98%, 99.5%, or essentially 100% monomeric and dimeric;
(b) is at least about 93%, 94%, 95%, 96% or 97% monomeric; and/or
(c) has an albumin polymer content of not greater, or less, than about 1.0% (w/w), 0.1 % (w/w) or 0.01% (w/w), wherein the albumin polymer is distinct from monomeric and dimeric forms of albumin.

7. The method of any preceding claim wherein the recombinant yeast-derived serum albumin preparation present in the cryopreservation medium and/or the storage medium:

(a) comprises, consists essentially of, or consists of, yeast-derived serum albumin protein, cations and balancing anions, water, and optionally octanoate and polysorbate 80;
(b) possesses an intact or substantially intact N-terminal sequence;
(c) when tested by mass spectrometry, displays substantially fewer peaks distinct from the main peak at about 66.4 kDa that is representative of native intact human serum albumin molecule, compared to recombinant plant-derived serum albumin protein;
(d) comprise albumin protein that has a free thiol group content that is greater than 62%, or has a free thiol group content that is at least 69%, at least 70%, at least 75%, at least 80%, at least 85%, at least 90%, at least 95%, about 96%, about 97%;

(e) comprise albumin protein that, when tested by size exclusion chromatography (SEC), displays an SEC profile excluding peaks with a peak retention time under 14 minutes and over 19 minutes, or an an SEC profile that excludes peaks with a peak retention time under 14 or 15 minutes and over 18 minutes;
(f) comprise albumin protein that, when tested by reversed phase high performance liquid chromatography (RP-HPLC), displays a single major peak, corresponding to albumin in the native monomeric form;
(g) comprise albumin protein that, when tested by mass spectrometry, is a product that displays fewer than 13, 12, 11, 10, 9, 8, 7, or 6 hexose modified lysine and/or arginine residues per protein, or displays about 1 to 11, 1 to 8, 1 to 5, 1 to 4, 1 to 3, 1 to 2, 1 or less than 1, hexose modified lysine and/or arginine residues per protein; and/or
(h) comprises albumin protein that is not glycated with plant-specific sugar, or comprises albumin protein that is not glycated with a plant-specific sugar selected from $\alpha$-1,3-fucose and/or $\beta$-1,2-xylose.

**8.** The method of any preceding claim wherein the recombinant yeast-derived serum albumin preparation present in the cryopreservation medium and/or the storage medium, and/or the cryopreservation medium and/or the storage medium itself:

(a) comprises octanoate at less than 35mM, 20 mM, 10 mM, 5 mM, 1 mM, 0.1 mM, 0.01 mM, 0.001 mM, is substantially free of octanoate, or is free of octanoate;
(b) has an overall fatty acid content less than or equal to 35mM, 20 mM, 10 mM, 5 mM, 1 mM, is substantially free of fatty acids, or is free of fatty acids;
(c) comprises detergent at a concentration less than 200 $mg.L^{-1}$, 100 $mg.L^{-1}$, 50 $mg.L^{-1}$, 10 $mg.L^{-1}$, 1 $mg.L^{-1}$, 0.1 $mg.L^{-1}$, 0.01 $mg.L^{-1}$, 0.001 $mg.L^{-1}$, is substantially free of the detergent, or is free of the detergent;
(d) comprises total free amino acid level and/or N-acetyl tryptophan levels less than 35mM, 20 mM, 10 mM, 5 mM1 mM, 0.1 mM, 0.01 mM, 0.005 mM, 0.001 mM, is substantially free of free amino acids and/or N-acetyl tryptophan in particular, or is free of free amino acids and/or of N-acetyl tryptophan in particular;
(e) is substantially free of, or completely free of, all of octanoate, free amino acids and/or N-acetyl tryptophan in particular, and detergent; and/or
(f) is free of one or more, such all, components selected from: haem, prekallikrein activator, pyrogens, hepatitis C and/or human viruses and/or has an aluminium concentration of less than 200 $\mu g.L^{-1}$, 100 $\mu g.L^{-1}$, 50 $\mu g.L^{-1}$, or within the range of about 10 $\mu g.L^{-1}$ to about 30 $\mu g.L^{-1}$.

**9.** The method of Claim 8, wherein the recombinant yeast-derived serum albumin preparation present in the cryopreservation medium and/or the storage medium, and/or the cryopreservation medium and/or the storage medium itself, comprises detergent at a concentration less than 200 $mg.L^{-1}$, 100 $mg.L^{-1}$, 50 $mg.L^{-1}$, 10 $mg.L^{-1}$, 1 $mg.L^{-1}$, 0.1 $mg.L^{-1}$, 0.01 $mg.L^{-1}$, 0.001 $mg.L^{-1}$, is substantially free of the detergent, or is free of the detergent,
wherein the detergent is polysorbate or polysorbate 80.

**10.** The method of Claim 8 or 9, wherein the recombinant yeast-derived serum albumin preparation present in the cryopreservation medium and/or the storage medium, and/or the cryopreservation medium and/or the storage medium itself, is substantially free of, or completely free of, all of octanoate, free amino acids and/or N-acetyl tryptophan in particular, and detergent,
wherein the detergent is polysorbate 80.

**11.** The method of any preceding claim, wherein:

(a) the cryopreservation medium comprises the recombinant serum albumin preparation and a cryopreservant, or
(b) the cryopreservation medium comprises, consists essentially of, or consists of an aqueous solution of the recombinant yeast-derived serum albumin preparation, a cryopreservant, and an ionic buffer; and wherein:

(i) the cryopreservation medium is not a stem cell culture growth media,
(ii) the cryopreservation medium is not a stem cell culture growth media, and does not support the growth of stem cells, or
(iii) the cryopreservation medium is not a stem cell culture growth media, does not support the growth of stem cells, and includes substantially no, or no, levels of any one or more, or all, of the components of a typical stem cell culture medium.

**12.** The method of Claim 11, wherein the cryopreservation medium is not a stem cell culture growth media, does not support the growth of stem cells, and includes substantially no, or no, levels of any one or more, or all, of the components of a typical stem cell culture medium, and
wherein the components of a typical stem cell culture medium include vitamins, hormones, growth factors, iron sources, free amino acids and/or glucose.

**13.** The method of any preceding claim, wherein:

(a) the storage medium comprises the recombinant yeast-derived serum albumin preparation, or
(b) the storage medium comprises, consists essentially of, or consists of an aqueous solution of the recombinant yeast-derived serum albumin preparation and an ionic buffer, and wherein:

(i) the storage medium is not a stem cell culture growth media,
(ii) the storage medium is not a stem cell culture growth media and does not support the growth of stem cells, or
(iii) the storage medium is not a stem cell culture growth media, does not support the growth of stem cells, and includes substantially no, or no, levels of any one or more, or all, of the components of a typical stem cell culture medium.

**14.** The method of Claim 13, wherein the storage medium is not a stem cell culture growth media, does not support the growth of stem cells, and includes substantially no, or no, levels of any one or more, or all, of the components of a typical stem cell culture medium, and
wherein the components of a typical stem cell culture medium include vitamins, hormones, growth factors, iron sources, free amino acids and/or glucose.

**15.** The method of any preceding claim, which method comprises:

(i) storing stem cells in the storage medium,
(ii) storing stem cells in the storage medium at a temperature of 2-8°C, or
(iii) storing stem cells in the storage medium at a temperature of 2-8°C for a period of time selected from greater than 24 hours; greater than 24 hours and up to about 48 hours; greater than 24 hours and up to about 72 hours; or for more than 72 hours, and
wherein, directly or indirectly after the step of storing stem cells in the storage medium in accordance with the method as defined by any of parts (i), (ii) or (iii) of this claim, then the method further comprises one or more steps, selected from the steps of: culturing the stem cells; expanding a culture of the stem cells; differentiation of the stem cells; immobilization of the stem cells, or cultured and/or differentiated cells derived therefrom; immobilization of the stem cells, or cultured and/or differentiated cells derived therefrom into tissue or a medical implant; and/or formulating stem cells, or cultured and/or differentiated cells or other products derived therefrom, in a pharmaceutically acceptable composition or veterinarially acceptable composition.

**16.** A cryopreservation medium for the cryopreservation of stem cells, wherein;

(i) the cryopreservation medium comprises a recombinant yeast-derived serum albumin preparation and a cryopreservant, or wherein the cryopreservation medium comprises a recombinant yeast-derived serum albumin preparation and a cryopreservant and is a cryopreservation medium as defined by any of the preceding claims,
(ii) the cryopreservation medium is a cryopreservation medium as defined by part (i) of this claim, which further comprises stem cells, or
(iii) the cryopreservation medium is a cryopreservation comprising stem cells as defined by part (ii) of this claim and wherein the cryopreservation medium is frozen, or is a cryopreservation medium comprising stem cells as defined by part (ii) of this claim which has been frozen and then thawed.

**17.** A storage medium for the storage of stem cells that have been frozen in a cryopreservation medium, thawed, and then transferred to the storage medium:

(i) wherein the storage medium comprises a recombinant yeast-derived serum albumin preparation and wherein the storage medium is not a stem cell culture growth medium, or wherein the storage medium is a storage medium as defined by any of Claims 1 to 115, and is not a stem cell culture growth medium,
(ii) wherein the storage medium is a storage medium as defined by part (i) of this claim and further comprises stem cells,
(iii) wherein the storage medium is a storage medium as defined by part (ii) of this claim and wherein the stem cells have been frozen in a cryopreservation medium, thawed, and then transferred to the storage medium, or (iv) wherein the storage medium is a storage medium as defined by part (iii) of this claim and the cryopreservation medium is a cryopreservation medium as defined by Claim 16.

**18.** The storage medium of Claim 17, wherein the storage medium:

(a) does not support the growth of stem cells; and/or
(b) includes substantially no, or no, levels of any one or more of vitamins, hormones, growth factors, iron sources, free amino acids and/or glucose.

**19.** The storage medium of Claim 17 or 18, which further comprises stem cells:

(i) that have been: (a) frozen in a cryopreservation medium, thawed, and then transferred to the storage medium, or (b) subjected to another physiological shock prior to being transferred to the storage medium,
(ii) that have been stored in the storage medium at a temperature of 2-8°C for a period of time selected from greater than 24 hours; greater than 24 hours and up to about 48 hours; greater than 24 hours and up to about 72 hours; or more than 72 hours, and
(iii) in which the viability of the stem cells at the end of the storage period is greater than 5%, 10%, 20%, 30%, 40%, 50%, 60%, 70%, 75%, 80%, 85%, 86%, 87%, 88%, 89%, 90%, 91%, 92%, 93%, 94%, 95% or more.

**20.** The use of a cryopreservation medium according to Claim 16:

(i) for the preservation of stem cells,
(ii) for the preservation of stem cells in a viable state following storage of the stem cells at 2-8°C for a period of time greater than 24 hours, greater than 24 hours and up to about 48 hours, greater than 24 hours and up to about 72 hours, or more than 72 hours; or
(iii) for the use as defined by part (i) or (ii) of this claim in which the viability of the stem cells at the end of the storage period is greater than 5%, 10%, 20%, 30%, 40%, 50%, 60%, 70%, 75%, 80%, 85%, 86%, 87%, 88%, 89%, 90%, 91%, 92%, 93%, 94%, 95% or more.

**21.** The use of Claim 20:

(a) for the preservation of stem cells by combining the stem cells with the cryopreservation medium to produce a mixture, and freezing the mixture to produce a frozen stem cell product, or subjecting the stem cells to another physiological shock, prior to storage at 2-8°C for a period of time greater than 24 hours, greater than 24 hours and up to about 48 hours, greater than 24 hours and up to about 72 hours, or more than 72 hours;
(b) for the preservation of stem cells by a method that further comprises the steps of thawing the frozen stem cell product, transferring the thawed cells to a storage medium, and storing stem cells in the storage medium at 2-8°C for a period of time greater than 24 hours, greater than 24 hours and up to about 48 hours, greater than 24 hours and up to about 72 hours, or more than 72 hours; or
(c) for the preservation of stem cells by combining the stem cells with the cryopreservation medium to produce a mixture, subjecting the stem cells to a physiological shock, transferring the cells to a storage medium, and storing stem cells in the storage medium at 2-8°C for a period of time greater than 24 hours, greater than 24 hours and up to about 48 hours, greater than 24 hours and up to about 72 hours, or more than 72 hours.

**22.** The use of a storage medium according to Claim 17, 18 or 19:

(i) for the preservation of stem cells, by storing stem cells in the storage medium,
(ii) for the preservation of stem cells, by storing stem cells in the storage medium, wherein the stem cells have been frozen in a cryopreservation medium, thawed, and then transferred to the storage medium prior to storage,
(iii) for the preservation of stem cells according to part (ii) of this claim wherein the cryopreservation medium is a cryopreservation medium according to Claim 16;
(iv) for the preservation of stem cells, by storing stem cells in the storage medium, wherein the stem cells have been mixed with a cryopreservation medium, subjected a physiological shock, and then transferred to the storage medium prior to storage, or
(v) for the preservation of stem cells according to part (iv) of this claim wherein the cryopreservation medium is a cryopreservation medium according to Claim 16.

**23.** The use of recombinant yeast-derived serum albumin:

(i) for improving the post-thawing viability of cryopreserved stem cells,
(ii) for improving the post-thawing viability of cryopreserved stem cells according to part (i) of this claim, wherein the improvement is compared to plasma-derived serum albumin that is used in the same concentration;
(iii) for improving the post-thawing viability of cryopreserved stem cells according to part (i) or (ii) of this claim, wherein the improvement is observable in the post-thawed stem cells, when stored in a storage medium at 2-8°C for a period of time greater than 24 hours, greater than 24 hours and up to about 48 hours, greater than 24 hours and up to about 72 hours, or more than 72 hours;
(iv) for improving the post-thawing viability of cryopreserved stem cells according to any of parts (i), (ii) or (iii) of this claim, wherein the recombinant yeast-derived serum albumin is used by formulating it into a cryopreservation medium and mixing the cryopreservation medium with stem cells prior to freezing,
(v) for improving the post-thawing viability of cryopreserved stem cells according to part (iv) of this claim, wherein the cryopreservation medium is a cryopreservation medium as defined by Claim 16;
(vi) for improving the post-thawing viability of cryopreserved stem cells according to any of parts (i) to (v) of this claim, wherein the recombinant yeast-derived serum albumin is used by formulating it into a storage medium and mixing the storage medium with stem cells after thawing, or
(vii) for improving the post-thawing viability of cryopreserved stem cells according to part (vi) of this claim, wherein the storage medium is a medium as defined by Claim 17, 18 or 19.

**24.** The use of recombinant yeast-derived serum albumin:

(i) for improving the viability of stem cells that are subjected to physiological shock,
(ii) for improving the viability of stem cells that are subjected to physiological shock according to part (i) of this claim, wherein the improvement is compared to the use of plasma-derived serum albumin that is used in the same concentration;
(iii) for improving the viability of stem cells that are subjected to physiological shock according to part (i) or (ii) of this claim, wherein the improvement is observable in the post-shock stem cells, when stored in a storage medium at 2-8°C for a period of time greater than 24 hours, greater than 24 hours and up to about 48 hours, greater than 24 hours and up to about 72 hours, or more than 72 hours;
(iv) for improving the viability of stem cells that are subjected to physiological shock according to any of parts (i), (ii) or (iii) of this claim, wherein the recombinant yeast-derived serum albumin is used by formulating it into a cryopreservation medium and mixing the cryopreservation medium with stem cells prior to the physiological shock,
(v) for improving the viability of stem cells that are subjected to physiological shock according to part (iv) of this claim, wherein the cryopreservation medium is a cryopreservation medium as defined by Claim 16;
(vi) for improving the viability of stem cells that are subjected to physiological shock according to any of parts (i) to (v) of this claim, wherein the recombinant yeast-derived serum albumin is used by formulating it into a storage medium and mixing the storage medium with stem cells after receiving the physiological shock, or
(vii) for improving the viability of stem cells that are subjected to physiological shock according to part (vi) of this claim, wherein the storage medium is a storage medium as defined by Claim 17, 18 or 19.

## Patentansprüche

**1.** Verfahren zur Erhaltung von Stammzellen, wobei das Verfahren die folgenden Schritte umfasst:

(i) Kombinieren der Stammzellen mit einem Kryokonservierungsmedium, um eine Mischung zu erzeugen, und Einfrieren der Mischung, um ein gefrorenes Stammzellenprodukt zu erzeugen,
(ii) Kombinieren der Stammzellen mit einem Kryokonservierungsmedium, um eine Mischung zu erzeugen, und Einfrieren der Mischung, um ein gefrorenes Stammzellenprodukt zu erzeugen, wobei das Verfahren ferner die folgenden Schritte umfasst: Auftauen des gefrorenen Stammzellenprodukts, Übertragen der aufgetauten Zellen auf ein Aufbewahrungsmedium und Aufbewahren von Stammzellen in dem Aufbewahrungsmedium, oder
(iii) Aufbewahren der Stammzellen in einem Aufbewahrungsmedium, wobei die Stammzellen in einem Kryokonservierungsmedium eingefroren, aufgetaut und anschließend vor der Aufbewahrung auf das Aufbewahrungsmedium übertragen wurden,
wobei das Kryokonservierungsmedium und/oder das Aufbewahrungsmedium ein aus Hefe abgeleitetes rekombinantes Serumalbuminpräparat umfasst.

2. Verfahren nach Anspruch 1, wobei das Verfahren die folgenden Schritte umfasst: Einfrieren von Stammzellen in dem Kryokonservierungsmedium, um ein gefrorenes Stammzellenprodukt zu erzeugen, Auftauen des gefrorenen Stammzellenprodukts, Übertragen der aufgetauten Zellen in das Aufbewahrungsmedium und Aufbewahren der Stammzellen in dem Aufbewahrungsmedium, wobei das Kryokonservierungsmedium und/oder das Aufbewahrungsmedium ein aus Hefe abgeleitetes rekombinantes Serumalbuminpräparat umfasst.

3. Verfahren nach einem der vorhergehenden Ansprüche, wobei das aus Hefe abgeleitete rekombinante Serumalbuminpräparat in dem Kryokonservierungsmedium und/oder das Aufbewahrungsmedium bei Mischung mit den Stammzellen in einer geeigneten Menge vorhanden ist, um eine Konzentration des aus Hefe abgeleiteten rekombinanten Serumalbuminpräparats bereitzustellen, das eine Konzentration aufweist, die ausgewählt ist aus: (i) größer als etwa 0,01 % (Gew./Vol.) und kleiner als 10 % (Gew./Vol.), (ii) von etwa 0,1 % (Gew./Vol.) bis etwa 5 % (Gew./Vol.) oder (iii) etwa 2 % (Gew./Vol.).

4. Verfahren nach einem der vorangehenden Ansprüche, wobei:

(a) die Stammzellen in dem Aufbewahrungsmedium bei einer Temperatur von 2 bis 8 °C aufbewahrt werden; und/oder
(b) wobei die Stammzellen in dem Aufbewahrungsmedium für einen Zeitraum aufbewahrt werden, der ausgewählt ist, aus: mehr als 24 Stunden; mehr als 24 Stunden und bis zu etwa 48 Stunden; mehr als 24 Stunden und bis zu etwa 72 Stunden; oder mehr als ungefähr 72 Stunden; oder
(c) wobei die Stammzellen in dem Aufbewahrungsmedium bei einer Temperatur von 2 bis 8 °C für einen Zeitraum aufbewahrt werden, der ausgewählt ist, aus: mehr als 24 Stunden; mehr als 24 Stunden und bis zu etwa 48 Stunden; mehr als 24 Stunden und bis zu etwa 72 Stunden; oder mehr als etwa 72 Stunden, und in welchem die Lebensfähigkeit der Stammzellen am Ende der Aufbewahrungszeit größer ist, als 5 %, 10 %, 20 %, 30 %, 40 %, 50 %, 60 %, 70 %, 75 %, 80 %, 85 %, 86 %, 87 %, 88 %, 89 %, 90 %, 91 %, 92 %, 93 %, 94 %, 95 % oder mehr.

5. Verfahren nach einem der vorhergehenden Ansprüche, wobei das in dem Kryokonservierungsmedium und/oder dem Aufbewahrungsmedium vorhandene aus Hefe abgeleitete rekombinante Serumalbuminprotein eine oder mehrere der folgenden Eigenschaften aufweist:

(a) weniger als 0,5 % (Gew./Gew.), 0,4 % (Gew./Gew.), 0,3 % (Gew./Gew.), 0,2 % (Gew./Gew.) oder 0,15 % (Gew./Gew.) ist an Concanavalin A gebunden; und/oder
(b) ein Glycationsniveau von weniger als 0,6; 0,10; 0,075 oder 0,05 Hexose-Mole/Protein-Mol.

6. Verfahren nach einem der vorhergehenden Ansprüche, wobei das aus Hefe abgeleitete rekombinante Serumalbuminprotein, das in dem Kryokonservierungsmedium und/oder dem Aufbewahrungsmedium vorliegt:

(a) mindestens etwa 95 %, 96 %, 97 %, 98 %, 99,5 % oder im Wesentlichen 100 % monomerisch und dimerisch ist;
(b) mindestens etwa 93 %, 94 %, 95 %, 96 % oder 97 % monomerisch ist; und/oder
(c) einen Albuminpolymergehalt von nicht mehr oder weniger als etwa 1,0 % (Gew./Gew.), 0,1 % (Gew./Gew.) oder 0,01 % (Gew./Gew.) aufweist, wobei sich das Albuminpolymer von monomeren und dimeren Albuminformen unterscheidet.

7. Verfahren nach einem der vorhergehenden Ansprüche, wobei das aus Hefe abgeleitete rekombinante Serumalbuminpräparat, das in dem Kryokonservierungsmedium und/oder dem Aufbewahrungsmedium vorhanden ist:

(a) umfasst, im Wesentlichen daraus besteht oder besteht: aus Hefe abgeleitetes Serumalbuminprotein, Kationen und ausgleichende Anionen, Wasser und optional Octanoat und Polysorbat 80;
(b) eine intakte oder im Wesentlichen intakte N-terminale Sequenz besitzt;
(c) bei einer massenspektrometrischen Prüfung, im Vergleich zu rekombinantem pflanzlichem Serumalbuminprotein, wesentlich weniger Peaks aufweist, die von dem Hauptpeak von etwa 66,4 kDa, der für ein natives intaktes menschliches Serumalbuminmolekül repräsentativ ist, abweichen;
(d) Albuminprotein umfasst, das einen Gehalt an freien Thiolgruppen von mehr als 62 % oder einen Gehalt an freien Thiolgruppen von mindestens 69 %, mindestens 70 %, mindestens 75 %, mindestens 80 %, mindestens 85 % aufweist mindestens 90 %, mindestens 95 %, etwa 96 %, etwa 97 % aufweist;
(e) Albuminprotein umfasst, das bei einer Prüfung durch Größenausschlusschromatographie (*Size Exclusion Chromatography,* SEC) ein SEC-Profil ohne Peaks mit einer Peakretentionszeit von weniger als 14 Minuten und mehr als 19 Minuten oder ein SEC-Profil ohne Peaks mit einer Peakretentionszeit von weniger als 14 oder 15 Minuten und mehr als 18 Minuten aufweist;
(f) Albuminprotein umfasst, das, bei einer Prüfung durch Umkehrphasen-Hochleistungsflüssigkeitschromatographie (*Reversed Phase High Performance Liquid Chromatography,* RP-HPLC), einen einzelnen Hauptpeak aufweist, der Albumin in der nativen monomeren Form entspricht;
(g) Albuminprotein umfasst, das, bei einer Prüfung durch Massenspektrometrie, ein Produkt ist, das weniger als 13, 12, 11, 10, 9, 8, 7 oder 6 hexosemodifizierte Lysin- und/oder Argininreste pro Protein aufweist oder ungefähr 1 bis 11, 1 bis 8, 1 bis 5, 1 bis 4, 1 bis 3, 1 bis 2, 1 oder weniger als 1 hexosemodifizierte Lysin- und/oder Argininreste pro Protein aufweist; und/oder
(h) Albuminprotein umfasst, das nicht mit pflanzenspezifischem Zucker glykiert ist, oder Albuminprotein umfasst, das nicht mit einem pflanzenspezifischen Zucker glykiert ist, der aus $\alpha$-1,3-Fucose und/oder $\beta$-1,2-Xylose ausgewählt ist.

**8.** Verfahren nach einem der vorhergehenden Ansprüche, wobei das aus Hefe abgeleitete rekombinante Serumalbuminpräparat in dem Kryokonservierungsmedium und/oder dem Aufbewahrungsmedium, und/oder das Kryokonservierungsmedium und/oder das Aufbewahrungsmedium selbst:

(a) bei weniger als 35 mM, 20 mM, 10 mM, 5 mM, 1 mM, 0,1 mM, 0,01 mM, 0,001 mM Octanoat umfasst, im Wesentlichen frei von Octanoat ist oder frei von Octanoat ist;
(b) einen Gesamtfettsäuregehalt von weniger als oder gleich 35 mM, 20 mM, 10 mM, 5 mM, 1 mM aufweist, im Wesentlichen frei von Fettsäuren ist oder frei von Fettsäuren ist;
(c) Detergens in einer Konzentration von weniger als 200 $mg.L^{-1}$, 100 $mg.L^{-1}$, 50 $mg.L^{-1}$, 10 $mg.L^{-1}$, 1 $mg.L^{-1}$, 0,1 $mg.L^{-1}$, 0,01 $mg.L^{-1}$, 0,001 $mg.L^{-1}$ umfasst, im Wesentlichen frei von dem Detergens ist oder frei von dem Detergens ist;
(d) einen Gesamtgehalt an freien Aminosäuren und/oder N-Acetyltryptophan von weniger als 35 mM, 20 mM, 10 mM, 5 mM, 1 mM, 0,1 mM, 0,01 mM, 0,005 mM, 0,001 mM umfasst, im Wesentlichen frei von an freien Aminosäuren und/oder insbesondere von N-Acetyltryptophan oder frei von freien Aminosäuren und/oder insbesondere von N-Acetyltryptophan ist;
(e) im Wesentlichen frei oder vollständig frei von jedem von Octanoat, freien Aminosäuren und/oder insbesondere N-Acetyltryptophan und Detergens ist; und/oder
(f) frei ist von einer oder mehreren dieser Komponenten, ausgewählt aus: Häm, Präkallikrein-Aktivator, Pyrogenen, Hepatitis C und/oder humanen Viren und/oder eine Aluminiumkonzentration von weniger als 200 $\mu g.L^{-1}$, 100 $\mu g.L^{-1}$, 50 $\mu g.L^{-1}$ aufweist, oder innerhalb des Bereichs von ungefähr 10 $\mu g.L^{-1}$ bis ungefähr 30 $\mu g.L^{-1}$ liegt.

**9.** Verfahren nach Anspruch 8, wobei das aus Hefe abgeleitete rekombinante Serumalbuminpräparat, das in dem Kryokonservierungsmedium und/oder dem Aufbewahrungsmedium und/oder dem Kryokonservierungsmedium und/oder dem Aufbewahrungsmedium selbst vorhanden ist, Detergens in einer Konzentration von weniger als 200 $mg.L^{-1}$, 100 $mg.L^{-1}$, 50 $mg.L^{-1}$, 10 $mg.L^{-1}$, 1 $mg.L^{-1}$, 0,1 $mg.L^{-1}$, 0,01 $mg.L^{-1}$, 0,001 $mg.L^{-1}$ umfasst, im Wesentlichen frei von dem Detergens ist, oder frei von dem Detergens ist, wobei das Detergens Polysorbat oder Polysorbat 80 ist.

**10.** Verfahren nach Anspruch 8 oder 9, wobei das aus Hefe abgeleitete rekombinante Serumalbuminpräparat, das in dem Kryokonservierungsmedium und/oder dem Aufbewahrungsmedium und/oder dem Kryokonservierungsmedium und/oder dem Aufbewahrungsmedium selbst vorhanden ist, im Wesentlichen frei ist von oder vollständig frei von jedem von Octanoat, freien Aminosäuren und/oder insbesondere von N-Acetyltryptophan und Detergens, wobei das Detergens Polysorbat 80 ist.

**11.** Verfahren nach einem der vorangehenden Ansprüche, wobei:

(a) das Kryokonservierungsmedium das rekombinante Serumalbuminpräparat und ein Kryokonservierungsmittel umfasst, oder
(b) das Kryokonservierungsmedium Folgendes umfasst, im Wesentlichen daraus besteht oder daraus besteht: eine wässrige Lösung des aus Hefe abgeleiteten rekombinanten Serumalbuminpräparats, ein Kryokonservierungsmittel und einen Ionenpuffer; und wobei:

(i) das Kryokonservierungsmedium kein Stammzellkultur-Wachstumsmedium ist,
(ii) das Kryokonservierungsmedium kein Stammzellkultur-Wachstumsmedium ist und das Wachstum von Stammzellen nicht unterstützt, oder
(iii) das Kryokonservierungsmedium kein Stammzellkultur-Wachstumsmedium ist, das Wachstum von Stammzellen nicht unterstützt und im Wesentlichen keine oder keine Gehalte einer oder mehrerer oder aller Komponenten eines typischen Stammzellkulturmediums enthält.

**12.** Verfahren nach Anspruch 11, wobei das Kryokonservierungsmedium kein Stammzellkultur-Wachstumsmedium ist, das Wachstum von Stammzellen nicht unterstützt und im Wesentlichen keine oder keine Gehalte einer oder mehrerer oder aller Komponenten eines typischen Stammzellkulturmediums enthält und wobei die Komponenten eines typischen Stammzellkulturmediums Vitamine, Hormone, Wachstumsfaktoren, Eisenquellen, freie Aminosäuren und/oder Glucose enthalten.

**13.** Verfahren nach einem der vorangehenden Ansprüche, wobei:

(a) das Aufbewahrungsmedium das aus Hefe abgeleitete rekombinante Serumalbuminpräparat umfasst, oder
(b) das Aufbewahrungsmedium Folgendes umfasst, im Wesentlichen daraus besteht oder daraus besteht: eine wässrige Lösung des aus Hefe abgeleiteten rekombinanten Serumalbuminpräparats und einen Ionenpuffer, und wobei:

(i) das Aufbewahrungsmedium kein Stammzellkultur-Wachstumsmedium ist,
(ii) das Aufbewahrungsmedium kein Stammzellkultur-Wachstumsmedium ist und das Wachstum von Stammzellen nicht unterstützt, oder
(iii) das Aufbewahrungsmedium kein Stammzellkultur-Wachstumsmedium ist, das Wachstum von Stammzellen nicht unterstützt und im Wesentlichen keine oder keine Gehalte einer oder mehrerer oder aller Komponenten eines typischen Stammzellkulturmediums enthält.

**14.** Verfahren nach Anspruch 13, wobei das Aufbewahrungsmedium kein Stammzellkultur-Wachstumsmedium ist, das Wachstum von Stammzellen nicht unterstützt und im Wesentlichen keine oder keine Gehalte einer oder mehrerer oder aller Komponenten eines typischen Stammzellkulturmediums enthält, und wobei die Komponenten eines typischen Stammzellkulturmediums Vitamine, Hormone, Wachstumsfaktoren, Eisenquellen, freie Aminosäuren und/oder Glucose enthalten.

**15.** Verfahren nach einem der vorhergehenden Ansprüche, das Verfahren Folgendes umfassend:

(i) Aufbewahren von Stammzellen in dem Aufbewahrungsmedium,
(ii) Aufbewahren von Stammzellen in dem Aufbewahrungsmedium bei einer Temperatur von 2 bis 8 °C oder
(iii) Aufbewahren von Stammzellen in dem Aufbewahrungsmedium bei einer Temperatur von 2 bis 8 °C für einen Zeitraum von mehr als 24 Stunden; mehr als 24 Stunden und bis zu etwa 48 Stunden; mehr als 24 Stunden und bis zu etwa 72 Stunden; oder für mehr als 72 Stunden, und
wobei direkt oder indirekt nach dem Schritt des Aufbewahrens von Stammzellen in dem Aufbewahrungsmedium nach dem Verfahren gemäß einem der Teile (i), (ii) oder (iii) dieses Anspruchs, das Verfahren ferner eines oder mehrere Schritte umfasst, ausgewählt aus den folgenden Schritten: Kultivieren der Stammzellen; Erweitern einer Kultur der Stammzellen; Differenzieren der Stammzellen; Immobilisieren der Stammzellen oder der kultivierten und/oder der davon abgeleiteten differenzierten Zellen; Immobilisieren der Stammzellen oder der kultivierten und/oder davon abgeleiteten differenzierten Zellen in Gewebe oder ein einem medizinischen Implantat; und/oder Formulieren von Stammzellen oder kultivierten und/oder differenzierten Zellen oder anderen davon abgeleiteten Produkten in einer pharmazeutisch verträglichen Zusammensetzung oder veterinärmedizinisch verträglichen Zusammensetzung.

**16.** Kryokonservierungsmedium für die Kryokonservierung von Stammzellen, wobei:

(i) das Kryokonservierungsmedium ein aus Hefe abgeleitetes rekombinantes Serumalbuminpräparat und ein Kryokonservierungsmittel umfasst, oder wobei das Kryokonservierungsmedium ein aus Hefe abgeleitetes rekombinantes Serumalbuminpräparat und ein Kryokonservierungsmittel umfasst und ein Kryokonservierungsmedium gemäß einem der vorhergehenden Ansprüche ist,
(ii) das Kryokonservierungsmedium ein Kryokonservierungsmedium gemäß Teil (i) dieses Anspruchs ist, das ferner Stammzellen umfasst, oder
(iii) das Kryokonservierungsmedium eine Kryokonservierung ist, die Stammzellen gemäß Teil (ii) dieses Anspruchs umfasst, und wobei das Kryokonservierungsmedium gefroren ist, oder ein Kryokonservierungsmedium ist, das Stammzellen gemäß einem Teil dieses Anspruchs umfasst, das gefroren und anschließend aufgetaut wurde.

**17.** Aufbewahrungsmedium zum Aufbewahren von Stammzellen, die in einem Kryokonservierungsmedium eingefroren, aufgetaut und anschließend auf das Aufbewahrungsmedium übertragen wurden:

(i) wobei das Aufbewahrungsmedium ein aus Hefe abgeleitetes rekombinantes Serumalbuminpräparat umfasst und wobei das Aufbewahrungsmedium kein Stammzellkultur-Wachstumsmedium ist, oder wobei das Aufbewahrungsmedium ein Aufbewahrungsmedium gemäß einem der Ansprüche 1 bis 115 ist und kein Stammzellkultur-Wachstumsmedium ist,
(ii) wobei das Aufbewahrungsmedium ein Aufbewahrungsmedium gemäß Teil (i) dieses Anspruchs ist und ferner Stammzellen umfasst,
(iii) wobei das Aufbewahrungsmedium ein Aufbewahrungsmedium gemäß Teil (ii) dieses Anspruchs ist und wobei die Stammzellen in einem Kryokonservierungsmedium eingefroren, aufgetaut und anschließend auf das Aufbewahrungsmedium übertragen wurden, oder
(iv) wobei das Aufbewahrungsmedium ein Aufbewahrungsmedium gemäß Teil (iii) dieses Anspruchs ist, und das Kryokonservierungsmedium ein Kryokonservierungsmedium gemäß Anspruch 16 ist.

**18.** Aufbewahrungsmedium nach Anspruch 17, wobei das Aufbewahrungsmedium:

(a) das Wachstum von Stammzellen nicht unterstützt; und/oder
(b) im Wesentlichen keine oder keine Gehalte an einem oder mehreren von Folgendem enthält: Vitaminen, Hormonen, Wachstumsfaktoren, Eisenquellen, freien Aminosäuren und/oder Glucose.

**19.** Aufbewahrungsmedium nach Anspruch 17 oder 18, welches ferner Stammzellen umfasst:

(i) die (a) in einem Kryokonservierungsmedium eingefroren, aufgetaut und anschließend auf das Aufbewahrungsmedium übertragen wurden oder (b) einem anderen physiologischen Schock ausgesetzt wurden, bevor sie auf das Aufbewahrungsmedium übertragen wurden,
(ii) die in dem Aufbewahrungsmedium bei einer Temperatur von 2 bis 8 °C für einen Zeitraum von mehr als 24 Stunden, mehr als 24 Stunden und bis zu etwa 48 Stunden, mehr als 24 Stunden und bis zu etwa 72 Stunden, oder mehr als 72 Stunden aufbewahrt wurden, und
(iii) wobei die Lebensfähigkeit der Stammzellen am Ende der Aufbewahrungszeit größer als 5 %, 10 %, 20 %, 30 %, 40 %, 50 %, 60 %, 70 %, 75 %, 80 %, 85 %, 86 %, 87 %, 88 %, 89 %, 90 %, 91 %, 92 %, 93 %, 94 %, 95 % oder mehr ist.

**20.** Verwendung eines Kryokonservierungsmediums nach Anspruch 16:

(i) zur Erhaltung von Stammzellen,
(ii) zum Konservieren von Stammzellen in einem lebensfähigen Zustand nach einer Aufbewahrung der Stammzellen bei 2 bis 8 °C für einen Zeitraum von mehr als 24 Stunden, mehr als 24 Stunden und bis zu etwa 48 Stunden, mehr als 24 Stunden und bis zu ungefähr 72 Stunden oder mehr als 72 Stunden; oder
(iii) zum Verwenden nach Teil (i) oder (ii) dieses Anspruchs, wobei die Lebensfähigkeit der Stammzellen am Ende der Lagerzeit größer als 5 %, 10 %, 20 %, 30 %, 40 %, 50 %, 60 %, 70 %, 75 %, 80 %, 85 %, 86 %, 87 %, 88 %, 89 %, 90 %, 91 %, 92 %, 93 %, 94 %, 95 % oder mehr ist.

**21.** Verwendung nach Anspruch 20:

(a) zum Konservieren von Stammzellen durch Kombinieren der Stammzellen mit dem Kryokonservierungsmedium, um eine Mischung zu erzeugen, und Einfrieren der Mischung, um ein gefrorenes Stammzellenprodukt zu erzeugen, oder Aussetzen der Stammzellen einem anderen physiologischen Schock vor der Aufbewahrung bei 2 bis 8 °C über einen Zeitraum von mehr als 24 Stunden, mehr als 24 Stunden und bis zu etwa 48 Stunden, mehr als 24 Stunden und bis zu etwa 72 Stunden oder mehr als 72 Stunden;
(b) zum Konservieren von Stammzellen durch ein Verfahren, das ferner die Schritte des Auftauens des gefrorenen Stammzellenprodukts, der Übertragung der aufgetauten Zellen auf ein Aufbewahrungsmedium und des Aufbewahrens von Stammzellen in dem Aufbewahrungsmedium bei 2 bis 8 ° C für einen Zeitraum umfasst mehr als 24 Stunden, mehr als 24 Stunden und bis zu etwa 48 Stunden, mehr als 24 Stunden und bis zu etwa 72 Stunden oder mehr als 72 Stunden umfasst; oder
(c) zum Konservieren von Stammzellen durch Kombinieren der Stammzellen mit dem Kryokonservierungsmedium, um eine Mischung zu erzeugen, die Stammzellen einem physiologischen Schock auszusetzen, Übertragen der Zellen auf ein Aufbewahrungsmedium und Aufbewahren der Stammzellen in dem Aufbewahrungsmedium bei 2 bis 8 °C für einen Zeitraum von mehr als 24 Stunden, mehr als 24 Stunden und bis zu etwa 48 Stunden, mehr als 24 Stunden und bis zu etwa 72 Stunden oder mehr als 72 Stunden.

**22.** Verwendung eines Aufbewahrungsmediums nach Anspruch 17, 18 oder 19:

(i) zum Konservieren von Stammzellen durch Aufbewahren von Stammzellen in dem Aufbewahrungsmedium,
(ii) zum Konservierung von Stammzellen durch Aufbewahren von Stammzellen in dem Aufbewahrungsmedium, wobei die Stammzellen in einem Kryokonservierungsmedium eingefroren, aufgetaut und anschließend vor der Aufbewahrung auf das Aufbewahrungsmedium übertragen wurden,
(iii) zum Konservieren von Stammzellen nach Teil (ii) dieses Anspruchs, wobei das Kryokonservierungsmedium ein Kryokonservierungsmedium nach Anspruch 16 ist;
(iv) zum Konservieren von Stammzellen durch Aufbewahren von Stammzellen in dem Aufbewahrungsmedium, wobei die Stammzellen mit einem Kryokonservierungsmedium gemischt, einem physiologischen Schock ausgesetzt und anschließend vor der Aufbewahrung auf das Aufbewahrungsmedium übertragen wurden, oder
(v) zum Konservieren von Stammzellen nach Teil (iv) dieses Anspruchs, wobei das Kryokonservierungsmedium ein Kryokonservierungsmedium nach Anspruch 16 ist.

**23.** Verwendung von aus Hefe abgeleitetem rekombinantem Serumalbumin:

(i) zum Verbessern der Lebensfähigkeit von kryokonservierten Stammzellen nach dem Auftauen,
(ii) zum Verbessern der Lebensfähigkeit von kryokonservierten Stammzellen nach dem Auftauen nach Teil (i) dieses Anspruchs, wobei die Verbesserung mit aus Plasma gewonnenem Serumalbumin, das in der gleichen Konzentration verwendet wird, verglichen wird;
(iii) zum Verbessern der Lebensfähigkeit von kryokonservierten Stammzellen nach dem Auftauen nach Teil (i) oder (ii) dieses Anspruchs, wobei die Verbesserung bei den nach dem Auftauen erhaltenen Stammzellen zu beobachten ist, wenn sie in einem Aufbewahrungsmedium bei 2 bis 8 °C für einen Zeitraum von mehr als 24 Stunden, mehr als 24 Stunden und bis zu etwa 48 Stunden, mehr als 24 Stunden und bis zu ungefähr 72 Stunden oder mehr als 72 Stunden aufbewahrt werden;
(iv) zum Verbessern der Lebensfähigkeit von kryokonservierten Stammzellen nach dem Auftauen nach einem der Teile (i), (ii) oder (iii) dieses Anspruchs, wobei das aus Hefe abgeleitete rekombinante Serumalbumin verwendet wird, indem es zu einem Kryokonservierungsmedium formuliert und das Kryokonservierungsmedium vor dem Einfrieren mit Stammzellen gemischt wird,
(v) zum Verbessern der Lebensfähigkeit von kryokonservierten Stammzellen nach dem Auftauen nach Teil (iv) dieses Anspruchs, wobei das Kryokonservierungsmedium ein Kryokonservierungsmedium gemäß Anspruch 16 ist;
(vi) zum Verbessern der Lebensfähigkeit von kryokonservierten Stammzellen nach dem Auftauen gemäß einem der Teile (i) bis (v) dieses Anspruchs, wobei das aus Hefe abgeleitete rekombinante Serumalbumin verwendet wird, indem es zu einem Aufbewahrungsmedium formuliert und das Aufbewahrungsmedium nach dem Auftauen mit Stammzellen gemischt wird, oder
(vii) zum Verbessern der Lebensfähigkeit von kryokonservierten Stammzellen nach dem Auftauen nach Teil (vi) dieses Anspruchs, wobei das Aufbewahrungsmedium ein Medium gemäß Anspruch 17, 18 oder 19 ist.

**24.** Verwendung eines aus Hefe abgeleiteten rekombinanten Serumalbumins:

(i) zur Verbesserung der Lebensfähigkeit von Stammzellen, die einem physiologischen Schock ausgesetzt sind,
(ii) zur Verbesserung der Lebensfähigkeit von Stammzellen, die einem physiologischen Schock nach Teil (i) dieses Anspruchs ausgesetzt sind, wobei die Verbesserung mit der Verwendung von aus Plasma gewonnenem Serumalbumin, das in der gleichen Konzentration verwendet wird, verglichen wird;
(iii) zur Verbesserung der Lebensfähigkeit von Stammzellen, die einem physiologischen Schock nach Teil (i) oder (ii) dieses Anspruchs ausgesetzt sind, wobei die Verbesserung bei den nach dem Schock erhaltenen Stammzellen zu beobachten ist, wenn sie in einem Aufbewahrungsmedium bei 2 bis 8 °C für einen Zeitraum von mehr als 24 Stunden, mehr als 24 Stunden und bis zu etwa 48 Stunden, mehr als 24 Stunden und bis zu ungefähr 72 Stunden oder mehr als 72 Stunden aufbewahrt werden;
(iv) zur Verbesserung der Lebensfähigkeit von Stammzellen, die einem physiologischen Schock nach einem der Teile (i), (ii) oder (iii) dieses Anspruchs ausgesetzt sind, wobei das aus Hefe abgeleitete rekombinante Serumalbumin verwendet wird, indem es zu einem Kryokonservierungsmedium formuliert und das Kryokonservierungsmedium vor dem der physiologischen Schock mit Stammzellen gemischt wird,
(v) zur Verbesserung der Lebensfähigkeit von Stammzellen, die einem physiologischen Schock nach Teil (iv) dieses Anspruchs ausgesetzt sind, wobei das Kryokonservierungsmedium ein Kryokonservierungsmedium gemäß Anspruch 16 ist;
(vi) zur Verbesserung der Lebensfähigkeit von Stammzellen, die einem physiologischen Schock nach einem der Teile (i) bis (v) dieses Anspruchs ausgesetzt sind, wobei das aus Hefe abgeleitete rekombinante Serumalbumin verwendet wird, indem es zu einem Aufbewahrungsmedium formuliert und das Aufbewahrungsmedium nach dem der physiologischen Schock mit Stammzellen gemischt wird, oder
(vii) zur Verbesserung der Lebensfähigkeit von Stammzellen, die einem physiologischen Schock gemäß Teil (vi) dieses Anspruchs ausgesetzt sind, wobei das Aufbewahrungsmedium ein Aufbewahrungsmedium gemäß Anspruch 17, 18 oder 19 ist.

## Revendications

1. Procédé de conservation de cellules souches, le procédé comprenant les étapes consistant à :

(i) combiner les cellules souches avec un milieu de cryoconservation pour obtenir un mélange, et congeler le mélange pour obtenir un produit de cellules souches congelé,
(ii) combiner les cellules souches avec un milieu de cryoconservation pour obtenir un mélange, et congeler le mélange pour obtenir un produit de cellules souches congelé, le procédé comprenant en outre les étapes consistant à décongeler le produit de cellules souches congelé, transférer les cellules décongelées sur un milieu de stockage, et à stocker les cellules souches dans le milieu de stockage, ou à
(iii) stocker les cellules souches dans un milieu de stockage, les cellules souches ayant été congelées dans un milieu de cryoconservation, décongelées, puis transférées sur le milieu de stockage avant le stockage ;
dans lequel le milieu de cryoconservation et/ou le milieu de stockage comprend une préparation de sérum-albumine recombinante dérivée d'une levure.

2. Procédé selon la revendication 1, le procédé comprenant les étapes consistant à congeler des cellules souches dans le milieu de cryoconservation de façon à obtenir un produit de cellules souches congelé, décongeler le produit de cellules souches congelé, transférer les cellules décongelées sur le milieu de stockage et stocker les cellules souches dans le milieu de stockage, le milieu de cryoconservation et/ou le milieu de stockage comprenant une préparation de sérum-albumine recombinante dérivée d'une levure.

3. Procédé selon l'une des revendications précédentes, dans lequel la préparation de sérum-albumine recombinante dérivée d'une levure est présente dans le milieu de cryoconservation et/ou dans le milieu de stockage, dans lequel, lorsqu'elle est mélangée avec les cellules souches en une quantité appropriée pour obtenir une concentration de la protéine de sérum-albumine recombinante dérivée d'une levure, la concentration est choisie parmi les valeurs suivantes : (i) supérieure à environ 0,01 % (p/v) et inférieure à 10 % (p/v), (ii) comprise entre environ 0,1 % (p/v) et environ 5 % (p/v), ou (iii) environ 2 % (p/v).

4. Procédé selon l'une des revendications précédentes, dans lequel :

(a) les cellules souches sont stockées dans le milieu de stockage à une température de 2 à 8 °C ; et/ou
(b) les cellules souches sont stockées dans le milieu de stockage pendant une durée choisie parmi les valeurs suivantes : supérieure à 24 heures ; supérieure à 24 heures et allant jusqu'à environ 48 heures ; supérieure à 24 heures et allant jusqu'à environ 72 heures ; ou supérieure à environ 72 heures ; ou
(c) les cellules souches sont stockées dans le milieu de stockage à une température de 2 à 8 °C pendant une durée choisie parmi les valeurs suivantes : supérieure à 24 heures ; supérieure à 24 heures et allant jusqu'à environ 48 heures ; supérieure à 24 heures et allant jusqu'à environ 72 heures ; ou supérieure à environ 72 heures ; et dans lequel la viabilité des cellules souches à la fin de la période de stockage est supérieure à 5 %, 10 %, 20 %, 30 %, 40 %, 50 %, 60 %, 70 %, 75 %, 80 %, 85 %, 86 %, 87 %, 88 %, 89 %, 90 %, 91 %, 92 %, 93 %, 94 %, 95 % ou plus.

5. Procédé selon l'une des revendications précédentes, dans lequel la protéine de sérum-albumine recombinante dérivée d'une levure présente dans le milieu de cryoconservation et/ou le milieu de stockage présente une ou plusieurs des propriétés suivantes :

(a) moins de 0,5 % (p/p), 0,4 % (p/p), 0,3 % (p/p), 0,2 % (p/p) ou 0,15 % (p/p) se lie à la concanavaline A ; et/ou
(b) un taux de glycation inférieur à 0,6, 0,10, 0,075 ou 0,05 mole d'hexose/mole de protéine.

6. Procédé selon l'une des revendications précédentes, dans lequel la protéine de sérum-albumine recombinante dérivée d'une levure présente dans le milieu de cryoconservation et/ou dans le milieu de stockage :

(a) est d'au moins environ 95 %, 96 %, 97 %, 98 %, 99,5 % ou essentiellement 100 % monomère et dimère ;
(b) est d'au moins environ 93 %, 94 %, 95 %, 96 % ou 97 % monomère ; et/ou
(c) a une teneur en polymère d'albumine non supérieure ou inférieure à environ 1,0 % (p/p), 0,1 % (p/p) ou 0,01 % (p/p), le polymère d'albumine étant distinct des formes monomères et dimères de l'albumine.

7. Procédé selon l'une des revendications précédentes, dans lequel la préparation de sérum-albumine recombinante dérivée d'une levure présente dans le milieu de cryoconservation et/ou dans le milieu de stockage :

(a) comprend, est essentiellement constituée, ou est constituée de protéine sérum-albumine dérivée d'une levure, de cations et d'anions d'équilibrage, d'eau, et éventuellement d'octanoate et de polysorbate 80 ;
(b) possède une séquence N-terminale intacte ou sensiblement intacte ;
(c) présente, lorsqu'elle est testée par spectrométrie de masse, sensiblement moins de pics distincts du pic principal à environ 66,4 kDa, ce qui est représentatif de la molécule de sérum-albumine humaine native intacte, par rapport à la protéine de sérum-albumine recombinante d'origine végétale ;
(d) comprend une protéine d'albumine ayant une teneur en groupe thiol libre qui est supérieure à 62 %, ou ayant une teneur en groupe thiol libre qui est d'au moins 69 %, d'au moins 70 %, d'au moins 75 %, d'au moins 80 %, d'au moins 85 %, d'au moins 90 %, d'au moins 95 %, d'environ 96 % et d'environ 97 % ;
(e) comprend une protéine d'albumine qui, lorsqu'elle est testée par chromatographie d'exclusion de taille (SEC), présente un profil SEC excluant des pics avec un temps de rétention de pic inférieur à 14 minutes et supérieur à 19 minutes, ou un profil SEC qui exclut les pics avec un temps de rétention de pic inférieur à 14 ou 15 minutes et supérieur à 18 minutes ;
(f) comprend une protéine d'albumine qui, lorsqu'elle est testée par chromatographie liquide haute performance en phase inversée (RP-HPLC), présente un pic majeur unique, correspondant à l'albumine sous sa forme monomère native ;
(g) comprend une protéine d'albumine qui, lorsqu'elle est testée par spectrométrie de masse, est un produit qui présente moins de 13, 12, 11, 10, 9, 8, 7 ou 6 résidus de lysine et/ou d'arginine modifiés par hexose par protéine, ou environ 1 à 11, 1 à 8, 1 à 5, 1 à 4, 1 à 3, 1 à 2, 1 ou moins d'un résidu de lysine et/ou d'arginine modifié par hexose par protéine ; et/ou
(h) comprend une protéine d'albumine qui n'est pas glycatée avec du sucre spécifique aux végétaux, ou comprend une protéine d'albumine qui n'est pas glycatée avec un sucre spécifique aux végétaux choisi parmi $\alpha$-1,3-fucose et/ou $\beta$-1,2-xylose.

8. Procédé selon l'une des revendications précédentes, dans lequel la préparation de sérum-albumine recombinante dérivée d'une levure présente dans le milieu de cryoconservation et/ou le milieu de stockage, et/ou le milieu de cryoconservation et/ou le milieu de stockage lui-même :

(a) comprend de l'octanoate à moins de 35 mM, 20 mM, 10 mM, 5 mM, 1 mM, 0,1 mM, 0,01 mM, 0,001 mM, est sensiblement exempt(e) d'octanoate, ou est exempt(e) d'octanoate ;
(b) a une teneur totale en acides gras inférieure ou égale à 35 mM, 20 mM, 10 mM, 5 mM, 1 mM, est sensiblement exempt(e) d'acides gras, ou est exempt(e) d'acides gras ;
(c) comprend un détergent à une concentration inférieure à 200 $mg.L^{-1}$, 100 $mg.L^{-1}$, 50 $mg.L^{-1}$, 10 $mg.L^{-1}$, 1 $mg.L^{-1}$, 0,1 $mg.L^{-1}$, 0,01 $mg.L^{-1}$, 0,001 $mg.L^{-1}$, est sensiblement exempt(e) du détergent, ou est exempt(e) du détergent ;
(d) comprend un niveau total d'acides aminés libres et/ou des niveaux de N-acétyl tryptophane inférieurs à 35 mM, 20 mM, 10 mM, 5 mM, 1 mM, 0,1 mM, 0,01 mM, 0,005 mM, 0,001 mM, est sensiblement exempt(e) d'acides aminés libres et/ou de N-acétyl tryptophane en particulier, ou est exempt(e) d'acides aminés libres et/ou de N-acétyl tryptophane en particulier ;
(e) est sensiblement exempt(e) ou totalement exempt(e) de tout octanoate, d'acides aminés libres et/ou de N-acétyl tryptophane en particulier, et de détergent ; et/ou
(f) est exempt(e) d'un ou de plusieurs, notamment de tous les composants choisis parmi : un hème, un activateur de prékallicréine, des pyrogènes, l'hépatite C et/ou des virus humains et/ou a une concentration en aluminium inférieure à 200 $\mu$g.L-1, 100 $\mu$g.L-1, 50 $\mu$g.L-1, ou comprise entre environ 10 $\mu g.L^{-1}$ et environ 30 $\mu g.L^{-1}$.

9. Procédé selon la revendication 8, dans lequel la préparation de sérum-albumine recombinante dérivée d'une levure présente dans le milieu de cryoconservation et/ou le milieu de stockage, et/ou le milieu de cryoconservation et/ou le milieu de stockage lui-même comprend un détergent à une concentration inférieure à 200 $mg.L^{-1}$, 100 $mg.L^{-1}$, 50 $mg.L^{-1}$, 10 $mg.L^{-1}$, 1 $mg.L^{-1}$, 0,1 $mg.L^{-1}$, 0,01 $mg.L^{-1}$, 0,001 $mg.L^{-1}$, est sensiblement exempt(e) du détergent, ou est exempt(e) du détergent, le détergent étant du polysorbate ou du polysorbate 80.

**10.** Procédé selon la revendication 8 ou 9, dans lequel la préparation de sérum-albumine recombinante dérivée d'une levure présente dans le milieu de cryoconservation et/ou le milieu de stockage, et/ou le milieu de cryoconservation et/ou le milieu de stockage lui-même est sensiblement exempt(e) ou totalement exempt(e) de tout octanoate, d'acides aminés libres et/ou de N-acétyl tryptophane en particulier, et de détergent, le détergent étant du polysorbate 80.

**11.** Procédé selon l'une des revendications précédentes, dans lequel :

(a) le milieu de cryoconservation comprend la préparation de sérum-albumine recombinante et un cryoconservateur, ou
(b) le milieu de cryoconservation comprend, est essentiellement constitué, ou est constitué d'une solution aqueuse de la préparation de sérum-albumine recombinante dérivée d'une levure, d'un cryoconservateur et d'un tampon ionique ; dans lequel :

(i) le milieu de cryoconservation n'est pas un milieu de croissance pour la culture de cellules souches,
(ii) le milieu de cryoconservation n'est pas un milieu de croissance pour la culture de cellules souches et ne favorise pas la croissance des cellules souches, ou
(iii) le milieu de cryoconservation n'est pas un milieu de croissance pour la culture de cellules souches, ne favorise pas la croissance des cellules souches et ne comprend sensiblement aucun, ou ne comprend aucun niveau d'un ou de plusieurs, ou de tous les composants d'un milieu de culture de cellules souches typique.

**12.** Procédé selon la revendication 11, dans lequel le milieu de cryoconservation n'est pas un milieu de croissance pour la culture de cellules souches, ne favorise pas la croissance des cellules souches et ne comprend sensiblement aucun, ou ne comprend aucun niveau d'un ou de plusieurs, ou de tous des composants d'un milieu de culture de cellules souches typique, et les composants d'un milieu de culture de cellules souches typique comprenant des vitamines, des hormones, des facteurs de croissance, des sources de fer, des acides aminés libres et/ou du glucose.

**13.** Procédé selon l'une des revendications précédentes, dans lequel :

(a) le milieu de stockage comprend la préparation de sérum-albumine recombinante dérivée d'une levure, ou
(b) le milieu de stockage comprend, est essentiellement constitué, ou est constitué d'une solution aqueuse de la préparation de sérum-albumine recombinante dérivée d'une levure et d'un tampon ionique, et dans lequel :

(i) le milieu de stockage n'est pas un milieu de croissance pour la culture de cellules souches,
(ii) le milieu de stockage n'est pas un milieu de croissance pour la culture de cellules souches et ne favorise pas la croissance des cellules souches, ou
(iii) le milieu de stockage n'est pas un milieu de croissance pour la culture de cellules souches, ne favorise pas la croissance des cellules souches et ne comprend sensiblement aucun, ou ne comprend aucun niveau d'un ou de plusieurs, ou de tous les composants d'un milieu de culture de cellules souches typique.

**14.** Procédé selon la revendication 13, dans lequel le milieu de stockage n'est pas un milieu de croissance pour la culture de cellules souches, ne favorise pas la croissance des cellules souches et ne comprend sensiblement aucun, ou ne comprend aucun niveau d'un ou de plusieurs, ou de tous des composants d'un milieu de culture de cellules souches typique, et les composants d'un milieu de culture de cellules souches typique comprenant des vitamines, des hormones, des facteurs de croissance, des sources de fer, des acides aminés libres et/ou du glucose.

**15.** Procédé selon l'une des revendications précédentes, ledit procédé consistant à :

(i) stocker des cellules souches dans le milieu de stockage,
(ii) stocker des cellules souches dans le milieu de stockage à une température de 2 à 8 °C, ou
(iii) stocker les cellules souches dans le milieu de stockage à une température de 2 à 8 °C pendant une durée choisie parmi les valeurs suivantes : supérieure à 24 heures ; supérieure à 24 heures et allant jusqu'à environ 48 heures ; supérieure à 24 heures et allant jusqu'à environ 72 heures ; ou supérieure à 72 heures, et
dans lequel, directement ou indirectement après l'étape de stockage de cellules souches dans le milieu de stockage conformément au procédé défini dans l'une des parties (i), (ii) ou (iii) selon la présente revendication, le procédé comprend en outre une ou plusieurs étapes choisies parmi les étapes consistant à : cultiver les cellules souches ; développer une culture des cellules souches ; différencier les cellules souches ; immobiliser les cellules souches ou les cellules cultivées et/ou différenciées qui en sont dérivées ; immobiliser les cellules souches ou les cellules cultivées et/ou différenciées qui en sont dérivées dans un tissu ou un implant médical ; et/ou formuler des cellules souches ou des cellules cultivées et/ou différenciées ou d'autres produits qui en sont dérivés dans une composition pharmaceutiquement acceptable ou une composition acceptable du point de vue vétérinaire.

**16.** Milieu de cryoconservation pour la cryoconservation de cellules souches,

(i) le milieu de cryoconservation comprenant une préparation de sérum-albumine recombinante dérivée d'une levure et un cryoconservateur, ou le milieu de cryoconservation comprenant une préparation de sérum-albumine recombinante dérivée d'une levure et un cryoconservateur, et étant un milieu de cryoconservation tel que défini dans l'une des revendications précédentes,
(ii) le milieu de cryoconservation étant un milieu de cryoconservation tel que défini dans la partie (i) selon la présente revendication, lequel comprend en outre des cellules souches, ou
(iii) le milieu de cryoconservation étant une cryoconservation comprenant des cellules souches telles que définies dans la partie (ii) selon la présente revendication, et le milieu de cryoconservation étant congelé, ou étant un milieu de cryoconservation comprenant des cellules souches telles que définies dans une partie selon la présente revendication, lequel milieu a été congelé puis décongelé.

**17.** Milieu de stockage destiné au stockage de cellules souches qui ont été congelées dans un milieu de cryoconservation, décongelées, puis transférées sur le milieu de stockage :

(i) le milieu de stockage comprenant une préparation de sérum-albumine recombinante dérivée d'une levure et le milieu de stockage n'étant pas un milieu de croissance pour la culture de cellules souches, ou le milieu de stockage étant un milieu de stockage tel que défini dans l'une des revendications 1 à 115, et n'étant pas un milieu de croissance pour la culture de cellules souches,
(ii) le milieu de stockage étant un milieu de stockage tel que défini dans la partie (i) selon la présente revendication, et comprenant en outre des cellules souches,
(iii) le milieu de stockage étant un milieu de stockage tel que défini dans la partie (ii) selon la présente revendication, et les cellules souches ayant été congelées dans un milieu de cryoconservation, décongelées, puis transférées sur le milieu de stockage, ou
(iv) le milieu de stockage étant un milieu de stockage tel que défini dans la partie (iii) selon la présente revendication, et le milieu de cryoconservation étant un milieu de cryoconservation tel que défini dans la revendication 16.

**18.** Milieu de stockage selon la revendication 17, le milieu de stockage :

(a) ne favorisant pas la croissance de cellules souches ; et/ou
(b) ne comprenant sensiblement aucun, ou ne comprenant aucun niveau d'un ou de plusieurs des éléments que sont des vitamines, des hormones, des facteurs de croissance, des sources de fer, des acides aminés libres et/ou du glucose.

**19.** Milieu de stockage selon la revendication 17 ou 18, comprenant en outre des cellules souches :

(i) qui ont été : (a) congelées dans un milieu de cryoconservation, décongelées, puis transférées sur le milieu de stockage, ou (b) soumises à un autre choc physiologique avant leur transfert sur le milieu de stockage,
(ii) qui ont été stockées dans le milieu de stockage à une température de 2 à 8 °C pendant une durée choisie parmi les valeurs suivantes : supérieure à 24 heures ; supérieure à 24 heures et allant jusqu'à environ 48 heures ; supérieure à 24 heures et allant jusqu'à environ 72 heures ; ou supérieure à 72 heures, et
(iii) la viabilité des cellules souches à la fin de la période de stockage étant supérieure à 5 %, 10 %, 20 %, 30 %, 40 %, 50 %, 60 %, 70 %, 75 %, 80 %, 85 %, 86 %, 87 %, 88 %, 89 %, 90 %, 91 %, 92 %, 93 %, 94 %, 95 % ou plus.

**20.** Utilisation d'un milieu de cryoconservation selon la revendication 16 :

(i) pour la conservation de cellules souches,

(ii) pour la conservation de cellules souches dans un état viable après stockage de celles-ci à une température de 2 à 8 °C pendant une durée supérieure à 24 heures, supérieure à 24 heures et allant jusqu'à environ 48 heures, supérieure à 24 heures et allant jusqu'à environ 72 heures, ou supérieure à 72 heures ; ou
(iii) telle que définie dans la partie (i) ou (ii) selon la présente revendication, dans laquelle la viabilité des cellules souches à la fin de la période de stockage est supérieure à 5 %, 10 %, 20 %, 30 %, 40 %, 50 %, 60 %, 70 %, 75 %, 80 %, 85 %, 86 %, 87 %, 88 %, 89 %, 90 %, 91 %, 92 %, 93 %, 94 %, 95 % ou plus.

**21.** Utilisation selon la revendication 20 :

(a) pour la conservation de cellules souches en combinant celles-ci avec le milieu de cryoconservation pour obtenir un mélange, et en congelant le mélange pour obtenir un produit de cellules souches congelé, ou en soumettant les cellules souches à un autre choc physiologique avant le stockage à une température de 2 à 8 °C pendant une durée supérieure à 24 heures, supérieure à 24 heures et allant jusqu'à environ 48 heures, supérieure à 24 heures et allant jusqu'à environ 72 heures, ou supérieure à 72 heures ;
(b) pour la conservation de cellules souches par un procédé comprenant en outre les étapes consistant à décongeler le produit de cellules souches congelé, à transférer les cellules décongelées sur un milieu de stockage et à stocker les cellules souches dans le milieu de stockage à une température de 2 à 8 °C pendant une durée supérieure à 24 heures, supérieure à 24 heures et allant jusqu'à environ 48 heures, supérieure à 24 heures et allant jusqu'à environ 72 heures, ou supérieure à 72 heures ; ou
(c) pour la conservation de cellules souches en combinant les cellules souches avec le milieu de cryoconservation pour obtenir un mélange, en soumettant les cellules souches à un choc physiologique, en transférant les cellules sur un milieu de stockage et en stockant les cellules souches dans le milieu de stockage à une température de 2 à 8 °C pendant une durée supérieure à 24 heures, supérieure à 24 heures et allant jusqu'à environ 48 heures, supérieure à 24 heures et allant jusqu'à environ 72 heures, ou supérieure à 72 heures.

**22.** Utilisation d'un milieu de stockage selon la revendication 17, 18 ou 19 :

(i) pour la conservation de cellules souches en stockant celles-ci dans le milieu de stockage,
(ii) pour la conservation de cellules souches en les stockant dans le milieu de stockage, les cellules souches ayant été congelées dans un milieu de cryoconservation, décongelées, puis transférées sur le milieu de stockage avant le stockage,
(iii) pour la conservation de cellules souches conformément à la partie (ii) selon la présente revendication, le milieu de cryoconservation étant un milieu de cryoconservation selon la revendication 16 ;
(iv) pour la conservation de cellules souches en stockant celles-ci dans le milieu de stockage, les cellules souches ayant été mélangées à un milieu de cryoconservation, soumises à un choc physiologique, puis transférées sur le milieu de stockage avant le stockage, ou
(v) pour la conservation de cellules souches conformément à la partie (iv) selon la présente revendication, le milieu de cryoconservation étant un milieu de cryoconservation selon la revendication 16.

**23.** Utilisation de sérum-albumine recombinante dérivée d'une levure :

(i) pour améliorer la viabilité de cellules souches cryoconservées après la décongélation,
(ii) pour améliorer la viabilité de cellules souches cryoconservées après décongélation conformément à la partie (i) selon la présente revendication, l'amélioration étant comparée à la sérum-albumine d'origine plasmatique utilisée avec la même concentration ;
(iii) pour améliorer la viabilité de cellules souches cryoconservées après décongélation conformément à la partie (i) ou (ii) selon la présente revendication, l'amélioration étant observable dans les cellules souches post-décongelées lorsqu'elles sont stockées dans un milieu de stockage à une température de 2 à 8 °C pendant une durée supérieure à 24 heures, supérieure à 24 heures et allant jusqu'à environ 48 heures, supérieure à 24 heures et allant jusqu'à environ 72 heures, ou supérieure à 72 heures ;
(iv) pour améliorer la viabilité de cellules souches cryoconservées après décongélation conformément à l'une des parties (i), (ii) ou (iii) selon la présente revendication, la sérum-albumine recombinante dérivée d'une levure étant utilisée en la formulant dans un milieu de cryoconservation et en mélangeant le milieu de cryoconservation avec des cellules souches avant la congélation,
(v) pour améliorer la viabilité de cellules souches cryoconservées après décongélation conformément à la partie (iv) selon la présente revendication, le milieu de cryoconservation étant un milieu de cryoconservation tel que défini dans la revendication 16 ;
(vi) pour améliorer la viabilité de cellules souches cryoconservées après décongélation conformément à l'une des parties (i) à (v) selon la présente revendication, la sérum-albumine recombinante dérivée d'une levure étant utilisée en la formulant dans un milieu de stockage et en mélangeant le milieu de stockage avec des cellules souches après décongélation, ou
(vii) pour améliorer la viabilité de cellules souches cryoconservées après décongélation conformément à la partie (vi) selon la présente revendication, le milieu de stockage étant un milieu tel que défini dans la revendication 17, 18 ou 19.

**24.** Utilisation de sérum-albumine recombinante dérivée d'une levure :

(i) pour améliorer la viabilité de cellules souches soumises à un choc physiologique,
(ii) pour améliorer la viabilité de cellules souches soumises à un choc physiologique conformément à la partie (i) selon la présente revendication, l'amélioration étant comparée à l'utilisation de sérum-albumine d'origine plasmatique utilisée avec la même concentration ;
(iii) pour améliorer la viabilité de cellules souches soumises à un choc physiologique conformément à la partie (i) ou (ii) selon la présente revendication, l'amélioration étant observable dans les cellules souches post-choc lorsqu'elles sont stockées dans un milieu de stockage à une température de 2 à 8 °C pendant une durée supérieure à 24 heures, supérieure à 24 heures et allant jusqu'à environ 48 heures, supérieure à 24 heures et allant jusqu'à environ 72 heures, ou supérieure à 72 heures ;
(iv) pour améliorer la viabilité de cellules souches soumises à un choc physiologique conformément à l'une des parties (i), (ii) ou (iii) selon la présente revendication, la sérum-albumine recombinante dérivée d'une levure étant utilisée en la formulant dans un milieu de cryoconservation et en mélangeant le milieu de cryoconservation avec des cellules souches avant le choc physiologique,
(v) pour améliorer la viabilité de cellules souches soumises à un choc physiologique conformément à la partie (iv) selon la présente revendication, le milieu de cryoconservation étant un milieu de cryoconservation tel que défini dans la revendication 16 ;
(vi) pour améliorer la viabilité de cellules souches soumises à un choc physiologique conformément à l'une des parties (i) à (v) selon la présente revendication, la sérum-albumine recombinante dérivée d'une levure étant utilisée en la formulant dans un milieu de stockage et en mélangeant le milieu de stockage avec des cellules souches après réception du choc physiologique, ou
(vii) pour améliorer la viabilité de cellules souches soumises à un choc physiologique conformément à la partie (vi) selon la présente revendication, le milieu de stockage étant un milieu de stockage tel que défini dans la revendication 17, 18 ou 19.

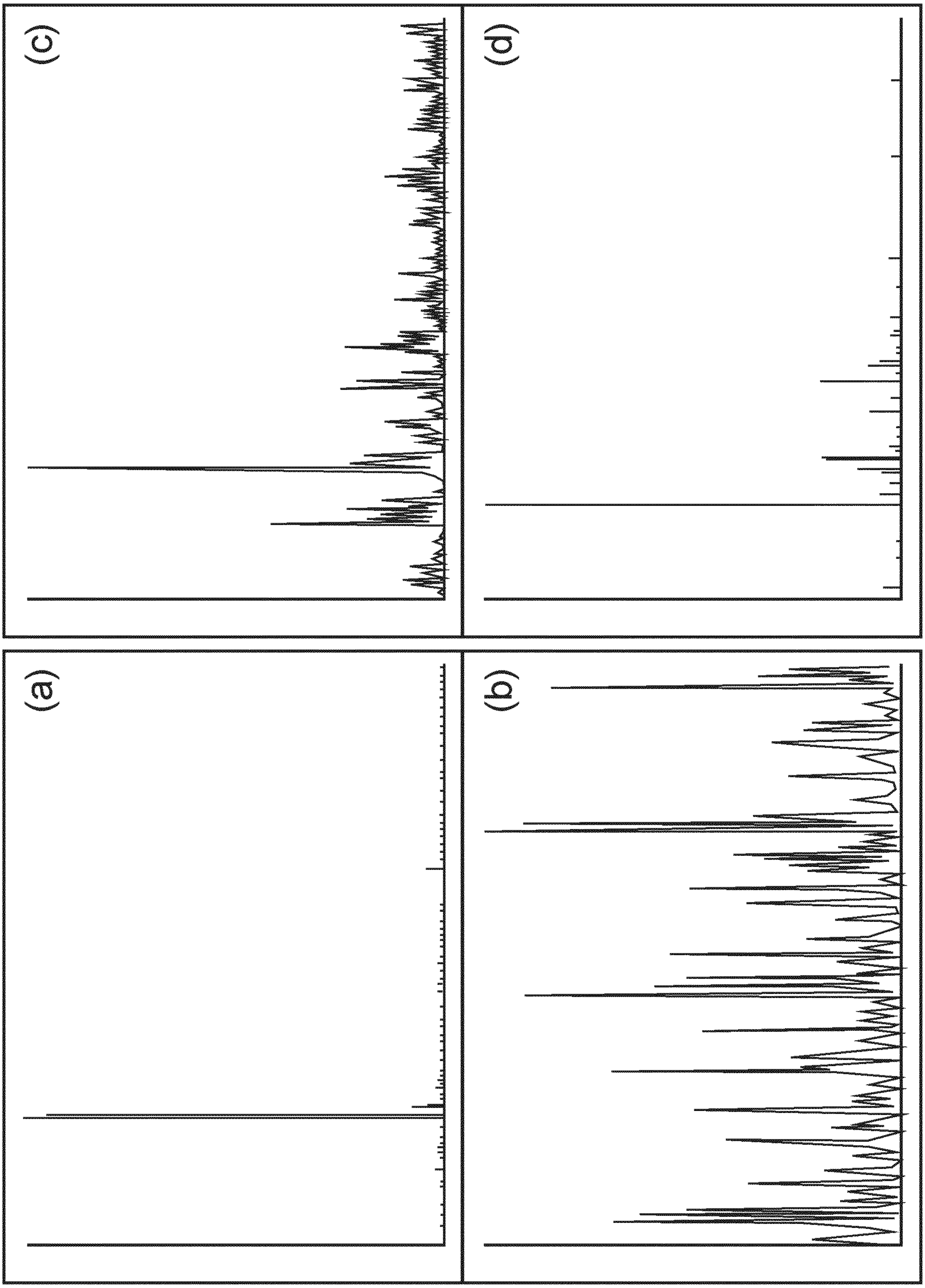


FIG. 1

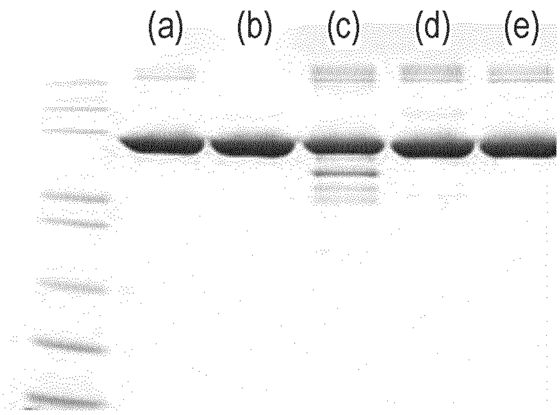
(a)
(b)
(c)
(d)
(e)

FIG. 2

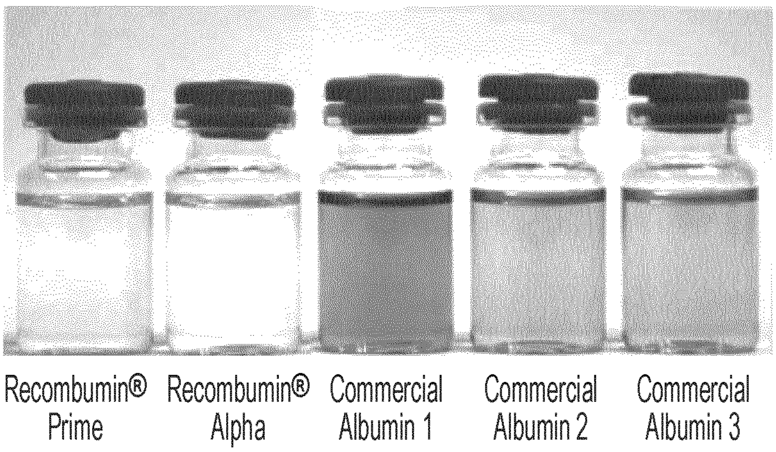
Recombumin® Prime
Recombumin® Alpha
Commercial Albumin 1
Commercial Albumin 2
Commercial Albumin 3

FIG. 3

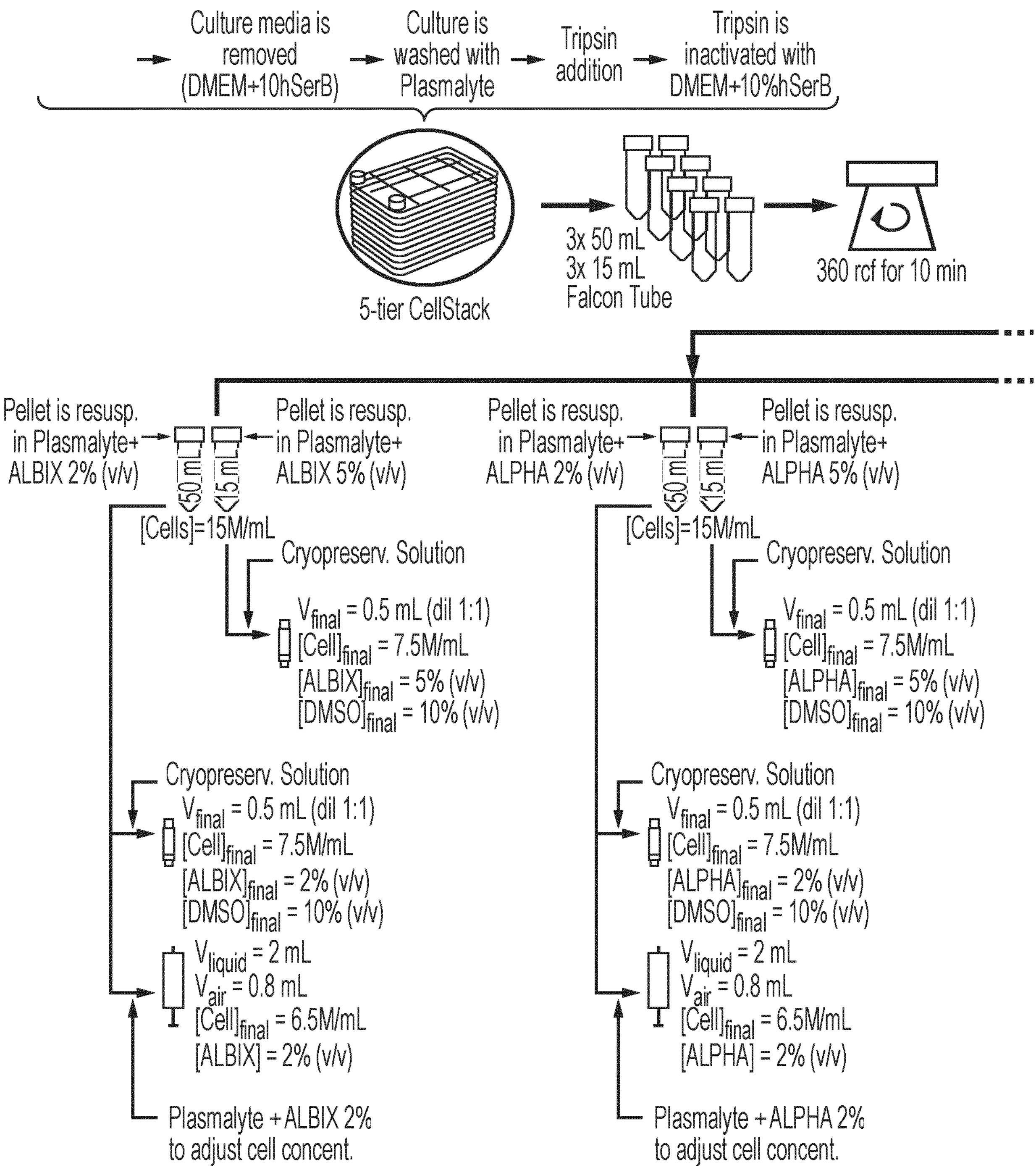
Culture media is removed (DMEM+10hSerB)
Culture is washed with Plasmalyte
Tripsin addition
Tripsin is inactivated with DMEM+10%hSerB
5-tier CellStack
3x 50 mL
3x 15 mL
Falcon Tube
360 rcf for 10 min
Pellet is resusp. in Plasmalyte+ ALBIX 2% (v/v)
Pellet is resusp. in Plasmalyte+ ALBIX 5% (v/v)
Pellet is resusp. in Plasmalyte+ ALPHA 2% (v/v)
Pellet is resusp. in Plasmalyte+ ALPHA 5% (v/v)
50 mL
15 mL
[Cells]=15M/mL
Cryopreserv. Solution
V final = 0.5 mL (dil 1:1)
[Cell] final = 7.5M/mL
[ALBIX] final = 5% (v/v)
[DMSO] final = 10% (v/v)
[ALPHA] final = 5% (v/v)
[ALBIX] final = 2% (v/v)
[ALPHA] final = 2% (v/v)
V liquid = 2 mL
V air = 0.8 mL
[Cell] final = 6.5M/mL
[ALBIX] = 2% (v/v)
[ALPHA] = 2% (v/v)
Plasmalyte + ALBIX 2% to adjust cell concent.
Plasmalyte + ALPHA 2% to adjust cell concent.

FIG. 4

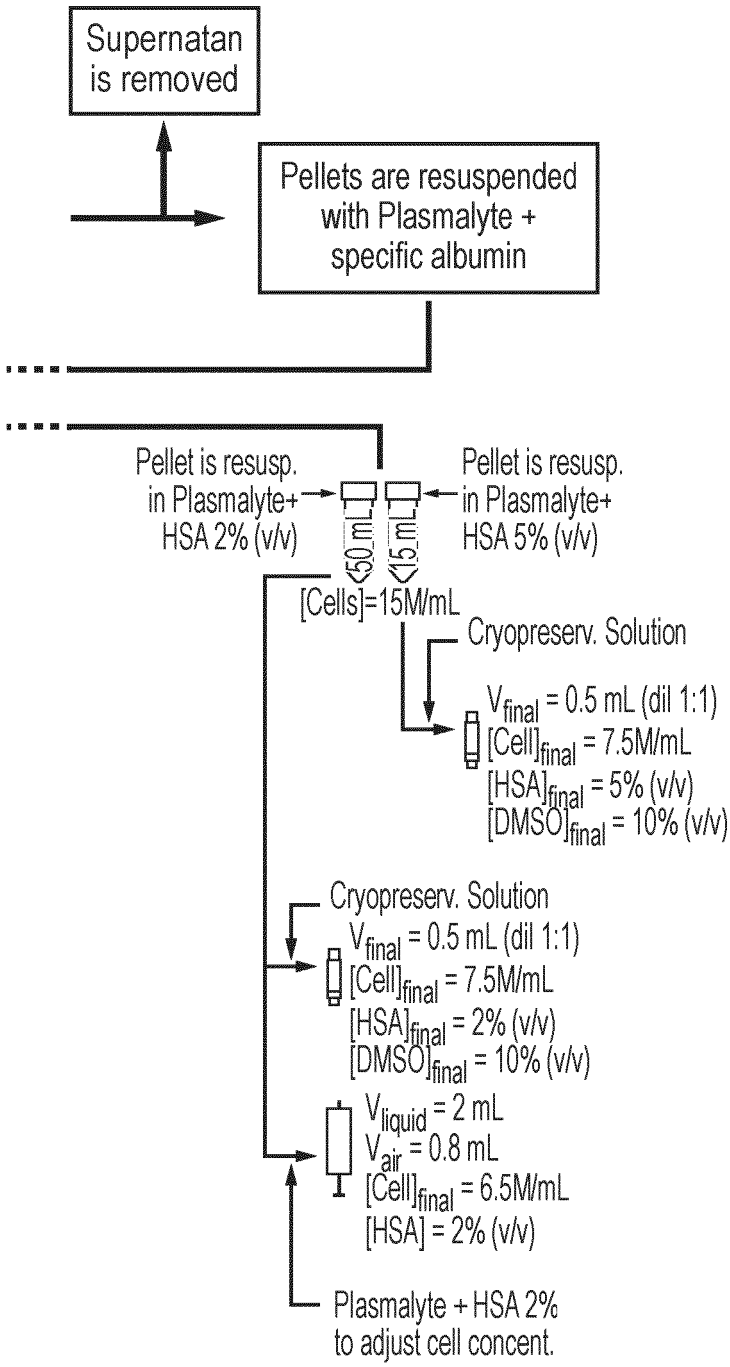


FIG. 4 (Continued)

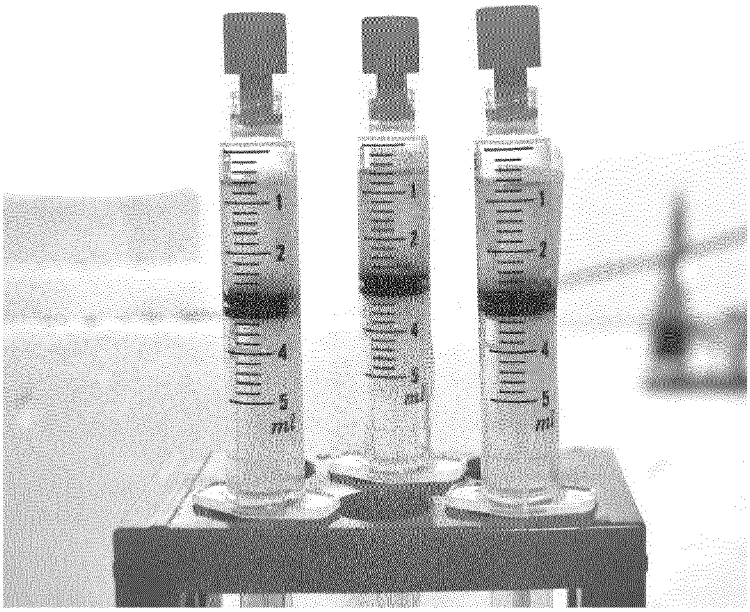


FIG. 5

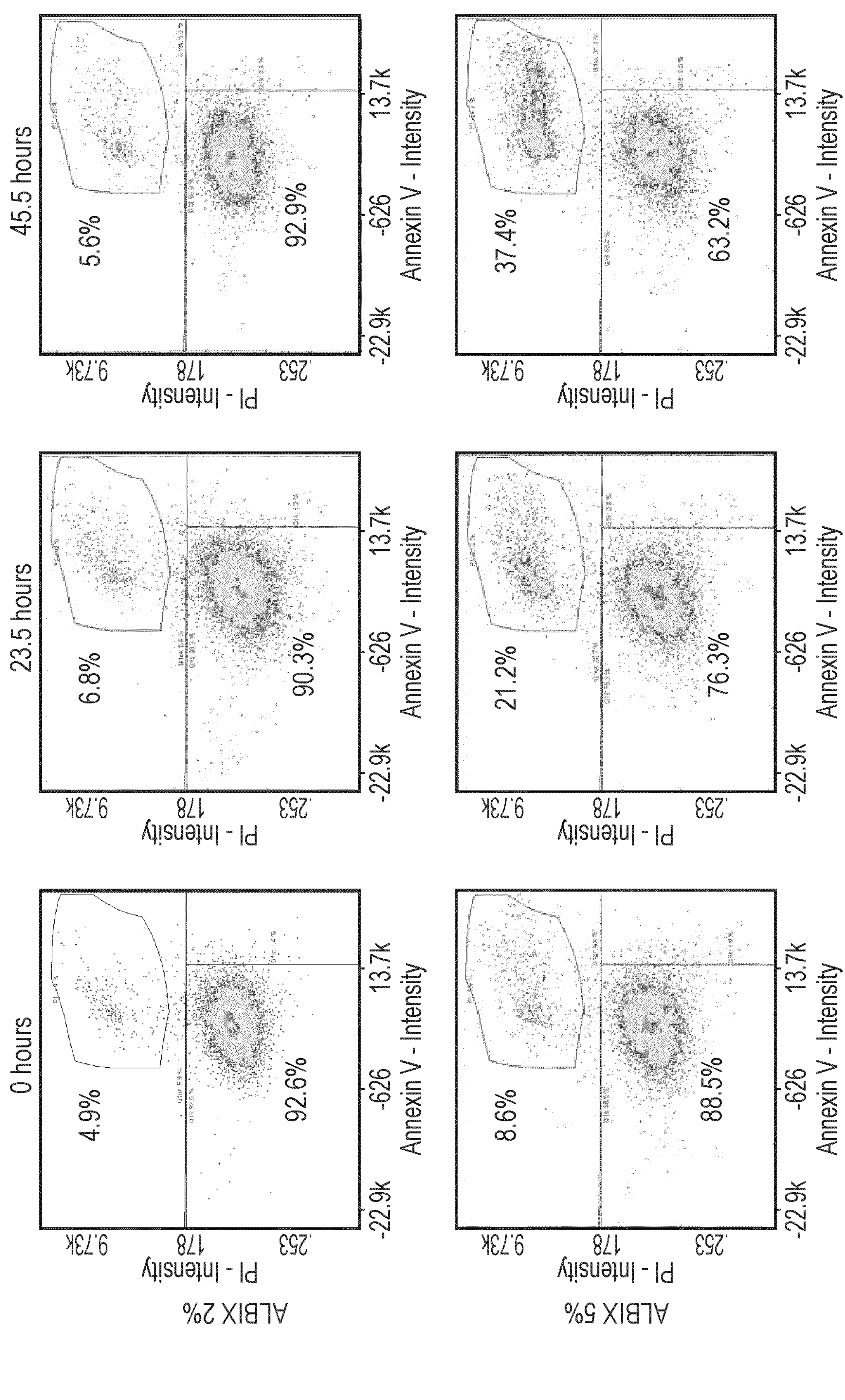
0 hours
23.5 hours
45.5 hours
ALBIX 2%
ALBIX 5%
4.9%
92.6%
6.8%
90.3%
5.6%
92.9%
8.6%
88.5%
21.2%
76.3%
37.4%
63.2%
PI - Intensity
9.73K
178
.253
Annexin V - Intensity
-22.9k
-626
13.7k

FIG. 6

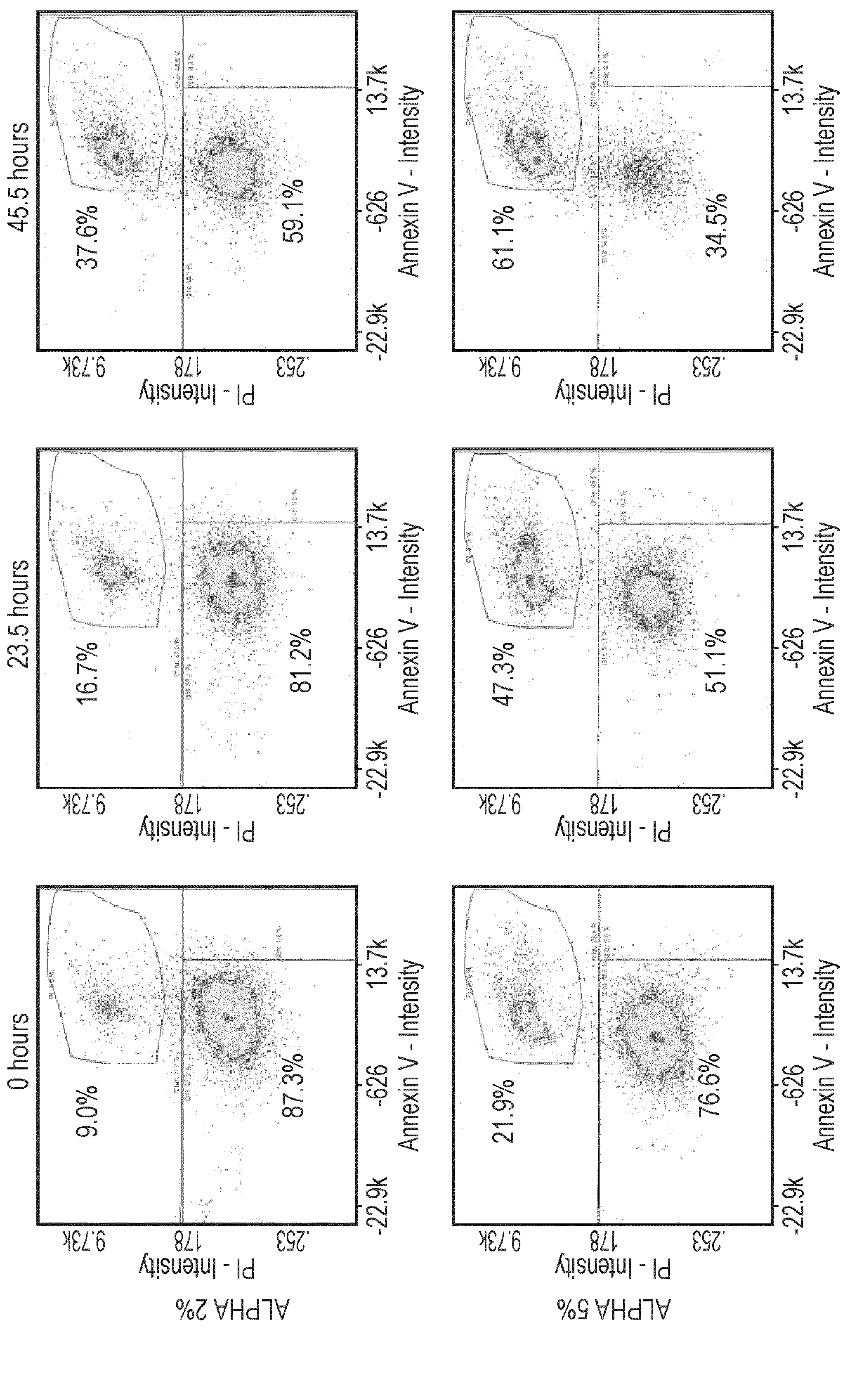


FIG. 6 (Continued)

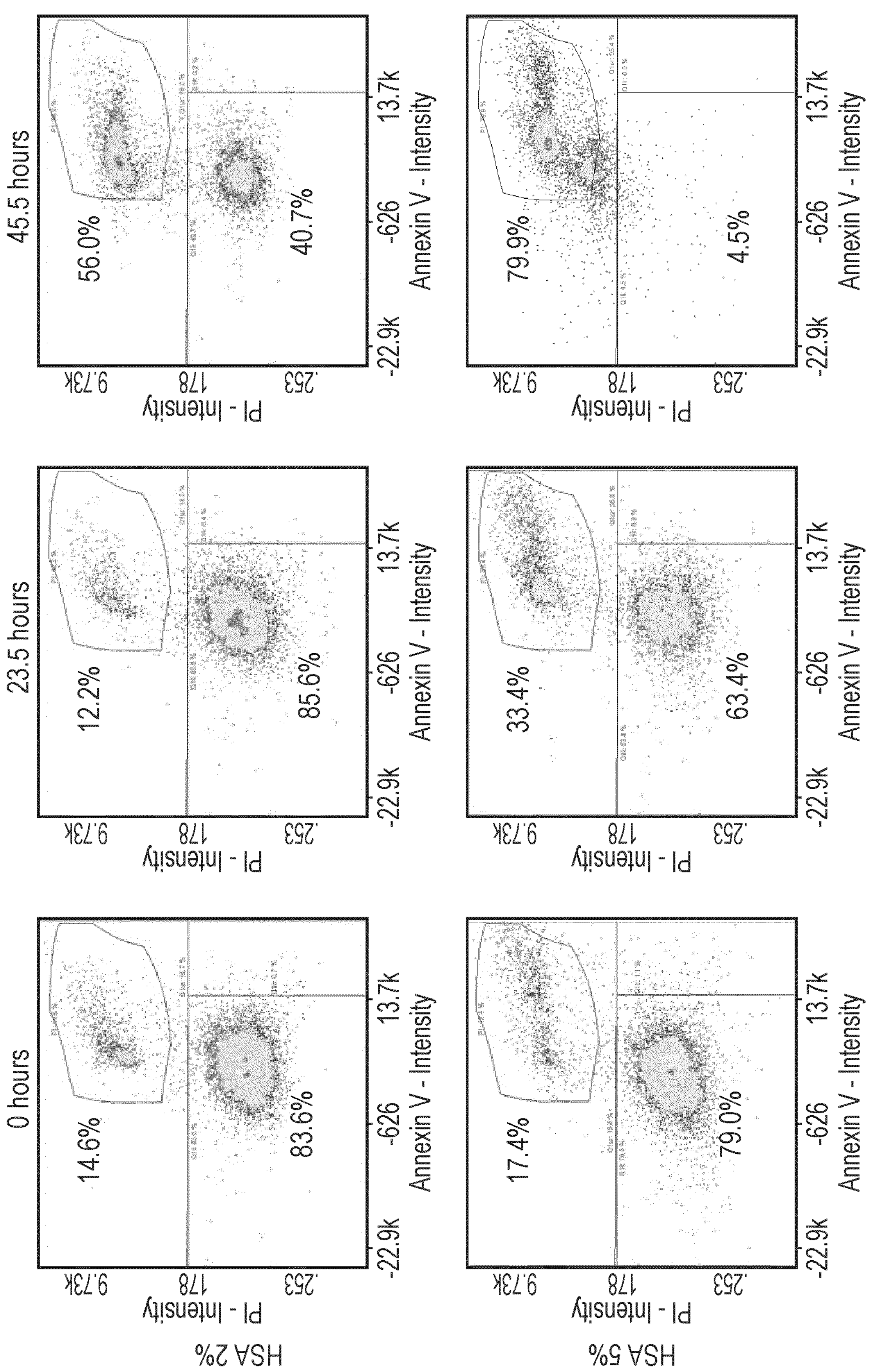


FIG. 6 (Continued)

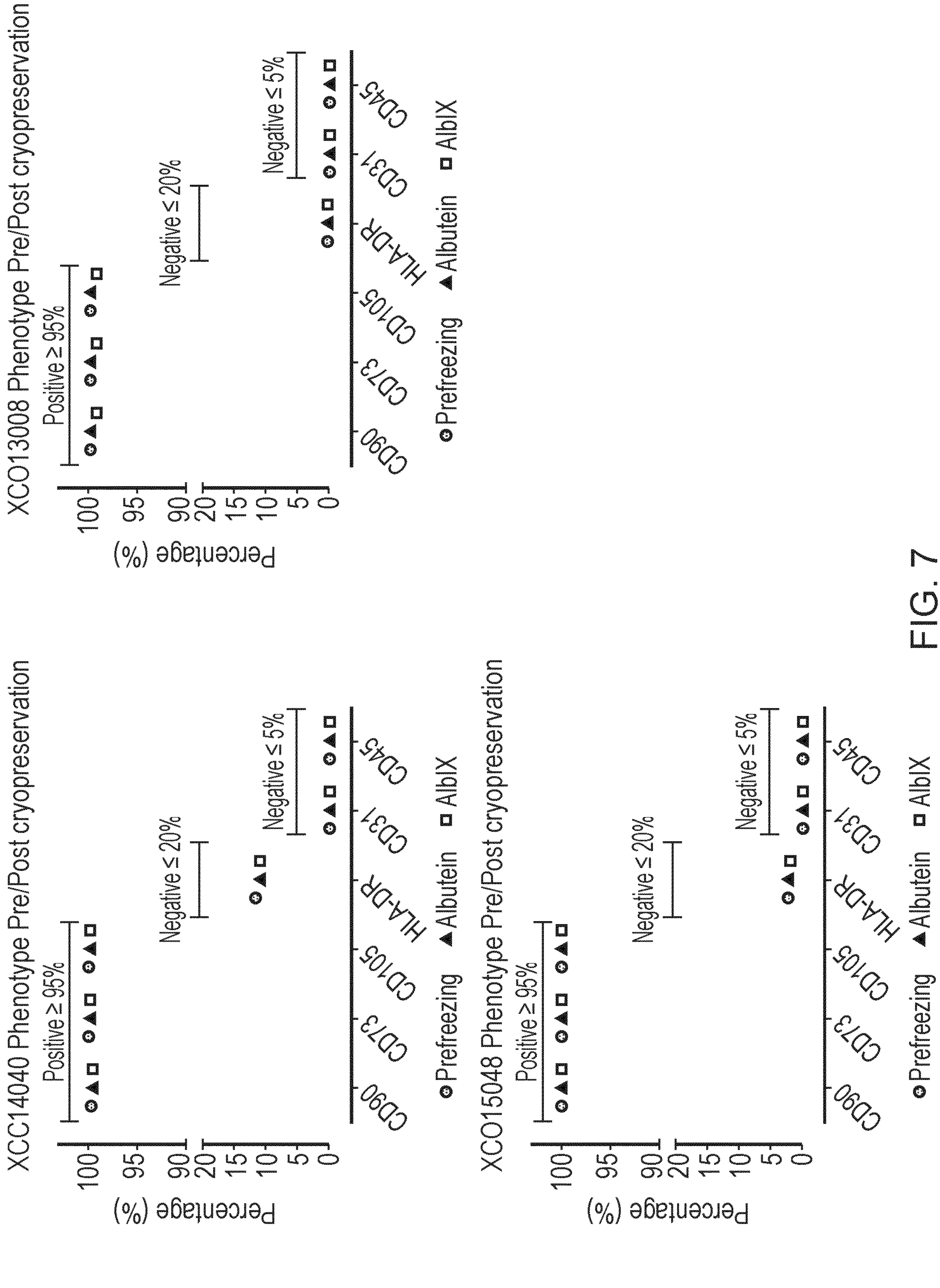
XCO13008 Phenotype Pre/Post cryopreservation
Percentage (%)
100
95
90
20
15
10
5
0
Positive ≥ 95%
Negative ≤ 20%
Negative ≤ 5%
CD90
CD73
CD105
HLA-DR
CD31
CD45
Prefreezing
Albutein
AlbIX
XCC14040 Phenotype Pre/Post cryopreservation
Percentage (%)
100
95
90
20
15
10
5
0
Positive ≥ 95%
Negative ≤ 20%
Negative ≤ 5%
CD90
CD73
CD105
HLA-DR
CD31
CD45
Prefreezing
Albutein
AlbIX
XCO15048 Phenotype Pre/Post cryopreservation
Percentage (%)
100
95
90
20
15
10
5
0
Positive ≥ 95%
Negative ≤ 20%
Negative ≤ 5%
CD90
CD73
CD105
HLA-DR
CD31
CD45
Prefreezing
Albutein
AlbIX

FIG. 7

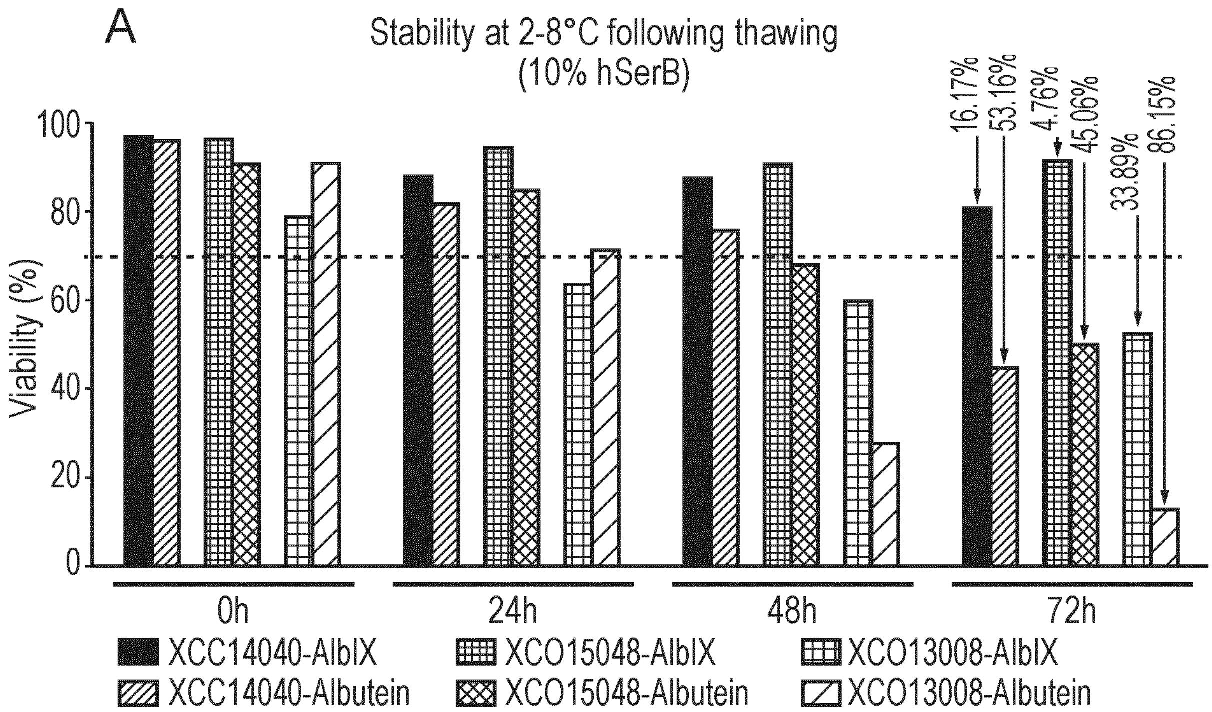


ANOVA Tukey Multiple comparison test:
AlbIX: no significant differences Albutein: t=0 vs 72h ($p<0.05$)

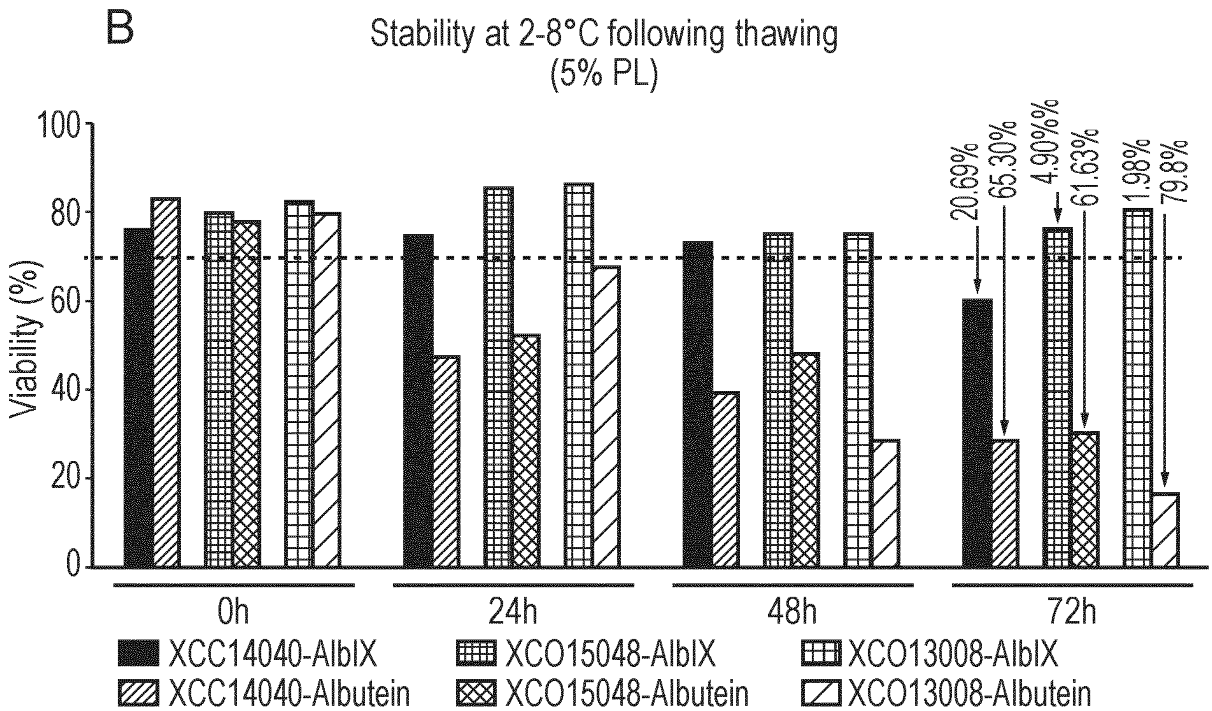


ANOVA Tukey Multiple comparison test: AlbIX: no significant differences
Albutein: From t=0 vs 24 ($p<0.05$): t=0 vs 48 and 72 ($p<0.001$)

FIG. 8

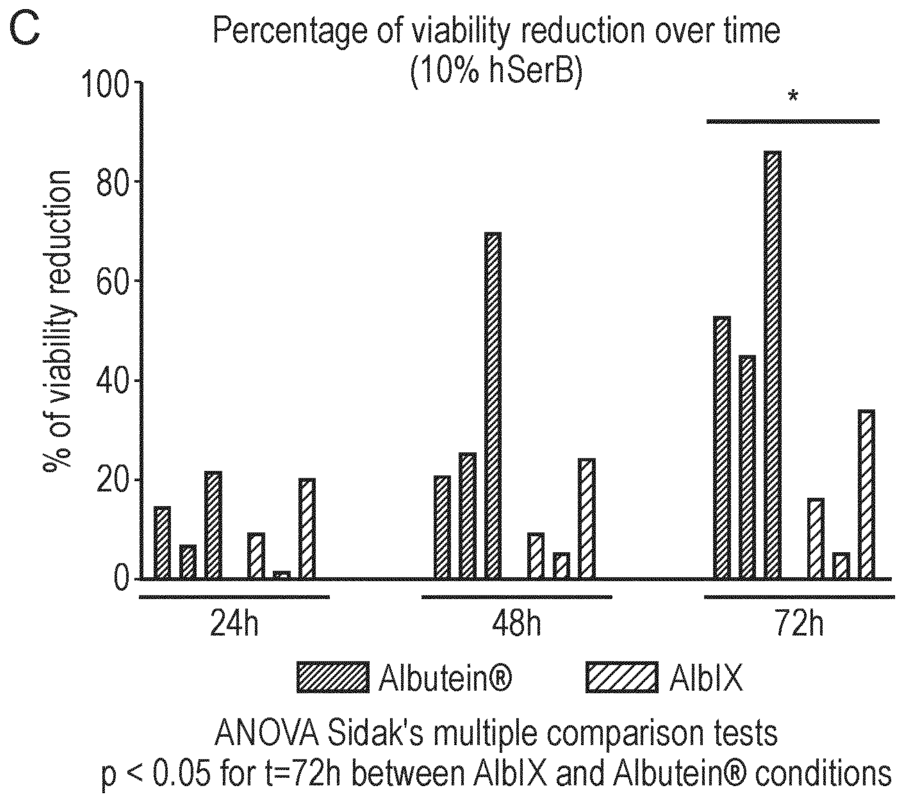


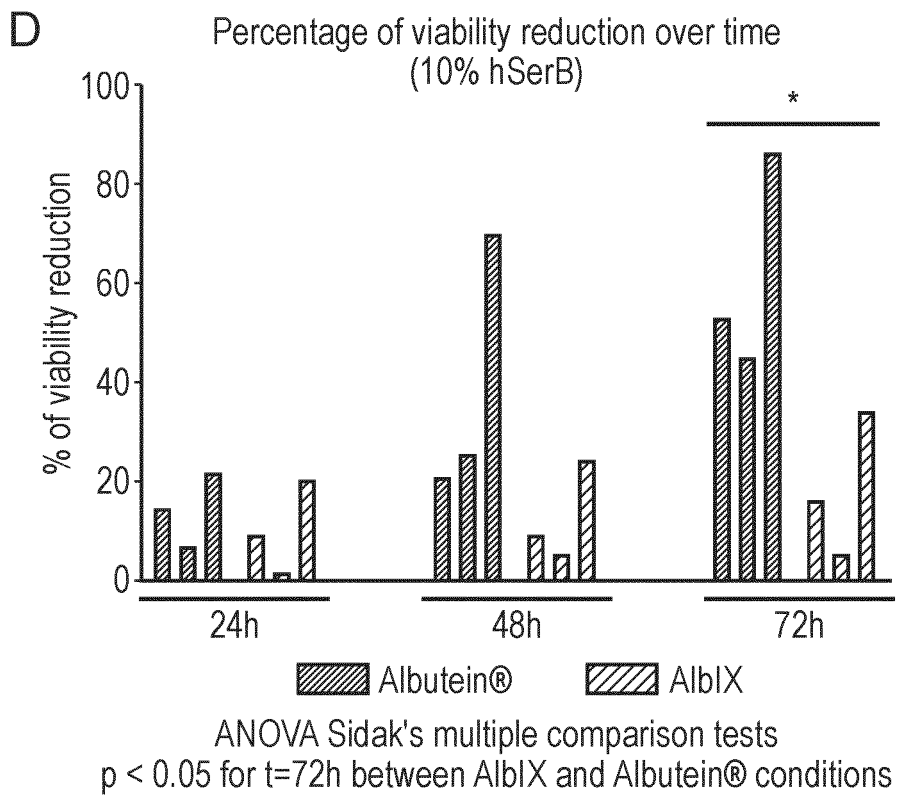


FIG. 8 (Continued)

| Fatty Acid | Average FA Content | | | |
|---|---|---|---|---|
| Lipid No. | mg/mL | %wt | mM | % mol |
| C6:0 | 0.001 | 0.039 | 0.01 | 0.09 |
| C8:0 | 0.033 | 1.268 | 0.23 | 2.36 |
| C9:0 | 0.000 | 0.000 | 0.00 | 0.00 |
| C10:0 | 0.001 | 0.039 | 0.01 | 0.06 |
| C11:0 | 0.000 | 0.000 | 0.00 | 0.00 |
| C12:0 | 0.011 | 0.429 | 0.05 | 0.58 |
| C13:0 | 0.000 | 0.000 | 0.00 | 0.00 |
| C14:0 | 0.229 | 8.933 | 1.00 | 10.51 |
| C14:1 | 0.001 | 0.039 | 0.00 | 0.05 |
| C15:0 | 0.010 | 0.390 | 0.04 | 0.43 |
| C15:1 | 0.000 | 0.000 | 0.00 | 0.00 |
| C16:0 | 0.866 | 33.762 | 3.38 | 35.37 |
| C16:1n7 | 0.024 | 0.917 | 0.09 | 0.97 |
| C16:2n4 | 0.000 | 0.000 | 0.00 | 0.00 |
| C16:3n4 | 0.013 | 0.488 | 0.05 | 0.52 |
| C17:0 | 0.014 | 0.527 | 0.05 | 0.52 |
| C17:1 | 0.000 | 0.000 | 0.00 | 0.00 |
| C18:0 | 0.584 | 22.762 | 2.05 | 21.49 |
| C18:1n7 | 0.005 | 0.195 | 0.02 | 0.19 |
| C18:1n9c | 0.227 | 8.836 | 0.80 | 8.40 |
| C18:1n9t | 0.049 | 1.892 | 0.17 | 1.80 |
| C18:2n6c | 0.118 | 4.603 | 0.42 | 4.41 |
| C18:2n6t | 0.000 | 0.000 | 0.00 | 0.00 |
| C18:3n3 | 0.000 | 0.000 | 0.00 | 0.00 |
| C18:4n3 | 0.000 | 0.000 | 0.00 | 0.00 |
| C19:0 | 0.000 | 0.000 | 0.00 | 0.00 |
| C20:0 | 0.187 | 7.275 | 0.60 | 6.25 |
| C20:1n9 | 0.000 | 0.000 | 0.00 | 0.00 |

FIG. 9

| **Fatty Acid** | **Average FA Content** | | | |
|---|---|---|---|---|
| **Lipid No.** | **mg/mL** | **%wt** | **mM** | **% mol** |
| C20:2n6 | 0.001 | 0.020 | 0.00 | 0.02 |
| C20:3n3 | 0.000 | 0.000 | 0.00 | 0.00 |
| C20:3n6 | 0.001 | 0.020 | 0.00 | 0.02 |
| C20:4n6 | 0.000 | 0.000 | 0.00 | 0.00 |
| C20:5n3 | 0.000 | 0.000 | 0.00 | 0.00 |
| C22:0 | 0.194 | 7.568 | 0.57 | 5.97 |
| C22:1n11 | 0.000 | 0.000 | 0.00 | 0.00 |
| C22:1n9 | 0.000 | 0.000 | 0.00 | 0.00 |
| C22:2n6 | 0.000 | 0.000 | 0.00 | 0.00 |
| **Sum** | 2.564 | 100.000 | 9.543 | 100.00 |

FIG. 9 (Continued)

| Metal | Conc (mg/kg solution) |
|---|---|
| Silver | <0.5 |
| Aluminium | <0.5 |
| Arsenic | <0.5 |
| Boron | <0.5 |
| Barium | <0.5 |
| Beryllium | <0.5 |
| Bismuth | <0.5 |
| Calcium | 2.6 |
| Cadmium | <0.5 |
| Cobalt | <0.5 |
| Chromium | <0.5 |
| Copper | <0.5 |
| Iron | <0.5 |
| Gallium | <0.5 |
| Indium | <0.5 |
| Potassium | 1 |
| Lithium | <0.5 |
| Magnesium | <0.5 |
| Manganese | <0.5 |
| Molybdenum | <0.5 |
| Nickel | <0.5 |
| Phosphorus | 2.6 |
| Lead | <0.5 |
| Palladium | <0.5 |
| Platinum | <0.5 |
| Antimony | <0.5 |
| Selenium | <0.5 |
| Silicon | <0.5 |
| Tin | <0.5 |
| Strontium | <0.5 |

FIG. 10

| **Metal** | **Conc (mg/kg solution)** |
|---|---|
| Titanium | <0.5 |
| Thallium | <0.5 |
| Vanadium | <0.5 |
| Zinc | <0.5 |
| Zirconium | <0.5 |

FIG. 10 (Continued)

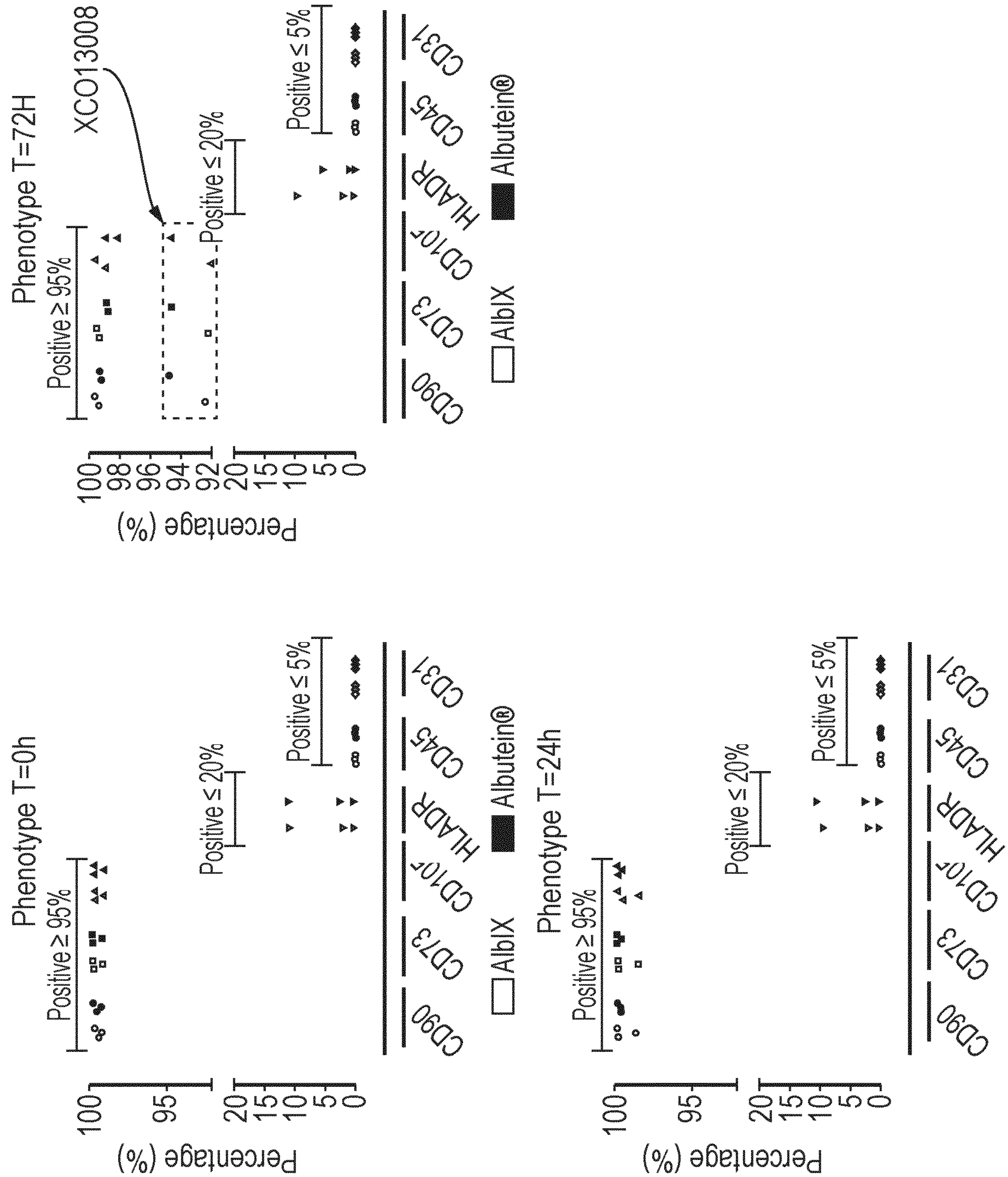
Phenotype T=0h
Phenotype T=24h
Phenotype T=72H
Percentage (%)
Positive ≥ 95%
Positive ≤ 20%
Positive ≤ 5%
XCO13008
CD90
CD73
CD105
HLADR
CD45
CD31
AlbIX
Albutein®

FIG. 11

A.

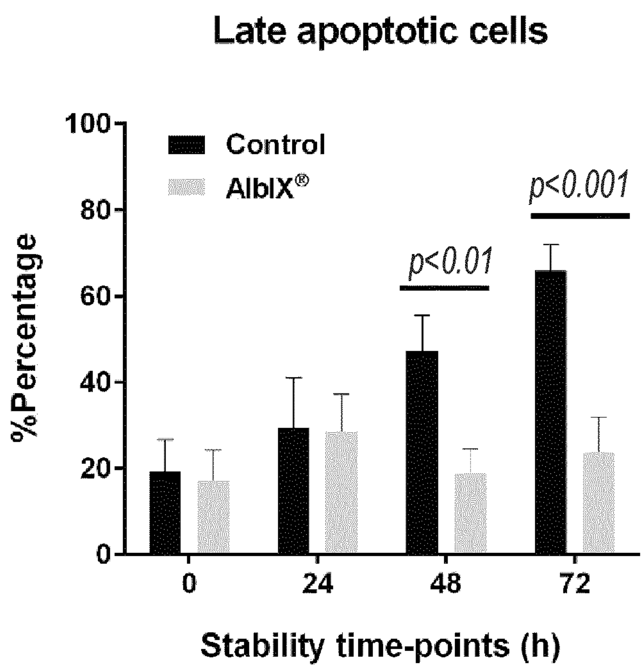


*Statistical analysis: ANOVA, Sidak's Multiple comparison test*

B.

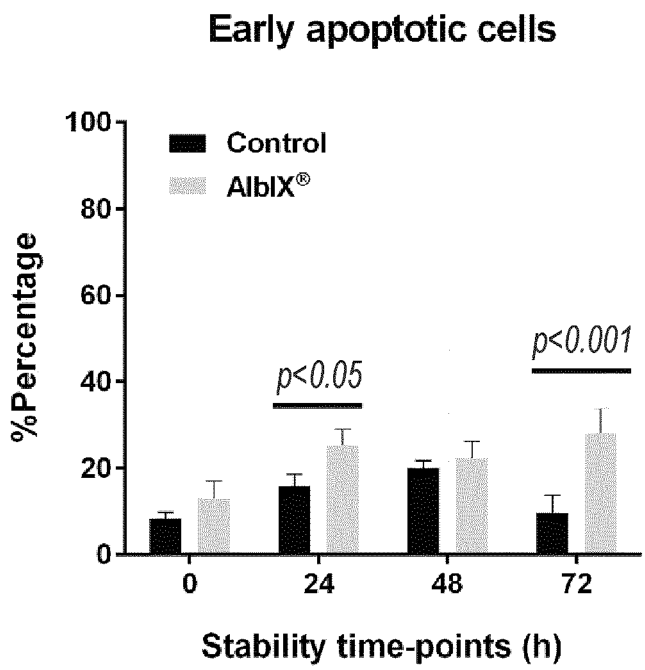


*Statistical analysis: ANOVA, Sidak's Multiple comparison test*

FIG. 12

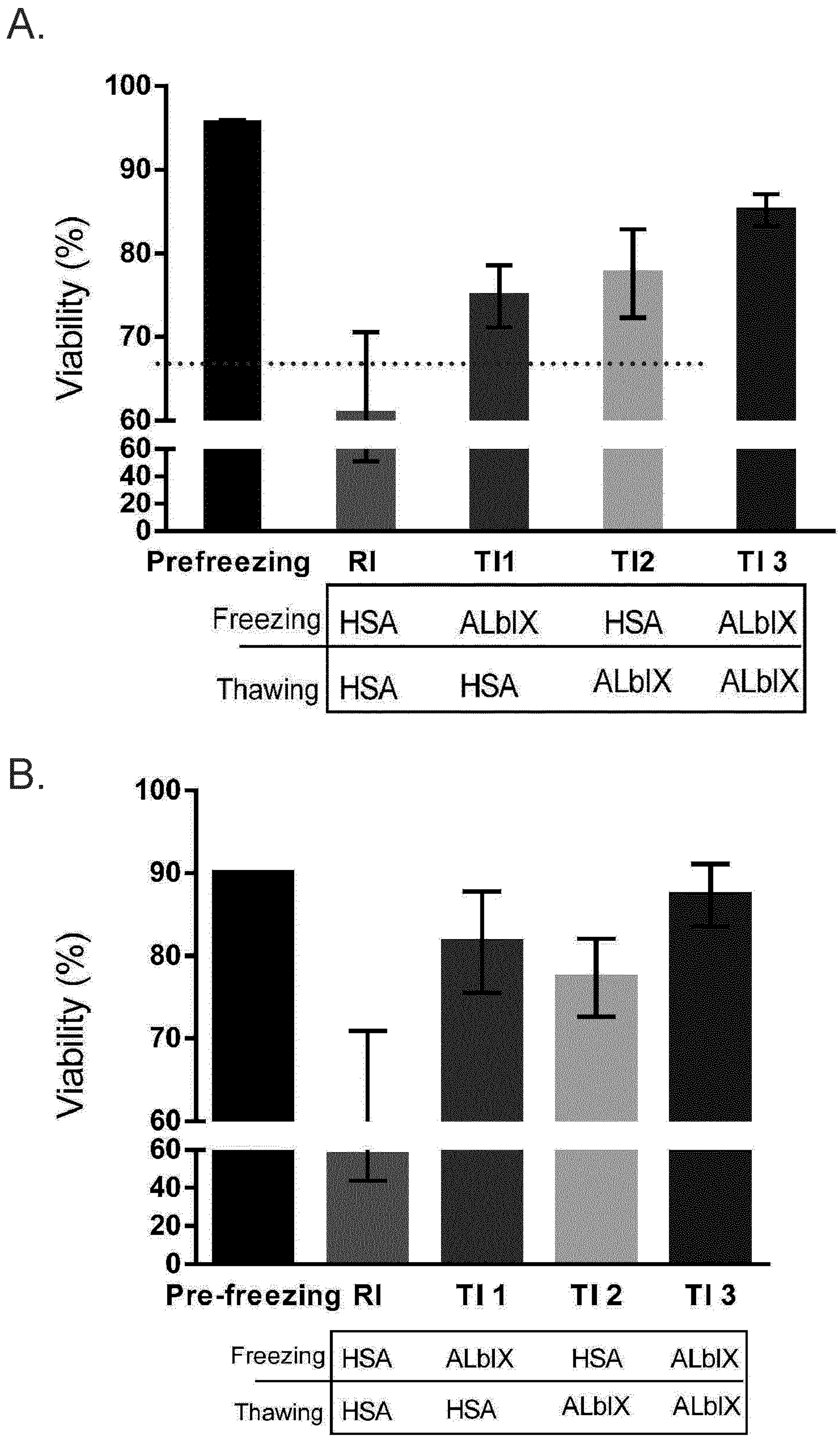
A.
100
90
80
70
60
60
40
20
0
Viability (%)
Prefreezing
RI
TI1
TI2
TI 3
Freezing
HSA
ALbIX
HSA
ALbIX
Thawing
HSA
HSA
ALbIX
ALbIX
B.
100
90
80
70
60
60
40
20
0
Viability (%)
Pre-freezing
RI
TI 1
TI 2
TI 3
Freezing
HSA
ALbIX
HSA
ALbIX
Thawing
HSA
HSA
ALbIX
ALbIX

FIG. 13

A.

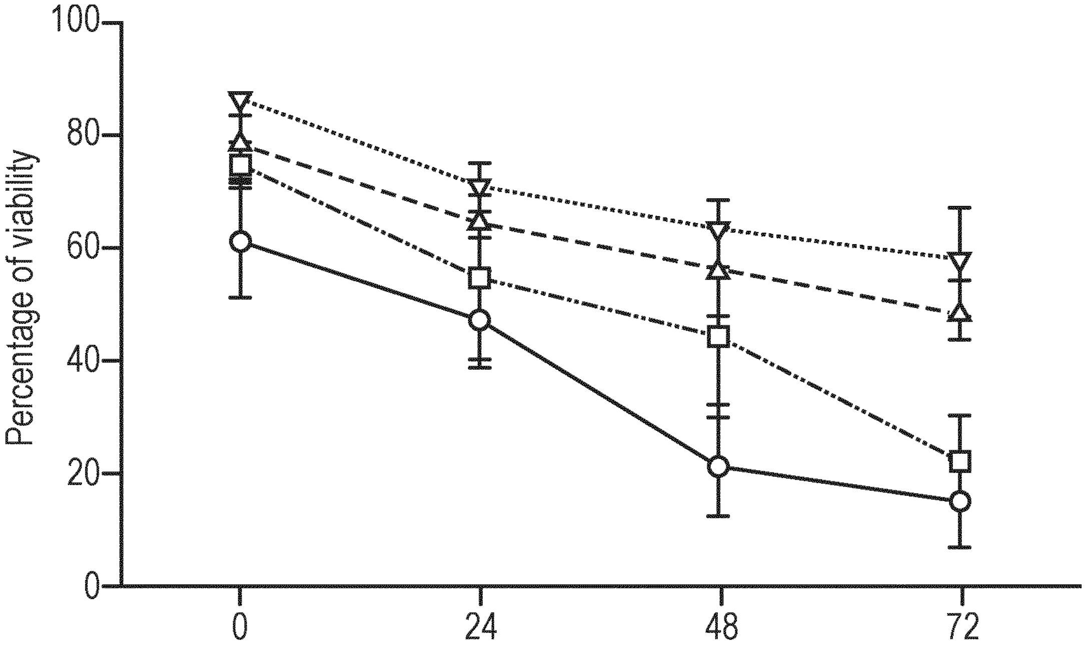
Percentage of viability
100
80
60
40
20
0
0
24
48
72

B.

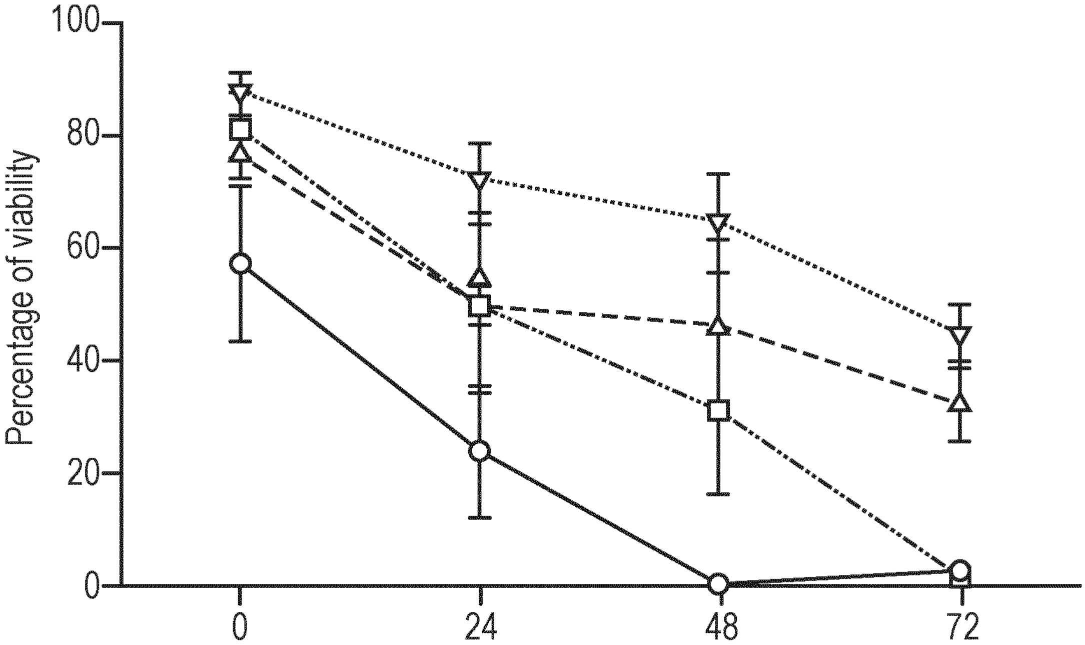
Percentage of viability
100
80
60
40
20
0
0
24
48
72

FIG. 14

A.

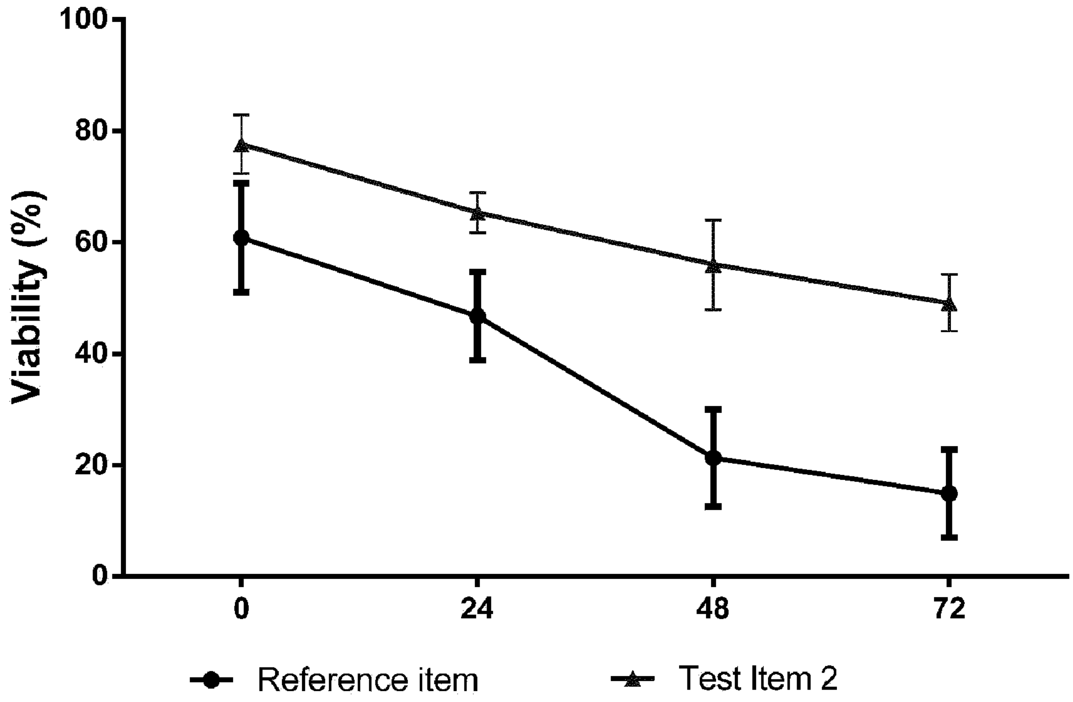
Viability (%)
100
80
60
40
20
0
0
24
48
72
Reference item
Test Item 2

B.

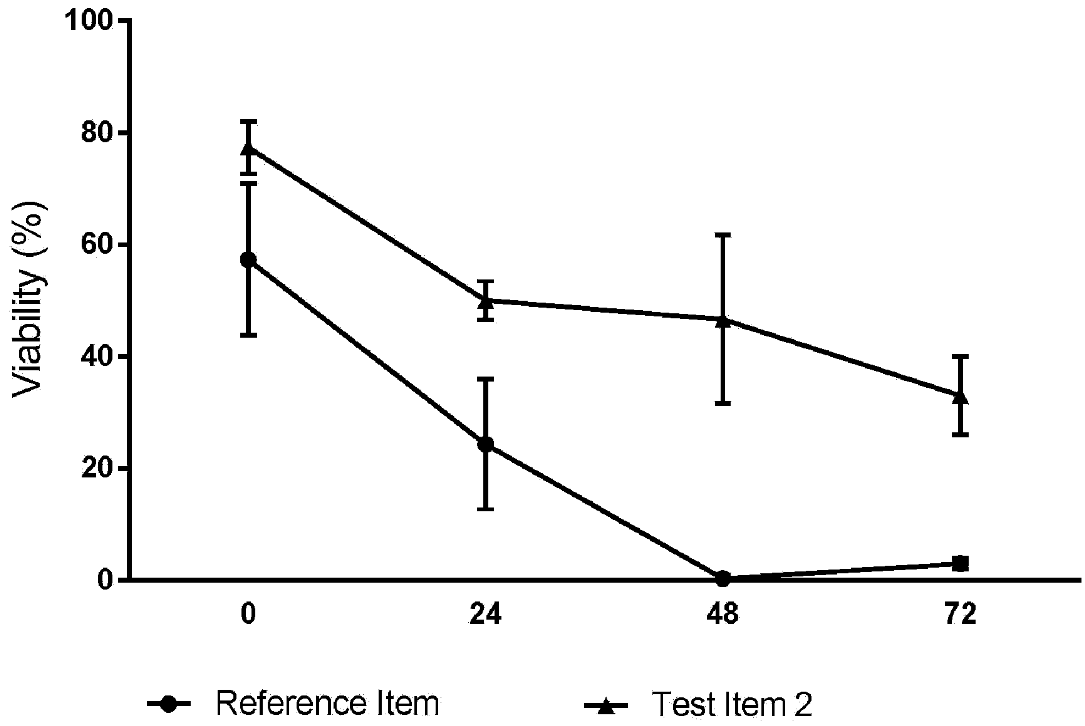
Viability (%)
100
80
60
40
20
0
0
24
48
72
Reference Item
Test Item 2

FIG. 15

A.

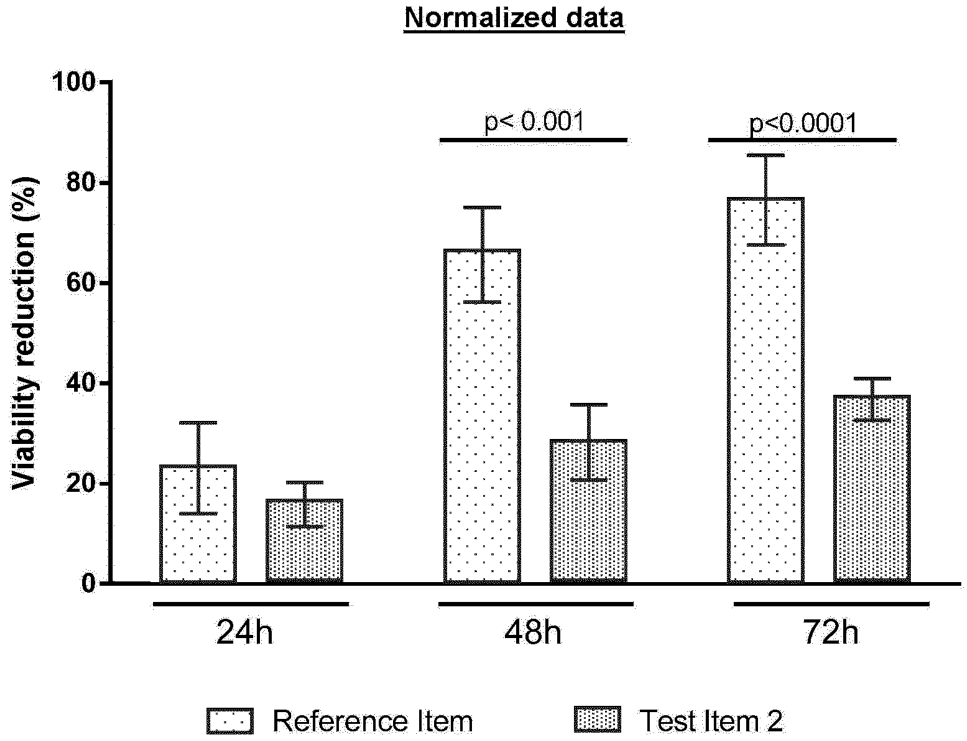
Normalized data
Viability reduction (%)
100
80
60
40
20
0
p< 0.001
p<0.0001
24h
48h
72h
Reference Item
Test Item 2

B.

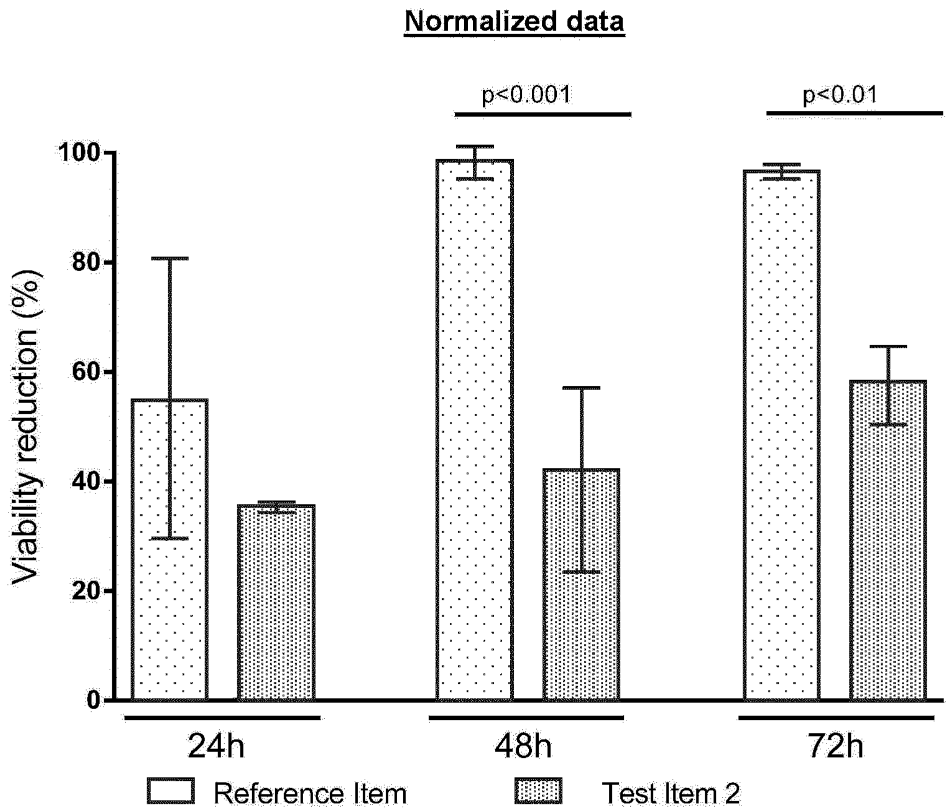
Normalized data
Viability reduction (%)
100
80
60
40
20
0
p<0.001
p<0.01
24h
48h
72h
Reference Item
Test Item 2

FIG. 16

A.

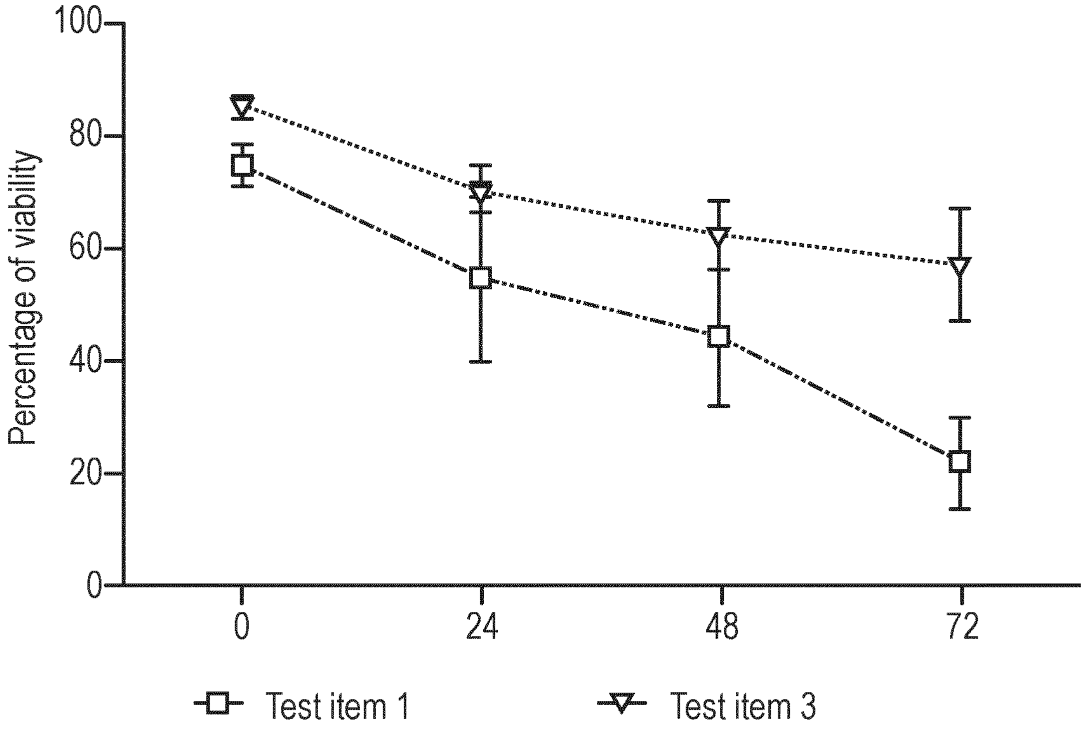
100
80
60
40
20
0
Percentage of viability
0
24
48
72
Test item 1
Test item 3

B.

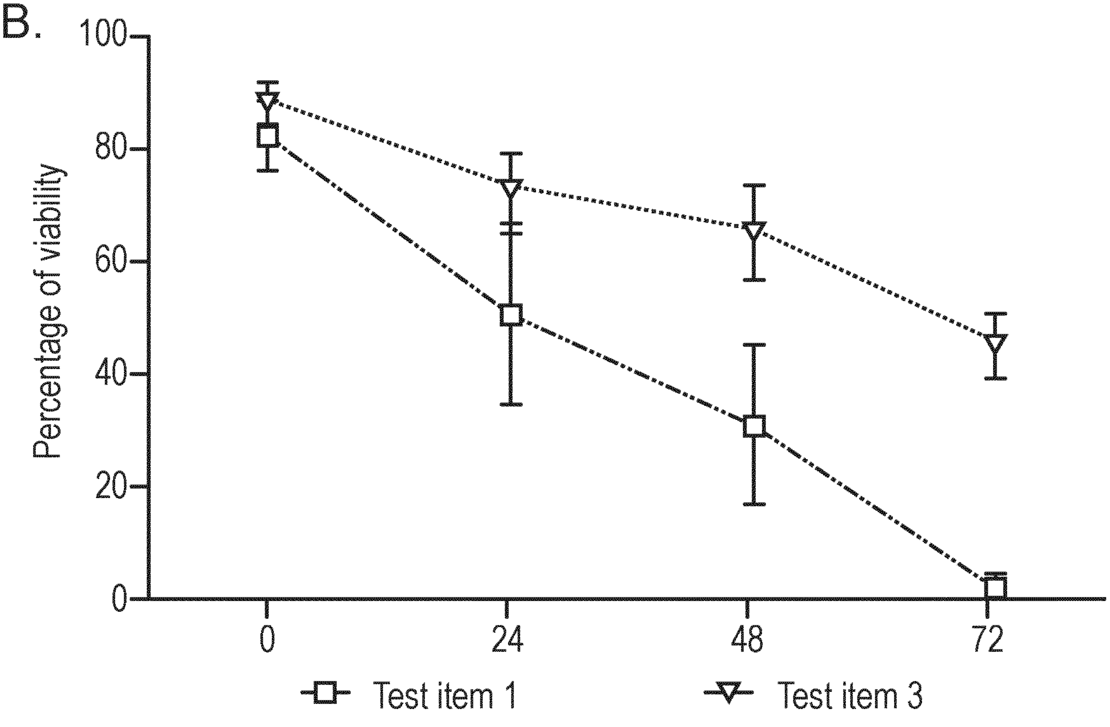
100
80
60
40
20
0
Percentage of viability
0
24
48
72
Test item 1
Test item 3

FIG. 17

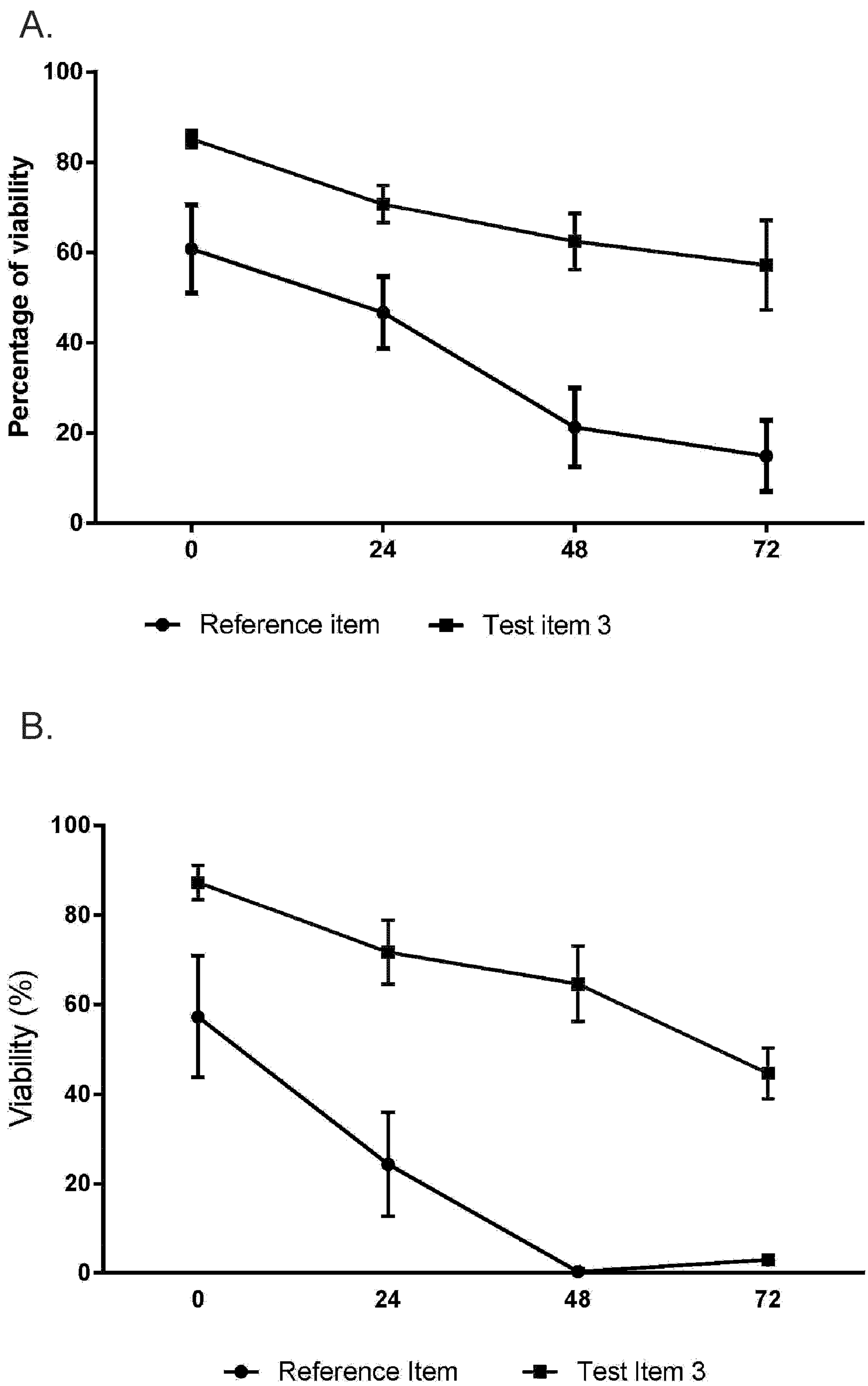
A.
Percentage of viability
100
80
60
40
20
0
0
24
48
72
Reference item
Test item 3
B.
Viability (%)
100
80
60
40
20
0
0
24
48
72
Reference Item
Test Item 3

FIG. 18

A.

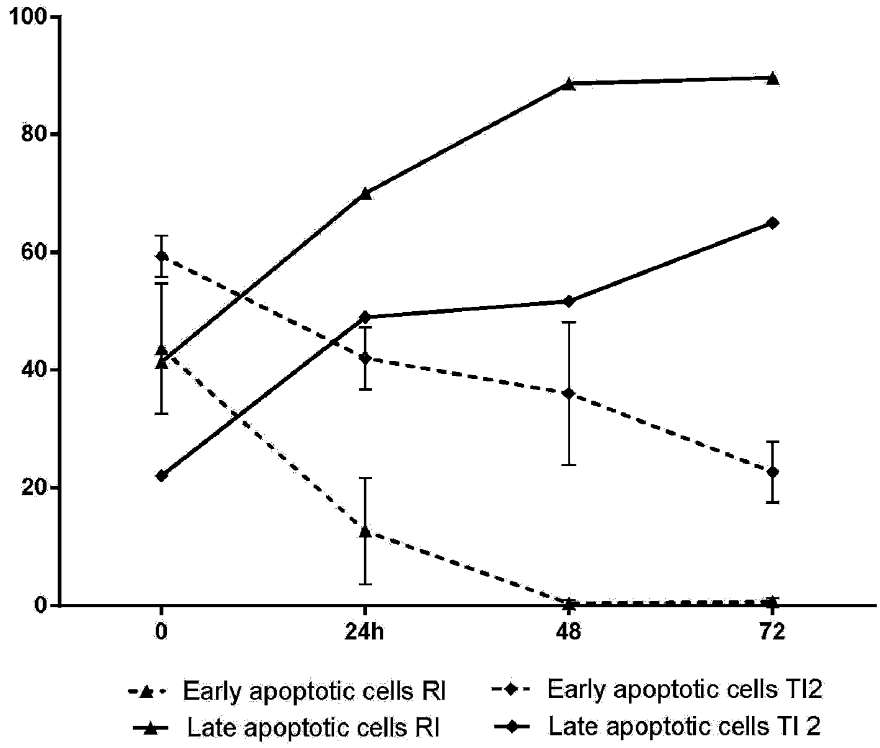
100
80
60
40
20
0
0
24h
48
72
Early apoptotic cells RI
Early apoptotic cells TI2
Late apoptotic cells RI
Late apoptotic cells TI 2

B.

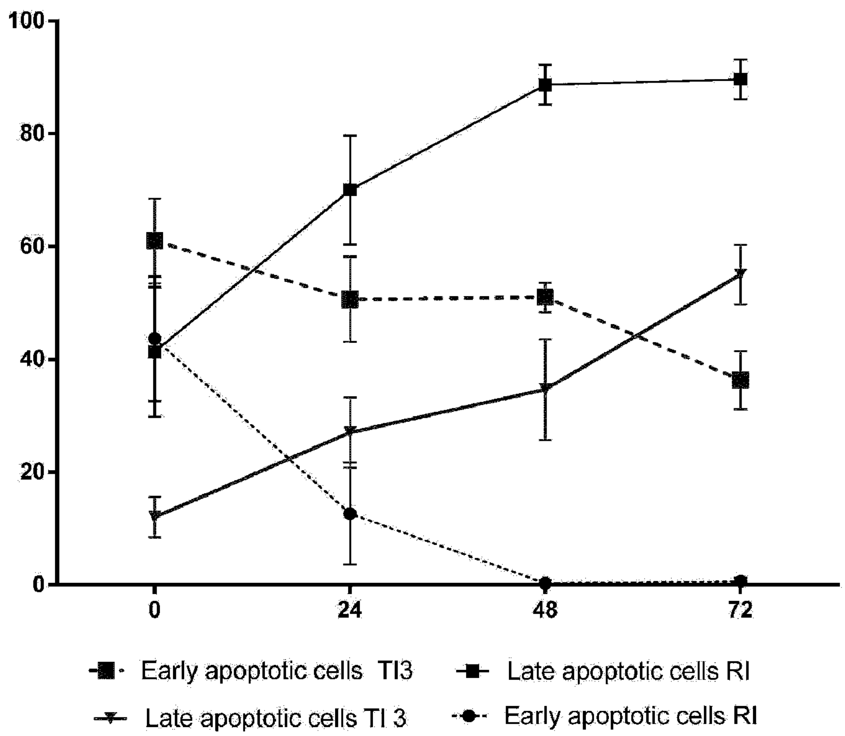
100
80
60
40
20
0
0
24
48
72
Early apoptotic cells TI3
Late apoptotic cells RI
Late apoptotic cells TI 3
Early apoptotic cells RI

FIG. 19

C.

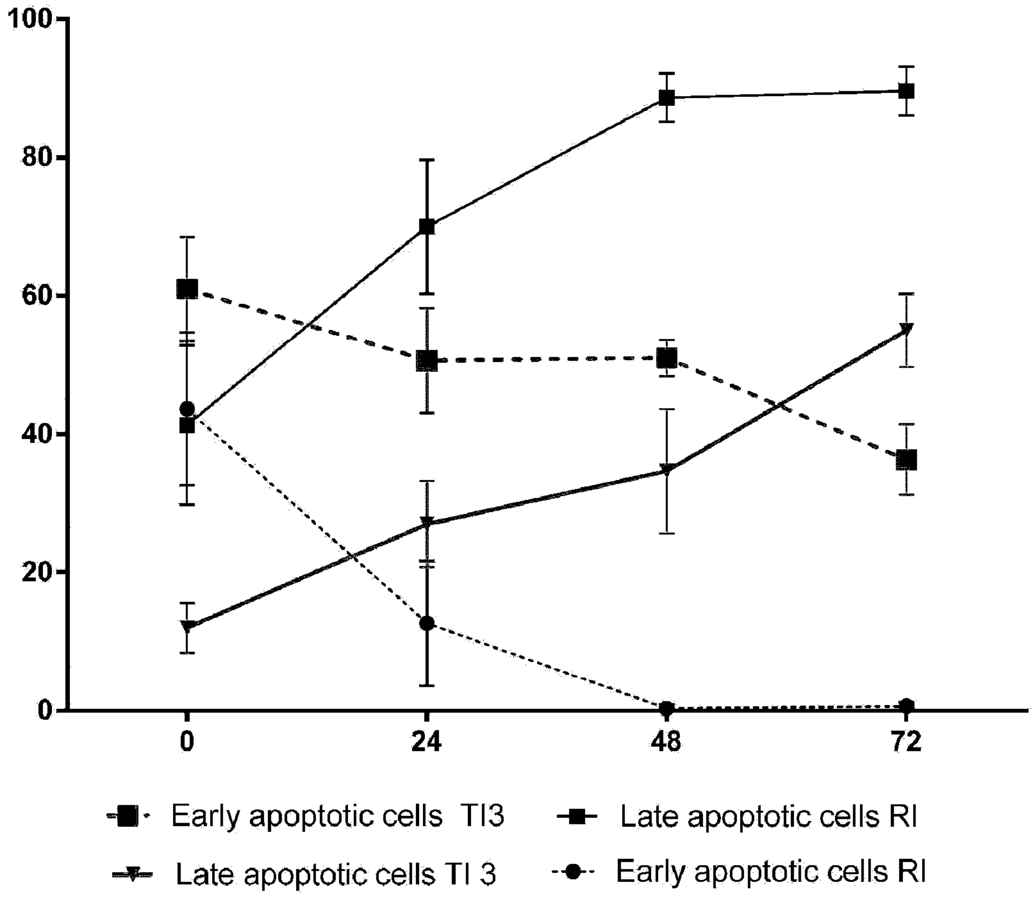


FIG. 19 (Continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2013157356 A
- WO 2000044772 A
- WO 2013006675 A
- EP 1329460 A1
- EP 1329461 A1
- EP 1329462 A1
- EP 1710250 A1
- WO 2010092135 A
- EP 11055577 W
- WO 2011124718 A
- EP 2010066572 W
- WO 2011051489 A
- EP 2011055577 W
- WO 9637515 A
- WO 2004099234 A
- WO 16192276 A


### Non-patent literature cited in the description


- **RUBENSTEIN et al.** *Proc. Natl. Acad. Sci. USA.,* 1995, vol. 92, 10119-10122
- **VIVES et al.** *Met. Eng.,* 2003, vol. 5, 124-132
- **TINTÓ et al.** *J. Biotechnol.,* vol. 95, 205-214
- **MILIAN et al.** *J. Biotech.,* 2015, vol. 209, 58-67
- **TINTÓ et al.** *J. Biotech.,* 2002, vol. 95, 205-214
- **FLEER.** *Bio/technology,* 1991, vol. 9, 968-975
- **KOBAYASHI.** *Therapeutic Apheresis,* 1998, vol. 2, 257-262
- **SLEEP.** *Bio/technology,* 1990, vol. 8, 42-46
- **KRAGH-HANSEN et al.** *Biol. Pharm. Bull.,* 2002, vol. 25, 695
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **FRAHM et al.** *PLOS One,* 2014, vol. 9 (1), e109893
- **GIRARD et al.** *Biomed. Chromatogr.,* vol. 12, 183-184
- **CODINACH et al.** *Cytotherapy,* 2016, vol. 18 (9), 1197-1208
- Annexin V Assay using the NucleoCounter® NC-3000™ System. No. 3017 Rev. 1.4. ChemoMetec A/S

SEQUENCE LISTING

<110> Albumedix A/S

<120> Uses of Recombinant Yeast-Derived Serum Albumin

<130> ALBBE/P64407PC

<140> UNKNOWN
<141> 2017-10-04

<150> 16192276.0
<151> 2016-10-04

<160> 19

<170> PatentIn version 3.5

<210> 1
<211> 1758
<212> DNA
<213> Homo sapiens

<220>
<221> misc_feature
<222> (1)..(1758)
<223> cDNA encoding HSA

<400> 1

```
gatgcacaca agagtgaggt tgctcatcgg tttaaagatt tgggagaaga aaatttcaaa      60
gccttggtgt tgattgcctt tgctcagtat cttcagcagt gtccatttga agatcatgta     120
aaattagtga atgaagtaac tgaatttgca aaaacatgtg ttgctgatga gtcagctgaa     180
aattgtgaca aatcacttca taccctttt ggagacaaat tatgcacagt tgcaactctt      240
cgtgaaacct atggtgaaat ggctgactgc tgtgcaaaac aagaacctga gagaaatgaa     300
tgcttcttgc aacacaaaga tgacaaccca aacctccccc gattggtgag accagaggtt     360
gatgtgatgt gcactgcttt tcatgacaat gaagagacat ttttgaaaaa atacttatat     420
gaaattgcca gaagacatcc ttacttttat gccccggaac tccttttctt tgctaaaagg     480
tataaagctg cttttacaga atgttgccaa gctgctgata aagctgcctg cctgttgcca     540
aagctcgatg aacttcggga tgaagggaag gcttcgtctg ccaaacagag actcaagtgt     600
gccagtctcc aaaaatttgg agaaagagct ttcaaagcat gggcagtagc tcgcctgagc     660
cagagatttc ccaaagctga gtttgcagaa gtttccaagt tagtgacaga tcttaccaaa     720
gtccacacgg aatgctgcca tggagatctg cttgaatgtg ctgatgacag ggcggacctt     780
gccaagtata tctgtgaaaa tcaagattcg atctccagta aactgaagga atgctgtgaa     840
aaacctctgt tggaaaaatc ccactgcatt gccgaagtgg aaaatgatga gatgcctgct     900
gacttgcctt cattagctgc tgattttgtt gaaagtaagg atgtttgcaa aaactatgct     960
gaggcaaagg atgtcttcct gggcatgttt ttgtatgaat atgcaagaag gcatcctgat    1020
```

```
tactctgtcg tgctgctgct gagacttgcc aagacatatg aaaccactct agagaagtgc     1080
tgtgccgctg cagatcctca tgaatgctat gccaaagtgt tcgatgaatt taaacctctt     1140
gtggaagagc ctcagaattt aatcaaacaa aattgtgagc tttttgagca gcttggagag     1200
tacaaattcc agaatgcgct attagttcgt tacaccaaga aagtacccca agtgtcaact     1260
ccaactcttg tagaggtctc aagaaaccta ggaaaagtgg gcagcaaatg ttgtaaacat     1320
cctgaagcaa aaagaatgcc ctgtgcagaa gactatctat ccgtggtcct gaaccagtta     1380
tgtgtgttgc atgagaaaac gccagtaagt gacagagtca ccaaatgctg cacagaatcc     1440
ttggtgaaca ggcgaccatg cttttcagct ctggaagtcg atgaaacata cgttcccaaa     1500
gagtttaatg ctgaaacatt caccttccat gcagatatat gcacactttc tgagaaggag     1560
agacaaatca agaaacaaac tgcacttgtt gagctcgtga aacacaagcc caaggcaaca     1620
aaagagcaac tgaaagctgt tatggatgat ttcgcagctt ttgtagagaa gtgctgcaag     1680
gctgacgata aggagacctg ctttgccgag gagggtaaaa aacttgttgc tgcaagtcaa     1740
gctgccttag gcttataa                                                   1758
```

<210> 2
<211> 585
<212> PRT
<213> Homo sapiens

<400> 2

```
Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Cys Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110
```

Pro Arg Leu Val Arg Pro Glu Val Asp Val Met Cys Thr Ala Phe His
115 120 125

Asp Asn Glu Glu Thr Phe Leu Lys Lys Tyr Leu Tyr Glu Ile Ala Arg
130 135 140

Arg His Pro Tyr Phe Tyr Ala Pro Glu Leu Leu Phe Phe Ala Lys Arg
145 150 155 160

Tyr Lys Ala Ala Phe Thr Glu Cys Cys Gln Ala Ala Asp Lys Ala Ala
165 170 175

Cys Leu Leu Pro Lys Leu Asp Glu Leu Arg Asp Glu Gly Lys Ala Ser
180 185 190

Ser Ala Lys Gln Arg Leu Lys Cys Ala Ser Leu Gln Lys Phe Gly Glu
195 200 205

Arg Ala Phe Lys Ala Trp Ala Val Ala Arg Leu Ser Gln Arg Phe Pro
210 215 220

Lys Ala Glu Phe Ala Glu Val Ser Lys Leu Val Thr Asp Leu Thr Lys
225 230 235 240

Val His Thr Glu Cys Cys His Gly Asp Leu Leu Glu Cys Ala Asp Asp
245 250 255

Arg Ala Asp Leu Ala Lys Tyr Ile Cys Glu Asn Gln Asp Ser Ile Ser
260 265 270

Ser Lys Leu Lys Glu Cys Cys Glu Lys Pro Leu Leu Glu Lys Ser His
275 280 285

Cys Ile Ala Glu Val Glu Asn Asp Glu Met Pro Ala Asp Leu Pro Ser
290 295 300

Leu Ala Ala Asp Phe Val Glu Ser Lys Asp Val Cys Lys Asn Tyr Ala
305 310 315 320

Glu Ala Lys Asp Val Phe Leu Gly Met Phe Leu Tyr Glu Tyr Ala Arg
325 330 335

Arg His Pro Asp Tyr Ser Val Val Leu Leu Leu Arg Leu Ala Lys Thr
340 345 350

Tyr Glu Thr Thr Leu Glu Lys Cys Cys Ala Ala Ala Asp Pro His Glu
355 360 365

```
Cys Tyr Ala Lys Val Phe Asp Glu Phe Lys Pro Leu Val Glu Glu Pro
    370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu
            580                 585
```

<210> 3
<211> 609
<212> PRT
<213> Homo sapiens

<400> 3

```
Met Lys Trp Val Thr Phe Ile Ser Leu Leu Phe Leu Phe Ser Ser Ala
1               5                   10                  15

Tyr Ser Arg Gly Val Phe Arg Arg Asp Ala His Lys Ser Glu Val Ala
            20                  25                  30

His Arg Phe Lys Asp Leu Gly Glu Glu Asn Phe Lys Ala Leu Val Leu
        35                  40                  45

Ile Ala Phe Ala Gln Tyr Leu Gln Gln Cys Pro Phe Glu Asp His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys
145                 150                 155                 160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
                245                 250                 255
```

```
Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

Phe Lys Pro Leu Val Glu Glu Pro Gln Asn Leu Ile Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ser Lys Cys Cys Lys His
    450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
```

500 505 510

Ser Ala Leu Glu Val Asp Glu Thr Tyr Val Pro Lys Glu Phe Asn Ala
515 520 525

Glu Thr Phe Thr Phe His Ala Asp Ile Cys Thr Leu Ser Glu Lys Glu
530 535 540

Arg Gln Ile Lys Lys Gln Thr Ala Leu Val Glu Leu Val Lys His Lys
545 550 555 560

Pro Lys Ala Thr Lys Glu Gln Leu Lys Ala Val Met Asp Asp Phe Ala
565 570 575

Ala Phe Val Glu Lys Cys Cys Lys Ala Asp Asp Lys Glu Thr Cys Phe
580 585 590

Ala Glu Glu Gly Lys Lys Leu Val Ala Ala Ser Gln Ala Ala Leu Gly
595 600 605

Leu

<210> 4
<211> 621
<212> PRT
<213> Pan troglodytes

<400> 4

```
Met Asn Glu Ser Ser Cys Cys Ser Thr Ser Leu Pro Ala Phe Gly Val
1               5                   10                  15

Ser Val Leu Asp Ser Gly His Ser Ser Ser Ser Ala Tyr Ser Arg Gly
            20                  25                  30

Val Phe Arg Arg Asp Ala His Lys Ser Glu Val Ala His Arg Phe Lys
        35                  40                  45

Asp Leu Gly Glu Glu Asn Phe Lys Ala Leu Val Leu Val Ala Phe Ala
    50                  55                  60

Gln Tyr Leu Gln Gln Cys Pro Phe Glu Asp His Val Lys Leu Val Asn
65                  70                  75                  80

Glu Val Thr Glu Phe Ala Lys Thr Cys Val Ala Asp Glu Ser Ala Glu
                85                  90                  95

Asn Cys Asp Lys Ser Leu His Thr Leu Phe Gly Asp Lys Leu Cys Thr
```

100 105 110

Val Ala Thr Leu Arg Glu Lys Tyr Gly Glu Met Ala Asp Cys Cys Ala
115 120 125

Lys Gln Glu Pro Glu Arg Asn Glu Cys Phe Leu Gln His Lys Asp Asp
130 135 140

Asn Pro Asn Leu Pro Arg Leu Val Arg Pro Glu Val Asp Val Met Cys
145 150 155 160

Thr Ala Phe His Asp Asn Glu Gly Thr Phe Leu Lys Lys Tyr Leu Tyr
165 170 175

Glu Val Ala Arg Arg His Pro Tyr Phe Tyr Ala Pro Glu Leu Leu Phe
180 185 190

Phe Ala Glu Arg Tyr Lys Ala Ala Phe Thr Glu Cys Cys Gln Ala Ala
195 200 205

Asp Lys Ala Ala Cys Leu Leu Pro Lys Leu Asp Glu Leu Arg Asp Glu
210 215 220

Gly Lys Ala Ser Ser Ala Lys Gln Arg Leu Lys Cys Ala Ser Leu Gln
225 230 235 240

Lys Phe Gly Glu Arg Ala Phe Lys Ala Trp Ala Val Ala Arg Leu Ser
245 250 255

Gln Arg Phe Pro Lys Ala Glu Phe Ala Glu Val Ser Lys Leu Val Thr
260 265 270

Asp Leu Thr Lys Val His Thr Glu Cys Cys His Gly Asp Leu Leu Glu
275 280 285

Cys Ala Asp Asp Arg Ala Asp Leu Ala Lys Tyr Ile Cys Glu Asn Gln
290 295 300

Asp Ser Ile Ser Ser Lys Leu Lys Glu Cys Cys Glu Lys Pro Leu Leu
305 310 315 320

Glu Lys Ser His Cys Leu Ala Glu Val Glu Asn Asp Glu Met Pro Ala
325 330 335

Asp Leu Pro Ser Leu Ala Ala Asp Phe Val Glu Ser Lys Glu Val Cys
340 345 350

```
Lys Asn Tyr Ala Glu Ala Lys Asp Val Phe Leu Gly Met Phe Leu Tyr
        355                 360                 365

Glu Tyr Ala Arg Arg His Pro Asp Tyr Ser Val Val Leu Leu Leu Arg
    370                 375                 380

Leu Ala Lys Thr Tyr Glu Thr Thr Leu Glu Lys Cys Cys Ala Ala Ala
385                 390                 395                 400

Asp Pro His Glu Cys Tyr Ala Lys Val Phe Asp Glu Phe Lys Pro Leu
                405                 410                 415

Val Glu Glu Pro Gln Asn Leu Ile Lys Gln Asn Cys Glu Leu Phe Glu
            420                 425                 430

Gln Leu Gly Glu Tyr Lys Phe Gln Asn Ala Leu Leu Val Arg Tyr Thr
        435                 440                 445

Lys Lys Val Pro Gln Val Ser Thr Pro Thr Leu Val Glu Val Ser Arg
    450                 455                 460

Asn Leu Gly Lys Val Gly Ser Lys Cys Cys Lys His Pro Glu Ala Lys
465                 470                 475                 480

Arg Met Pro Cys Ala Glu Asp Tyr Leu Ser Val Val Leu Asn Gln Leu
                485                 490                 495

Cys Val Leu His Glu Lys Thr Pro Val Ser Asp Arg Val Thr Lys Cys
            500                 505                 510

Cys Thr Glu Ser Leu Val Asn Arg Arg Pro Cys Phe Ser Ala Leu Glu
        515                 520                 525

Val Asp Glu Thr Tyr Val Pro Lys Glu Phe Asn Ala Glu Thr Phe Thr
    530                 535                 540

Phe His Ala Asp Ile Cys Thr Leu Ser Glu Lys Glu Arg Gln Ile Lys
545                 550                 555                 560

Lys Gln Thr Ala Leu Val Glu Leu Val Lys His Lys Pro Lys Ala Thr
                565                 570                 575

Lys Glu Gln Leu Lys Ala Val Met Asp Asp Phe Ala Ala Phe Val Glu
            580                 585                 590

Lys Cys Cys Lys Ala Asp Asp Lys Glu Thr Cys Phe Ala Glu Glu Gly
        595                 600                 605
```

```
Lys Lys Leu Val Ala Ala Ser Gln Ala Ala Leu Gly Leu
    610                 615                 620
```

<210> 5
<211> 608
<212> PRT
<213> Macaca mulatta

<400> 5

```
Met Lys Trp Val Thr Phe Ile Ser Leu Leu Phe Leu Phe Ser Ser Ala
1               5                   10                  15

Tyr Ser Arg Gly Val Phe Arg Arg Asp Thr His Lys Ser Glu Val Ala
            20                  25                  30

His Arg Phe Lys Asp Leu Gly Glu Glu His Phe Lys Gly Leu Val Leu
        35                  40                  45

Val Ala Phe Ser Gln Tyr Leu Gln Gln Cys Pro Phe Glu Glu His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Pro Leu Val Arg Pro Glu Val
    130                 135                 140

Asp Val Met Cys Thr Ala Phe His Asp Asn Glu Ala Thr Phe Leu Lys
145                 150                 155                 160

Lys Tyr Leu Tyr Glu Val Ala Arg Arg His Pro Tyr Phe Tyr Ala Pro
                165                 170                 175

Glu Leu Leu Phe Phe Ala Ala Arg Tyr Lys Ala Ala Phe Ala Glu Cys
            180                 185                 190

Cys Gln Ala Ala Asp Lys Ala Ala Cys Leu Leu Pro Lys Leu Asp Glu
        195                 200                 205
```

```
Leu Arg Asp Glu Gly Lys Ala Ser Ser Ala Lys Gln Arg Leu Lys Cys
    210                 215                 220

Ala Ser Leu Gln Lys Phe Gly Asp Arg Ala Phe Lys Ala Trp Ala Val
225                 230                 235                 240

Ala Arg Leu Ser Gln Lys Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Met
        275                 280                 285

Cys Glu Asn Gln Asp Ser Ile Ser Ser Lys Leu Lys Glu Cys Cys Asp
    290                 295                 300

Lys Pro Leu Leu Glu Lys Ser His Cys Leu Ala Glu Val Glu Asn Asp
305                 310                 315                 320

Glu Met Pro Ala Asp Leu Pro Ser Leu Ala Ala Asp Tyr Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Met
        355                 360                 365

Leu Leu Leu Arg Leu Ala Lys Ala Tyr Glu Ala Thr Leu Glu Lys Cys
    370                 375                 380

Cys Ala Ala Ala Asp Pro His Glu Cys Tyr Ala Lys Val Phe Asp Glu
385                 390                 395                 400

Phe Gln Pro Leu Val Glu Glu Pro Gln Asn Leu Val Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ala Lys Cys Cys Lys Leu
    450                 455                 460
```

```
Pro Glu Ala Lys Arg Met Pro Cys Ala Glu Asp Tyr Leu Ser Val Val
465                 470                 475                 480

Leu Asn Arg Leu Cys Val Leu His Glu Lys Thr Pro Val Ser Glu Lys
                485                 490                 495

Val Thr Lys Cys Cys Thr Glu Ser Leu Val Asn Arg Arg Pro Cys Phe
            500                 505                 510

Ser Ala Leu Glu Leu Asp Glu Ala Tyr Val Pro Lys Ala Phe Asn Ala
        515                 520                 525

Glu Thr Phe Thr Phe His Ala Asp Met Cys Thr Leu Ser Glu Lys Glu
    530                 535                 540

Lys Gln Val Lys Lys Gln Thr Ala Leu Val Glu Leu Val Lys His Lys
545                 550                 555                 560

Pro Lys Ala Thr Lys Glu Gln Leu Lys Gly Val Met Asp Asn Phe Ala
                565                 570                 575

Ala Phe Val Glu Lys Cys Cys Lys Ala Asp Asp Lys Glu Ala Cys Phe
            580                 585                 590

Ala Glu Glu Gly Pro Lys Phe Val Ala Ala Ser Gln Ala Ala Leu Ala
        595                 600                 605
```

<210> 6
<211> 608
<212> PRT
<213> Mesocricetus auratus

<400> 6

```
Met Lys Trp Val Thr Phe Leu Leu Leu Leu Phe Val Ser Asp Ser Ala
1               5                   10                  15

Phe Ser Arg Gly Leu Phe Arg Arg Asp Ala His Lys Ser Glu Ile Ala
            20                  25                  30

His Arg Phe Lys Asp Leu Gly Glu Gln His Phe Lys Gly Leu Val Leu
        35                  40                  45

Ile Ala Phe Ser Gln Phe Leu Gln Lys Cys Pro Tyr Glu Glu His Val
    50                  55                  60

Lys Leu Val Asn Glu Val Thr Asp Phe Ala Lys Thr Cys Val Ala Asp
65                  70                  75                  80
```

Glu Ser Ala Glu Asn Cys Asp Lys Ser Leu His Thr Leu Phe Gly Asp
85 90 95

Lys Leu Cys Ala Ile Pro Thr Leu Arg Asp Ser Tyr Gly Glu Leu Ala
100 105 110

Asp Cys Cys Ala Lys Lys Glu Pro Glu Arg Asn Glu Cys Phe Leu Lys
115 120 125

His Lys Asp Asp His Pro Asn Leu Pro Pro Phe Val Arg Pro Asp Ala
130 135 140

Glu Ala Met Cys Thr Ser Phe Gln Glu Asn Ala Val Thr Phe Met Gly
145 150 155 160

His Tyr Leu His Glu Val Ala Arg Arg His Pro Tyr Phe Tyr Ala Pro
165 170 175

Glu Leu Leu Tyr Tyr Ala Glu Lys Tyr Ser Ala Ile Met Thr Glu Cys
180 185 190

Cys Gly Glu Ala Asp Lys Ala Ala Cys Ile Thr Pro Lys Leu Asp Ala
195 200 205

Leu Lys Glu Lys Ala Leu Ala Ser Ser Val Asn Gln Arg Leu Lys Cys
210 215 220

Ser Ser Leu Gln Arg Phe Gly Gln Arg Ala Phe Lys Ala Trp Ala Val
225 230 235 240

Ala Arg Met Ser Gln Lys Phe Pro Lys Ala Asp Phe Ala Glu Ile Thr
245 250 255

Lys Leu Ala Thr Asp Leu Thr Lys Leu Thr Glu Glu Cys Cys His Gly
260 265 270

Asp Leu Leu Glu Cys Ala Asp Asp Arg Ala Glu Leu Ala Lys Tyr Met
275 280 285

Cys Glu Asn Gln Ala Ser Ile Ser Ser Lys Leu Gln Ala Cys Cys Asp
290 295 300

Lys Pro Val Leu Lys Lys Ser His Cys Leu Ser Glu Val Glu Asn Asp
305 310 315 320

Asp Leu Pro Ala Asp Leu Pro Ser Leu Ala Ala Asp Phe Val Glu Asp

```
                325                 330                 335

Lys Glu Val Cys Lys Asn Tyr Ala Glu Ala Lys Asp Val Phe Leu Gly
            340                 345                 350

Thr Phe Leu Tyr Glu Tyr Ala Arg Arg His Pro Asp Tyr Ser Val Ala
        355                 360                 365

Leu Leu Leu Arg Leu Ala Lys Lys Tyr Glu Ala Thr Leu Glu Lys Cys
    370                 375                 380

Cys Ala Glu Ala Asp Pro Ser Ala Cys Tyr Gly Lys Val Leu Asp Glu
385                 390                 395                 400

Phe Gln Pro Leu Val Glu Glu Pro Lys Asn Leu Val Lys Ala Asn Cys
                405                 410                 415

Glu Leu Phe Glu Lys Leu Gly Glu Tyr Gly Phe Gln Asn Ala Leu Ile
            420                 425                 430

Val Arg Tyr Thr Gln Lys Ala Pro Gln Val Ser Thr Pro Thr Leu Val
        435                 440                 445

Glu Ala Ala Arg Asn Leu Gly Lys Val Gly Ser Lys Cys Cys Val Leu
    450                 455                 460

Pro Glu Ala Gln Arg Leu Pro Cys Val Glu Asp Tyr Ile Ser Ala Ile
465                 470                 475                 480

Leu Asn Arg Val Cys Val Leu His Glu Lys Thr Pro Val Ser Glu Gln
                485                 490                 495

Val Thr Lys Cys Cys Thr Gly Ser Val Val Glu Arg Arg Pro Cys Phe
            500                 505                 510

Ser Ala Leu Pro Val Asp Glu Thr Tyr Val Pro Lys Glu Phe Lys Ala
        515                 520                 525

Glu Thr Phe Thr Phe His Ala Asp Ile Cys Ser Leu Pro Glu Lys Glu
    530                 535                 540

Lys Gln Met Lys Lys Gln Ala Ala Leu Val Glu Leu Val Lys His Lys
545                 550                 555                 560

Pro Lys Ala Thr Gly Pro Gln Leu Arg Thr Val Leu Gly Glu Phe Thr
                565                 570                 575
```

```
Ala Phe Leu Asp Lys Cys Cys Lys Ala Glu Asp Lys Glu Ala Cys Phe
            580                 585                 590

Ser Glu Asp Gly Pro Lys Leu Val Ala Ser Ser Gln Ala Ala Leu Ala
        595                 600                 605
```

<210> 7
<211> 608
<212> PRT
<213> Cavia porcellus

<400> 7

```
Met Lys Trp Val Thr Phe Ile Ser Leu Leu Phe Leu Phe Ser Ser Val
1               5                   10                  15

Tyr Ser Arg Gly Val Phe Arg Arg Glu Ala His Lys Ser Glu Ile Ala
            20                  25                  30

His Arg Phe Asn Asp Leu Gly Glu Gly His Phe Lys Gly Leu Val Leu
        35                  40                  45

Ile Thr Leu Ser Gln His Leu Gln Lys Ser Pro Phe Glu Glu His Val
    50                  55                  60

Lys Leu Val Asn Glu Val Thr Asp Phe Ala Lys Ala Cys Val Ala Asp
65                  70                  75                  80

Glu Ser Ala Gln Asn Cys Gly Lys Ala Ile Ala Thr Leu Phe Gly Asp
                85                  90                  95

Lys Val Cys Ala Ile Pro Ser Leu Arg Glu Thr Tyr Gly Glu Leu Ala
            100                 105                 110

Asp Cys Cys Ala Lys Glu Asp Pro Asp Arg Val Glu Cys Phe Leu Gln
        115                 120                 125

His Lys Asp Asp Asn Pro Asn Leu Pro Pro Phe Glu Arg Pro Glu Pro
    130                 135                 140

Glu Ala Leu Cys Thr Ala Phe Lys Glu Asn Asn Asp Arg Phe Ile Gly
145                 150                 155                 160

His Tyr Leu Tyr Glu Val Ser Arg Arg His Pro Tyr Phe Tyr Ala Pro
                165                 170                 175

Glu Leu Leu Tyr Tyr Ala Glu Lys Tyr Lys Asn Ala Leu Thr Glu Cys
            180                 185                 190
```

```
Cys Glu Ala Ala Asp Lys Ala Ala Cys Leu Thr Pro Lys Leu Asp Ala
        195                 200                 205

Ile Lys Glu Lys Ala Leu Val Ser Ser Ala Gln Gln Arg Leu Lys Cys
    210                 215                 220

Ala Ser Leu Gln Lys Phe Gly Glu Arg Ala Phe Lys Ala Trp Ser Val
225                 230                 235                 240

Ala Arg Leu Ser Gln Lys Phe Pro Lys Ala Glu Phe Ala Glu Ile Ser
                245                 250                 255

Thr Ile Val Thr Ser Leu Thr Lys Val Thr Lys Glu Cys Cys His Gly
            260                 265                 270

Asp Leu Leu Glu Cys Ala Asp Asp Arg Gln Glu Leu Ala Lys Tyr Met
        275                 280                 285

Cys Glu His Gln Asp Ser Ile Ser Ser Lys Leu Lys Glu Cys Cys Val
    290                 295                 300

Lys Pro Thr Leu Gln Lys Ala His Cys Ile Leu Glu Ile Gln Arg Asp
305                 310                 315                 320

Glu Leu Pro Thr Glu Leu Pro Asp Leu Ala Val Asp Phe Val Glu Asp
                325                 330                 335

Lys Glu Val Cys Lys Asn Phe Ala Glu Ala Lys Asp Val Phe Leu Gly
            340                 345                 350

Thr Phe Leu Tyr Glu Tyr Ser Arg Arg His Pro Glu Tyr Ser Ile Gly
        355                 360                 365

Met Leu Leu Arg Ile Ala Lys Gly Tyr Glu Ala Lys Leu Glu Lys Cys
    370                 375                 380

Cys Ala Glu Ala Asp Pro His Ala Cys Tyr Ala Lys Val Phe Asp Glu
385                 390                 395                 400

Leu Gln Pro Leu Ile Asp Glu Pro Lys Lys Leu Val Gln Gln Asn Cys
                405                 410                 415

Glu Leu Phe Asp Lys Leu Gly Glu Tyr Gly Phe Gln Asn Ala Leu Ala
            420                 425                 430

Val Arg Tyr Thr Gln Lys Ala Pro Gln Val Ser Thr Pro Thr Leu Val
        435                 440                 445
```

```
Glu Tyr Ala Arg Lys Leu Gly Ser Val Gly Thr Lys Cys Cys Ser Leu
    450                 455                 460

Pro Glu Thr Glu Arg Leu Ser Cys Thr Glu Asn Tyr Leu Ala Leu Ile
465                 470                 475                 480

Leu Asn Arg Leu Cys Ile Leu His Glu Lys Thr Pro Val Ser Glu Arg
                485                 490                 495

Val Thr Lys Cys Cys Thr Glu Ser Leu Val Asn Arg Arg Pro Cys Phe
            500                 505                 510

Ser Ala Leu His Val Asp Glu Thr Tyr Val Pro Lys Pro Phe His Ala
        515                 520                 525

Asp Ser Phe Thr Phe His Ala Asp Ile Cys Thr Leu Pro Glu Lys Glu
    530                 535                 540

Lys Gln Val Lys Lys Gln Met Ala Leu Val Glu Leu Val Lys His Lys
545                 550                 555                 560

Pro Lys Ala Ser Glu Glu Gln Met Lys Thr Val Met Gly Asp Phe Ala
                565                 570                 575

Ala Phe Leu Lys Lys Cys Cys Asp Ala Asp Asn Lys Glu Ala Cys Phe
            580                 585                 590

Thr Glu Asp Gly Pro Lys Leu Val Ala Lys Cys Gln Ala Thr Leu Ala
        595                 600                 605
```

<210> 8
<211> 608
<212> PRT
<213> Mus musculus

<400> 8

```
Met Lys Trp Val Thr Phe Leu Leu Leu Leu Phe Val Ser Gly Ser Ala
1               5                   10                  15

Phe Ser Arg Gly Val Phe Arg Arg Glu Ala His Lys Ser Glu Ile Ala
            20                  25                  30

His Arg Tyr Asn Asp Leu Gly Glu Gln His Phe Lys Gly Leu Val Leu
        35                  40                  45

Ile Ala Phe Ser Gln Tyr Leu Gln Lys Cys Ser Tyr Asp Glu His Ala
    50                  55                  60
```

```
Lys Leu Val Gln Glu Val Thr Asp Phe Ala Lys Thr Cys Val Ala Asp
65                  70                  75                  80

Glu Ser Ala Ala Asn Cys Asp Lys Ser Leu His Thr Leu Phe Gly Asp
                85                  90                  95

Lys Leu Cys Ala Ile Pro Asn Leu Arg Glu Asn Tyr Gly Glu Leu Ala
            100                 105                 110

Asp Cys Cys Thr Lys Gln Glu Pro Glu Arg Asn Glu Cys Phe Leu Gln
        115                 120                 125

His Lys Asp Asp Asn Pro Ser Leu Pro Pro Phe Glu Arg Pro Glu Ala
    130                 135                 140

Glu Ala Met Cys Thr Ser Phe Lys Glu Asn Pro Thr Thr Phe Met Gly
145                 150                 155                 160

His Tyr Leu His Glu Val Ala Arg Arg His Pro Tyr Phe Tyr Ala Pro
                165                 170                 175

Glu Leu Leu Tyr Tyr Ala Glu Gln Tyr Asn Glu Ile Leu Thr Gln Cys
            180                 185                 190

Cys Ala Glu Ala Asp Lys Glu Ser Cys Leu Thr Pro Lys Leu Asp Gly
        195                 200                 205

Val Lys Glu Lys Ala Leu Val Ser Ser Val Arg Gln Arg Met Lys Cys
    210                 215                 220

Ser Ser Met Gln Lys Phe Gly Glu Arg Ala Phe Lys Ala Trp Ala Val
225                 230                 235                 240

Ala Arg Leu Ser Gln Thr Phe Pro Asn Ala Asp Phe Ala Glu Ile Thr
                245                 250                 255

Lys Leu Ala Thr Asp Leu Thr Lys Val Asn Lys Glu Cys Cys His Gly
            260                 265                 270

Asp Leu Leu Glu Cys Ala Asp Asp Arg Ala Glu Leu Ala Lys Tyr Met
        275                 280                 285

Cys Glu Asn Gln Ala Thr Ile Ser Ser Lys Leu Gln Thr Cys Cys Asp
    290                 295                 300

Lys Pro Leu Leu Lys Lys Ala His Cys Leu Ser Glu Val Glu His Asp
305                 310                 315                 320
```

Thr Met Pro Ala Asp Leu Pro Ala Ile Ala Ala Asp Phe Val Glu Asp
325 330 335

Gln Glu Val Cys Lys Asn Tyr Ala Glu Ala Lys Asp Val Phe Leu Gly
340 345 350

Thr Phe Leu Tyr Glu Tyr Ser Arg Arg His Pro Asp Tyr Ser Val Ser
355 360 365

Leu Leu Leu Arg Leu Ala Lys Lys Tyr Glu Ala Thr Leu Glu Lys Cys
370 375 380

Cys Ala Glu Ala Asn Pro Pro Ala Cys Tyr Gly Thr Val Leu Ala Glu
385 390 395 400

Phe Gln Pro Leu Val Glu Glu Pro Lys Asn Leu Val Lys Thr Asn Cys
405 410 415

Asp Leu Tyr Glu Lys Leu Gly Glu Tyr Gly Phe Gln Asn Ala Ile Leu
420 425 430

Val Arg Tyr Thr Gln Lys Ala Pro Gln Val Ser Thr Pro Thr Leu Val
435 440 445

Glu Ala Ala Arg Asn Leu Gly Arg Val Gly Thr Lys Cys Cys Thr Leu
450 455 460

Pro Glu Asp Gln Arg Leu Pro Cys Val Glu Asp Tyr Leu Ser Ala Ile
465 470 475 480

Leu Asn Arg Val Cys Leu Leu His Glu Lys Thr Pro Val Ser Glu His
485 490 495

Val Thr Lys Cys Cys Ser Gly Ser Leu Val Glu Arg Arg Pro Cys Phe
500 505 510

Ser Ala Leu Thr Val Asp Glu Thr Tyr Val Pro Lys Glu Phe Lys Ala
515 520 525

Glu Thr Phe Thr Phe His Ser Asp Ile Cys Thr Leu Pro Glu Lys Glu
530 535 540

Lys Gln Ile Lys Lys Gln Thr Ala Leu Ala Glu Leu Val Lys His Lys
545 550 555 560

Pro Lys Ala Thr Ala Glu Gln Leu Lys Thr Val Met Asp Asp Phe Ala

565 570 575

Gln Phe Leu Asp Thr Cys Cys Lys Ala Ala Asp Lys Asp Thr Cys Phe
580 585 590

Ser Thr Glu Gly Pro Asn Leu Val Thr Arg Cys Lys Asp Ala Leu Ala
595 600 605

<210> 9
<211> 608
<212> PRT
<213> Rattus norvegicus

<400> 9

```
Met Lys Trp Val Thr Phe Leu Leu Leu Leu Phe Ile Ser Gly Ser Ala
1               5                   10                  15

Phe Ser Arg Gly Val Phe Arg Arg Glu Ala His Lys Ser Glu Ile Ala
            20                  25                  30

His Arg Phe Lys Asp Leu Gly Glu Gln His Phe Lys Gly Leu Val Leu
        35                  40                  45

Ile Ala Phe Ser Gln Tyr Leu Gln Lys Cys Pro Tyr Glu Glu His Ile
    50                  55                  60

Lys Leu Val Gln Glu Val Thr Asp Phe Ala Lys Thr Cys Val Ala Asp
65                  70                  75                  80

Glu Asn Ala Glu Asn Cys Asp Lys Ser Ile His Thr Leu Phe Gly Asp
                85                  90                  95

Lys Leu Cys Ala Ile Pro Lys Leu Arg Asp Asn Tyr Gly Glu Leu Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Pro Phe Gln Arg Pro Glu Ala
    130                 135                 140

Glu Ala Met Cys Thr Ser Phe Gln Glu Asn Pro Thr Ser Phe Leu Gly
145                 150                 155                 160

His Tyr Leu His Glu Val Ala Arg Arg His Pro Tyr Phe Tyr Ala Pro
                165                 170                 175

Glu Leu Leu Tyr Tyr Ala Glu Lys Tyr Asn Glu Val Leu Thr Gln Cys
```

```
                180                 185                 190

Cys Thr Glu Ser Asp Lys Ala Ala Cys Leu Thr Pro Lys Leu Asp Ala
            195                 200                 205

Val Lys Glu Lys Ala Leu Val Ala Ala Val Arg Gln Arg Met Lys Cys
    210                 215                 220

Ser Ser Met Gln Arg Phe Gly Glu Arg Ala Phe Lys Ala Trp Ala Val
225                 230                 235                 240

Ala Arg Met Ser Gln Arg Phe Pro Asn Ala Glu Phe Ala Glu Ile Thr
                245                 250                 255

Lys Leu Ala Thr Asp Val Thr Lys Ile Asn Lys Glu Cys Cys His Gly
            260                 265                 270

Asp Leu Leu Glu Cys Ala Asp Asp Arg Ala Glu Leu Ala Lys Tyr Met
        275                 280                 285

Cys Glu Asn Gln Ala Thr Ile Ser Ser Lys Leu Gln Ala Cys Cys Asp
    290                 295                 300

Lys Pro Val Leu Gln Lys Ser Gln Cys Leu Ala Glu Ile Glu His Asp
305                 310                 315                 320

Asn Ile Pro Ala Asp Leu Pro Ser Ile Ala Ala Asp Phe Val Glu Asp
                325                 330                 335

Lys Glu Val Cys Lys Asn Tyr Ala Glu Ala Lys Asp Val Phe Leu Gly
            340                 345                 350

Thr Phe Leu Tyr Glu Tyr Ser Arg Arg His Pro Asp Tyr Ser Val Ser
        355                 360                 365

Leu Leu Leu Arg Leu Ala Lys Lys Tyr Glu Ala Thr Leu Glu Lys Cys
    370                 375                 380

Cys Ala Glu Gly Asp Pro Pro Ala Cys Tyr Gly Thr Val Leu Ala Glu
385                 390                 395                 400

Phe Gln Pro Leu Val Glu Glu Pro Lys Asn Leu Val Lys Thr Asn Cys
                405                 410                 415

Glu Leu Tyr Glu Lys Leu Gly Glu Tyr Gly Phe Gln Asn Ala Val Leu
            420                 425                 430
```

Val Arg Tyr Thr Gln Lys Ala Pro Gln Val Ser Thr Pro Thr Leu Val
435 440 445

Glu Ala Ala Arg Asn Leu Gly Arg Val Gly Thr Lys Cys Cys Thr Leu
450 455 460

Pro Glu Ala Gln Arg Leu Pro Cys Val Glu Asp Tyr Leu Ser Ala Ile
465 470 475 480

Leu Asn Arg Leu Cys Val Leu His Glu Lys Thr Pro Val Ser Glu Lys
485 490 495

Val Thr Lys Cys Cys Ser Gly Ser Leu Val Glu Arg Arg Pro Cys Phe
500 505 510

Ser Ala Leu Thr Val Asp Glu Thr Tyr Val Pro Lys Glu Phe Lys Ala
515 520 525

Glu Thr Phe Thr Phe His Ser Asp Ile Cys Thr Leu Pro Asp Lys Glu
530 535 540

Lys Gln Ile Lys Lys Gln Thr Ala Leu Ala Glu Leu Val Lys His Lys
545 550 555 560

Pro Lys Ala Thr Glu Asp Gln Leu Lys Thr Val Met Gly Asp Phe Ala
565 570 575

Gln Phe Val Asp Lys Cys Cys Lys Ala Ala Asp Lys Asp Asn Cys Phe
580 585 590

Ala Thr Glu Gly Pro Asn Leu Val Ala Arg Ser Lys Glu Ala Leu Ala
595 600 605

<210> 10
<211> 607
<212> PRT
<213> Bos taurus

<400> 10

Met Lys Trp Val Thr Phe Ile Ser Leu Leu Leu Leu Phe Ser Ser Ala
1 5 10 15

Tyr Ser Arg Gly Val Phe Arg Arg Asp Thr His Lys Ser Glu Ile Ala
20 25 30

His Arg Phe Lys Asp Leu Gly Glu Glu His Phe Lys Gly Leu Val Leu
35 40 45

```
Ile Ala Phe Ser Gln Tyr Leu Gln Gln Cys Pro Phe Asp Glu His Val
    50                  55                  60

Lys Leu Val Asn Glu Leu Thr Glu Phe Ala Lys Thr Cys Val Ala Asp
65                  70                  75                  80

Glu Ser His Ala Gly Cys Glu Lys Ser Leu His Thr Leu Phe Gly Asp
                85                  90                  95

Glu Leu Cys Lys Val Ala Ser Leu Arg Glu Thr Tyr Gly Asp Met Ala
            100                 105                 110

Asp Cys Cys Glu Lys Gln Glu Pro Glu Arg Asn Glu Cys Phe Leu Ser
        115                 120                 125

His Lys Asp Asp Ser Pro Asp Leu Pro Lys Leu Lys Pro Asp Pro Asn
    130                 135                 140

Thr Leu Cys Asp Glu Phe Lys Ala Asp Glu Lys Lys Phe Trp Gly Lys
145                 150                 155                 160

Tyr Leu Tyr Glu Ile Ala Arg Arg His Pro Tyr Phe Tyr Ala Pro Glu
                165                 170                 175

Leu Leu Tyr Tyr Ala Asn Lys Tyr Asn Gly Val Phe Gln Glu Cys Cys
            180                 185                 190

Gln Ala Glu Asp Lys Gly Ala Cys Leu Leu Pro Lys Ile Glu Thr Met
        195                 200                 205

Arg Glu Lys Val Leu Ala Ser Ser Ala Arg Gln Arg Leu Arg Cys Ala
    210                 215                 220

Ser Ile Gln Lys Phe Gly Glu Arg Ala Leu Lys Ala Trp Ser Val Ala
225                 230                 235                 240

Arg Leu Ser Gln Lys Phe Pro Lys Ala Glu Phe Val Glu Val Thr Lys
                245                 250                 255

Leu Val Thr Asp Leu Thr Lys Val His Lys Glu Cys Cys His Gly Asp
            260                 265                 270

Leu Leu Glu Cys Ala Asp Asp Arg Ala Asp Leu Ala Lys Tyr Ile Cys
        275                 280                 285

Asp Asn Gln Asp Thr Ile Ser Ser Lys Leu Lys Glu Cys Cys Asp Lys
    290                 295                 300
```

```
Pro Leu Leu Glu Lys Ser His Cys Ile Ala Glu Val Glu Lys Asp Ala
305                 310                 315                 320

Ile Pro Glu Asn Leu Pro Pro Leu Thr Ala Asp Phe Ala Glu Asp Lys
                325                 330                 335

Asp Val Cys Lys Asn Tyr Gln Glu Ala Lys Asp Ala Phe Leu Gly Ser
            340                 345                 350

Phe Leu Tyr Glu Tyr Ser Arg Arg His Pro Glu Tyr Ala Val Ser Val
        355                 360                 365

Leu Leu Arg Leu Ala Lys Glu Tyr Glu Ala Thr Leu Glu Glu Cys Cys
    370                 375                 380

Ala Lys Asp Asp Pro His Ala Cys Tyr Ser Thr Val Phe Asp Lys Leu
385                 390                 395                 400

Lys His Leu Val Asp Glu Pro Gln Asn Leu Ile Lys Gln Asn Cys Asp
                405                 410                 415

Gln Phe Glu Lys Leu Gly Glu Tyr Gly Phe Gln Asn Ala Leu Ile Val
            420                 425                 430

Arg Tyr Thr Arg Lys Val Pro Gln Val Ser Thr Pro Thr Leu Val Glu
        435                 440                 445

Val Ser Arg Ser Leu Gly Lys Val Gly Thr Arg Cys Cys Thr Lys Pro
    450                 455                 460

Glu Ser Glu Arg Met Pro Cys Thr Glu Asp Tyr Leu Ser Leu Ile Leu
465                 470                 475                 480

Asn Arg Leu Cys Val Leu His Glu Lys Thr Pro Val Ser Glu Lys Val
                485                 490                 495

Thr Lys Cys Cys Thr Glu Ser Leu Val Asn Arg Arg Pro Cys Phe Ser
            500                 505                 510

Ala Leu Thr Pro Asp Glu Thr Tyr Val Pro Lys Ala Phe Asp Glu Lys
        515                 520                 525

Leu Phe Thr Phe His Ala Asp Ile Cys Thr Leu Pro Asp Thr Glu Lys
    530                 535                 540

Gln Ile Lys Lys Gln Thr Ala Leu Val Glu Leu Leu Lys His Lys Pro
545                 550                 555                 560
```

Lys Ala Thr Glu Glu Gln Leu Lys Thr Val Met Glu Asn Phe Val Ala
565 570 575

Phe Val Asp Lys Cys Cys Ala Ala Asp Asp Lys Glu Ala Cys Phe Ala
580 585 590

Val Glu Gly Pro Lys Leu Val Val Ser Thr Gln Thr Ala Leu Ala
595 600 605

<210> 11
<211> 607
<212> PRT
<213> Equus caballus

<400> 11

Met Lys Trp Val Thr Phe Val Ser Leu Leu Phe Leu Phe Ser Ser Ala
1 5 10 15

Tyr Ser Arg Gly Val Leu Arg Arg Asp Thr His Lys Ser Glu Ile Ala
20 25 30

His Arg Phe Asn Asp Leu Gly Glu Lys His Phe Lys Gly Leu Val Leu
35 40 45

Val Ala Phe Ser Gln Tyr Leu Gln Gln Cys Pro Phe Glu Asp His Val
50 55 60

Lys Leu Val Asn Glu Val Thr Glu Phe Ala Lys Lys Cys Ala Ala Asp
65 70 75 80

Glu Ser Ala Glu Asn Cys Asp Lys Ser Leu His Thr Leu Phe Gly Asp
85 90 95

Lys Leu Cys Thr Val Ala Thr Leu Arg Ala Thr Tyr Gly Glu Leu Ala
100 105 110

Asp Cys Cys Glu Lys Gln Glu Pro Glu Arg Asn Glu Cys Phe Leu Thr
115 120 125

His Lys Asp Asp His Pro Asn Leu Pro Lys Leu Lys Pro Glu Pro Asp
130 135 140

Ala Gln Cys Ala Ala Phe Gln Glu Asp Pro Asp Lys Phe Leu Gly Lys
145 150 155 160

Tyr Leu Tyr Glu Val Ala Arg Arg His Pro Tyr Phe Tyr Gly Pro Glu
165 170 175

```
Leu Leu Phe His Ala Glu Glu Tyr Lys Ala Asp Phe Thr Glu Cys Cys
            180                 185                 190

Pro Ala Asp Asp Lys Leu Ala Cys Leu Ile Pro Lys Leu Asp Ala Leu
        195                 200                 205

Lys Glu Arg Ile Leu Leu Ser Ser Ala Lys Glu Arg Leu Lys Cys Ser
    210                 215                 220

Ser Phe Gln Asn Phe Gly Glu Arg Ala Val Lys Ala Trp Ser Val Ala
225                 230                 235                 240

Arg Leu Ser Gln Lys Phe Pro Lys Ala Asp Phe Ala Glu Val Ser Lys
                245                 250                 255

Ile Val Thr Asp Leu Thr Lys Val His Lys Glu Cys Cys His Gly Asp
            260                 265                 270

Leu Leu Glu Cys Ala Asp Asp Arg Ala Asp Leu Ala Lys Tyr Ile Cys
        275                 280                 285

Glu His Gln Asp Ser Ile Ser Gly Lys Leu Lys Ala Cys Cys Asp Lys
    290                 295                 300

Pro Leu Leu Gln Lys Ser His Cys Ile Ala Glu Val Lys Glu Asp Asp
305                 310                 315                 320

Leu Pro Ser Asp Leu Pro Ala Leu Ala Ala Asp Phe Ala Glu Asp Lys
                325                 330                 335

Glu Ile Cys Lys His Tyr Lys Asp Ala Lys Asp Val Phe Leu Gly Thr
            340                 345                 350

Phe Leu Tyr Glu Tyr Ser Arg Arg His Pro Asp Tyr Ser Val Ser Leu
        355                 360                 365

Leu Leu Arg Ile Ala Lys Thr Tyr Glu Ala Thr Leu Glu Lys Cys Cys
    370                 375                 380

Ala Glu Ala Asp Pro Pro Ala Cys Tyr Arg Thr Val Phe Asp Gln Phe
385                 390                 395                 400

Thr Pro Leu Val Glu Glu Pro Lys Ser Leu Val Lys Lys Asn Cys Asp
                405                 410                 415
```

420 425 430

Arg Tyr Thr Lys Lys Ala Pro Gln Val Ser Thr Pro Thr Leu Val Glu
435 440 445

Ile Gly Arg Thr Leu Gly Lys Val Gly Ser Arg Cys Cys Lys Leu Pro
450 455 460

Glu Ser Glu Arg Leu Pro Cys Ser Glu Asn His Leu Ala Leu Ala Leu
465 470 475 480

Asn Arg Leu Cys Val Leu His Glu Lys Thr Pro Val Ser Glu Lys Ile
485 490 495

Thr Lys Cys Cys Thr Asp Ser Leu Ala Glu Arg Arg Pro Cys Phe Ser
500 505 510

Ala Leu Glu Leu Asp Glu Gly Tyr Val Pro Lys Glu Phe Lys Ala Glu
515 520 525

Thr Phe Thr Phe His Ala Asp Ile Cys Thr Leu Pro Glu Asp Glu Lys
530 535 540

Gln Ile Lys Lys Gln Ser Ala Leu Ala Glu Leu Val Lys His Lys Pro
545 550 555 560

Lys Ala Thr Lys Glu Gln Leu Lys Thr Val Leu Gly Asn Phe Ser Ala
565 570 575

Phe Val Ala Lys Cys Cys Gly Arg Glu Asp Lys Glu Ala Cys Phe Ala
580 585 590

Glu Glu Gly Pro Lys Leu Val Ala Ser Ser Gln Leu Ala Leu Ala
595 600 605

<210> 12
<211> 607
<212> PRT
<213> Equus asinus

<400> 12

```
Met Lys Trp Val Thr Phe Val Ser Leu Leu Phe Leu Phe Ser Ser Ala
1               5                   10                  15

Tyr Phe Arg Gly Val Leu Arg Arg Asp Thr His Lys Ser Glu Ile Ala
            20                  25                  30

His Arg Phe Asn Asp Leu Gly Glu Lys His Phe Lys Gly Leu Val Leu
```

```
            35                  40                  45

Val Ala Phe Ser Gln Tyr Leu Gln Gln Cys Pro Phe Glu Asp His Val
    50                  55                  60

Lys Leu Val Asn Glu Val Thr Glu Phe Ala Lys Lys Cys Ala Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Ala Thr Tyr Gly Glu Leu Ala
            100                 105                 110

Asp Cys Cys Glu Lys Gln Glu Pro Glu Arg Asn Glu Cys Phe Leu Thr
        115                 120                 125

His Lys Asp Asp His Pro Asn Leu Pro Lys Leu Lys Pro Glu Pro Asp
    130                 135                 140

Ala Gln Cys Ala Ala Phe Gln Glu Asp Pro Asp Lys Phe Leu Gly Lys
145                 150                 155                 160

Tyr Leu Tyr Glu Val Ala Arg Arg His Pro Tyr Phe Tyr Gly Pro Glu
                165                 170                 175

Leu Leu Phe His Ala Glu Glu Tyr Lys Ala Asp Phe Thr Glu Cys Cys
            180                 185                 190

Pro Ala Asp Asp Lys Ala Gly Cys Leu Ile Pro Lys Leu Asp Ala Leu
        195                 200                 205

Lys Glu Arg Ile Leu Leu Ser Ser Ala Lys Glu Arg Leu Lys Cys Ser
    210                 215                 220

Ser Phe Gln Lys Phe Gly Glu Arg Ala Phe Lys Ala Trp Ser Val Ala
225                 230                 235                 240

Arg Leu Ser Gln Lys Phe Pro Lys Ala Asp Phe Ala Glu Val Ser Lys
                245                 250                 255

Ile Val Thr Asp Leu Thr Lys Val His Lys Glu Cys Cys His Gly Asp
            260                 265                 270

Leu Leu Glu Cys Ala Asp Asp Arg Ala Asp Leu Thr Lys Tyr Ile Cys
        275                 280                 285
```

Glu His Gln Asp Ser Ile Ser Gly Lys Leu Lys Ala Cys Cys Asp Lys
    290                 295                 300

Pro Leu Leu Gln Lys Ser His Cys Ile Ala Glu Val Lys Glu Asp Asp
305                 310                 315                 320

Leu Pro Ser Asp Leu Pro Ala Leu Ala Ala Asp Phe Ala Glu Asp Lys
                325                 330                 335

Glu Ile Cys Lys His Tyr Lys Asp Ala Lys Asp Val Phe Leu Gly Thr
            340                 345                 350

Phe Leu Tyr Glu Tyr Ser Arg Arg His Pro Asp Tyr Ser Val Ser Leu
        355                 360                 365

Leu Leu Arg Ile Ala Lys Thr Tyr Glu Ala Thr Leu Glu Lys Cys Cys
    370                 375                 380

Ala Glu Ala Asp Pro Pro Ala Cys Tyr Ala Thr Val Phe Asp Gln Phe
385                 390                 395                 400

Thr Pro Leu Val Glu Glu Pro Lys Ser Leu Val Lys Lys Asn Cys Asp
                405                 410                 415

Leu Phe Glu Glu Val Gly Glu Tyr Asp Phe Gln Asn Ala Leu Ile Val
            420                 425                 430

Arg Tyr Thr Lys Lys Ala Pro Gln Val Ser Thr Pro Thr Leu Val Glu
        435                 440                 445

Ile Gly Arg Thr Leu Gly Lys Val Gly Ser Arg Cys Cys Lys Leu Pro
    450                 455                 460

Glu Ser Glu Arg Leu Pro Cys Ser Glu Asn His Leu Ala Leu Ala Leu
465                 470                 475                 480

Asn Arg Leu Cys Val Leu His Glu Lys Thr Pro Val Ser Glu Lys Ile
                485                 490                 495

Thr Lys Cys Cys Thr Asp Ser Leu Ala Glu Arg Arg Pro Cys Phe Ser
            500                 505                 510

Ala Leu Glu Leu Asp Glu Gly Tyr Ile Pro Lys Glu Phe Lys Ala Glu
        515                 520                 525

Thr Phe Thr Phe His Ala Asp Ile Cys Thr Leu Pro Glu Asp Glu Lys
    530                 535                 540

Gln Ile Lys Lys Gln Ser Ala Leu Ala Glu Leu Val Lys His Lys Pro
545 550 555 560

Lys Ala Thr Lys Glu Gln Leu Lys Thr Val Leu Gly Asn Phe Ser Ala
565 570 575

Phe Val Ala Lys Cys Cys Gly Ala Glu Asp Lys Glu Ala Cys Phe Ala
580 585 590

Glu Glu Gly Pro Lys Leu Val Ala Ser Ser Gln Leu Ala Leu Ala
595 600 605

<210> 13
<211> 608
<212> PRT
<213> Oryctolagus cuniculus

<400> 13

Met Lys Trp Val Thr Phe Ile Ser Leu Leu Phe Leu Phe Ser Ser Ala
1 5 10 15

Tyr Ser Arg Gly Val Phe Arg Arg Glu Ala His Lys Ser Glu Ile Ala
20 25 30

His Arg Phe Asn Asp Val Gly Glu Glu His Phe Ile Gly Leu Val Leu
35 40 45

Ile Thr Phe Ser Gln Tyr Leu Gln Lys Cys Pro Tyr Glu Glu His Ala
50 55 60

Lys Leu Val Lys Glu Val Thr Asp Leu Ala Lys Ala Cys Val Ala Asp
65 70 75 80

Glu Ser Ala Ala Asn Cys Asp Lys Ser Leu His Asp Ile Phe Gly Asp
85 90 95

Lys Ile Cys Ala Leu Pro Ser Leu Arg Asp Thr Tyr Gly Asp Val Ala
100 105 110

Asp Cys Cys Glu Lys Lys Glu Pro Glu Arg Asn Glu Cys Phe Leu His
115 120 125

His Lys Asp Asp Lys Pro Asp Leu Pro Pro Phe Ala Arg Pro Glu Ala
130 135 140

Asp Val Leu Cys Lys Ala Phe His Asp Asp Glu Lys Ala Phe Phe Gly
145 150 155 160

```
His Tyr Leu Tyr Glu Val Ala Arg Arg His Pro Tyr Phe Tyr Ala Pro
                165                 170                 175

Glu Leu Leu Tyr Tyr Ala Gln Lys Tyr Lys Ala Ile Leu Thr Glu Cys
            180                 185                 190

Cys Glu Ala Ala Asp Lys Gly Ala Cys Leu Thr Pro Lys Leu Asp Ala
        195                 200                 205

Leu Glu Gly Lys Ser Leu Ile Ser Ala Ala Gln Glu Arg Leu Arg Cys
    210                 215                 220

Ala Ser Ile Gln Lys Phe Gly Asp Arg Ala Tyr Lys Ala Trp Ala Leu
225                 230                 235                 240

Val Arg Leu Ser Gln Arg Phe Pro Lys Ala Asp Phe Thr Asp Ile Ser
                245                 250                 255

Lys Ile Val Thr Asp Leu Thr Lys Val His Lys Glu Cys Cys His Gly
            260                 265                 270

Asp Leu Leu Glu Cys Ala Asp Asp Arg Ala Asp Leu Ala Lys Tyr Met
        275                 280                 285

Cys Glu His Gln Glu Thr Ile Ser Ser His Leu Lys Glu Cys Cys Asp
    290                 295                 300

Lys Pro Ile Leu Glu Lys Ala His Cys Ile Tyr Gly Leu His Asn Asp
305                 310                 315                 320

Glu Thr Pro Ala Gly Leu Pro Ala Val Ala Glu Glu Phe Val Glu Asp
                325                 330                 335

Lys Asp Val Cys Lys Asn Tyr Glu Glu Ala Lys Asp Leu Phe Leu Gly
            340                 345                 350

Lys Phe Leu Tyr Glu Tyr Ser Arg Arg His Pro Asp Tyr Ser Val Val
        355                 360                 365

Leu Leu Leu Arg Leu Gly Lys Ala Tyr Glu Ala Thr Leu Lys Lys Cys
    370                 375                 380

Cys Ala Thr Asp Asp Pro His Ala Cys Tyr Ala Lys Val Leu Asp Glu
385                 390                 395                 400

Phe Gln Pro Leu Val Asp Glu Pro Lys Asn Leu Val Lys Gln Asn Cys
                405                 410                 415
```

Glu Leu Tyr Glu Gln Leu Gly Asp Tyr Asn Phe Gln Asn Ala Leu Leu
420 425 430

Val Arg Tyr Thr Lys Lys Val Pro Gln Val Ser Thr Pro Thr Leu Val
435 440 445

Glu Ile Ser Arg Ser Leu Gly Lys Val Gly Ser Lys Cys Cys Lys His
450 455 460

Pro Glu Ala Glu Arg Leu Pro Cys Val Glu Asp Tyr Leu Ser Val Val
465 470 475 480

Leu Asn Arg Leu Cys Val Leu His Glu Lys Thr Pro Val Ser Glu Lys
485 490 495

Val Thr Lys Cys Cys Ser Glu Ser Leu Val Asp Arg Arg Pro Cys Phe
500 505 510

Ser Ala Leu Gly Pro Asp Glu Thr Tyr Val Pro Lys Glu Phe Asn Ala
515 520 525

Glu Thr Phe Thr Phe His Ala Asp Ile Cys Thr Leu Pro Glu Thr Glu
530 535 540

Arg Lys Ile Lys Lys Gln Thr Ala Leu Val Glu Leu Val Lys His Lys
545 550 555 560

Pro His Ala Thr Asn Asp Gln Leu Lys Thr Val Val Gly Glu Phe Thr
565 570 575

Ala Leu Leu Asp Lys Cys Cys Ser Ala Glu Asp Lys Glu Ala Cys Phe
580 585 590

Ala Val Glu Gly Pro Lys Leu Val Glu Ser Ser Lys Ala Thr Leu Gly
595 600 605

<210> 14
<211> 583
<212> PRT
<213> Capra hircus

<400> 14

Asp Thr His Lys Ser Glu Ile Ala His Arg Phe Asn Asp Leu Gly Glu
1 5 10 15

Glu Asn Phe Gln Gly Leu Val Leu Ile Ala Phe Ser Gln Tyr Leu Gln
20 25 30

```
Gln Cys Pro Phe Asp Glu His Val Lys Leu Val Lys Glu Leu Thr Glu
        35                  40                  45

Phe Ala Lys Thr Cys Val Ala Asp Glu Ser His Ala Gly Cys Asp Lys
    50                  55                  60

Ser Leu His Thr Leu Phe Gly Asp Glu Leu Cys Lys Val Ala Thr Leu
65                  70                  75                  80

Arg Glu Thr Tyr Gly Asp Met Ala Asp Cys Cys Glu Lys Gln Glu Pro
                85                  90                  95

Glu Arg Asn Glu Cys Phe Leu Lys His Lys Asp Asp Ser Pro Asp Leu
            100                 105                 110

Pro Lys Leu Lys Pro Glu Pro Asp Thr Leu Cys Ala Glu Phe Lys Ala
        115                 120                 125

Asp Glu Lys Lys Phe Trp Gly Lys Tyr Leu Tyr Glu Val Ala Arg Arg
    130                 135                 140

His Pro Tyr Phe Tyr Ala Pro Glu Leu Leu Tyr Tyr Ala Asn Lys Tyr
145                 150                 155                 160

Asn Gly Val Phe Gln Glu Cys Cys Gln Ala Glu Asp Lys Gly Ala Cys
                165                 170                 175

Leu Leu Pro Lys Ile Glu Thr Met Arg Glu Lys Val Leu Ala Ser Ser
            180                 185                 190

Ala Arg Gln Arg Leu Arg Cys Ala Ser Ile Gln Lys Phe Gly Glu Arg
        195                 200                 205

Ala Leu Lys Ala Trp Ser Val Ala Arg Leu Ser Gln Lys Phe Pro Lys
    210                 215                 220

Ala Asp Phe Thr Asp Val Thr Lys Ile Val Thr Asp Leu Thr Lys Val
225                 230                 235                 240

His Lys Glu Cys Cys His Gly Asp Leu Leu Glu Cys Ala Asp Asp Arg
                245                 250                 255

Ala Asp Leu Ala Lys Tyr Ile Cys Asp His Gln Asp Thr Leu Ser Ser
            260                 265                 270

Lys Leu Lys Glu Cys Cys Asp Lys Pro Val Leu Glu Lys Ser His Cys
```

```
        275                 280                 285

Ile Ala Glu Ile Asp Lys Asp Ala Val Pro Glu Asn Leu Pro Pro Leu
    290                 295                 300

Thr Ala Asp Phe Ala Glu Asp Lys Glu Val Cys Lys Asn Tyr Gln Glu
305                 310                 315                 320

Ala Lys Asp Val Phe Leu Gly Ser Phe Leu Tyr Glu Tyr Ser Arg Arg
                325                 330                 335

His Pro Glu Tyr Ala Val Ser Val Leu Leu Arg Leu Ala Lys Glu Tyr
            340                 345                 350

Glu Ala Thr Leu Glu Asp Cys Cys Ala Lys Glu Asp Pro His Ala Cys
        355                 360                 365

Tyr Ala Thr Val Phe Asp Lys Leu Lys His Leu Val Asp Glu Pro Gln
    370                 375                 380

Asn Leu Ile Lys Lys Asn Cys Glu Leu Phe Glu Lys His Gly Glu Tyr
385                 390                 395                 400

Gly Phe Gln Asn Ala Leu Ile Val Arg Tyr Thr Arg Lys Ala Pro Gln
                405                 410                 415

Val Ser Thr Pro Thr Leu Val Glu Ile Ser Arg Ser Leu Gly Lys Val
            420                 425                 430

Gly Thr Lys Cys Cys Ala Lys Pro Glu Ser Glu Arg Met Pro Cys Thr
        435                 440                 445

Glu Asp Tyr Leu Ser Leu Ile Leu Asn Arg Leu Cys Val Leu His Glu
    450                 455                 460

Lys Thr Pro Val Ser Glu Lys Val Thr Lys Cys Cys Thr Glu Ser Leu
465                 470                 475                 480

Val Asn Arg Arg Pro Cys Phe Ser Asp Leu Thr Leu Asp Glu Thr Tyr
                485                 490                 495

Val Pro Lys Pro Phe Asp Gly Glu Ser Phe Thr Phe His Ala Asp Ile
            500                 505                 510

Cys Thr Leu Pro Asp Thr Glu Lys Gln Ile Lys Lys Gln Thr Ala Leu
        515                 520                 525
```

```
Val Glu Leu Leu Lys His Lys Pro Lys Ala Thr Asp Glu Gln Leu Lys
    530                 535                 540

Thr Val Met Glu Asn Phe Val Ala Phe Val Asp Lys Cys Cys Ala Ala
545                 550                 555                 560

Asp Asp Lys Glu Gly Cys Phe Leu Leu Glu Gly Pro Lys Leu Val Ala
                565                 570                 575

Ser Thr Gln Ala Ala Leu Ala
            580
```

<210> 15
<211> 607
<212> PRT
<213> Ovis aries

<400> 15

```
Met Lys Trp Val Thr Phe Ile Ser Leu Leu Leu Leu Phe Ser Ser Ala
1               5                   10                  15

Tyr Ser Arg Gly Val Phe Arg Arg Asp Thr His Lys Ser Glu Ile Ala
            20                  25                  30

His Arg Phe Asn Asp Leu Gly Glu Glu Asn Phe Gln Gly Leu Val Leu
        35                  40                  45

Ile Ala Phe Ser Gln Tyr Leu Gln Gln Cys Pro Phe Asp Glu His Val
    50                  55                  60

Lys Leu Val Lys Glu Leu Thr Glu Phe Ala Lys Thr Cys Val Ala Asp
65                  70                  75                  80

Glu Ser His Ala Gly Cys Asp Lys Ser Leu His Thr Leu Phe Gly Asp
                85                  90                  95

Glu Leu Cys Lys Val Ala Thr Leu Arg Glu Thr Tyr Gly Asp Met Ala
            100                 105                 110

Asp Cys Cys Glu Lys Gln Glu Pro Glu Arg Asn Glu Cys Phe Leu Asn
        115                 120                 125

His Lys Asp Asp Ser Pro Asp Leu Pro Lys Leu Lys Pro Glu Pro Asp
    130                 135                 140

Thr Leu Cys Ala Glu Phe Lys Ala Asp Glu Lys Lys Phe Trp Gly Lys
145                 150                 155                 160
```

```
Tyr Leu Tyr Glu Val Ala Arg Arg His Pro Tyr Phe Tyr Ala Pro Glu
                165                 170                 175

Leu Leu Tyr Tyr Ala Asn Lys Tyr Asn Gly Val Phe Gln Glu Cys Cys
            180                 185                 190

Gln Ala Glu Asp Lys Gly Ala Cys Leu Leu Pro Lys Ile Asp Ala Met
        195                 200                 205

Arg Glu Lys Val Leu Ala Ser Ser Ala Arg Gln Arg Leu Arg Cys Ala
    210                 215                 220

Ser Ile Gln Lys Phe Gly Glu Arg Ala Leu Lys Ala Trp Ser Val Ala
225                 230                 235                 240

Arg Leu Ser Gln Lys Phe Pro Lys Ala Asp Phe Thr Asp Val Thr Lys
                245                 250                 255

Ile Val Thr Asp Leu Thr Lys Val His Lys Glu Cys Cys His Gly Asp
            260                 265                 270

Leu Leu Glu Cys Ala Asp Asp Arg Ala Asp Leu Ala Lys Tyr Ile Cys
        275                 280                 285

Asp His Gln Asp Ala Leu Ser Ser Lys Leu Lys Glu Cys Cys Asp Lys
    290                 295                 300

Pro Val Leu Glu Lys Ser His Cys Ile Ala Glu Val Asp Lys Asp Ala
305                 310                 315                 320

Val Pro Glu Asn Leu Pro Pro Leu Thr Ala Asp Phe Ala Glu Asp Lys
                325                 330                 335

Glu Val Cys Lys Asn Tyr Gln Glu Ala Lys Asp Val Phe Leu Gly Ser
            340                 345                 350

Phe Leu Tyr Glu Tyr Ser Arg Arg His Pro Glu Tyr Ala Val Ser Val
        355                 360                 365

Leu Leu Arg Leu Ala Lys Glu Tyr Glu Ala Thr Leu Glu Asp Cys Cys
    370                 375                 380

Ala Lys Glu Asp Pro His Ala Cys Tyr Ala Thr Val Phe Asp Lys Leu
385                 390                 395                 400

Lys His Leu Val Asp Glu Pro Gln Asn Leu Ile Lys Lys Asn Cys Glu
                405                 410                 415
```

Leu Phe Glu Lys His Gly Glu Tyr Gly Phe Gln Asn Ala Leu Ile Val
420 425 430

Arg Tyr Thr Arg Lys Ala Pro Gln Val Ser Thr Pro Thr Leu Val Glu
435 440 445

Ile Ser Arg Ser Leu Gly Lys Val Gly Thr Lys Cys Cys Ala Lys Pro
450 455 460

Glu Ser Glu Arg Met Pro Cys Thr Glu Asp Tyr Leu Ser Leu Ile Leu
465 470 475 480

Asn Arg Leu Cys Val Leu His Glu Lys Thr Pro Val Ser Glu Lys Val
485 490 495

Thr Lys Cys Cys Thr Glu Ser Leu Val Asn Arg Arg Pro Cys Phe Ser
500 505 510

Asp Leu Thr Leu Asp Glu Thr Tyr Val Pro Lys Pro Phe Asp Glu Lys
515 520 525

Phe Phe Thr Phe His Ala Asp Ile Cys Thr Leu Pro Asp Thr Glu Lys
530 535 540

Gln Ile Lys Lys Gln Thr Ala Leu Val Glu Leu Leu Lys His Lys Pro
545 550 555 560

Lys Ala Thr Asp Glu Gln Leu Lys Thr Val Met Glu Asn Phe Val Ala
565 570 575

Phe Val Asp Lys Cys Cys Ala Ala Asp Asp Lys Glu Gly Cys Phe Val
580 585 590

Leu Glu Gly Pro Lys Leu Val Ala Ser Thr Gln Ala Ala Leu Ala
595 600 605

<210> 16
<211> 608
<212> PRT
<213> canis lupus familiaris

<400> 16

Met Lys Trp Val Thr Phe Ile Ser Leu Phe Phe Leu Phe Ser Ser Ala
1 5 10 15

Tyr Ser Arg Gly Leu Val Arg Arg Glu Ala Tyr Lys Ser Glu Ile Ala
20 25 30

```
His Arg Tyr Asn Asp Leu Gly Glu Glu His Phe Arg Gly Leu Val Leu
        35                  40                  45

Val Ala Phe Ser Gln Tyr Leu Gln Gln Cys Pro Phe Glu Asp His Val
    50                  55                  60

Lys Leu Ala Lys Glu Val Thr Glu Phe Ala Lys Ala Cys Ala Ala Glu
65                  70                  75                  80

Glu Ser Gly Ala Asn Cys Asp Lys Ser Leu His Thr Leu Phe Gly Asp
                85                  90                  95

Lys Leu Cys Thr Val Ala Ser Leu Arg Asp Lys Tyr Gly Asp Met Ala
            100                 105                 110

Asp Cys Cys Glu Lys Gln Glu Pro Asp Arg Asn Glu Cys Phe Leu Ala
        115                 120                 125

His Lys Asp Asp Asn Pro Gly Phe Pro Pro Leu Val Ala Pro Glu Pro
    130                 135                 140

Asp Ala Leu Cys Ala Ala Phe Gln Asp Asn Glu Gln Leu Phe Leu Gly
145                 150                 155                 160

Lys Tyr Leu Tyr Glu Ile Ala Arg Arg His Pro Tyr Phe Tyr Ala Pro
                165                 170                 175

Glu Leu Leu Tyr Tyr Ala Gln Gln Tyr Lys Gly Val Phe Ala Glu Cys
            180                 185                 190

Cys Gln Ala Ala Asp Lys Ala Ala Cys Leu Gly Pro Lys Ile Glu Ala
        195                 200                 205

Leu Arg Glu Lys Val Leu Leu Ser Ser Ala Lys Glu Arg Phe Lys Cys
    210                 215                 220

Ala Ser Leu Gln Lys Phe Gly Asp Arg Ala Phe Lys Ala Trp Ser Val
225                 230                 235                 240

Ala Arg Leu Ser Gln Arg Phe Pro Lys Ala Asp Phe Ala Glu Ile Ser
                245                 250                 255

Lys Val Val Thr Asp Leu Thr Lys Val His Lys Glu Cys Cys His Gly
            260                 265                 270

Asp Leu Leu Glu Cys Ala Asp Asp Arg Ala Asp Leu Ala Lys Tyr Met
        275                 280                 285
```

```
Cys Glu Asn Gln Asp Ser Ile Ser Thr Lys Leu Lys Glu Cys Cys Asp
    290                 295                 300

Lys Pro Val Leu Glu Lys Ser Gln Cys Leu Ala Glu Val Glu Arg Asp
305                 310                 315                 320

Glu Leu Pro Gly Asp Leu Pro Ser Leu Ala Ala Asp Phe Val Glu Asp
                325                 330                 335

Lys Glu Val Cys Lys Asn Tyr Gln Glu Ala Lys Asp Val Phe Leu Gly
            340                 345                 350

Thr Phe Leu Tyr Glu Tyr Ala Arg Arg His Pro Glu Tyr Ser Val Ser
        355                 360                 365

Leu Leu Leu Arg Leu Ala Lys Glu Tyr Glu Ala Thr Leu Glu Lys Cys
    370                 375                 380

Cys Ala Thr Asp Asp Pro Pro Thr Cys Tyr Ala Lys Val Leu Asp Glu
385                 390                 395                 400

Phe Lys Pro Leu Val Asp Glu Pro Gln Asn Leu Val Lys Thr Asn Cys
                405                 410                 415

Glu Leu Phe Glu Lys Leu Gly Glu Tyr Gly Phe Gln Asn Ala Leu Leu
            420                 425                 430

Val Arg Tyr Thr Lys Lys Ala Pro Gln Val Ser Thr Pro Thr Leu Val
        435                 440                 445

Glu Val Ser Arg Lys Leu Gly Lys Val Gly Thr Lys Cys Cys Lys Lys
    450                 455                 460

Pro Glu Ser Glu Arg Met Ser Cys Ala Glu Asp Phe Leu Ser Val Val
465                 470                 475                 480

Leu Asn Arg Leu Cys Val Leu His Glu Lys Thr Pro Val Ser Glu Arg
                485                 490                 495

Val Thr Lys Cys Cys Ser Glu Ser Leu Val Asn Arg Arg Pro Cys Phe
            500                 505                 510

Ser Gly Leu Glu Val Asp Glu Thr Tyr Val Pro Lys Glu Phe Asn Ala
        515                 520                 525

Glu Thr Phe Thr Phe His Ala Asp Leu Cys Thr Leu Pro Glu Ala Glu
```

```
            530                 535                 540

        Lys Gln Val Lys Lys Gln Thr Ala Leu Val Glu Leu Leu Lys His Lys
        545                 550                 555                 560

        Pro Lys Ala Thr Asp Glu Gln Leu Lys Thr Val Met Gly Asp Phe Gly
                        565                 570                 575

        Ala Phe Val Glu Lys Cys Cys Ala Ala Glu Asn Lys Glu Gly Cys Phe
                    580                 585                 590

        Ser Glu Glu Gly Pro Lys Leu Val Ala Ala Ala Gln Ala Ala Leu Val
                595                 600                 605
```

<210> 17
<211> 615
<212> PRT
<213> Gallus gallus

<400> 17

```
Met Lys Trp Val Thr Leu Ile Ser Phe Ile Phe Leu Phe Ser Ser Ala
1               5                   10                  15

Thr Ser Arg Asn Leu Gln Arg Phe Ala Arg Asp Ala Glu His Lys Ser
            20                  25                  30

Glu Ile Ala His Arg Tyr Asn Asp Leu Lys Glu Glu Thr Phe Lys Ala
        35                  40                  45

Val Ala Met Ile Thr Phe Ala Gln Tyr Leu Gln Arg Cys Ser Tyr Glu
    50                  55                  60

Gly Leu Ser Lys Leu Val Lys Asp Val Val Asp Leu Ala Gln Lys Cys
65                  70                  75                  80

Val Ala Asn Glu Asp Ala Pro Glu Cys Ser Lys Pro Leu Pro Ser Ile
                85                  90                  95

Ile Leu Asp Glu Ile Cys Gln Val Glu Lys Leu Arg Asp Ser Tyr Gly
            100                 105                 110

Ala Met Ala Asp Cys Cys Ser Lys Ala Asp Pro Glu Arg Asn Glu Cys
        115                 120                 125

Phe Leu Ser Phe Lys Val Ser Gln Pro Asp Phe Val Gln Pro Tyr Gln
    130                 135                 140

Arg Pro Ala Ser Asp Val Ile Cys Gln Glu Tyr Gln Asp Asn Arg Val
```

145 150 155 160

Ser Phe Leu Gly His Phe Ile Tyr Ser Val Ala Arg Arg His Pro Phe
165 170 175

Leu Tyr Ala Pro Ala Ile Leu Ser Phe Ala Val Asp Phe Glu His Ala
180 185 190

Leu Gln Ser Cys Cys Lys Glu Ser Asp Val Gly Ala Cys Leu Asp Thr
195 200 205

Lys Glu Ile Val Met Arg Glu Lys Ala Lys Gly Val Ser Val Lys Gln
210 215 220

Gln Tyr Phe Cys Gly Ile Leu Lys Gln Phe Gly Asp Arg Val Phe Gln
225 230 235 240

Ala Arg Gln Leu Ile Tyr Leu Ser Gln Lys Tyr Pro Lys Ala Pro Phe
245 250 255

Ser Glu Val Ser Lys Phe Val His Asp Ser Ile Gly Val His Lys Glu
260 265 270

Cys Cys Glu Gly Asp Met Val Glu Cys Met Asp Asp Met Ala Arg Met
275 280 285

Met Ser Asn Leu Cys Ser Gln Gln Asp Val Phe Ser Gly Lys Ile Lys
290 295 300

Asp Cys Cys Glu Lys Pro Ile Val Glu Arg Ser Gln Cys Ile Met Glu
305 310 315 320

Ala Glu Phe Asp Glu Lys Pro Ala Asp Leu Pro Ser Leu Val Glu Lys
325 330 335

Tyr Ile Glu Asp Lys Glu Val Cys Lys Ser Phe Glu Ala Gly His Asp
340 345 350

Ala Phe Met Ala Glu Phe Val Tyr Glu Tyr Ser Arg Arg His Pro Glu
355 360 365

Phe Ser Ile Gln Leu Ile Met Arg Ile Ala Lys Gly Tyr Glu Ser Leu
370 375 380

Leu Glu Lys Cys Cys Lys Thr Asp Asn Pro Ala Glu Cys Tyr Ala Asn
385 390 395 400

```
Ala Gln Glu Gln Leu Asn Gln His Ile Lys Glu Thr Gln Asp Val Val
                405                 410                 415

Lys Thr Asn Cys Asp Leu Leu His Asp His Gly Glu Ala Asp Phe Leu
            420                 425                 430

Lys Ser Ile Leu Ile Arg Tyr Thr Lys Lys Met Pro Gln Val Pro Thr
        435                 440                 445

Asp Leu Leu Leu Glu Thr Gly Lys Lys Met Thr Thr Ile Gly Thr Lys
    450                 455                 460

Cys Cys Gln Leu Gly Glu Asp Arg Arg Met Ala Cys Ser Glu Gly Tyr
465                 470                 475                 480

Leu Ser Ile Val Ile His Asp Thr Cys Arg Lys Gln Glu Thr Thr Pro
                485                 490                 495

Ile Asn Asp Asn Val Ser Gln Cys Cys Ser Gln Leu Tyr Ala Asn Arg
            500                 505                 510

Arg Pro Cys Phe Thr Ala Met Gly Val Asp Thr Lys Tyr Val Pro Pro
        515                 520                 525

Pro Phe Asn Pro Asp Met Phe Ser Phe Asp Glu Lys Leu Cys Ser Ala
    530                 535                 540

Pro Ala Glu Glu Arg Glu Val Gly Gln Met Lys Leu Leu Ile Asn Leu
545                 550                 555                 560

Ile Lys Arg Lys Pro Gln Met Thr Glu Glu Gln Ile Lys Thr Ile Ala
                565                 570                 575

Asp Gly Phe Thr Ala Met Val Asp Lys Cys Cys Lys Gln Ser Asp Ile
            580                 585                 590

Asn Thr Cys Phe Gly Glu Glu Gly Ala Asn Leu Ile Val Gln Ser Arg
        595                 600                 605

Ala Thr Leu Gly Ile Gly Ala
    610                 615
```

<210> 18
<211> 607
<212> PRT
<213> Sus scrofa

<400> 18

```
Met Lys Trp Val Thr Phe Ile Ser Leu Leu Phe Leu Phe Ser Ser Ala
1               5                   10                  15

Tyr Ser Arg Gly Val Phe Arg Arg Asp Thr Tyr Lys Ser Glu Ile Ala
            20                  25                  30

His Arg Phe Lys Asp Leu Gly Glu Gln Tyr Phe Lys Gly Leu Val Leu
        35                  40                  45

Ile Ala Phe Ser Gln His Leu Gln Gln Cys Pro Tyr Glu Glu His Val
    50                  55                  60

Lys Leu Val Arg Glu Val Thr Glu Phe Ala Lys Thr Cys Val Ala Asp
65                  70                  75                  80

Glu Ser Ala Glu Asn Cys Asp Lys Ser Ile His Thr Leu Phe Gly Asp
                85                  90                  95

Lys Leu Cys Ala Ile Pro Ser Leu Arg Glu His Tyr Gly Asp Leu Ala
            100                 105                 110

Asp Cys Cys Glu Lys Glu Glu Pro Glu Arg Asn Glu Cys Phe Leu Gln
        115                 120                 125

His Lys Asn Asp Asn Pro Asp Ile Pro Lys Leu Lys Pro Asp Pro Val
    130                 135                 140

Ala Leu Cys Ala Asp Phe Gln Glu Asp Glu Gln Lys Phe Trp Gly Lys
145                 150                 155                 160

Tyr Leu Tyr Glu Ile Ala Arg Arg His Pro Tyr Phe Tyr Ala Pro Glu
                165                 170                 175

Leu Leu Tyr Tyr Ala Ile Ile Tyr Lys Asp Val Phe Ser Glu Cys Cys
            180                 185                 190

Gln Ala Ala Asp Lys Ala Ala Cys Leu Leu Pro Lys Ile Glu His Leu
        195                 200                 205

Arg Glu Lys Val Leu Thr Ser Ala Ala Lys Gln Arg Leu Lys Cys Ala
    210                 215                 220

Ser Ile Gln Lys Phe Gly Glu Arg Ala Phe Lys Ala Trp Ser Leu Ala
225                 230                 235                 240

Arg Leu Ser Gln Arg Phe Pro Lys Ala Asp Phe Thr Glu Ile Ser Lys
                245                 250                 255
```

```
Ile Val Thr Asp Leu Ala Lys Val His Lys Glu Cys Cys His Gly Asp
            260                 265                 270

Leu Leu Glu Cys Ala Asp Asp Arg Ala Asp Leu Ala Lys Tyr Ile Cys
        275                 280                 285

Glu Asn Gln Asp Thr Ile Ser Thr Lys Leu Lys Glu Cys Cys Asp Lys
    290                 295                 300

Pro Leu Leu Glu Lys Ser His Cys Ile Ala Glu Ala Lys Arg Asp Glu
305                 310                 315                 320

Leu Pro Ala Asp Leu Asn Pro Leu Glu His Asp Phe Val Glu Asp Lys
                325                 330                 335

Glu Val Cys Lys Asn Tyr Lys Glu Ala Lys His Val Phe Leu Gly Thr
            340                 345                 350

Phe Leu Tyr Glu Tyr Ser Arg Arg His Pro Asp Tyr Ser Val Ser Leu
        355                 360                 365

Leu Leu Arg Ile Ala Lys Ile Tyr Glu Ala Thr Leu Glu Asp Cys Cys
    370                 375                 380

Ala Lys Glu Asp Pro Pro Ala Cys Tyr Ala Thr Val Phe Asp Lys Phe
385                 390                 395                 400

Gln Pro Leu Val Asp Glu Pro Lys Asn Leu Ile Lys Gln Asn Cys Glu
                405                 410                 415

Leu Phe Glu Lys Leu Gly Glu Tyr Gly Phe Gln Asn Ala Leu Ile Val
            420                 425                 430

Arg Tyr Thr Lys Lys Val Pro Gln Val Ser Thr Pro Thr Leu Val Glu
        435                 440                 445

Val Ala Arg Lys Leu Gly Leu Val Gly Ser Arg Cys Cys Lys Arg Pro
    450                 455                 460

Glu Glu Glu Arg Leu Ser Cys Ala Glu Asp Tyr Leu Ser Leu Val Leu
465                 470                 475                 480

Asn Arg Leu Cys Val Leu His Glu Lys Thr Pro Val Ser Glu Lys Val
                485                 490                 495

Thr Lys Cys Cys Thr Glu Ser Leu Val Asn Arg Arg Pro Cys Phe Ser
            500                 505                 510
```

Ala Leu Thr Pro Asp Glu Thr Tyr Lys Pro Lys Glu Phe Val Glu Gly
515 520 525

Thr Phe Thr Phe His Ala Asp Leu Cys Thr Leu Pro Glu Asp Glu Lys
530 535 540

Gln Ile Lys Lys Gln Thr Ala Leu Val Glu Leu Leu Lys His Lys Pro
545 550 555 560

His Ala Thr Glu Glu Gln Leu Arg Thr Val Leu Gly Asn Phe Ala Ala
565 570 575

Phe Val Gln Lys Cys Cys Ala Ala Pro Asp His Glu Ala Cys Phe Ala
580 585 590

Val Glu Gly Pro Lys Phe Val Ile Glu Ile Arg Gly Ile Leu Ala
595 600 605

<210> 19
<211> 584
<212> PRT
<213> Mus musculus

<400> 19

Glu Ala His Lys Ser Glu Ile Ala His Arg Tyr Asn Asp Leu Gly Glu
1 5 10 15

Gln His Phe Lys Gly Leu Val Leu Ile Ala Phe Ser Gln Tyr Leu Gln
20 25 30

Lys Cys Ser Tyr Asp Glu His Ala Lys Leu Val Gln Glu Val Thr Asp
35 40 45

Phe Ala Lys Thr Cys Val Ala Asp Glu Ser Ala Ala Asn Cys Asp Lys
50 55 60

Ser Leu His Thr Leu Phe Gly Asp Lys Leu Cys Ala Ile Pro Asn Leu
65 70 75 80

Arg Glu Asn Tyr Gly Glu Leu Ala Asp Cys Cys Thr Lys Gln Glu Pro
85 90 95

Glu Arg Asn Glu Cys Phe Leu Gln His Lys Asp Asp Asn Pro Ser Leu
100 105 110

Pro Pro Phe Glu Arg Pro Glu Ala Glu Ala Met Cys Thr Ser Phe Lys
115 120 125

```
Glu Asn Pro Thr Thr Phe Met Gly His Tyr Leu His Glu Val Ala Arg
    130                 135                 140

Arg His Pro Tyr Phe Tyr Ala Pro Glu Leu Leu Tyr Tyr Ala Glu Gln
145                 150                 155                 160

Tyr Asn Glu Ile Leu Thr Gln Cys Cys Ala Glu Ala Asp Lys Glu Ser
                165                 170                 175

Cys Leu Thr Pro Lys Leu Asp Gly Val Lys Glu Lys Ala Leu Val Ser
            180                 185                 190

Ser Val Arg Gln Arg Met Lys Cys Ser Ser Met Gln Lys Phe Gly Glu
        195                 200                 205

Arg Ala Phe Lys Ala Trp Ala Val Ala Arg Leu Ser Gln Thr Phe Pro
    210                 215                 220

Asn Ala Asp Phe Ala Glu Ile Thr Lys Leu Ala Thr Asp Leu Thr Lys
225                 230                 235                 240

Val Asn Lys Glu Cys Cys His Gly Asp Leu Leu Glu Cys Ala Asp Asp
                245                 250                 255

Arg Ala Glu Leu Ala Lys Tyr Met Cys Glu Asn Gln Ala Thr Ile Ser
            260                 265                 270

Ser Lys Leu Gln Thr Cys Cys Asp Lys Pro Leu Leu Lys Lys Ala His
        275                 280                 285

Cys Leu Ser Glu Val Glu His Asp Thr Met Pro Ala Asp Leu Pro Ala
    290                 295                 300

Ile Ala Ala Asp Phe Val Glu Asp Gln Glu Val Cys Lys Asn Tyr Ala
305                 310                 315                 320

Glu Ala Lys Asp Val Phe Leu Gly Thr Phe Leu Tyr Glu Tyr Ser Arg
                325                 330                 335

Arg His Pro Asp Tyr Ser Val Ser Leu Leu Leu Arg Leu Ala Lys Lys
            340                 345                 350

Tyr Glu Ala Thr Leu Glu Lys Cys Cys Ala Glu Ala Asn Pro Pro Ala
        355                 360                 365

Cys Tyr Gly Thr Val Leu Ala Glu Phe Gln Pro Leu Val Glu Glu Pro
```

```
        370                 375                 380

Lys Asn Leu Val Lys Thr Asn Cys Asp Leu Tyr Glu Lys Leu Gly Glu
385                 390                 395                 400

Tyr Gly Phe Gln Asn Ala Ile Leu Val Arg Tyr Thr Gln Lys Ala Pro
                405                 410                 415

Gln Val Ser Thr Pro Thr Leu Val Glu Ala Ala Arg Asn Leu Gly Arg
            420                 425                 430

Val Gly Thr Lys Cys Cys Thr Leu Pro Glu Asp Gln Arg Leu Pro Cys
        435                 440                 445

Val Glu Asp Tyr Leu Ser Ala Ile Leu Asn Arg Val Cys Leu Leu His
    450                 455                 460

Glu Lys Thr Pro Val Ser Glu His Val Thr Lys Cys Cys Ser Gly Ser
465                 470                 475                 480

Leu Val Glu Arg Arg Pro Cys Phe Ser Ala Leu Thr Val Asp Glu Thr
                485                 490                 495

Tyr Val Pro Lys Glu Phe Lys Ala Glu Thr Phe Thr Phe His Ser Asp
            500                 505                 510

Ile Cys Thr Leu Pro Glu Lys Glu Lys Gln Ile Lys Lys Gln Thr Ala
        515                 520                 525

Leu Ala Glu Leu Val Lys His Lys Pro Lys Ala Thr Ala Glu Gln Leu
    530                 535                 540

Lys Thr Val Met Asp Asp Phe Ala Gln Phe Leu Asp Thr Cys Cys Lys
545                 550                 555                 560

Ala Ala Asp Lys Asp Thr Cys Phe Ser Thr Glu Gly Pro Asn Leu Val
                565                 570                 575

Thr Arg Cys Lys Asp Ala Leu Ala
            580
```